# EUROPEAN PATENT APPLICATION

(11) **EP 1 470 824 A1**
(43) Date of publication of application: **27.10.2004**
(21) Application number: 02080206.2
(22) Date of filing: 10.12.2002
(51) Int. Cl.: A61K 47/48, C07K 16/18

(54) **Affinity proteins for controlled application of cosmetic substances**

(71) Applicant: L-MAbs B.V., 6708 PW Wageningen (NL)
(72) Inventor: Houtzager, Erwin, 3958 XD Amerongen (NL); Vijn, Irma Maria Caecilia, 6721 ZJ Bennekom (NL); Sijmons, Peter Christiaan, 1075 GK Amsterdam (NL); Mudge, Grant, West Redding, Connecticut 06496 (US); Fadel, Addi, Shelton, Connecticut 06484 (US); Valinotti, Tony, Sandy Hook, Connecticut 06482 (US)
(74) Representative: Prins, Adrianus Willem, Mr. Ir.

(57) **Abstract**

Provided are means and methods for applying cosmetic substance to a desired target. The method comprising providing a conjugate of a proteinaceous substance having a specific affinity for said target molecule linked to a cosmetic substance, whereby the resulting connection between cosmetic substance and target molecule can be disrupted upon the presence of a chemical and/or physical signal.

SEQUENCE LISTING

<110> L-MAbs B.V.

<120> Affinity proteins for controlled application of
      cosmetic substances

<130> P62519EP00

<140> 02080206.2
<141> 2002-12-10

<160> 171

<170> PatentIn Ver. 2.1

<210> 1
<211> 133
<212> PRT
<213> Artificial Sequence

<220>
<223> Description of Artificial Sequence: sequence of
      iMab100

<220>
<221> SITE
<222> (1)..(133)

<400> 1
Asn Val Lys Leu Val Glu Lys Gly Gly Asn Phe Val Glu Asn Asp Asp
  1               5                  10                  15

Asp Leu Lys Leu Thr Cys Arg Ala Glu Gly Tyr Thr Ile Gly Pro Tyr
             20                  25                  30

Cys Met Gly Trp Phe Arg Gln Ala Pro Asn Asp Asp Ser Thr Asn Val
         35                  40                  45

Ala Thr Ile Asn Met Gly Gly Gly Ile Thr Tyr Tyr Gly Asp Ser Val
     50                  55                  60

Lys Glu Arg Phe Asp Ile Arg Arg Asp Asn Ala Ser Asn Thr Val Thr
 65                  70                  75                  80

Leu Ser Met Asp Asp Leu Gln Pro Glu Asp Ser Ala Glu Tyr Asn Cys
                 85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser Arg Gly Gln Gly Thr Asp
        115                 120                 125

Val Thr Val Ser Ser
    130

<210> 2
<211> 133
<212> PRT
<213> Artificial Sequence

<220>
<223> Description of Artificial Sequence: iMab without
      glucosylation site

<220>
<221> SITE
<222> (1)..(133)

<400> 2
Asn Val Lys Leu Val Glu Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Cys Met Gly Trp Phe Arg Gln Ala Pro Asn Asp Asp Ser Thr Asn Val
        35                  40                  45

Ala Thr Ile Asn Met Gly Gly Gly Ile Thr Tyr Tyr Gly Asp Ser Val
    50                  55                  60

Lys Glu Arg Phe Asp Ile Arg Arg Asp Gln Ala Ser Asn Thr Val Thr
65                  70                  75                  80

Leu Ser Met Asp Asp Leu Gln Pro Glu Asp Ser Ala Glu Tyr Asn Cys
                85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser Arg Gly Gln Gly Thr Asp
        115                 120                 125

Val Thr Val Ser Ser
    130


<210> 3
<211> 23
<212> PRT
<213> Artificial Sequence

<220>
<223> Description of Artificial Sequence: AR4

<220>
<221> SITE
<222> (1)..(23)

<400> 3
Cys Ala Ala Gln Thr Gly Gly Pro Pro Ala Pro Tyr Tyr Cys Thr Glu

1                 5                   10                  15

Tyr Gly Ser Pro Asp Ser Trp
            20


<210> 4
<211> 20
<212> PRT
<213> Artificial Sequence

<220>
<223> Description of Artificial Sequence: AR4

<220>
<221> SITE
<222> (1)..(20)

<400> 4
Cys Ala Ala Val Leu Gly Cys Gly Tyr Cys Asp Tyr Asp Asp Gly Asp
 1               5                   10                  15

Val Gly Ser Trp
            20


<210> 5
<211> 19
<212> PRT
<213> Artificial Sequence

<220>
<223> Description of Artificial Sequence: AR4

<220>
<221> SITE
<222> (1)..(19)

<400> 5
Cys Ala Ala Thr Glu Asn Phe Arg Ile Ala Arg Glu Gly Tyr Glu Tyr
 1               5                   10                  15

Asp Tyr Trp


<210> 6
<211> 19
<212> PRT
<213> Artificial Sequence

<220>
<223> Description of Artificial Sequence: AR4

<220>
<221> SITE
<222> (1)..(19)

<400> 6
Cys Ala Ala Thr Ser Asp Phe Arg Ile Ala Arg Glu Asp Tyr Glu Tyr
1               5                   10                  15

Asp Tyr Trp

<210> 7
<211> 15
<212> PRT
<213> Artificial Sequence

<220>
<223> Description of Artificial Sequence: spacer

<220>
<221> SITE
<222> (1)..(15)

<400> 7
Ser Gly Gly Gly Gly Ser Gly Gly Gly Gly Ser Gly Gly Gly Gly
1               5                   10                  15

<210> 8
<211> 116
<212> PRT
<213> Artificial Sequence

<220>
<223> Description of Artificial Sequence: VAP1 / VAP2

<220>
<221> SITE
<222> (1)..(116)

<400> 8
Asn Val Lys Leu Val Glu Lys Gly Gly Asn Phe Val Glu Asn Asp Asp
1               5                   10                  15

Asp Leu Lys Leu Thr Cys Arg Ala Glu Xaa Xaa Xaa Xaa Xaa Xaa Met
            20                  25                  30

Gly Trp Phe Arg Gln Ala Pro Asn Asp Asp Ser Thr Asn Val Ala Thr
        35                  40                  45

Ile Xaa Xaa Xaa Xaa Xaa Xaa Tyr Gly Asp Ser Val Lys Glu Arg Phe
    50                  55                  60

Asp Ile Arg Arg Asp Xaa Xaa Xaa Xaa Xaa Xaa Asn Thr Val Thr Leu
65                  70                  75                  80

Ser Met Asp Asp Leu Gln Pro Glu Asp Ser Ala Glu Tyr Asn Cys Xaa
                85                  90                  95

Xaa Xaa Xaa Xaa Xaa Asp Ser His Tyr Arg Gly Gln Gly Thr Asp Val
            100                 105                 110

Thr Val Ser Ser
        115


<210> 9
<211> 15
<212> PRT
<213> Artificial Sequence

<220>
<223> Description of Artificial Sequence: linker

<220>
<221> SITE
<222> (1)..(15)

<400> 9
Gly Gly Gly Gly Ser Gly Gly Gly Gly Ser Gly Gly Gly Gly Ser
  1               5                  10                  15


<210> 10
<211> 136
<212> PRT
<213> Artificial Sequence

<220>
<223> Description of Artificial Sequence: iMab100

<220>
<221> SITE
<222> (1)..(136)

<400> 10
Asn Val Lys Leu Val Glu Lys Gly Gly Asn Phe Val Glu Asn Asp Asp
  1               5                  10                  15

Asp Leu Lys Leu Thr Cys Arg Ala Glu Gly Tyr Thr Ile Gly Pro Tyr
             20                  25                  30

Cys Met Gly Trp Phe Arg Gln Ala Pro Asn Asp Asp Ser Thr Asn Val
         35                  40                  45

Ala Thr Ile Asn Met Gly Gly Gly Ile Thr Tyr Tyr Gly Asp Ser Val
     50                  55                  60

Lys Glu Arg Phe Asp Ile Arg Arg Asp Asn Ala Ser Asn Thr Val Thr
 65                  70                  75                  80

Leu Ser Met Asp Asp Leu Gln Pro Glu Asp Ser Ala Glu Tyr Asn Cys
                 85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser His Tyr Arg Gly Gln Gly
        115                 120                 125

Thr Asp Val Thr Val Ser Ser Ala
    130                 135


<210> 11
<211> 143
<212> PRT
<213> Artificial Sequence

<220>
<223> Description of Artificial Sequence: iMab502

<220>
<221> SITE
<222> (1)..(143)

<400> 11
Ser Val Lys Phe Val Cys Lys Val Leu Pro Asn Phe Trp Glu Asn Asn
  1               5                  10                  15

Lys Asp Leu Pro Ile Lys Phe Thr Val Arg Ala Ser Gly Tyr Thr Ile
             20                  25                  30

Gly Pro Thr Cys Val Gly Val Phe Ala Gln Asn Pro Glu Asp Asp Ser
         35                  40                  45

Thr Asn Val Ala Thr Ile Asn Met Gly Gly Gly Ile Thr Tyr Tyr Gly
     50                  55                  60

Asp Ser Val Lys Leu Arg Phe Asp Ile Arg Arg Asp Asn Ala Lys Val
 65                  70                  75                  80

Thr Arg Thr Asn Ser Leu Asp Asp Val Gln Pro Glu Gly Arg Gly Lys
                 85                  90                  95

Ser Phe Glu Leu Thr Cys Ala Ala Asp Ser Thr Ile Tyr Ala Ser Tyr
            100                 105                 110

Tyr Glu Cys Gly His Gly Ile Ser Thr Gly Gly Tyr Gly Tyr Asp Gln
        115                 120                 125

Val Ala Arg Tyr His Arg Gly Ile Asp Ile Thr Val Asp Gly Pro
    130                 135                 140


<210> 12
<211> 140
<212> PRT
<213> Artificial Sequence

<220>
<223> Description of Artificial Sequence: iMab702

<220>
<221> SITE
<222> (1)..(140)

<400> 12
Ala Val Lys Ser Val Phe Lys Val Ser Thr Asn Phe Ile Glu Asn Asp
1               5                   10                  15

Gly Thr Met Asp Ser Lys Leu Thr Phe Arg Ala Ser Gly Tyr Thr Ile
            20                  25                  30

Gly Pro Gln Cys Leu Gly Phe Phe Gln Gln Gly Val Pro Asp Asp Ser
        35                  40                  45

Thr Asn Val Ala Thr Ile Asn Met Gly Gly Gly Ile Thr Tyr Tyr Gly
    50                  55                  60

Asp Ser Val Lys Ser Ile Phe Asp Ile Arg Arg Asp Asn Ala Lys Asp
65                  70                  75                  80

Thr Tyr Thr Ala Ser Val Asp Asp Asn Gln Pro Glu Asp Val Glu Ile
                85                  90                  95

Thr Cys Ala Ala Asp Ser Thr Ile Tyr Ala Ser Tyr Tyr Glu Cys Gly
            100                 105                 110

His Gly Ile Ser Thr Gly Gly Tyr Gly Tyr Asp Leu Ile Leu Arg Thr
        115                 120                 125

Leu Gln Lys Gly Ile Asp Leu Phe Val Val Pro Thr
    130                 135                 140


<210> 13
<211> 133
<212> PRT
<213> Artificial Sequence

<220>
<223> Description of Artificial Sequence: iMab1202
      (1EJ6)

<220>
<221> SITE
<222> (1)..(133)

<400> 13
Ile Val Lys Leu Val Met Glu Lys Arg Gly Asn Phe Glu Asn Gly Gln
1               5                   10                  15

Asp Cys Lys Leu Thr Ile Arg Ala Ser Gly Tyr Thr Ile Gly Pro Ala
            20                  25                  30

Cys Asp Gly Phe Phe Cys Gln Phe Pro Ser Asp Asp Ser Phe Ser Thr
        35                  40                  45

Glu Asp Asn Met Gly Gly Gly Ile Thr Val Asn Asp Ala Met Lys Pro

    50                  55                  60

Gln Phe Asp Ile Arg Arg Asp Asn Ala Lys Gly Thr Trp Thr Leu Ser
65                  70                  75                  80

Met Asp Phe Gln Pro Glu Gly Ile Tyr Glu Met Gln Cys Ala Ala Asp
                85                  90                  95

Ser Thr Ile Tyr Ala Ser Tyr Tyr Glu Cys Gly His Gly Ile Ser Thr
            100                 105                 110

Gly Gly Tyr Gly Tyr Asp Asn Pro Val Arg Leu Gly Gly Phe Asp Val
        115                 120                 125

Asp Val Pro Asp Val
    130

<210> 14
<211> 136
<212> PRT
<213> Artificial Sequence

<220>
<223> Description of Artificial Sequence: iMab1302

<220>
<221> SITE
<222> (1)..(136)

<400> 14
Val Val Lys Val Val Ile Lys Pro Ser Gln Asn Phe Ile Glu Asn Gly
1               5                   10                  15

Glu Asp Lys Lys Phe Thr Cys Arg Ala Ser Gly Tyr Thr Ile Gly Pro
            20                  25                  30

Lys Cys Ile Gly Trp Phe Ser Gln Asn Pro Glu Asp Asp Ser Thr Asn
        35                  40                  45

Val Ala Thr Ile Asn Met Gly Gly Gly Ile Thr Tyr Tyr Gly Asp Ser
    50                  55                  60

Val Lys Glu Arg Phe Asp Ile Arg Arg Asp Asn Ala Lys Asp Thr Ser
65                  70                  75                  80

Thr Leu Ser Ile Asp Asp Ala Gln Pro Glu Asp Ala Gly Ile Tyr Lys
                85                  90                  95

Cys Ala Ala Asp Ser Thr Ile Tyr Ala Ser Tyr Tyr Glu Cys Gly His
            100                 105                 110

Gly Ile Ser Thr Gly Gly Tyr Gly Tyr Asp Ser Glu Ala Thr Val Gly
        115                 120                 125

Val Asp Ile Phe Val Lys Leu Met
    130                 135

<210> 15
<211> 136
<212> PRT
<213> Artificial Sequence

<220>
<223> Description of Artificial Sequence: iMab1502
      (1NEU)

<220>
<221> SITE
<222> (1)..(136)

<400> 15
Asn Val Lys Val Val Thr Lys Arg Glu Asn Phe Gly Glu Asn Gly Ser
1               5                   10                  15

Asp Val Lys Leu Thr Cys Arg Ala Ser Gly Tyr Thr Ile Gly Pro Ile
            20                  25                  30

Cys Phe Gly Trp Phe Tyr Gln Pro Glu Gly Asp Asp Ser Ala Ile Ser
        35                  40                  45

Ile Phe His Asn Met Gly Gly Gly Ile Thr Asp Glu Val Asp Thr Phe
    50                  55                  60

Lys Glu Arg Phe Asp Ile Arg Arg Asp Asn Ala Lys Lys Thr Gly Thr
65                  70                  75                  80

Ile Ser Ile Asp Asp Leu Gln Pro Ser Asp Asn Glu Thr Phe Thr Cys
                85                  90                  95

Ala Ala Asp Ser Thr Ile Tyr Ala Ser Tyr Tyr Glu Cys Gly His Gly
            100                 105                 110

Ile Ser Thr Gly Gly Tyr Gly Tyr Asp Gly Lys Thr Arg Gln Val Gly
        115                 120                 125

Leu Asp Val Phe Val Lys Val Pro
    130                 135


<210> 16
<211> 137
<212> PRT
<213> Artificial Sequence

<220>
<223> Description of Artificial Sequence: iMab1602

<220>
<221> SITE
<222> (1)..(137)

<400> 16

Ala Val Lys Pro Val Ile Gly Ser Lys Ala Pro Asn Phe Gly Glu Asn
1               5                   10                  15

Gly Asp Val Lys Thr Ile Asp Arg Ala Ser Gly Tyr Thr Ile Gly Pro
            20                  25                  30

Thr Cys Gly Gly Val Phe Phe Gln Gly Pro Thr Asp Asp Ser Thr Asn
        35                  40                  45

Val Ala Thr Ile Asn Met Gly Gly Gly Ile Thr Tyr Tyr Gly Asp Ser
    50                  55                  60

Val Lys Glu Thr Phe Asp Ile Arg Arg Asp Asn Ala Lys Ser Thr Arg
65                  70                  75                  80

Thr Glu Ser Tyr Asp Asp Asn Gln Pro Glu Gly Leu Thr Glu Val Lys
                85                  90                  95

Cys Ala Ala Asp Ser Thr Ile Tyr Ala Ser Tyr Tyr Glu Cys Gly His
            100                 105                 110

Gly Ile Ser Thr Gly Gly Tyr Gly Tyr Asp Val Ser Ser Arg Leu Tyr
        115                 120                 125

Gly Tyr Asp Ile Leu Val Gly Thr Gln
    130                 135


<210> 17
<211> 135
<212> PRT
<213> Artificial Sequence

<220>
<223> Description of Artificial Sequence: VAP amino acid
      sequence of iMab100

<220>
<221> SITE
<222> (1)..(135)

<400> 17
Asn Val Lys Leu Val Glu Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Cys Met Gly Trp Phe Arg Gln Ala Pro Asn Asp Asp Ser Thr Asn Val
        35                  40                  45

Ala Thr Ile Asn Met Gly Gly Gly Ile Thr Tyr Tyr Gly Asp Ser Val
    50                  55                  60

Lys Glu Arg Phe Asp Ile Arg Arg Asp Asn Ala Ser Asn Thr Val Thr
65                  70                  75                  80

Leu Ser Met Asp Asp Leu Gln Pro Glu Asp Ser Ala Glu Tyr Asn Cys
                85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser His Tyr Arg Gly Gln Gly
        115                 120                 125

Thr Asp Val Thr Val Ser Ser
    130                 135


<210> 18
<211> 111
<212> PRT
<213> Artificial Sequence

<220>
<223> Description of Artificial Sequence: VAP amino acid
      sequence of iMab101

<220>
<221> SITE
<222> (1)..(111)

<400> 18
Val Lys Leu Val Glu Lys Gly Gly Asn Phe Val Glu Asn Asp Asp Asp
  1               5                  10                  15

Leu Lys Leu Thr Cys Arg Ala Ser Gly Tyr Thr Ile Gly Pro Tyr Cys
             20                  25                  30

Met Gly Trp Phe Arg Gln Ala Pro Asn Asp Asp Ser Thr Asn Val Ala
         35                  40                  45

Thr Ile Asn Met Gly Thr Val Thr Leu Ser Met Asp Asp Leu Gln Pro
     50                  55                  60

Glu Asp Ser Ala Glu Tyr Asn Cys Ala Ala Asp Ser Thr Ile Tyr Ala
 65                  70                  75                  80

Ser Tyr Tyr Glu Cys Gly His Gly Leu Ser Thr Gly Gly Tyr Gly Tyr
                 85                  90                  95

Asp Ser His Tyr Arg Gly Gln Gly Thr Asp Val Thr Val Ser Ser
            100                 105                 110


<210> 19
<211> 96
<212> PRT
<213> Artificial Sequence

<220>
<223> Description of Artificial Sequence: VAP amino acid
      sequence of iMab102

<220>
<221> SITE
<222> (1)..(96)

<400> 19
Asp Leu Lys Leu Thr Cys Arg Ala Ser Gly Tyr Thr Ile Gly Pro Tyr
1               5                   10                  15

Cys Met Gly Trp Phe Arg Gln Ala Pro Asn Asp Asp Ser Thr Asn Val
            20                  25                  30

Ala Thr Ile Asn Met Gly Thr Val Thr Leu Ser Met Asp Asp Leu Gln
        35                  40                  45

Pro Glu Asp Ser Ala Glu Tyr Asn Cys Ala Ala Asp Ser Thr Ile Tyr
    50                  55                  60

Ala Ser Tyr Tyr Glu Cys Gly His Gly Leu Ser Thr Gly Gly Tyr Gly
65                  70                  75                  80

Tyr Asp Ser His Tyr Arg Gly Gln Gly Thr Asp Val Thr Val Ser Ser
                85                  90                  95


<210> 20
<211> 135
<212> PRT
<213> Artificial Sequence

<220>
<223> Description of Artificial Sequence: VAP amino acid
      sequence of iMab111

<220>
<221> SITE
<222> (1)..(135)

<400> 20
Asn Val Lys Leu Val Cys Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Cys Met Gly Trp Phe Arg Gln Ala Pro Asn Asp Asp Ser Thr Asn Val
        35                  40                  45

Ala Thr Ile Asn Met Gly Gly Gly Ile Thr Tyr Tyr Gly Asp Ser Val
    50                  55                  60

Lys Glu Arg Phe Asp Ile Arg Arg Asp Asn Ala Ser Asn Thr Val Thr
65                  70                  75                  80

Leu Ser Met Asp Asp Leu Gln Pro Glu Asp Ser Ala Glu Tyr Asn Cys
                85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser His Tyr Arg Cys Gln Gly
        115                 120                 125

Thr Asp Val Thr Val Ser Ser
    130                 135


<210> 21
<211> 135
<212> PRT
<213> Artificial Sequence

<220>
<223> Description of Artificial Sequence: VAP amino acid
      sequence of iMab112

<220>
<221> SITE
<222> (1)..(135)

<400> 21
Asn Val Lys Leu Val Glu Lys Gly Gly Asn Phe Val Glu Asn Asp Asp
  1               5                  10                  15

Asp Leu Lys Leu Thr Cys Arg Ala Glu Gly Tyr Thr Ile Gly Pro Tyr
             20                  25                  30

Cys Met Gly Trp Phe Cys Gln Ala Pro Asn Asp Asp Ser Thr Cys Val
         35                  40                  45

Ala Thr Ile Asn Met Gly Gly Gly Ile Thr Tyr Tyr Gly Asp Ser Val
     50                  55                  60

Lys Glu Arg Phe Asp Ile Arg Arg Asp Asn Ala Ser Asn Thr Val Thr
 65                  70                  75                  80

Leu Ser Met Asp Asp Leu Gln Pro Glu Asp Ser Ala Glu Tyr Asn Cys
                 85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser His Tyr Arg Gly Gln Gly
        115                 120                 125

Thr Asp Val Thr Val Ser Ser
    130                 135


<210> 22
<211> 135
<212> PRT
<213> Artificial Sequence

<220>
<223> Description of Artificial Sequence: VAP amino acid
      sequence of iMab113

<220>
<221> SITE
<222> (1)..(135)

<400> 22
Asn Val Lys Leu Val Glu Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Ser Met Gly Trp Phe Arg Gln Ala Pro Asn Asp Asp Ser Thr Asn Val
        35                  40                  45

Ser Cys Ile Asn Met Gly Gly Gly Ile Thr Tyr Tyr Gly Asp Ser Val
    50                  55                  60

Lys Glu Arg Phe Asp Ile Arg Arg Asp Asn Ala Ser Asn Thr Val Thr
65                  70                  75                  80

Leu Ser Met Asp Asp Leu Gln Pro Glu Asp Ser Ala Glu Tyr Asn Cys
                85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser His Tyr Arg Gly Gln Gly
        115                 120                 125

Thr Asp Val Thr Val Ser Ser
    130                 135


<210> 23
<211> 135
<212> PRT
<213> Artificial Sequence

<220>
<223> Description of Artificial Sequence: VAP amino acid
      sequence of iMab114

<220>
<221> SITE
<222> (1)..(135)

<400> 23
Asn Val Lys Leu Val Glu Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Ser Met Gly Trp Phe Arg Gln Ala Pro Asn Asp Asp Ser Thr Asn Val
        35                  40                  45

Ala Thr Ile Asn Met Gly Gly Gly Ile Thr Tyr Tyr Gly Asp Ser Val
    50                  55                  60

Lys Glu Arg Phe Asp Ile Arg Arg Asp Asn Ala Ser Asn Thr Val Thr
65                  70                  75                  80

Leu Ser Met Asp Asp Leu Gln Pro Glu Asp Ser Ala Glu Tyr Asn Cys
                85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser His Tyr Arg Gly Gln Gly
        115                 120                 125

Thr Asp Val Thr Val Ser Ser
    130                 135


<210> 24
<211> 135
<212> PRT
<213> Artificial Sequence

<220>
<223> Description of Artificial Sequence: VAP amino acid
      sequence of iMab115

<220>
<221> SITE
<222> (1)..(135)

<400> 24
Asn Val Lys Leu Val Glu Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Cys Met Gly Trp Phe Arg Gln Ala Pro Asn Asp Asp Ser Thr Asn Val
        35                  40                  45

Ala Thr Ile Asn Met Gly Gly Gly Ile Thr Tyr Tyr Gly Asp Ser Val
    50                  55                  60

Lys Glu Arg Phe Asp Ile Arg Arg Asp Gln Ala Ser Asn Thr Val Thr
65                  70                  75                  80

Leu Ser Met Asp Asp Leu Gln Pro Glu Asp Ser Ala Glu Tyr Asn Cys
                85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser His Tyr Arg Gly Gln Gly
        115                 120                 125

Thr Asp Val Thr Val Ser Ser
    130                 135


<210> 25
<211> 135
<212> PRT
<213> Artificial Sequence

<220>
<223> Description of Artificial Sequence: VAP amino acid
      sequence of iMab116

<220>
<221> SITE
<222> (1)..(135)

<400> 25
Asn Val Lys Leu Val Glu Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Cys Met Gly Trp Phe Arg Gln Ala Pro Asn Asp Asp Ser Thr Asn Val
        35                  40                  45

Ala Thr Ile Asn Met Gly Gly Gly Ile Thr Tyr Tyr Gly Asp Ser Val
    50                  55                  60

Lys Glu Arg Phe Asp Ile Arg Arg Asp Asn Ala Ser Asn Thr Val Thr
65                  70                  75                  80

Leu Ser Met Asp Asp Leu Gln Pro Glu Asp Ser Ala Glu Tyr Asn Gly
                85                  90                  95

Ala Gly Asp Ser Thr Ile Tyr Gly Ser Tyr Tyr Glu Cys Gly His Gly
            100                 105                 110

Leu Ser Thr Gly Gly Tyr Gly Tyr Asp Ser His Tyr Arg Gly Gln Gly
        115                 120                 125

Thr Asp Val Thr Val Ser Ser
    130                 135


<210> 26
<211> 126
<212> PRT
<213> Artificial Sequence

<220>
<223> Description of Artificial Sequence: VAP amino acid
      sequence of iMab121

<220>
<221> SITE
<222> (1)..(126)

<400> 26
Asn Val Lys Leu Val Glu Lys Gly Gly Asn Phe Val Glu Asn Asp Asp
1               5                   10                  15

Asp Leu Lys Leu Thr Cys Arg Ala Ser Gly Arg Ser Phe Ser Ser Tyr
            20                  25                  30

Ile Met Gly Trp Phe Arg Gln Ala Pro Asn Asp Asp Ser Thr Asn Val
        35                  40                  45

Ala Thr Ile Ser Glu Thr Gly Gly Asp Ile Val Tyr Thr Asn Tyr Gly
    50                  55                  60

Asp Ser Val Lys Glu Arg Phe Asp Ile Arg Arg Asp Ile Ala Ser Asn
65                  70                  75                  80

Thr Val Thr Leu Ser Met Asp Asp Leu Gln Pro Glu Asp Ser Ala Glu
                85                  90                  95

Tyr Asn Cys Ala Gly Ser Val Tyr Gly Ser Gly Trp Arg Pro Asp Arg
            100                 105                 110

Tyr Asp Tyr Arg Gly Gln Gly Thr Asp Val Thr Val Ser Ser
        115                 120                 125


<210> 27
<211> 85
<212> PRT
<213> Artificial Sequence

<220>
<223> Description of Artificial Sequence: VAP amino acid
      sequence of iMab124

<220>
<221> SITE
<222> (1)..(85)

<400> 27
Asp Asp Leu Lys Leu Thr Cys Arg Ala Ser Gly Arg Ser Phe Ser Ser
1               5                   10                  15

Tyr Ile Met Gly Trp Phe Arg Gln Ala Pro Asn Asp Asp Ser Thr Asn
            20                  25                  30

Val Ala Thr Ile Ser Glu Thr Thr Val Thr Leu Ser Met Asp Asp Leu
        35                  40                  45

Gln Pro Glu Asp Ser Ala Glu Tyr Asn Cys Ala Gly Ser Val Tyr Gly
    50                  55                  60

Ser Gly Trp Arg Pro Asp Arg Tyr Asp Tyr Arg Gly Gln Gly Thr Asp
65                  70                  75                  80

Val Thr Val Ser Ser
                85

<210> 28
<211> 125
<212> PRT
<213> Artificial Sequence

<220>
<223> Description of Artificial Sequence: VAP amino acid
      sequence of iMab122

<220>
<221> SITE
<222> (1)..(125)

<400> 28
Asn Val Lys Leu Val Glu Lys Gly Gly Asn Phe Val Glu Asn Asp Asp
1               5                   10                  15

Asp Leu Lys Leu Thr Cys Arg Ala Ser Gly Arg Thr Phe Ser Ser Arg
            20                  25                  30

Thr Met Gly Trp Phe Arg Gln Ala Pro Asn Asp Asp Ser Thr Asn Val
        35                  40                  45

Ala Thr Ile Arg Trp Asn Gly Gly Ser Thr Tyr Tyr Thr Asn Tyr Gly
    50                  55                  60

Asp Ser Val Lys Glu Arg Phe Asp Ile Arg Val Asp Gln Ala Ser Asn
65                  70                  75                  80

Thr Val Thr Leu Ser Met Asp Asp Leu Gln Pro Glu Asp Ser Ala Glu
                85                  90                  95

Tyr Asn Cys Ala Gly Thr Asp Ile Gly Asp Gly Trp Ser Gly Arg Tyr
            100                 105                 110

Asp Tyr Arg Gly Gln Gly Thr Asp Val Thr Val Ser Ser
        115                 120                 125

<210> 29
<211> 84
<212> PRT
<213> Artificial Sequence

<220>
<223> Description of Artificial Sequence: VAP amino acid
      sequence of iMab125

<220>
<221> SITE

<222> (1)..(84)

<400> 29
Asp Asp Leu Lys Leu Thr Cys Arg Ala Ser Gly Arg Thr Phe Ser Ser
1               5                   10                  15

Arg Thr Met Gly Trp Phe Arg Gln Ala Pro Asn Asp Asp Ser Thr Asn
            20                  25                  30

Val Ala Thr Ile Arg Trp Asn Thr Val Thr Leu Ser Met Asp Asp Leu
        35                  40                  45

Gln Pro Glu Asp Ser Ala Glu Tyr Asn Cys Ala Gly Thr Asp Ile Gly
    50                  55                  60

Asp Gly Trp Ser Gly Arg Tyr Asp Tyr Arg Gly Gln Gly Thr Asp Val
65                  70                  75                  80

Thr Val Ser Ser

<210> 30
<211> 124
<212> PRT
<213> Artificial Sequence

<220>
<223> Description of Artificial Sequence: VAP amino acid
      sequence of iMab123

<220>
<221> SITE
<222> (1)..(124)

<400> 30
Asn Val Lys Leu Val Glu Lys Gly Gly Asn Phe Val Glu Asn Asp Asp
1               5                   10                  15

Asp Leu Lys Leu Thr Cys Arg Ala Ser Gly Arg Thr Phe Ser Arg Ala
            20                  25                  30

Ala Met Gly Trp Phe Arg Gln Ala Pro Asn Asp Asp Ser Thr Asn Val
        35                  40                  45

Ala Thr Ile Thr Trp Ser Gly Arg His Thr Arg Tyr Gly Asp Ser Val
    50                  55                  60

Lys Glu Arg Phe Asp Ile Arg Arg Asp Gln Ala Ser Asn Thr Val Thr
65                  70                  75                  80

Leu Ser Met Asp Asp Leu Gln Pro Glu Asp Ser Ala Glu Tyr Asn Cys
                85                  90                  95

Ala Gly Glu Gly Ser Asn Thr Ala Ser Thr Ser Pro Arg Pro Tyr Asp
            100                 105                 110

Tyr Arg Gly Gln Gly Thr Asp Val Thr Val Ser Ser
        115                 120

<210> 31
<211> 121
<212> PRT
<213> Artificial Sequence

<220>
<223> Description of Artificial Sequence: VAP amino acid
      sequence of iMab130

<220>
<221> SITE
<222> (1)..(121)

<400> 31
Asn Val Lys Leu Val Glu Lys Gly Gly Asn Phe Val Glu Asn Asp Asp
1               5                   10                  15

Asp Leu Lys Leu Thr Cys Arg Ala Ser Gly Tyr Ala Tyr Thr Tyr Ile
            20                  25                  30

Tyr Met Gly Trp Phe Arg Gln Ala Pro Asn Asp Asp Ser Thr Asn Val
        35                  40                  45

Ala Thr Ile Asp Ser Gly Gly Gly Gly Thr Leu Tyr Gly Asp Ser Val
    50                  55                  60

Lys Glu Arg Phe Asp Ile Arg Arg Asp Lys Gly Ser Asn Thr Val Thr
65                  70                  75                  80

Leu Ser Met Asp Asp Leu Gln Pro Glu Asp Ser Ala Glu Tyr Asn Cys
                85                  90                  95

Ala Ala Gly Gly Tyr Glu Leu Arg Asp Arg Thr Tyr Gly Gln Arg Gly
            100                 105                 110

Gln Gly Thr Asp Val Thr Val Ser Ser
        115                 120

<210> 32
<211> 109
<212> PRT
<213> Artificial Sequence

<220>
<223> Description of Artificial Sequence: VAP amino acid
      sequence of iMab201

<220>
<221> SITE
<222> (1)..(109)

<400> 32

Val Gln Leu Gln Ala Ser Gly Gly Gly Ser Val Gln Ala Gly Gly Ser
1               5                   10                  15

Leu Arg Leu Ser Cys Arg Ala Ser Gly Tyr Thr Ile Gly Pro Tyr Cys
            20                  25                  30

Met Gly Trp Phe Arg Gln Ala Pro Gly Asp Asp Ser Glu Gly Val Ala
        35                  40                  45

Ala Ile Asn Met Gly Thr Val Tyr Leu Leu Met Asn Ser Leu Glu Pro
    50                  55                  60

Glu Asp Thr Ala Ile Tyr Tyr Cys Ala Ala Asp Ser Thr Ile Tyr Ala
65                  70                  75                  80

Ser Tyr Tyr Glu Cys Gly His Gly Leu Ser Thr Gly Gly Tyr Gly Tyr
                85                  90                  95

Asp Ser Trp Gly Gln Gly Thr Gln Val Thr Val Ser Ser
            100                 105

<210> 33
<211> 109
<212> PRT
<213> Artificial Sequence

<220>
<223> Description of Artificial Sequence: VAP amino acid
      sequence of iMab300

<220>
<221> SITE
<222> (1)..(109)

<400> 33
Val Gln Leu Gln Gln Pro Gly Ser Asn Leu Val Arg Pro Gly Ala Ser
1               5                   10                  15

Val Lys Leu Ser Cys Lys Ala Ser Gly Tyr Thr Ile Gly Pro Ser Cys
            20                  25                  30

Ile His Trp Ala Lys Gln Arg Pro Gly Asp Gly Leu Glu Trp Ile Gly
        35                  40                  45

Glu Ile Asn Met Gly Thr Ala Tyr Val Asp Leu Ser Ser Leu Thr Ser
    50                  55                  60

Glu Asp Ser Ala Val Tyr Tyr Cys Ala Ala Asp Ser Thr Ile Tyr Ala
65                  70                  75                  80

Ser Tyr Tyr Glu Cys Gly His Gly Leu Ser Thr Gly Gly Tyr Gly Tyr
                85                  90                  95

Asp Tyr Trp Gly Gln Gly Thr Thr Leu Thr Val Ser Ser
            100                 105

<210> 34
<211> 95
<212> PRT
<213> Artificial Sequence

<220>
<223> Description of Artificial Sequence: VAP amino acid
      sequence of iMab302

<220>
<221> SITE
<222> (1)..(95)

<400> 34
Ala Ser Val Lys Leu Ser Cys Lys Ala Ser Gly Tyr Thr Ile Gly Pro
1               5                   10                  15

Ser Cys Ile His Trp Ala Lys Gln Arg Pro Gly Asp Gly Leu Glu Trp
            20                  25                  30

Ile Gly Glu Ile Asn Met Gly Thr Ala Tyr Val Asp Leu Ser Ser Leu
        35                  40                  45

Thr Ser Glu Asp Ser Ala Val Tyr Tyr Cys Ala Ala Asp Ser Thr Ile
    50                  55                  60

Tyr Ala Ser Tyr Tyr Glu Cys Gly His Gly Leu Ser Thr Gly Gly Tyr
65                  70                  75                  80

Gly Tyr Asp Tyr Trp Gly Gln Gly Thr Thr Leu Thr Val Ser Ser
                85                  90                  95


<210> 35
<211> 109
<212> PRT
<213> Artificial Sequence

<220>
<223> Description of Artificial Sequence: VAP amino acid
      sequence of iMab400

<220>
<221> SITE
<222> (1)..(109)

<400> 35
Val Gln Leu Val Glu Ser Gly Gly Gly Leu Val Gln Pro Gly Gly Ser
1               5                   10                  15

Leu Arg Leu Ser Cys Arg Ala Ser Gly Tyr Thr Ile Gly Pro Tyr Cys
            20                  25                  30

Met Asn Trp Val Arg Gln Ala Pro Gly Asp Gly Leu Glu Trp Val Gly
        35                  40                  45

Trp Ile Asn Met Gly Thr Ala Tyr Leu Gln Met Asn Ser Leu Arg Ala
    50                  55                  60

Glu Asp Thr Ala Val Tyr Tyr Cys Ala Ala Asp Ser Thr Ile Tyr Ala
65                  70                  75                  80

Ser Tyr Tyr Glu Cys Gly His Gly Leu Ser Thr Gly Gly Tyr Gly Tyr
                85                  90                  95

Asp Val Trp Gly Gln Gly Thr Leu Val Thr Val Ser Ser
            100                 105


<210> 36
<211> 113
<212> PRT
<213> Artificial Sequence

<220>
<223> Description of Artificial Sequence: VAP amino acid
      sequence of iMab500

<220>
<221> SITE
<222> (1)..(113)

<400> 36
Pro Asn Phe Leu Cys Ser Val Leu Pro Thr His Trp Arg Cys Asn Lys
 1               5                  10                  15

Thr Leu Pro Ile Ala Phe Lys Cys Arg Ala Ser Gly Tyr Thr Ile Gly
            20                  25                  30

Pro Thr Cys Val Thr Val Met Ala Gly Asn Asp Glu Asp Tyr Ser Asn
        35                  40                  45

Met Gly Ala Arg Phe Asn Asp Leu Arg Phe Val Gly Arg Ser Gly Arg
    50                  55                  60

Gly Lys Ser Phe Thr Leu Thr Cys Ala Ala Asp Ser Thr Ile Tyr Ala
65                  70                  75                  80

Ser Tyr Tyr Glu Cys Gly His Gly Leu Ser Thr Gly Gly Tyr Gly Tyr
                85                  90                  95

Pro Gln Val Ala Thr Tyr His Arg Ala Ile Lys Ile Thr Val Asp Gly
            100                 105                 110

Pro


<210> 37
<211> 143
<212> PRT
<213> Artificial Sequence

<220>
<223> Description of Artificial Sequence: VAP amino acid
      sequence of iMab502

<220>
<221> SITE
<222> (1)..(143)

<400> 37
Ser Val Lys Phe Val Cys Lys Val Leu Pro Asn Phe Trp Glu Asn Asn
1               5                   10                  15

Lys Asp Leu Pro Ile Lys Phe Thr Val Arg Ala Ser Gly Tyr Thr Ile
            20                  25                  30

Gly Pro Thr Cys Val Gly Val Phe Ala Gln Asn Pro Glu Asp Asp Ser
        35                  40                  45

Thr Asn Val Ala Thr Ile Asn Met Gly Gly Gly Ile Thr Tyr Tyr Gly
    50                  55                  60

Asp Ser Val Lys Leu Arg Phe Asp Ile Arg Arg Asp Asn Ala Lys Val
65                  70                  75                  80

Thr Arg Thr Asn Ser Leu Asp Asp Val Gln Pro Glu Gly Arg Gly Lys
                85                  90                  95

Ser Phe Glu Leu Thr Cys Ala Ala Asp Ser Thr Ile Tyr Ala Ser Tyr
            100                 105                 110

Tyr Glu Cys Gly His Gly Leu Ser Thr Gly Gly Tyr Gly Tyr Asp Gln
        115                 120                 125

Val Ala Arg Tyr His Arg Gly Ile Asp Ile Thr Val Asp Gly Pro
    130                 135                 140


<210> 38
<211> 109
<212> PRT
<213> Artificial Sequence

<220>
<223> Description of Artificial Sequence: VAP amino acid
      sequence of iMab600

<220>
<221> SITE
<222> (1)..(109)

<400> 38
Ala Pro Val Gly Leu Lys Ala Arg Asn Ala Asp Glu Ser Gly His Val
1               5                   10                  15

Val Leu Arg Cys Arg Ala Ser Gly Tyr Thr Ile Gly Pro Ile Cys Tyr
            20                  25                  30

Glu Val Asp Val Ser Ala Gly Gln Asp Ala Gly Ser Val Gln Arg Val
        35                  40                  45

Glu Ile Asn Met Gly Arg Thr Glu Ser Val Leu Ser Asn Leu Arg Gly
    50                  55                  60

Arg Thr Arg Tyr Thr Phe Ala Cys Ala Ala Asp Ser Thr Ile Tyr Ala
65                  70                  75                  80

Ser Tyr Tyr Glu Cys Gly His Gly Leu Ser Thr Gly Gly Tyr Gly Tyr
                85                  90                  95

Ser Glu Trp Ser Glu Pro Val Ser Leu Leu Thr Pro Ser
            100                 105


<210> 39
<211> 103
<212> PRT
<213> Artificial Sequence

<220>
<223> Description of Artificial Sequence: VAP amino acid
      sequence of iMab700

<220>
<221> SITE
<222> (1)..(103)

<400> 39
Asp Lys Ser Thr Leu Ala Ala Val Pro Thr Ser Ile Ile Ala Asp Gly
 1               5                  10                  15

Leu Met Ala Ser Thr Ile Thr Cys Glu Ala Ser Gly Tyr Thr Ile Gly
            20                  25                  30

Pro Ala Cys Val Ala Phe Asp Thr Thr Leu Gly Asn Asn Met Gly Thr
        35                  40                  45

Tyr Ser Ala Pro Leu Thr Ser Thr Thr Leu Gly Val Ala Thr Val Thr
    50                  55                  60

Cys Ala Ala Asp Ser Thr Ile Tyr Ala Ser Tyr Tyr Glu Cys Gly His
65                  70                  75                  80

Gly Leu Ser Thr Gly Gly Tyr Gly Tyr Ala Ala Phe Ser Val Pro Ser
                85                  90                  95

Val Thr Val Asn Phe Thr Ala
            100


<210> 40
<211> 140
<212> PRT
<213> Artificial Sequence

<220>
<223> Description of Artificial Sequence: VAP amino acid
      sequence of iMab702

<220>
<221> SITE
<222> (1)..(140)

<400> 40
Ala Val Lys Ser Val Phe Lys Val Ser Thr Asn Phe Ile Glu Asn Asp
1               5                   10                  15

Gly Thr Met Asp Ser Lys Leu Thr Phe Arg Ala Ser Gly Tyr Thr Ile
            20                  25                  30

Gly Pro Gln Cys Leu Gly Phe Phe Gln Gln Gly Val Pro Asp Asp Ser
        35                  40                  45

Thr Asn Val Ala Thr Ile Asn Met Gly Gly Gly Ile Thr Tyr Tyr Gly
    50                  55                  60

Asp Ser Val Lys Ser Ile Phe Asp Ile Arg Arg Asp Asn Ala Lys Asp
65                  70                  75                  80

Thr Tyr Thr Ala Ser Val Asp Asp Asn Gln Pro Glu Asp Val Glu Ile
                85                  90                  95

Thr Cys Ala Ala Asp Ser Thr Ile Tyr Ala Ser Tyr Tyr Glu Cys Gly
            100                 105                 110

His Gly Leu Ser Thr Gly Gly Tyr Gly Tyr Asp Leu Ile Leu Arg Thr
        115                 120                 125

Leu Gln Lys Gly Ile Asp Leu Phe Val Val Pro Thr
    130                 135                 140


<210> 41
<211> 86
<212> PRT
<213> Artificial Sequence

<220>
<223> Description of Artificial Sequence: VAP amino acid
      sequence of iMab701

<220>
<221> SITE
<222> (1)..(86)

<400> 41
Met Ala Ser Thr Ile Thr Cys Glu Ala Ser Gly Tyr Thr Ile Gly Pro
1               5                   10                  15

Ala Cys Val Ala Phe Asp Thr Thr Leu Gly Asn Asn Met Gly Thr Tyr
            20                  25                  30

Ser Ala Pro Leu Thr Ser Thr Thr Leu Gly Val Ala Thr Val Thr Cys
        35                  40                  45

Ala Ala Asp Ser Thr Ile Tyr Ala Ser Tyr Tyr Glu Cys Gly His Gly
    50                  55                  60

Leu Ser Thr Gly Gly Tyr Gly Tyr Ala Ala Phe Ser Val Pro Ser Val
65                  70                  75                  80

Thr Val Asn Phe Thr Ala
                85


<210> 42
<211> 110
<212> PRT
<213> Artificial Sequence

<220>
<223> Description of Artificial Sequence: VAP amino acid
      sequence of iMab800

<220>
<221> SITE
<222> (1)..(110)

<400> 42
Gly Arg Ser Ser Phe Thr Val Ser Thr Pro Asp Ile Leu Ala Asp Gly
1               5                   10                  15

Thr Met Ser Ser Thr Leu Ser Cys Arg Ala Ser Gly Tyr Thr Ile Gly
            20                  25                  30

Pro Gln Cys Leu Ser Phe Thr Gln Asn Gly Val Pro Val Ser Ile Ser
        35                  40                  45

Pro Ile Asn Met Gly Ser Tyr Thr Ala Thr Val Val Gly Asn Ser Val
    50                  55                  60

Gly Asp Val Thr Ile Thr Cys Ala Ala Asp Ser Thr Ile Tyr Ala Ser
65                  70                  75                  80

Tyr Tyr Glu Cys Gly His Gly Leu Ser Thr Gly Gly Tyr Gly Tyr Thr
                85                  90                  95

Leu Ile Leu Ser Thr Leu Gln Lys Lys Ile Ser Leu Phe Pro
            100                 105                 110


<210> 43
<211> 107
<212> PRT
<213> Artificial Sequence

<220>
<223> Description of Artificial Sequence: VAP amino acid
      sequence of iMab900

<220>
<221> SITE
<222> (1)..(107)

<400> 43
Leu Thr Leu Thr Ala Ala Val Ile Gly Asp Gly Ala Pro Ala Asn Gly
1               5                   10                  15

Lys Thr Ala Ile Thr Val Glu Cys Thr Ala Ser Gly Tyr Thr Ile Gly
            20                  25                  30

Pro Gln Cys Val Val Ile Thr Thr Asn Asn Gly Ala Leu Pro Asn Lys
        35                  40                  45

Ile Thr Glu Asn Met Gly Val Ala Arg Ile Ala Leu Thr Asn Thr Thr
    50                  55                  60

Asp Gly Val Thr Val Val Thr Cys Ala Ala Asp Ser Thr Ile Tyr Ala
65                  70                  75                  80

Ser Tyr Tyr Glu Cys Gly His Gly Leu Ser Thr Gly Gly Tyr Gly Tyr
                85                  90                  95

Gln Arg Gln Ser Val Asp Thr His Phe Val Lys
            100                 105


<210> 44
<211> 83
<212> PRT
<213> Artificial Sequence

<220>
<223> Description of Artificial Sequence: VAP amino acid
      sequence of iMab1000

<220>
<221> SITE
<222> (1)..(83)

<400> 44
His Lys Pro Val Ile Glu Lys Val Asp Gly Gly Tyr Leu Cys Lys Ala
1               5                   10                  15

Ser Gly Tyr Thr Ile Gly Pro Glu Cys Ile Glu Leu Leu Ala Asp Gly
            20                  25                  30

Arg Ser Tyr Thr Lys Asn Met Gly Glu Ala Phe Phe Ala Ile Asp Ala
        35                  40                  45

Ser Lys Val Thr Cys Ala Ala Asp Ser Thr Ile Tyr Ala Ser Tyr Tyr
    50                  55                  60

Glu Cys Gly His Gly Leu Ser Thr Gly Gly Tyr Gly Tyr His Trp Lys
65                  70                  75                  80

Ala Glu Asn

<210> 45
<211> 76
<212> PRT
<213> Artificial Sequence

<220>
<223> Description of Artificial Sequence: VAP amino acid
      sequence of iMab1001

<220>
<221> SITE
<222> (1)..(76)

<400> 45
Val Asp Gly Gly Tyr Leu Cys Lys Ala Ser Gly Tyr Thr Ile Gly Pro
1               5                   10                  15

Glu Cys Ile Glu Leu Leu Ala Asp Gly Arg Ser Tyr Thr Lys Asn Met
            20                  25                  30

Gly Glu Ala Phe Phe Ala Ile Asp Ala Ser Lys Val Thr Cys Ala Ala
        35                  40                  45

Asp Ser Thr Ile Tyr Ala Ser Tyr Tyr Glu Cys Gly His Gly Leu Ser
    50                  55                  60

Thr Gly Gly Tyr Gly Tyr His Trp Lys Ala Glu Asn
65                  70                  75

<210> 46
<211> 109
<212> PRT
<213> Artificial Sequence

<220>
<223> Description of Artificial Sequence: VAP amino acid
      sequence of iMab1100

<220>
<221> SITE
<222> (1)..(109)

<400> 46
Ala Pro Val Gly Leu Lys Ala Arg Leu Ala Asp Glu Ser Gly His Val
1               5                   10                  15

Val Leu Arg Cys Arg Ala Ser Gly Tyr Thr Ile Gly Pro Ile Cys Tyr
            20                  25                  30

Glu Val Asp Val Ser Ala Gly Asn Asp Ala Gly Ser Val Gln Arg Val
        35                  40                  45

Glu Ile Leu Asn Met Gly Thr Glu Ser Val Leu Ser Asn Leu Arg Gly
    50                  55                  60

Arg Thr Arg Tyr Thr Phe Ala Cys Ala Ala Asp Ser Thr Ile Tyr Ala
65                  70                  75                  80

Ser Tyr Tyr Glu Cys Gly His Gly Leu Ser Thr Gly Gly Tyr Gly Tyr
                85                  90                  95

Ser Ala Trp Ser Glu Pro Val Ser Leu Leu Thr Pro Ser
            100                 105


<210> 47
<211> 104
<212> PRT
<213> Artificial Sequence

<220>
<223> Description of Artificial Sequence: VAP amino acid
      sequence of iMab1200

<220>
<221> SITE
<222> (1)..(104)

<400> 47
His Gly Leu Pro Met Glu Lys Arg Gly Asn Phe Ile Val Gly Gln Asn
 1               5                  10                  15

Cys Ser Leu Thr Cys Pro Ala Ser Gly Tyr Thr Ile Gly Pro Gln Cys
            20                  25                  30

Val Phe Asn Cys Tyr Phe Asn Ser Ala Leu Ala Phe Ser Thr Glu Asn
        35                  40                  45

Met Gly Glu Trp Thr Leu Asp Met Val Phe Ser Asp Ala Gly Ile Tyr
    50                  55                  60

Thr Met Cys Ala Ala Asp Ser Thr Ile Tyr Ala Ser Tyr Tyr Glu Cys
65                  70                  75                  80

Gly His Gly Leu Ser Thr Gly Gly Tyr Gly Tyr Asn Pro Val Ser Leu
                85                  90                  95

Gly Ser Phe Val Val Asp Ser Pro
            100


<210> 48
<211> 133
<212> PRT
<213> Artificial Sequence

<220>
<223> Description of Artificial Sequence: VAP amino acid
      sequence of iMab1202

<220>
<221> SITE
<222> (1)..(133)

<400> 48
Ile Val Lys Leu Val Met Glu Lys Arg Gly Asn Phe Glu Asn Gly Gln
1               5                   10                  15

Asp Cys Lys Leu Thr Ile Arg Ala Ser Gly Tyr Thr Ile Gly Pro Ala
            20                  25                  30

Cys Asp Gly Phe Phe Cys Gln Phe Pro Ser Asp Asp Ser Phe Ser Thr
        35                  40                  45

Glu Asp Asn Met Gly Gly Gly Ile Thr Val Asn Asp Ala Met Lys Pro
    50                  55                  60

Gln Phe Asp Ile Arg Arg Asp Asn Ala Lys Gly Thr Trp Thr Leu Ser
65                  70                  75                  80

Met Asp Phe Gln Pro Glu Gly Ile Tyr Glu Met Gln Cys Ala Ala Asp
                85                  90                  95

Ser Thr Ile Tyr Ala Ser Tyr Tyr Glu Cys Gly His Gly Leu Ser Thr
            100                 105                 110

Gly Gly Tyr Gly Tyr Asp Asn Pro Val Arg Leu Gly Gly Phe Asp Val
        115                 120                 125

Asp Val Pro Asp Val
    130


<210> 49
<211> 101
<212> PRT
<213> Artificial Sequence

<220>
<223> Description of Artificial Sequence: VAP amino acid
      sequence of iMab1300

<220>
<221> SITE
<222> (1)..(101)

<400> 49
Leu Gln Val Asp Ile Lys Pro Ser Gln Gly Glu Ile Ser Val Gly Glu
1               5                   10                  15

Ser Lys Phe Phe Leu Cys Gln Ala Ser Gly Tyr Thr Ile Gly Pro Cys
            20                  25                  30

Ile Ser Trp Phe Ser Pro Asn Gly Glu Lys Leu Asn Met Gly Ser Ser
        35                  40                  45

Thr Leu Thr Ile Tyr Asn Ala Asn Ile Asp Asp Ala Gly Ile Tyr Lys
    50                  55                  60

Cys Ala Ala Asp Ser Thr Ile Tyr Ala Ser Tyr Tyr Glu Cys Gly His
65                  70                  75                  80

Gly Leu Ser Thr Gly Gly Tyr Gly Tyr Gln Ser Glu Ala Thr Val Asn
                85                  90                  95

Val Lys Ile Phe Gln
            100


<210> 50
<211> 136
<212> PRT
<213> Artificial Sequence

<220>
<223> Description of Artificial Sequence: VAP amino acid
      sequence of iMab1302

<220>
<221> SITE
<222> (1)..(136)

<400> 50
Val Val Lys Val Val Ile Lys Pro Ser Gln Asn Phe Ile Glu Asn Gly
1               5                   10                  15

Glu Asp Lys Lys Phe Thr Cys Arg Ala Ser Gly Tyr Thr Ile Gly Pro
            20                  25                  30

Lys Cys Ile Gly Trp Phe Ser Gln Asn Pro Glu Asp Asp Ser Thr Asn
        35                  40                  45

Val Ala Thr Ile Asn Met Gly Gly Gly Ile Thr Tyr Tyr Gly Asp Ser
    50                  55                  60

Val Lys Glu Arg Phe Asp Ile Arg Arg Asp Asn Ala Lys Asp Thr Ser
65                  70                  75                  80

Thr Leu Ser Ile Asp Asp Ala Gln Pro Glu Asp Ala Gly Ile Tyr Lys
                85                  90                  95

Cys Ala Ala Asp Ser Thr Ile Tyr Ala Ser Tyr Tyr Glu Cys Gly His
            100                 105                 110

Gly Leu Ser Thr Gly Gly Tyr Gly Tyr Asp Ser Glu Ala Thr Val Gly
        115                 120                 125

Val Asp Ile Phe Val Lys Leu Met
    130                 135


<210> 51
<211> 86

<212> PRT
<213> Artificial Sequence

<220>
<223> Description of Artificial Sequence: VAP amino acid
      sequence of iMab1301

<220>
<221> SITE
<222> (1)..(86)

<400> 51
Glu Ser Lys Phe Phe Leu Cys Gln Ala Ser Gly Tyr Thr Ile Gly Pro
1               5                   10                  15

Cys Ile Ser Trp Phe Ser Pro Asn Gly Glu Lys Leu Asn Met Gly Ser
            20                  25                  30

Ser Thr Leu Thr Ile Tyr Asn Ala Asn Ile Asp Asp Ala Gly Ile Tyr
        35                  40                  45

Lys Cys Ala Ala Asp Ser Thr Ile Tyr Ala Ser Tyr Tyr Glu Cys Gly
    50                  55                  60

His Gly Leu Ser Thr Gly Gly Tyr Gly Tyr Gln Ser Glu Ala Thr Val
65                  70                  75                  80

Asn Val Lys Ile Phe Gln
                85


<210> 52
<211> 104
<212> PRT
<213> Artificial Sequence

<220>
<223> Description of Artificial Sequence: VAP amino acid
      sequence of iMab1400

<220>
<221> SITE
<222> (1)..(104)

<400> 52
Val Pro Arg Asp Leu Glu Val Val Ala Ala Thr Pro Thr Ser Leu Leu
1               5                   10                  15

Ile Ser Cys Asp Ala Ser Gly Tyr Thr Ile Gly Pro Tyr Cys Ile Thr
            20                  25                  30

Tyr Gly Glu Thr Gly Gly Asn Ser Pro Val Gln Glu Phe Thr Val Pro
        35                  40                  45

Asn Met Gly Lys Ser Thr Ala Thr Ile Ser Gly Leu Lys Pro Gly Val
    50                  55                  60

Asp Tyr Thr Ile Thr Cys Ala Ala Asp Ser Thr Ile Tyr Ala Ser Tyr
65                  70                  75                  80

Tyr Glu Cys Gly His Gly Leu Ser Thr Gly Gly Tyr Gly Tyr Ser Lys
                85                  90                  95

Pro Ile Ser Ile Asn Tyr Arg Thr
            100


<210> 53
<211> 110
<212> PRT
<213> Artificial Sequence

<220>
<223> Description of Artificial Sequence: VAP amino acid
      sequence of iMab1500

<220>
<221> SITE
<222> (1)..(110)

<400> 53
Ile Lys Val Tyr Thr Asp Arg Glu Asn Tyr Gly Ala Val Gly Ser Gln
1               5                   10                  15

Val Thr Leu His Cys Ser Ala Ser Gly Tyr Thr Ile Gly Pro Ile Cys
            20                  25                  30

Phe Thr Trp Arg Tyr Gln Pro Glu Gly Asp Arg Asp Ala Ile Ser Ile
        35                  40                  45

Phe His Tyr Asn Met Gly Asp Gly Ser Ile Val Ile His Asn Leu Asp
    50                  55                  60

Tyr Ser Asp Asn Gly Thr Phe Thr Cys Ala Ala Asp Ser Thr Ile Tyr
65                  70                  75                  80

Ala Ser Tyr Tyr Glu Cys Gly His Gly Leu Ser Thr Gly Gly Tyr Gly
                85                  90                  95

Tyr Val Gly Lys Thr Ser Gln Val Thr Leu Tyr Val Phe Glu
            100                 105                 110


<210> 54
<211> 136
<212> PRT
<213> Artificial Sequence

<220>
<223> Description of Artificial Sequence: VAP amino acid
      sequence of iMab1502

<220>
<221> SITE

<222> (1)..(136)

<400> 54
Asn Val Lys Val Val Thr Lys Arg Glu Asn Phe Gly Glu Asn Gly Ser
1               5                   10                  15

Asp Val Lys Leu Thr Cys Arg Ala Ser Gly Tyr Thr Ile Gly Pro Ile
            20                  25                  30

Cys Phe Gly Trp Phe Tyr Gln Pro Glu Gly Asp Asp Ser Ala Ile Ser
        35                  40                  45

Ile Phe His Asn Met Gly Gly Gly Ile Thr Asp Glu Val Asp Thr Phe
    50                  55                  60

Lys Glu Arg Phe Asp Ile Arg Arg Asp Asn Ala Lys Lys Thr Gly Thr
65                  70                  75                  80

Ile Ser Ile Asp Asp Leu Gln Pro Ser Asp Asn Glu Thr Phe Thr Cys
                85                  90                  95

Ala Ala Asp Ser Thr Ile Tyr Ala Ser Tyr Tyr Glu Cys Gly His Gly
            100                 105                 110

Leu Ser Thr Gly Gly Tyr Gly Tyr Asp Gly Lys Thr Arg Gln Val Gly
        115                 120                 125

Leu Asp Val Phe Val Lys Val Pro
    130                 135


<210> 55
<211> 96
<212> PRT
<213> Artificial Sequence

<220>
<223> Description of Artificial Sequence: VAP amino acid
      sequence of iMab1501

<220>
<221> SITE
<222> (1)..(96)

<400> 55
Ser Gln Val Thr Leu His Cys Ser Ala Ser Gly Tyr Thr Ile Gly Pro
1               5                   10                  15

Ile Cys Phe Thr Trp Arg Tyr Gln Pro Glu Gly Asp Arg Asp Ala Ile
            20                  25                  30

Ser Ile Phe His Tyr Asn Met Gly Asp Gly Ser Ile Val Ile His Asn
        35                  40                  45

Leu Asp Tyr Ser Asp Asn Gly Thr Phe Thr Cys Ala Ala Asp Ser Thr
    50                  55                  60

Ile Tyr Ala Ser Tyr Tyr Glu Cys Gly His Gly Ile Ser Thr Gly Gly
65                  70                  75                  80

Tyr Gly Tyr Val Gly Lys Thr Ser Gln Val Thr Leu Tyr Val Phe Glu
                85                  90                  95

<210> 56
<211> 102
<212> PRT
<213> Artificial Sequence

<220>
<223> Description of Artificial Sequence: VAP amino acid
      sequence of iMab1600

<220>
<221> SITE
<222> (1)..(102)

<400> 56
Ser Lys Pro Gln Ile Gly Ser Val Ala Pro Asn Met Gly Ile Pro Gly
  1               5                  10                  15

Asn Asp Val Thr Ile Thr Cys Arg Ala Ser Gly Tyr Thr Ile Gly Pro
             20                  25                  30

Thr Cys Gly Thr Val Thr Phe Gly Gly Val Thr Asn Met Gly Asn Arg
         35                  40                  45

Ile Glu Val Tyr Val Pro Asn Met Ala Ala Gly Leu Thr Asp Val Lys
     50                  55                  60

Cys Ala Ala Asp Ser Thr Ile Tyr Ala Ser Tyr Tyr Glu Cys Gly His
 65                  70                  75                  80

Gly Leu Ser Thr Gly Gly Tyr Gly Tyr Gly Val Ser Ser Asn Leu Tyr
                85                  90                  95

Ser Tyr Asn Ile Leu Ser
            100

<210> 57
<211> 137
<212> PRT
<213> Artificial Sequence

<220>
<223> Description of Artificial Sequence: VAP amino acid
      sequence of iMab1602

<220>
<221> SITE
<222> (1)..(137)

<400> 57

Ala Val Lys Pro Val Ile Gly Ser Lys Ala Pro Asn Phe Gly Glu Asn
1               5                   10                  15

Gly Asp Val Lys Thr Ile Asp Arg Ala Ser Gly Tyr Thr Ile Gly Pro
            20                  25                  30

Thr Cys Gly Gly Val Phe Phe Gln Gly Pro Thr Asp Asp Ser Thr Asn
        35                  40                  45

Val Ala Thr Ile Asn Met Gly Gly Gly Ile Thr Tyr Tyr Gly Asp Ser
    50                  55                  60

Val Lys Glu Thr Phe Asp Ile Arg Arg Asp Asn Ala Lys Ser Thr Arg
65                  70                  75                  80

Thr Glu Ser Tyr Asp Asp Asn Gln Pro Glu Gly Leu Thr Glu Val Lys
                85                  90                  95

Cys Ala Ala Asp Ser Thr Ile Tyr Ala Ser Tyr Tyr Glu Cys Gly His
            100                 105                 110

Gly Leu Ser Thr Gly Gly Tyr Gly Tyr Asp Val Ser Ser Arg Leu Tyr
        115                 120                 125

Gly Tyr Asp Ile Leu Val Gly Thr Gln
    130                 135


<210> 58
<211> 104
<212> PRT
<213> Artificial Sequence

<220>
<223> Description of Artificial Sequence: VAP amino acid
      sequence of iMab1700

<220>
<221> SITE
<222> (1)..(104)

<400> 58
Lys Asp Pro Glu Ile His Leu Ser Gly Pro Leu Glu Ala Gly Lys Pro
1               5                   10                  15

Ile Thr Val Lys Cys Ser Ala Ser Gly Tyr Thr Ile Gly Pro Leu Cys
            20                  25                  30

Ile Asp Leu Leu Lys Gly Asp His Leu Met Lys Ser Gln Glu Phe Asn
        35                  40                  45

Met Gly Ser Leu Glu Val Thr Phe Thr Pro Val Ile Glu Asp Ile Gly
    50                  55                  60

Lys Val Leu Val Cys Ala Ala Asp Ser Thr Ile Tyr Ala Ser Tyr Tyr
65                  70                  75                  80

Glu Cys Gly His Gly Leu Ser Thr Gly Gly Tyr Gly Tyr Val Arg Gln
                85                  90                  95

Ala Val Lys Glu Leu Gln Val Asp
            100


<210> 59
<211> 90
<212> PRT
<213> Artificial Sequence

<220>
<223> Description of Artificial Sequence: VAP amino acid
      sequence of iMab1701

<220>
<221> SITE
<222> (1)..(90)

<400> 59
Lys Pro Ile Thr Val Lys Cys Ser Ala Ser Gly Tyr Thr Ile Gly Pro
 1               5                  10                  15

Leu Cys Ile Asp Leu Leu Lys Gly Asp His Leu Met Lys Ser Gln Glu
            20                  25                  30

Phe Asn Met Gly Ser Leu Glu Val Thr Phe Thr Pro Val Ile Glu Asp
        35                  40                  45

Ile Gly Lys Val Leu Val Cys Ala Ala Asp Ser Thr Ile Tyr Ala Ser
    50                  55                  60

Tyr Tyr Glu Cys Gly His Gly Leu Ser Thr Gly Gly Tyr Gly Tyr Val
65                  70                  75                  80

Arg Gln Ala Val Lys Glu Leu Gln Val Asp
                85                  90


<210> 60
<211> 402
<212> DNA
<213> Artificial Sequence

<220>
<223> Description of Artificial Sequence: DNA sequence
      iMab D 100

<220>
<221> misc_feature
<222> (1)..(402)

<400> 60
aatgtgaaac tggttgaaaa aggtggcaat ttcgtcgaaa acgatgacga tcttaagctc 60
acgtgccgtg ctgaaggtta caccattggc ccgtactgca tgggttggtt ccgtcaggcg 120
ccgaacgacg acagtactaa cgtggccacg atcaacatgg gtggcggtat tacgtactac 180

ggtgactccg tcaaagagcg cttcgatatc cgtcgcgaca acgcgtccaa caccgttacc 240
ttatcgatgg acgatctgca accggaagac tctgcagaat acaattgtgc aggtgattct 300
accatttacg cgagctatta tgaatgtggt catggcctga gtaccggcgg ttacggctac 360
gatagccact accgtggtca gggtaccgac gttaccgtct cg 402

<210> 61
<211> 351
<212> DNA
<213> Artificial Sequence

<220>
<223> Description of Artificial Sequence: DNA sequence
      iMab D 101

<220>
<221> misc_feature
<222> (1)..(351)

<400> 61
catatggtta aactggttga aaaaggtggt aacttcgttg aaaacgacga cgacctgaaa 60
ctgacctgcc gtgcttccgg ttacaccatc ggtccgtact gcatgggttg gttccgtcag 120
gctccgaacg acgactccac caacgttgct accatcaaca tgggtaccgt taccctgtcc 180
atggacgacc tgcagccgga agactccgct gaatacaact gcgctgctga ctccaccatc 240
tacgcttcct actacgaatg cggtcacggt atctccaccg gtggttacgg ttacgactcc 300
cactaccgtg gtcagggtac cgacgttacc gtttcctcgg ccagctcggc c 351

<210> 62
<211> 306
<212> DNA
<213> Artificial Sequence

<220>
<223> Description of Artificial Sequence: DNA sequence
      iMab D 102

<220>
<221> misc_feature
<222> (1)..(306)

<400> 62
catatggacc tgaaactgac ctgccgtgct tccggttaca ccatcggtcc gtactgcatg 60
ggttggttcc gtcaggctcc gaacgacgac tccaccaacg ttgctaccat caacatgggt 120
accgttaccc tgtccatgga cgacctgcag ccggaagact ccgctgaata caactgcgct 180
gctgactcca ccatctacgc ttcctactac gaatgcggtc acggtatctc caccggtggt 240
tacggttacg actcccacta ccgtggtcag ggtaccgacg ttaccgtttc ctcggccagc 300
tcggcc 306

<210> 63
<211> 423
<212> DNA
<213> Artificial Sequence

<220>
<223> Description of Artificial Sequence: DNA sequence

iMab D111

<220>
<221> misc_feature
<222> (1)..(423)

<400> 63
catatgaatg tgaaactggt ttgtaaaggt ggcaatttcg tcgaaaacga tgacgatctt 60
aagctcacgt gccgtgctga aggttacacc attggcccgt actgcatggg ttggttccgt 120
caggcgccga acgacgacag tactaacgtg gccacgatca acatgggtgg cggtattacg 180
tactacggtg actccgtcaa agagcgcttc gatatccgtc gcgacaacgc gtccaacacc 240
gttaccttat cgatggacga tctgcaaccg gaagactctg cagaatacaa ttgtgcaggt 300
gattctacca tttacgcgag ctattatgaa tgtggtcatg gcctgagtac cggcggttac 360
ggctacgata gccactaccg ttgccagggt accgacgtta ccgtctcgtc ggccagctcg 420
gcc 423


<210> 64
<211> 405
<212> DNA
<213> Artificial Sequence

<220>
<223> Description of Artificial Sequence: DNA sequence
      iMab D112

<220>
<221> misc_feature
<222> (1)..(405)

<400> 64
aatgtgaaac tggttgaaaa aggtggcaat ttcgtcgaaa acgatgacga tcttaagctc 60
acgtgccgtg ctgaaggtta caccattggc ccgtactgca tgggttggtt ctgtcaggcg 120
ccgaacgacg acagtacttg cgtggccacg atcaacatgg gtggcggtat tacgtactac 180
ggtgactccg tcaaagagcg cttcgatatc cgtcgcgaca acgcgtccaa caccgttacc 240
ttatcgatgg acgatctgca accggaagac tctgcagaat acaattgtgc aggtgattct 300
accatttacg cgagctatta tgaatgtggt catggcctga gtaccggcgg ttacggctac 360
gatagccact accgtggtca gggtaccgac gttaccgtct cgtcg 405


<210> 65
<211> 405
<212> DNA
<213> Artificial Sequence

<220>
<223> Description of Artificial Sequence: DNA sequence
      iMab D113

<220>
<221> misc_feature
<222> (1)..(405)

<400> 65
aatgtgaaac tggttgaaaa aggtggcaat ttcgtcgaaa acgatgacga tcttaagctc 60
acgtgccgtg ctgaaggtta caccattggc ccgtactcca tgggttggtt ccgtcaggcg 120
ccgaacgacg acagtactaa cgtgtcctgc atcaacatgg gtggcggtat tacgtactac 180

ggtgactccg tcaaagagcg cttcgatatc cgtcgcgaca acgcgtccaa caccgttacc 240
ttatcgatgg acgatctgca accggaagac tctgcagaat acaattgtgc aggtgattct 300
accatttacg cgagctatta tgaatgtggt catggcctga gtaccggcgg ttacggctac 360
gatagccact accgtggtca gggtaccgac gttaccgtct cgtcg 405

<210> 66
<211> 405
<212> DNA
<213> Artificial Sequence

<220>
<223> Description of Artificial Sequence: DNA sequence
      iMab D114

<220>
<221> misc_feature
<222> (1)..(405)

<400> 66
aatgtgaaac tggttgaaaa aggtggcaat ttcgtcgaaa acgatgacga tcttaagctc 60
acgtgccgtg ctgaaggtta caccattggc ccgtactcca tgggttggtt ccgtcaggcg 120
ccgaacgacg acagtactaa cgtggccacg atcaacatgg gtggcggtat tacgtactac 180
ggtgactccg tcaaagagcg cttcgatatc cgtcgcgaca acgcgtccaa caccgttacc 240
ttatcgatgg acgatctgca accggaagac tctgcagaat acaattgtgc aggtgattct 300
accatttacg cgagctatta tgaatgtggt catggcctga gtaccggcgg ttacggctac 360
gatagccact accgtggtca gggtaccgac gttaccgtct cgtcg 405

<210> 67
<211> 405
<212> DNA
<213> Artificial Sequence

<220>
<223> Description of Artificial Sequence: DNA sequence
      iMab D115

<220>
<221> misc_feature
<222> (1)..(405)

<400> 67
aatgtgaaac tggttgaaaa aggtggcaat ttcgtcgaaa acgatgacga tcttaagctc 60
acgtgccgtg ctgaaggtta caccattggc ccgtactgca tgggttggtt ccgtcaggcg 120
ccgaacgacg acagtactaa cgtggccacg atcaacatgg gtggcggtat tacgtactac 180
ggtgactccg tcaaagagcg cttcgatatc cgtcgcgacc aggcgtccaa caccgttacc 240
ttatcgatgg acgatctgca accggaagac tctgcagaat acaattgtgc aggtgattct 300
accatttacg cgagctatta tgaatgtggt catggcctga gtaccggcgg ttacggctac 360
gatagccact accgtggtca gggtaccgac gttaccgtct cgtcg 405

<210> 68
<211> 423
<212> DNA
<213> Artificial Sequence

<220>
<223> Description of Artificial Sequence: DNA sequence
      iMab D116

<220>
<221> misc_feature
<222> (1)..(423)

<400> 68
catatgaatg tgaaactggt tgaaaaaggt ggcaatttcg tcgaaaacga tgacgatctt 60
aagctcacgt gtcgtgctga aggttacacc attggcccgt actgcatggg ttggttccgt 120
caggcgccga acgacgacag tactaacgtg gccacgatca acatgggtgg cggtattacg 180
tactacggtg actccgtcaa agagcgcttc gatatccgtc gcgacaacgc gtccaacacc 240
gttaccttat cgatggacga tctgcaaccg gaagactctg cagaatacaa tggtgcaggt 300
gattctacca tttacgggag ctattatgaa tgtggtcatg gcctgagtac cggcggttac 360
ggctacgata gccactaccg tggtcagggt accgacgtta ccgtctcgtc ggccagctcg 420
gcc                                                               423


<210> 69
<211> 399
<212> DNA
<213> Artificial Sequence

<220>
<223> Description of Artificial Sequence: DNA sequence
      iMab D120

<220>
<221> misc_feature
<222> (1)..(399)

<400> 69
aatgtgaaac tggttgaaaa aggtggcaat ttcgtcgaaa acgatgacga tcttaagctc 60
acgtgccgtg ctgaaggtta caccattggc ccgtactgca tgggttggtt ccgtcaggcg 120
ccgaacgacg acagtactaa cgtggccacg atcaacatgg gtggcggtat tacgtactac 180
ggtgactccg tcaaagagcg cttcgatatc cgtcgcgaca acgcgtccaa caccgttacc 240
ttatcgatgg acgatctgca accggaagac tctgcagaat acaattgtgc aggtgattct 300
accatttacg cgagctatta tgaatgtggt catggcctga gtaccggcgg ttacggctac 360
gatagccgtg gtcagggtac cgacgttacc gtctcgtcg                        399


<210> 70
<211> 396
<212> DNA
<213> Artificial Sequence

<220>
<223> Description of Artificial Sequence: DNA sequence
      iMab D121

<220>
<221> misc_feature
<222> (1)..(396)

<400> 70
catatgaacg ttaaactggt tgaaaaaggt ggtaacttcg ttgaaaacga cgacgacctg 60

aaactgacct gccgtgcttc cggtcgttcc ttctcctcct acatcatggg ttggttccgt 120
caggctccga acgacgactc caccaacgtt gctaccatct ccgaaaccgg tggtgacatc 180
gtttacacca actacggtga ctccgttaaa gaacgtttcg acatccgtcg tgacatcgct 240
tccaacaccg ttaccctgtc catggacgac ctgcagccgg aagactccgc tgaatacaac 300
tgcgctggtt ccgtttacgg ttccggttgg cgtccggacc gttacgacta ccgtggtcag 360
ggtaccgacg ttaccgtttc ctcggccagc tcggcc 396

<210> 71
<211> 393
<212> DNA
<213> Artificial Sequence

<220>
<223> Description of Artificial Sequence: DNA sequence
      iMab D122

<220>
<221> misc_feature
<222> (1)..(393)

<400> 71
catatgaacg ttaaactggt tgaaaaaggt ggtaacttcg ttgaaaacga cgacgacctg 60
aaactgacct gccgtgcttc cggtcgtacc ttctcctccc gtaccatggg ttggttccgt 120
caggctccga acgacgactc caccaacgtt gctaccatcc gttggaacgg tggttccacc 180
tactacacca actacggtga ctccgttaaa gaacgtttcg acatccgtgt tgaccaggct 240
tccaacaccg ttaccctgtc catggacgac ctgcagccgg aagactccgc tgaatacaac 300
tgcgctggta ccgacatcgg tgacggttgg tccggtcgtt acgactaccg tggtcagggt 360
accgacgtta ccgtttcctc ggccagctcg gcc 393

<210> 72
<211> 390
<212> DNA
<213> Artificial Sequence

<220>
<223> Description of Artificial Sequence: DNA sequence
      iMab D123

<220>
<221> misc_feature
<222> (1)..(390)

<400> 72
catatgaacg ttaaactggt tgaaaaaggt ggtaacttcg ttgaaaacga cgacgacctg 60
aaactgacct gccgtgcttc cggtcgtacc ttctcccgtg ctgctatggg ttggttccgt 120
caggctccga acgacgactc caccaacgtt gctaccatca cctggtccgg tcgtcacacc 180
cgttacggtg actccgttaa agaacgtttc gacatccgtc gtgaccaggc ttccaacacc 240
gttaccctgt ccatggacga cctgcagccg gaagactccg ctgaatacaa ctgcgctggt 300
gaaggttcca acaccgcttc cacctccccg cgtccgtacg actaccgtgg tcagggtacc 360
gacgttaccg tttcctcggc cagctcggcc 390

<210> 73
<211> 273
<212> DNA

<213> Artificial Sequence

<220>
<223> Description of Artificial Sequence: DNA sequence
      iMab D124

<220>
<221> misc_feature
<222> (1)..(273)

<400> 73
catatggacg acctgaaact gacctgccgt gcttccggtc gttccttctc ctcctacatc 60
atgggttggt tccgtcaggc tccgaacgac gactccacca acgttgctac catctccgaa 120
accaccgtta ccctgtccat ggacgacctg cagccggaag actccgctga atacaactgc 180
gctggttccg tttacggttc cggttggcgt ccggaccgtt acgactaccg tggtcagggt 240
accgacgtta ccgtttcctc ggccagctcg gcc                              273


<210> 74
<211> 270
<212> DNA
<213> Artificial Sequence

<220>
<223> Description of Artificial Sequence: DNA sequence
      iMab D125

<220>
<221> misc_feature
<222> (1)..(270)

<400> 74
catatggacg acctgaaact gacctgccgt gcttccggtc gtaccttctc ctcccgtacc 60
atgggttggt tccgtcaggc tccgaacgac gactccacca acgttgctac catccgttgg 120
aacaccgtta ccctgtccat ggacgacctg cagccggaag actccgctga atacaactgc 180
gctggtaccg acatcggtga cggttggtcc ggtcgttacg actaccgtgg tcagggtacc 240
gacgttaccg tttcctcggc cagctcggcc                                  270


<210> 75
<211> 375
<212> DNA
<213> Artificial Sequence

<220>
<223> Description of Artificial Sequence: DNA sequence
      iMab D130

<220>
<221> misc_feature
<222> (1)..(375)

<400> 75
aatgtgaaac tggttgaaaa aggtggcaat ttcgtcgaaa acgatgacga tcttaagctc 60
acgtgccgtg ctagcggtta cgcctacacg tatatctaca tgggttggtt ccgtcaggcg 120
ccgaacgacg acagtactaa cgtggccacc atcgactcgg gtggcggcgg taccctgtac 180
ggtgactccg tcaaagagcg cttcgatatc cgtcgcgaca aaggctccaa caccgttacc 240

ttatcgatgg acgatctgca accggaagac tctgcagaat acaattgtgc agcgggtggc 300
tacgaactgc gcgaccgcac ctacggtcag cgtggtcagg gtaccgacgt taccgtctcg 360
tcggccagct cggcc 375

<210> 76
<211> 345
<212> DNA
<213> Artificial Sequence

<220>
<223> Description of Artificial Sequence: DNA sequence
      iMab D201

<220>
<221> misc_feature
<222> (1)..(345)

<400> 76
catatggttc agctgcaggc ttccggtggt ggttccgttc aggctggtgg ttccctgcgt 60
ctgtcctgcc gtgcttccgg ttacaccatc ggtccgtact gcatgggttg gttccgtcag 120
gctccgggtg acgactccga aggtgttgct gctatcaaca tgggtaccgt ttacctgctg 180
atgaactccc tggaaccgga agacaccgct atctactact gcgctgctga ctccaccatc 240
tacgcttcct actacgaatg cggtcacggt atctccaccg gtggttacgg ttacgactcc 300
tggggtcagg gtacccaggt taccgtttcc tcggccagct cggcc 345

<210> 77
<211> 345
<212> DNA
<213> Artificial Sequence

<220>
<223> Description of Artificial Sequence: DNA sequence
      iMab D300

<220>
<221> misc_feature
<222> (1)..(345)

<400> 77
catatggttc agctgcagca gccgggttcc aacctggttc gtccgggtgc ttccgttaaa 60
ctgtcctgca aagcttccgg ttacaccatc ggtccgtcct gcatccactg ggctaaacag 120
cgtccgggtg acggtctgga atggatcggt gaaatcaaca tgggtaccgc ttacgttgac 180
ctgtcctccc tgacctccga agactccgct gtttactact gcgctgctga ctccaccatc 240
tacgcttcct actacgaatg cggtcacggt atctccaccg gtggttacgg ttacgactac 300
tggggtcagg gtaccaccct gaccgtttcc tcggccagct cggcc 345

<210> 78
<211> 303
<212> DNA
<213> Artificial Sequence

<220>
<223> Description of Artificial Sequence: DNA sequence
      iMab D302

<220>
<221> misc_feature
<222> (1)..(303)

<400> 78
catatggctt ccgttaaact gtcctgcaaa gcttccggtt acaccatcgg tccgtcctgc 60
atccactggg ctaaacagcg tccgggtgac ggtctggaat ggatcggtga aatcaacatg 120
ggtaccgctt acgttgacct gtcctccctg acctccgaag actccgctgt ttactactgc 180
gctgctgact ccaccatcta cgcttcctac tacgaatgcg gtcacggtat ctccaccggt 240
ggttacggtt acgactactg gggtcagggt accaccctga ccgtttcctc ggccagctcg 300
gcc 303

<210> 79
<211> 345
<212> DNA
<213> Artificial Sequence

<220>
<223> Description of Artificial Sequence: DNA sequence
      iMab D400

<220>
<221> misc_feature
<222> (1)..(345)

<400> 79
catatggttc agctggttga atccggtggt ggtctggttc agccgggtgg ttccctgcgt 60
ctgtcctgcc gtgcttccgg ttacaccatc ggtccgtact gcatgaactg ggttcgtcag 120
gctccgggtg acggtctgga atgggttggt tggatcaaca tgggtaccgc ttacctgcag 180
atgaactccc tgcgtgctga agacaccgct gtttactact gcgctgctga ctccaccatc 240
tacgcttcct actacgaatg cggtcacggt atctccaccg gtggttacgg ttacgacgtt 300
tggggtcagg gtaccctggt taccgtttcc tcggccagct cggcc 345

<210> 80
<211> 357
<212> DNA
<213> Artificial Sequence

<220>
<223> Description of Artificial Sequence: DNA sequence
      iMab D500

<220>
<221> misc_feature
<222> (1)..(357)

<400> 80
catatgccga acttcctgtg ctccgttctg ccgacccact ggcgttgcaa caaaaccctg 60
ccgatcgctt tcaaatgccg tgcttccggt tacaccatcg gtccgacctg cgttaccgtt 120
atggctggta acgacgaaga ctactccaac atgggtgctc gtttcaacga cctgcgtttc 180
gttggtcgtt ccggtcgtgg taaatccttc accctgacct gcgctgctga ctccaccatc 240
tacgcttcct actacgaatg cggtcacggt atctccaccg gtggttacgg ttacccgcag 300
gttgctacct accaccgtgc tatcaaaatc accgttgacg gtccggccag ctcggcc 357

<210> 81
<211> 444
<212> DNA
<213> Artificial Sequence

<220>
<223> Description of Artificial Sequence: DNA sequence
      iMab D502

<220>
<221> misc_feature
<222> (1)..(444)

<400> 81
catatgtccg ttaaattcgt ttgcaaagtt ctgccgaact tctgggaaaa caacaaagac 60
ctgccgatca aattcaccgt tcgtgcttcc ggttacacca tcggtccgac ctgcgttggt 120
gttttcgctc agaacccgga agacgactcc accaacgttg ctaccatcaa catgggtggt 180
ggtatcacct actacggtga ctccgttaaa ctgcgtttcg acatccgtcg tgacaacgct 240
aaagttaccc gtaccaactc cctggacgac gttcagccgg aaggtcgtgg taaatccttc 300
gaactgacct gcgctgcaga ctccaccatc tacgcttcct actacgaatg cggtcacggt 360
ctgtccaccg gtggttacgg ttacgaccag gttgctcgtt accaccgtgg tatcgacatc 420
accgtctcgt cggccagctc ggcc                                        444


<210> 82
<211> 345
<212> DNA
<213> Artificial Sequence

<220>
<223> Description of Artificial Sequence: DNA sequence
      iMab D600

<220>
<221> misc_feature
<222> (1)..(345)

<400> 82
catatggctc cggttggtct gaaagctcgt aacgctgacg aatccggtca cgttgttctg 60
cgttgccgtg cttccggtta caccatcggt ccgatctgct acgaagttga cgtttccgct 120
ggtcaggacg ctggttccgt tcagcgtgtt gaaatcaaca tgggtcgtac cgaatccgtt 180
ctgtccaacc tgcgtggtcg tacccgttac accttcgctt gcgctgctga ctccaccatc 240
tacgcttcct actacgaatg cggtcacggt atctccaccg gtggttacgg ttactccgaa 300
tggtccgaac cggtttccct gctgaccccg tcggccagct cggcc                 345


<210> 83
<211> 327
<212> DNA
<213> Artificial Sequence

<220>
<223> Description of Artificial Sequence: DNA sequence
      iMab D700

<220>

<221> misc_feature
<222> (1)..(327)

<400> 83
catatggaca aatccaccct ggctgctgtt ccgacctcca tcatcgctga cggtctgatg 60
gcttccacca tcacctgcga agcttccggt tacaccatcg gtccggcttg cgttgctttc 120
gacaccaccc tgggtaacaa catgggtacc tactccgctc cgctgacctc caccaccctg 180
ggtgttgcta ccgttacctg cgctgctgac tccaccatct acgcttccta ctacgaatgc 240
ggtcacggta tctccaccgg tggttacggt tacgctgctt tctccgttcc gtccgttacc 300
gttaacttca ccgcggccag ctcggcc 327


<210> 84
<211> 276
<212> DNA
<213> Artificial Sequence

<220>
<223> Description of Artificial Sequence: DNA sequence
      iMab D701

<220>
<221> misc_feature
<222> (1)..(276)

<400> 84
catatgatgg cttccaccat cacctgcgaa gcttccggtt acaccatcgg tccggcttgc 60
gttgctttcg acaccaccct gggtaacaac atgggtacct actccgctcc gctgacctcc 120
accaccctgg gtgttgctac cgttacctgc gctgctgact ccaccatcta cgcttcctac 180
tacgaatgcg gtcacggtat ctccaccggt ggttacggtt acgctgcttt ctccgttccg 240
tccgttaccg ttaacttcac cgcggccagc tcggcc 276


<210> 85
<211> 435
<212> DNA
<213> Artificial Sequence

<220>
<223> Description of Artificial Sequence: DNA sequence
      iMab D702

<220>
<221> misc_feature
<222> (1)..(435)

<400> 85
catatggctg ttaaatccgt tttcaaagtt tccaccaact tcatcgaaaa cgacggcacc 60
atggactcca aactgacctt ccgtgcttcc ggttacacca tcggtccgca gtgcctgggt 120
ttcttccagc agggtgttcc ggacgactcc accaacgttg ctaccatcaa catgggtggt 180
ggtatcacct actacggtga ctccgttaaa tccatcttcg acatccgtcg tgacaacgct 240
aaagacacct acaccgcttc cgttgacgac aaccagccgg aagacgttga aatcacctgc 300
gctgcagact ccaccatcta cgcttcctac tacgaatgcg gtcacggtct gtccaccggt 360
ggttacggtt acgacctgat cctgcgtacc ctgcaaaaag gtatcgacct gttcgtctcg 420
tcggccagct cggcc 435

<210> 86
<211> 348
<212> DNA
<213> Artificial Sequence

<220>
<223> Description of Artificial Sequence: DNA sequence
      iMab D800

<220>
<221> misc_feature
<222> (1)..(348)

<400> 86
catatgggtc gttcctcctt caccgtttcc accccggaca tcctggctga cggtaccatg 60
tcctccaccc tgtcctgccg tgcttccggt tacaccatcg gtccgcagtg cctgtccttc 120
acccagaacg gtgttccggt ttccatctcc ccgatcaaca tgggttccta caccgctacc 180
gttgttggta actccgttgg tgacgttacc atcacctgcg ctgctgactc caccatctac 240
gcttcctact acgaatgcgg tcacggtatc tccaccggtg gttacggtta caccctgatc 300
ctgtccaccc tgcagaaaaa aatctccctg ttcccggcca gctcggcc 348


<210> 87
<211> 339
<212> DNA
<213> Artificial Sequence

<220>
<223> Description of Artificial Sequence: DNA sequence
      iMab D900

<220>
<221> misc_feature
<222> (1)..(339)

<400> 87
catatgctga ccctgaccgc tgctgttatc ggtgacggtg ctccggctaa cggtaaaacc 60
gctatcaccg ttgaatgcac cgcttccggt tacaccatcg gtccgcagtg cgttgttatc 120
accaccaaca acggtgctct gccgaacaaa atcaccgaaa acatgggtgt tgctcgtatc 180
gctctgacca acaccaccga cggtgttacc gttgttacct gcgctgctga ctccaccatc 240
tacgcttcct actacgaatg cggtcacggt atctccaccg gtggttacgg ttaccagcgt 300
cagtccgttg acacccactt cgttaaggcc agctcggcc 339


<210> 88
<211> 270
<212> DNA
<213> Artificial Sequence

<220>
<223> Description of Artificial Sequence: DNA sequence
      iMab D1000

<220>
<221> misc_feature
<222> (1)..(270)

<400> 88
catatgcaca aaccggttat cgaaaaagtt gacggtggtt acctgtgcaa agcttccggt 60
tacaccatcg gtccggaatg catcgaactg ctggctgacg gtcgttccta caccaaaaac 120
atgggtgaag ctttcttcgc tatcgacgct tccaaagtta cctgcgctgc tgactccacc 180
atctacgctt cctactacga atgcggtcac ggtatctcca ccggtggtta cggttaccac 240
tggaaagctg aaaactcggc cagctcggcc 270

<210> 89
<211> 249
<212> DNA
<213> Artificial Sequence

<220>
<223> Description of Artificial Sequence: DNA sequence
      iMab D1001

<220>
<221> misc_feature
<222> (1)..(249)

<400> 89
catatggttg acggtggtta cctgtgcaaa gcttccggtt acaccatcgg tccggaatgc 60
atcgaactgc tggctgacgg tcgttcctac accaaaaaca tgggtgaagc tttcttcgct 120
atcgacgctt ccaaagttac ctgcgctgct gactccacca tctacgcttc ctactacgaa 180
tgcggtcacg gtatctccac cggtggttac ggttaccact ggaaagctga aaattcggcc 240
agctcggcc 249

<210> 90
<211> 345
<212> DNA
<213> Artificial Sequence

<220>
<223> Description of Artificial Sequence: DNA sequence
      iMab D1100

<220>
<221> misc_feature
<222> (1)..(345)

<400> 90
catatggctc cggttggtct gaaagctcgt ctggctgacg aatccggtca cgttgttctg 60
cgttgccgtg cttccggtta caccatcggt ccgatctgct acgaagttga cgtttccgct 120
ggtaacgacg ctggttccgt tcagcgtgtt gaaatcctga acatgggtac cgaatccgtt 180
ctgtccaacc tgcgtggtcg tacccgttac accttcgctt gcgctgctga ctccaccatc 240
tacgcttcct actacgaatg cggtcacggt atctccaccg gtggttacgg ttactccgct 300
tggtccgaac cggtttccct gctgaccccg tcggccagct cggcc 345

<210> 91
<211> 330
<212> DNA
<213> Artificial Sequence

<220>

<223> Description of Artificial Sequence: DNA sequence
      iMab D1200

<220>
<221> misc_feature
<222> (1)..(330)

<400> 91
catatgcacg gtctgccgat ggaaaaacgt ggtaacttca tcgttggtca gaactgctcc 60
ctgacctgcc cggcttccgg ttacaccatc ggtccgcagt gcgttttcaa ctgctacttc 120
aactccgctc tggctttctc caccgaaaac atgggtgaat ggaccctgga catggttttc 180
tccgacgctg gtatctacac catgtgcgct gctgactcca ccatctacgc ttcctactac 240
gaatgcggtc acggtatctc caccggtggt tacggttaca acccggtttc cctgggttcc 300
ttcgttgttg actccccggc cagctcggcc                                  330


<210> 92
<211> 414
<212> DNA
<213> Artificial Sequence

<220>
<223> Description of Artificial Sequence: DNA sequence
      iMab D1202

<220>
<221> misc_feature
<222> (1)..(414)

<400> 92
catatgatcg ttaaactggt tatggaaaaa cgtggtaact tcgaaaacgg tcaggactgc 60
aaactgacca tccgtgcttc cggttacacc atcggtccgg cttgcgacgg tttcttctgc 120
cagttcccgt ccgacgactc cttctccacc gaagacaaca tgggtggtgg tatcaccgtt 180
aacgacgcta tgaaaccgca gttcgacatc cgtcgtgaca acgctaaagg cacctggacc 240
ctgtccatgg acttccagcc ggaaggtatc tacgaaatgc agtgcgctgc agactccacc 300
atctacgctt cctactacga atgcggtcac ggtctgtcca ccggtggtta cggttacgac 360
aacccggttc gtctgggtgg tttcgacgtt gacgtctcgt cggccagctc ggcc       414


<210> 93
<211> 321
<212> DNA
<213> Artificial Sequence

<220>
<223> Description of Artificial Sequence: DNA sequence
      iMab D1300

<220>
<221> misc_feature
<222> (1)..(321)

<400> 93
catatgctgc aggttgacat caaaccgtcc cagggtgaaa tctccgttgg tgaatccaaa 60
ttcttcctgt gccaggcttc cggttacacc atcggtccgt gcatctcctg gttctccccg 120
aacggtgaaa aactgaacat gggttcctcc accctgacca tctacaacgc taacatcgac 180
gacgctggta tctacaaatg cgctgctgac tccaccatct acgcttccta ctacgaatgc 240

ggtcacggta tctccaccgg tggttacggt taccagtccg aagctaccgt taacgttaaa 300
atcttccagg ccagctcggc c 321

<210> 94
<211> 276
<212> DNA
<213> Artificial Sequence

<220>
<223> Description of Artificial Sequence: DNA sequence
iMab D 1301

<220>
<221> misc_feature
<222> (1)..(276)

<400> 94
catatggaat ccaaattctt cctgtgccag gcttccggtt acaccatcgg tccgtgcatc 60
tcctggttct ccccgaacgg tgaaaaactg aacatgggtt cctccaccct gaccatctac 120
aacgctaaca tcgacgacgc tggtatctac aaatgcgctg ctgactccac catctacgct 180
tcctactacg aatgcggtca cggtatctcc accggtggtt acggttacca gtccgaagct 240
accgttaacg ttaaaatctt ccaggccagc tcggcc 276

<210> 95
<211> 423
<212> DNA
<213> Artificial Sequence

<220>
<223> Description of Artificial Sequence: DNA sequence
iMab D 1302

<220>
<221> misc_feature
<222> (1)..(423)

<400> 95
catatggttg ttaaagttgt tatcaaaccg tcccagaact tcatcgaaaa cggtgaagac 60
aaaaaattca cctgccgtgc ttccggttac accatcggtc cgaaatgcat cggttggttc 120
tcccagaacc cggaagacga ctccaccaac gttgctacca tcaacatggg tggtggtatc 180
acctactacg gtgactccgt taaagaacgt ttcgacatcc gtcgtgacaa cgctaaagac 240
acctccaccc tgtccatcga cgacgctcag ccggaagacg ctggtatcta caaatgcgct 300
gcagactcca ccatctacgc ttcctactac gaatgcggtc acggtctgtc caccggtggt 360
tacggttacg actccgaagc taccgttggt gttgacatct tcgtctcgtc ggccagctcg 420
gcc 423

<210> 96
<211> 330
<212> DNA
<213> Artificial Sequence

<220>
<223> Description of Artificial Sequence: DNA sequence
iMab D1400

<220>
<221> misc_feature
<222> (1)..(330)

<400> 96
catatggttc cgcgtgacct ggaagttgtt gctgctaccc cgacctccct gctgatctcc 60
tgcgacgctt ccggttacac catcggtccg tactgcatca cctacggtga aaccggtggt 120
aactccccgg ttcaggaatt caccgttccg aacatgggta aatccaccgc taccatctcc 180
ggtctgaaac cgggtgttga ctacaccatc acctgcgctg ctgactccac catctacgct 240
tcctactacg aatgcggtca cggtatctcc accggtggtt acggttactc caaaccgatc 300
tccatcaact accgtacggc cagctcggcc 330


<210> 97
<211> 348
<212> DNA
<213> Artificial Sequence

<220>
<223> Description of Artificial Sequence: DNA sequence
      iMab D1500

<220>
<221> misc_feature
<222> (1)..(348)

<400> 97
catatgatca aagtttacac cgaccgtgaa aactacggtg ctgttggttc ccaggttacc 60
ctgcactgct ccgcttccgg ttacaccatc ggtccgatct gcttcacctg gcgttaccag 120
ccggaaggtg accgtgacgc tatctccatc ttccactaca acatgggtga cggttccatc 180
gttatccaca acctggacta ctccgacaac ggtaccttca cctgcgctgc tgactccacc 240
atctacgctt cctactacga atgcggtcac ggtatctcca ccggtggtta cggttacgtt 300
ggtaaaacct cccaggttac cctgtacgtt ttcgaggcca gctcggcc 348


<210> 98
<211> 306
<212> DNA
<213> Artificial Sequence

<220>
<223> Description of Artificial Sequence: DNA sequence
      iMab D1501

<220>
<221> misc_feature
<222> (1)..(306)

<400> 98
catatgtccc aggttaccct gcactgctcc gcttccggtt acaccatcgg tccgatctgc 60
ttcacctggc gttaccagcc ggaaggtgac cgtgacgcta tctccatctt ccactacaac 120
atgggtgacg gttccatcgt tatccacaac ctggactact ccgacaacgg taccttcacc 180
tgcgctgctg actccaccat ctacgcttcc tactacgaat gcggtcacgg tatctccacc 240
ggtggttacg gttacgttgg taaaacctcc caggttaccc tgtacgtttt cgaggccagc 300
tcggcc 306

<210> 99
<211> 423
<212> DNA
<213> Artificial Sequence

<220>
<223> Description of Artificial Sequence: DNA sequence
      iMab D1502

<220>
<221> misc_feature
<222> (1)..(423)

<400> 99
catatgaacg ttaaagtggt taccaaacgt gaaaacttcg gtgaaaacgg ttccgacgtt 60
aaactgacct gccgtgcttc cggttacacc atcggtccga tctgcttcgg ttggttctac 120
cagccggaag gtgacgactc cgctatctcc atcttccaca acatgggtgg tggtatcacc 180
gacgaagttg acaccttcaa agaacgtttc gacatccgtc gtgacaacgc taaaaaaacc 240
ggcaccatct ccatcgacga cctgcaaccg tccgacaacg aaaccttcac ctgcgctgca 300
gactccacca tctacgcttc ctactacgaa tgcggtcacg gtctgtccac cggtggttac 360
ggttacgacg gtaaaacccg tcaggttggt ctggacgttt tcgtctcgtc ggccagctcg 420
gcc                                                               423


<210> 100
<211> 348
<212> DNA
<213> Artificial Sequence

<220>
<223> Description of Artificial Sequence: DNA sequence
      iMab D1600

<220>
<221> misc_feature
<222> (1)..(348)

<400> 100
catatgatca aagtttacac cgaccgtgaa aactacggtg ctgttggttc ccaggttacc 60
ctgcactgct ccgcttccgg ttacaccatc ggtccgatct gcttcacctg gcgttaccag 120
ccggaaggtg accgtgacgc tatctccatc ttccactaca acatgggtga cggttccatc 180
gttatccaca acctggacta ctccgacaac ggtaccttca cctgcgctgc tgactccacc 240
atctacgctt cctactacga atgcggtcac ggtatctcca ccggtggtta cggttacgtt 300
ggtaaaacct cccaggttac cctgtacgtt ttcgaggcca gctcggcc              348


<210> 101
<211> 426
<212> DNA
<213> Artificial Sequence

<220>
<223> Description of Artificial Sequence: DNA sequence
      iMab D1602

<220>

<221> misc_feature
<222> (1)..(426)

<400> 101
catatggctg ttaaaccggt tatcggttcc aaagctccga acttcggtga aaacggtgac 60
gttaaaacca tcgaccgtgc ttccggttac accatcggtc cgacctgcgg tggtgttttc 120
ttccagggtc cgaccgacga ctccaccaac gttgctacca tcaacatggg tggtggtatc 180
acctactacg gtgactccgt taaagaaacc ttcgacatcc gtcgtgacaa cgctaaatcc 240
acccgtaccg aatcctacga cgacaaccag ccggaaggtc tgaccgaagt taaatgcgct 300
gcagactcca ccatctacgc ttcctactac gaatgcggtc acggtctgtc caccggtggt 360
tacggttacg acgtttcctc ccgtctgtac ggttacgaca tcctggtctc gtcggccagc 420
tcggcc 426

<210> 102
<211> 333
<212> DNA
<213> Artificial Sequence

<220>
<223> Description of Artificial Sequence: DNA sequence
      iMab D1700

<220>
<221> misc_feature
<222> (1)..(333)

<400> 102
catatgaaag acccggaaat ccacctgtcc ggtccgctgg aagctggtaa accgatcacc 60
gttaaatgct ccgcttccgg ttacaccatc ggtccgctgt gcatcgacct gctgaaaggt 120
gaccacctga tgaaatccca ggaattcaac atgggttccc tggaagttac cttcaccccg 180
gttatcgaag acatcggtaa agttctggtt tgcgctgctg actccaccat ctacgcttcc 240
tactacgaat gcggtcacgg tatctccacc ggtggttacg gttacgttcg tcaggctgtt 300
aaagaactgc aggttgactc ggccagctcg gcc 333

<210> 103
<211> 291
<212> DNA
<213> Artificial Sequence

<220>
<223> Description of Artificial Sequence: DNA sequence
      iMab D1701

<220>
<221> misc_feature
<222> (1)..(291)

<400> 103
catatgaaac cgatcaccgt taaatgctcc gcttccggtt acaccatcgg tccgctgtgc 60
atcgacctgc tgaaaggtga ccacctgatg aaatcccagg aattcaacat gggttccctg 120
gaagttacct tcaccccggt tatcgaagac atcggtaaag ttctggtttg cgctgctgac 180
tccaccatct acgcttccta ctacgaatgc ggtcacggta tctccaccgg tggttacggt 240
tacgttcgtc aggctgttaa agaactgcag gttgactcgg ccagctcggc c 291

<210> 104
<211> 19
<212> DNA
<213> Artificial Sequence

<220>
<223> Description of Artificial Sequence: primer Pr4

<220>
<221> misc_feature
<222> (1)..(19)

<400> 104
caggaaaaca gctatgacc                                                    19


<210> 105
<211> 18
<212> DNA
<213> Artificial Sequence

<220>
<223> Description of Artificial Sequence: primer Pr5

<220>
<221> misc_feature
<222> (1)..(18)

<400> 105
tgtaaaacga cggccagt                                                     18


<210> 106
<211> 22
<212> DNA
<213> Artificial Sequence

<220>
<223> Description of Artificial Sequence: primer Pr8

<220>
<221> misc_feature
<222> (1)..(22)

<400> 106
cctgaaacct gaggacacgg cc                                                22


<210> 107
<211> 21
<212> DNA
<213> Artificial Sequence

<220>
<223> Description of Artificial Sequence: primer Pr9

<220>

<221> misc_feature
<222> (1)..(21)

<400> 107
cagggtcccc ttgtgcccca g                                                21


<210> 108
<211> 23
<212> DNA
<213> Artificial Sequence

<220>
<223> Description of Artificial Sequence: primer Pr33

<220>
<221> misc_feature
<222> (1)..(23)

<400> 108
gctatgccat agcattttta tcc                                              23


<210> 109
<211> 20
<212> DNA
<213> Artificial Sequence

<220>
<223> Description of Artificial Sequence: primer Pr35

<220>
<221> misc_feature
<222> (1)..(20)

<400> 109
acagccaagc tggagaccgt                                                  20


<210> 110
<211> 27
<212> DNA
<213> Artificial Sequence

<220>
<223> Description of Artificial Sequence: primer Pr49

<220>
<221> misc_feature
<222> (1)..(27)

<400> 110
ggtgacctgg gtacccttgt gccccgg                                          27


<210> 111
<211> 22

<212> DNA
<213> Artificial Sequence

<220>
<223> Description of Artificial Sequence: primer Pr56

<220>
<221> misc_feature
<222> (1)..(22)

<400> 111
ggagcgctga gggggtctca tg                                                22


<210> 112
<211> 24
<212> DNA
<213> Artificial Sequence

<220>
<223> Description of Artificial Sequence: primer Pr73

<220>
<221> misc_feature
<222> (1)..(24)

<400> 112
gaggacactg ccgtatatta cttg                                              24


<210> 113
<211> 24
<212> DNA
<213> Artificial Sequence

<220>
<223> Description of Artificial Sequence: primer Pr75

<220>
<221> misc_feature
<222> (1)..(24)

<400> 113
gaggacactg cagaatataa cttg                                              24


<210> 114
<211> 22
<212> DNA
<213> Artificial Sequence

<220>
<223> Description of Artificial Sequence: primer Pr76

<220>
<221> misc_feature
<222> (1)..(22)

<400> 114
ccagggaagg cagcgctgag tt                                                22


<210> 115
<211> 46
<212> DNA
<213> Artificial Sequence

<220>
<223> Description of Artificial Sequence: primer Pr80

<220>
<221> misc_feature
<222> (1)..(46)

<400> 115
gatgacgatc ttaagctcac gnnncgtgct gaaggttaca ccattg                      46


<210> 116
<211> 47
<212> DNA
<213> Artificial Sequence

<220>
<223> Description of Artificial Sequence: primer Pr81

<220>
<221> misc_feature
<222> (1)..(47)

<400> 116
cgtaaatggt agaatcacct gcnnnattgt attctgcaga gtcttcc                     47


<210> 117
<211> 40
<212> DNA
<213> Artificial Sequence

<220>
<223> Description of Artificial Sequence: primer Pr82

<220>
<221> misc_feature
<222> (1)..(40)

<400> 117
ccgcaatgtg aaactggttt gtaaaggtgg caatttcgtc                             40


<210> 118
<211> 41
<212> DNA
<213> Artificial Sequence

<220>
<223> Description of Artificial Sequence: primer Pr83

<220>
<221> misc_feature
<222> (1)..(41)

<400> 118
cggtaacgtc ggtaccctgg caacggtagt ggctatcgta g                41


<210> 119
<211> 30
<212> DNA
<213> Artificial Sequence

<220>
<223> Description of Artificial Sequence: primer Pr120

<220>
<221> misc_feature
<222> (1)..(30)

<400> 119
aggcgggcgg ccgcaatgtg aaactggttg                30


<210> 120
<211> 30
<212> DNA
<213> Artificial Sequence

<220>
<223> Description of Artificial Sequence: primer Pr121

<220>
<221> misc_feature
<222> (1)..(30)

<400> 120
caccggccga gctggccgac gagacggtaa                30


<210> 121
<211> 32
<212> DNA
<213> Artificial Sequence

<220>
<223> Description of Artificial Sequence: primer Pr129

<220>
<221> misc_feature
<222> (1)..(32)

<400> 121

tatacatatg aatgtgaaac tggttgaaaa ag                                    32


<210> 122
<211> 46
<212> DNA
<213> Artificial Sequence

<220>
<223> Description of Artificial Sequence: primer Pr136

<220>
<221> misc_feature
<222> (1)..(46)

<400> 122
cttcgatatc cgtcgcgacg atgcgtccaa caccgttacc ttatcg                    46


<210> 123
<211> 24
<212> DNA
<213> Artificial Sequence

<220>
<223> Description of Artificial Sequence: primer Pr299

<220>
<221> misc_feature
<222> (1)..(24)

<400> 123
gaggacacgg ccacatacta ctgt                                            24


<210> 124
<211> 24
<212> DNA
<213> Artificial Sequence

<220>
<223> Description of Artificial Sequence: primer Pr300

<220>
<221> misc_feature
<222> (1)..(24)

<400> 124
gaccaggagt ccttggcccc aggc                                            24


<210> 125
<211> 21
<212> DNA
<213> Artificial Sequence

<220>

<223> Description of Artificial Sequence: primer Pr301

<220>
<221> misc_feature
<222> (1)..(21)

<400> 125
gaccaggagt ccttggcccc a                                                21


<210> 126
<211> 25
<212> DNA
<213> Artificial Sequence

<220>
<223> Description of Artificial Sequence: primer Pr302

<220>
<221> misc_feature
<222> (1)..(25)

<400> 126
gttgtggttt tggtgtcttg ggttc                                            25


<210> 127
<211> 27
<212> DNA
<213> Artificial Sequence

<220>
<223> Description of Artificial Sequence: primer Pr303

<220>
<221> misc_feature
<222> (1)..(27)

<400> 127
cttggattct gttgtaggat tgggttg                                          27


<210> 128
<211> 19
<212> DNA
<213> Artificial Sequence

<220>
<223> Description of Artificial Sequence: primer Pr304

<220>
<221> misc_feature
<222> (1)..(19)

<400> 128
ggggtcttcg ctgtggtgc                                                   19

<210> 129
<211> 20
<212> DNA
<213> Artificial Sequence

<220>
<223> Description of Artificial Sequence: primer Pr305

<220>
<221> misc_feature
<222> (1)..(20)

<400> 129
cttggagctg ggtcttcgc                                                    20


<210> 130
<211> 99
<212> DNA
<213> Artificial Sequence

<220>
<223> Description of Artificial Sequence: primer Pr306

<220>
<221> misc_feature
<222> (1)..(99)

<400> 130
ccggatcctt agtggtgatg gtgatggtgg cttttgccca ggcggttcat ttctatatcg 60
gtatagctgc caccgccacc ggccgagctg gccgacgag                          99


<210> 131
<211> 110
<212> PRT
<213> Artificial Sequence

<220>
<223> Description of Artificial Sequence: IMABIS003

<220>
<221> SITE
<222> (1)..(110)

<400> 131
Ile Val Leu Thr Gln Ser Pro Ala Ser Leu Ala Val Ser Leu Gly Gln
  1               5                  10                  15

Arg Ala Thr Ile Ser Cys Arg Ala Ser Gly Tyr Thr Ile Gly Pro Ser
             20                  25                  30

Phe Met Asn Trp Phe Gln Gln Lys Pro Gly Gln Pro Pro Lys Leu Leu
         35                  40                  45

Ile Tyr Ala Asn Met Gly Asp Phe Ser Leu Asn Ile His Pro Met Glu

50                  55                  60

Glu Glu Asp Thr Ala Met Tyr Phe Cys Ala Ala Asp Ser Thr Ile Tyr
65                  70                  75                  80

Ala Ser Tyr Tyr Glu Cys Gly His Gly Ile Ser Thr Gly Gly Tyr Gly
                85                  90                  95

Tyr Leu Thr Phe Gly Ala Gly Thr Lys Val Glu Leu Lys Arg
            100                 105                 110


<210> 132
<211> 98
<212> PRT
<213> Artificial Sequence

<220>
<223> Description of Artificial Sequence: IMABIS004

<220>
<221> SITE
<222> (1)..(98)

<400> 132
Pro Thr Val Ser Ile Phe Pro Pro Ser Ser Glu Gln Leu Thr Ser Gly
1               5                   10                  15

Gly Ala Ser Val Val Cys Phe Ala Ser Gly Tyr Thr Ile Gly Pro Ile
            20                  25                  30

Asn Val Lys Trp Lys Ile Asp Gly Ser Glu Asn Met Gly Ser Ser Thr
        35                  40                  45

Leu Thr Leu Thr Lys Asp Glu Tyr Glu Arg His Asn Ser Tyr Thr Cys
    50                  55                  60

Ala Ala Asp Ser Thr Ile Tyr Ala Ser Tyr Tyr Glu Cys Gly His Gly
65                  70                  75                  80

Ile Ser Thr Gly Gly Tyr Gly Tyr Pro Ile Val Lys Ser Phe Asn Arg
                85                  90                  95

Asn Glu


<210> 133
<211> 115
<212> PRT
<213> Artificial Sequence

<220>
<223> Description of Artificial Sequence: IMABIS006

<220>
<221> SITE

<222> (1)..(115)

<400> 133
Thr Pro Pro Ser Val Tyr Pro Leu Ala Pro Gly Ser Ala Ala Gln Thr
1               5                   10                  15

Asn Ser Met Val Thr Leu Gly Cys Leu Val Lys Ala Ser Gly Tyr Thr
            20                  25                  30

Ile Gly Pro Glu Pro Val Thr Val Thr Trp Asn Ser Gly Ser Leu Ser
        35                  40                  45

Ser Gly Val His Thr Phe Pro Asn Met Gly Thr Leu Ser Ser Ser Val
    50                  55                  60

Thr Val Pro Ser Ser Thr Trp Pro Ser Glu Thr Val Thr Cys Asn Cys
65                  70                  75                  80

Ala Ala Asp Ser Thr Ile Tyr Ala Ser Tyr Tyr Glu Cys Gly His Gly
                85                  90                  95

Ile Ser Thr Gly Gly Tyr Gly Tyr Ser Thr Lys Val Asp Lys Lys Ile
            100                 105                 110

Val Pro Lys
        115


<210> 134
<211> 106
<212> PRT
<213> Artificial Sequence

<220>
<223> Description of Artificial Sequence: IMABIS007

<220>
<221> SITE
<222> (1)..(106)

<400> 134
Ile Ala Ser Pro Ala Lys Thr His Glu Lys Thr Pro Ile Glu Gly Arg
1               5                   10                  15

Pro Phe Gln Leu Asp Cys Val Ala Ser Gly Tyr Thr Ile Gly Pro Leu
            20                  25                  30

Ile Thr Trp Lys Lys Arg Leu Ser Gly Ala Asp Pro Asn Asn Met Gly
        35                  40                  45

Gly Asn Leu Tyr Phe Thr Ile Val Thr Lys Glu Asp Val Ser Asp Ile
    50                  55                  60

Tyr Lys Tyr Val Cys Ala Ala Asp Ser Thr Ile Tyr Ala Ser Tyr Tyr
65                  70                  75                  80

Glu Cys Gly His Gly Ile Ser Thr Gly Gly Tyr Gly Tyr Glu Val Val

85                  90                  95

Leu Val Glu Tyr Glu Ile Lys Gly Val Thr
            100                 105


<210> 135
<211> 113
<212> PRT
<213> Artificial Sequence

<220>
<223> Description of Artificial Sequence: IMABIS008

<220>
<221> SITE
<222> (1)..(113)

<400> 135
Pro Val Leu Lys Asp Gln Pro Ala Glu Val Leu Phe Arg Glu Asn Asn
 1               5                  10                  15

Pro Thr Val Leu Glu Cys Ile Ala Ser Gly Tyr Thr Ile Gly Pro Val
            20                  25                  30

Lys Tyr Ser Trp Lys Lys Asp Gly Lys Ser Tyr Asn Trp Gln Glu His
        35                  40                  45

Asn Ala Ala Leu Arg Lys Asn Met Gly Glu Gly Ser Leu Val Phe Leu
    50                  55                  60

Arg Pro Gln Ala Ser Asp Glu Gly His Tyr Gln Cys Ala Ala Asp Ser
 65                 70                  75                  80

Thr Ile Tyr Ala Ser Tyr Tyr Glu Cys Gly His Gly Ile Ser Thr Gly
                85                  90                  95

Gly Tyr Gly Tyr Val Ala Ser Ser Arg Val Ile Ser Phe Arg Lys Thr
            100                 105                 110

Tyr



<210> 136
<211> 99
<212> PRT
<213> Artificial Sequence

<220>
<223> Description of Artificial Sequence: IMABIS009

<220>
<221> SITE
<222> (1)..(99)

<400> 136

Lys Tyr Glu Gln Lys Pro Glu Lys Val Ile Val Val Lys Gln Gly Gln
1               5                   10                  15

Asp Val Thr Ile Pro Cys Lys Ala Ser Gly Tyr Thr Ile Gly Pro Pro
            20                  25                  30

Asn Val Val Trp Ser His Asn Ala Lys Pro Asn Met Gly Asp Ser Gly
        35                  40                  45

Leu Val Ile Lys Gly Val Lys Asn Gly Asp Lys Gly Tyr Tyr Gly Cys
    50                  55                  60

Ala Ala Asp Ser Thr Ile Tyr Ala Ser Tyr Tyr Glu Cys Gly His Gly
65                  70                  75                  80

Ile Ser Thr Gly Gly Tyr Gly Tyr Asp Lys Tyr Phe Glu Thr Leu Val
                85                  90                  95

Gln Val Asn

<210> 137
<211> 101
<212> PRT
<213> Artificial Sequence

<220>
<223> Description of Artificial Sequence: IMABIS010

<220>
<221> SITE
<222> (1)..(101)

<400> 137
Val Pro Gln Tyr Val Ser Lys Asp Met Met Ala Lys Ala Gly Asp Val
1               5                   10                  15

Thr Met Ile Tyr Cys Met Ala Ser Gly Tyr Thr Ile Gly Pro Gly Tyr
            20                  25                  30

Pro Asn Tyr Phe Lys Asn Gly Lys Asp Val Asn Asn Met Gly Gly Lys
        35                  40                  45

Arg Leu Leu Phe Lys Thr Thr Leu Pro Glu Asp Glu Gly Val Tyr Thr
    50                  55                  60

Cys Ala Ala Asp Ser Thr Ile Tyr Ala Ser Tyr Tyr Glu Cys Gly His
65                  70                  75                  80

Gly Ile Ser Thr Gly Gly Tyr Gly Tyr Pro Gln Lys His Ser Leu Lys
                85                  90                  95

Leu Thr Val Val Ser
            100

<210> 138
<211> 110
<212> PRT
<213> Artificial Sequence

<220>
<223> Description of Artificial Sequence: IMABIS012

<220>
<221> SITE
<222> (1)..(110)

<400> 138
Ile Gln Met Thr Gln Ser Pro Ser Ser Leu Ser Ala Ser Val Gly Asp
1               5                   10                  15

Arg Val Thr Ile Thr Cys Ser Ala Ser Gly Tyr Thr Ile Gly Pro Asn
            20                  25                  30

Tyr Leu Asn Trp Tyr Gln Gln Lys Pro Gly Lys Ala Pro Lys Val Leu
        35                  40                  45

Ile Tyr Phe Asn Met Gly Asp Phe Thr Leu Thr Ile Ser Ser Leu Gln
    50                  55                  60

Pro Glu Asp Phe Ala Thr Tyr Tyr Cys Ala Ala Asp Ser Thr Ile Tyr
65                  70                  75                  80

Ala Ser Tyr Tyr Glu Cys Gly His Gly Ile Ser Thr Gly Gly Tyr Gly
                85                  90                  95

Tyr Trp Thr Phe Gly Gln Gly Thr Lys Val Glu Ile Lys Arg
            100                 105                 110


<210> 139
<211> 101
<212> PRT
<213> Artificial Sequence

<220>
<223> Description of Artificial Sequence: IMABIS013

<220>
<221> SITE
<222> (1)..(101)

<400> 139
Pro Ser Val Phe Ile Phe Pro Pro Ser Asp Glu Gln Leu Lys Ser Gly
1               5                   10                  15

Thr Ala Ser Val Val Cys Leu Ala Ser Gly Tyr Thr Ile Gly Pro Ala
            20                  25                  30

Lys Val Gln Trp Lys Val Asp Asn Ala Leu Gln Ser Asn Met Gly Ser
        35                  40                  45

Ser Thr Leu Thr Leu Ser Lys Ala Asp Tyr Glu Lys His Lys Val Tyr
    50                  55                  60

Ala Cys Ala Ala Asp Ser Thr Ile Tyr Ala Ser Tyr Tyr Glu Cys Gly
65                  70                  75                  80

His Gly Ile Ser Thr Gly Gly Tyr Gly Tyr Pro Val Thr Lys Ser Phe
                85                  90                  95

Asn Arg Gly Glu Cys
            100


<210> 140
<211> 116
<212> PRT
<213> Artificial Sequence

<220>
<223> Description of Artificial Sequence: IMABIS014

<220>
<221> SITE
<222> (1)..(116)

<400> 140
Lys Gly Pro Ser Val Phe Pro Leu Ala Pro Ser Ser Lys Ser Thr Ser
1               5                   10                  15

Gly Gly Thr Ala Ala Leu Gly Cys Leu Val Lys Ala Ser Gly Tyr Thr
            20                  25                  30

Ile Gly Pro Glu Pro Val Thr Val Ser Trp Asn Ser Gly Ala Leu Thr
        35                  40                  45

Ser Gly Val His Thr Phe Pro Asn Met Gly Ser Leu Ser Ser Val Val
    50                  55                  60

Thr Val Pro Ser Ser Ser Leu Gly Thr Gln Thr Tyr Ile Cys Asn Cys
65                  70                  75                  80

Ala Ala Asp Ser Thr Ile Tyr Ala Ser Tyr Tyr Glu Cys Gly His Gly
                85                  90                  95

Ile Ser Thr Gly Gly Tyr Gly Tyr Asn Thr Lys Val Asp Lys Lys Val
            100                 105                 110

Glu Pro Lys Ser
        115


<210> 141
<211> 110
<212> PRT
<213> Artificial Sequence

<220>

<223> Description of Artificial Sequence: IMABIS015

<220>
<221> SITE
<222> (1)..(110)

<400> 141
Asn Pro Pro His Asn Leu Ser Val Ile Asn Ser Glu Glu Leu Ser Ser
1               5                   10                  15

Ile Leu Lys Leu Thr Trp Thr Ala Ser Gly Tyr Thr Ile Gly Pro Leu
            20                  25                  30

Lys Tyr Asn Ile Gln Tyr Arg Thr Lys Asp Ala Ser Thr Trp Ser Gln
        35                  40                  45

Ile Pro Pro Asn Met Gly Arg Ser Ser Phe Thr Val Gln Asp Leu Lys
    50                  55                  60

Pro Phe Thr Glu Tyr Val Phe Arg Cys Ala Ala Asp Ser Thr Ile Tyr
65                  70                  75                  80

Ala Ser Tyr Tyr Glu Cys Gly His Gly Ile Ser Thr Gly Gly Tyr Gly
                85                  90                  95

Tyr Ser Asp Trp Ser Glu Glu Ala Ser Gly Ile Thr Tyr Glu
            100                 105                 110


<210> 142
<211> 108
<212> PRT
<213> Artificial Sequence

<220>
<223> Description of Artificial Sequence: IMABIS016

<220>
<221> SITE
<222> (1)..(108)

<400> 142
Glu Lys Pro Lys Asn Leu Ser Cys Ile Val Asn Glu Gly Lys Lys Met
1               5                   10                  15

Arg Cys Glu Trp Asp Ala Ser Gly Tyr Thr Ile Gly Pro Thr Asn Phe
            20                  25                  30

Thr Leu Lys Ser Glu Trp Ala Thr His Lys Phe Ala Asp Cys Lys Ala
        35                  40                  45

Asn Met Gly Pro Thr Ser Cys Thr Val Asp Tyr Ser Thr Val Tyr Phe
    50                  55                  60

Val Asn Ile Glu Val Trp Cys Ala Ala Asp Ser Thr Ile Tyr Ala Ser
65                  70                  75                  80

Tyr Tyr Glu Cys Gly His Gly Ile Ser Thr Gly Gly Tyr Gly Tyr Lys
                85                  90                  95

Val Thr Ser Asp His Ile Asn Phe Asp Pro Val Tyr
            100                 105


<210> 143
<211> 106
<212> PRT
<213> Artificial Sequence

<220>
<223> Description of Artificial Sequence: IMABIS018

<220>
<221> SITE
<222> (1)..(106)

<400> 143
Asn Ala Pro Lys Leu Thr Gly Ile Thr Cys Gln Ala Asp Lys Ala Glu
  1               5                  10                  15

Ile His Trp Glu Ala Ser Gly Tyr Thr Ile Gly Pro Leu His Tyr Thr
             20                  25                  30

Ile Gln Phe Asn Thr Ser Phe Thr Pro Ala Ser Trp Asp Ala Ala Tyr
         35                  40                  45

Glu Lys Asn Met Gly Asp Ser Ser Phe Val Val Gln Met Ser Pro Trp
     50                  55                  60

Ala Asn Tyr Thr Phe Arg Cys Ala Ala Asp Ser Thr Ile Tyr Ala Ser
 65                  70                  75                  80

Tyr Tyr Glu Cys Gly His Gly Ile Ser Thr Gly Gly Tyr Gly Tyr Ser
                85                  90                  95

Pro Pro Ser Ala His Ser Asp Ser Cys Thr
            100                 105


<210> 144
<211> 107
<212> PRT
<213> Artificial Sequence

<220>
<223> Description of Artificial Sequence: IMABIS019

<220>
<221> SITE
<222> (1)..(107)

<400> 144
Gly Pro Glu Glu Leu Leu Cys Phe Thr Glu Arg Leu Glu Asp Leu Val
  1               5                  10                  15

Cys Phe Trp Glu Ala Ser Gly Tyr Thr Ile Gly Pro Gly Gln Tyr Ser
            20                  25                  30

Phe Ser Tyr Gln Leu Glu Asp Glu Pro Trp Lys Leu Cys Arg Asn Met
        35                  40                  45

Gly Arg Phe Trp Cys Ser Leu Pro Thr Ala Asp Thr Ser Ser Phe Val
    50                  55                  60

Pro Leu Glu Leu Arg Cys Ala Ala Asp Ser Thr Ile Tyr Ala Ser Tyr
65                  70                  75                  80

Tyr Glu Cys Gly His Gly Ile Ser Thr Gly Gly Tyr Gly Tyr Gly Ala
                85                  90                  95

Pro Arg Tyr His Arg Val Ile His Ile Asn Glu
            100                 105


<210> 145
<211> 109
<212> PRT
<213> Artificial Sequence

<220>
<223> Description of Artificial Sequence: IMABIS020

<220>
<221> SITE
<222> (1)..(109)

<400> 145
Ala Pro Val Gly Leu Val Ala Arg Leu Ala Asp Glu Ser Gly His Val
  1               5                  10                  15

Val Leu Arg Trp Leu Ala Ser Gly Tyr Thr Ile Gly Pro Ile Arg Tyr
            20                  25                  30

Glu Val Asp Val Ser Ala Gly Gln Gly Ala Gly Ser Val Gln Arg Val
        35                  40                  45

Glu Ile Asn Met Gly Arg Thr Glu Cys Val Leu Ser Asn Leu Arg Gly
    50                  55                  60

Arg Thr Arg Tyr Thr Phe Ala Cys Ala Ala Asp Ser Thr Ile Tyr Ala
65                  70                  75                  80

Ser Tyr Tyr Glu Cys Gly His Gly Ile Ser Thr Gly Gly Tyr Gly Tyr
                85                  90                  95

Ser Glu Trp Ser Glu Pro Val Ser Leu Leu Thr Pro Ser
            100                 105


<210> 146
<211> 108

<212> PRT
<213> Artificial Sequence

<220>
<223> Description of Artificial Sequence: IMABIS025

<220>
<221> SITE
<222> (1)..(108)

<400> 146
Gly Pro Glu Glu Leu Leu Cys Phe Thr Glu Arg Leu Glu Asp Leu Val
1               5                   10                  15

Cys Phe Trp Glu Ala Ser Gly Tyr Thr Ile Gly Pro Pro Gly Asn Tyr
            20                  25                  30

Ser Phe Ser Tyr Gln Leu Glu Asp Glu Pro Trp Lys Leu Cys Arg Asn
        35                  40                  45

Met Gly Arg Phe Trp Cys Ser Leu Pro Thr Ala Asp Thr Ser Ser Phe
    50                  55                  60

Val Pro Leu Glu Leu Arg Cys Ala Ala Asp Ser Thr Ile Tyr Ala Ser
65                  70                  75                  80

Tyr Tyr Glu Cys Gly His Gly Ile Ser Thr Gly Gly Tyr Gly Tyr Gly
                85                  90                  95

Ala Pro Arg Tyr His Arg Val Ile His Ile Asn Glu
            100                 105


<210> 147
<211> 109
<212> PRT
<213> Artificial Sequence

<220>
<223> Description of Artificial Sequence: IMABIS027

<220>
<221> SITE
<222> (1)..(109)

<400> 147
Ala Pro Val Gly Leu Val Ala Arg Leu Ala Asp Glu Ser Gly His Val
1               5                   10                  15

Val Leu Arg Trp Leu Ala Ser Gly Tyr Thr Ile Gly Pro Ile Arg Tyr
            20                  25                  30

Glu Val Asp Val Ser Ala Gly Gln Gly Ala Gly Ser Val Gln Arg Val
        35                  40                  45

Glu Ile Leu Asn Met Gly Thr Glu Cys Val Leu Ser Asn Leu Arg Gly
    50                  55                  60

Arg Thr Arg Tyr Thr Phe Ala Cys Ala Ala Asp Ser Thr Ile Tyr Ala
65                  70                  75                  80

Ser Tyr Tyr Glu Cys Gly His Gly Ile Ser Thr Gly Gly Tyr Gly Tyr
                85                  90                  95

Ser Glu Trp Ser Glu Pro Val Ser Leu Leu Thr Pro Ser
            100                 105


<210> 148
<211> 107
<212> PRT
<213> Artificial Sequence

<220>
<223> Description of Artificial Sequence: IMABIS028

<220>
<221> SITE
<222> (1)..(107)

<400> 148
Gly Pro Glu Glu Leu Leu Cys Phe Thr Glu Arg Leu Glu Asp Leu Val
 1               5                  10                  15

Cys Phe Trp Glu Ala Ser Gly Tyr Thr Ile Gly Pro Gly Gln Tyr Ser
            20                  25                  30

Phe Ser Tyr Gln Leu Glu Asp Glu Pro Trp Lys Leu Cys Arg Asn Met
        35                  40                  45

Gly Arg Phe Trp Cys Ser Leu Pro Thr Ala Asp Thr Ser Ser Phe Val
    50                  55                  60

Pro Leu Glu Leu Arg Cys Ala Ala Asp Ser Thr Ile Tyr Ala Ser Tyr
65                  70                  75                  80

Tyr Glu Cys Gly His Gly Ile Ser Thr Gly Gly Tyr Gly Tyr Gly Ala
                85                  90                  95

Pro Arg Tyr His Arg Val Ile His Ile Asn Glu
            100                 105


<210> 149
<211> 99
<212> PRT
<213> Artificial Sequence

<220>
<223> Description of Artificial Sequence: IMABIS031

<220>
<221> SITE
<222> (1)..(99)

<400> 149
Leu Met Phe Lys Asn Ala Pro Thr Pro Gln Glu Phe Lys Glu Gly Glu
1               5                   10                  15

Asp Ala Val Ile Val Cys Asp Ala Ser Gly Tyr Thr Ile Gly Pro Pro
            20                  25                  30

Thr Ile Ile Trp Lys His Lys Gly Arg Asp Val Asn Met Gly Asn Asn
        35                  40                  45

Tyr Leu Gln Ile Arg Gly Ile Lys Lys Thr Asp Glu Gly Thr Tyr Arg
    50                  55                  60

Cys Ala Ala Asp Ser Thr Ile Tyr Ala Ser Tyr Tyr Glu Cys Gly His
65                  70                  75                  80

Gly Ile Ser Thr Gly Gly Tyr Gly Tyr Ile Asn Phe Lys Asp Ile Gln
                85                  90                  95

Val Ile Val



<210> 150
<211> 108
<212> PRT
<213> Artificial Sequence

<220>
<223> Description of Artificial Sequence: IMABIS032

<220>
<221> SITE
<222> (1)..(108)

<400> 150
Asp Ser Pro Thr Gly Ile Asp Phe Ser Asp Ile Thr Ala Asn Ser Phe
1               5                   10                  15

Thr Val His Trp Ile Ala Ser Gly Tyr Thr Ile Gly Pro Thr Gly Tyr
            20                  25                  30

Arg Ile Arg His His Pro Glu His Phe Ser Gly Arg Pro Arg Glu Asp
        35                  40                  45

Arg Val Asn Met Gly Arg Asn Ser Ile Thr Leu Thr Asn Leu Thr Pro
    50                  55                  60

Gly Thr Glu Tyr Val Val Ser Cys Ala Ala Asp Ser Thr Ile Tyr Ala
65                  70                  75                  80

Ser Tyr Tyr Glu Cys Gly His Gly Ile Ser Thr Gly Gly Tyr Gly Tyr
                85                  90                  95

Ser Pro Leu Leu Ile Gly Gln Gln Ser Thr Val Ser
            100                 105

<210> 151
<211> 110
<212> PRT
<213> Artificial Sequence

<220>
<223> Description of Artificial Sequence: IMABIS034

<220>
<221> SITE
<222> (1)..(110)

<400> 151
Ser Pro Pro Thr Asn Leu His Leu Glu Ala Asn Pro Asp Thr Gly Val
1               5                   10                  15

Leu Thr Val Ser Trp Glu Ala Ser Gly Tyr Thr Ile Gly Pro Thr Gly
            20                  25                  30

Tyr Arg Ile Thr Thr Thr Pro Thr Asn Gly Gln Gln Gly Asn Ser Leu
        35                  40                  45

Glu Glu Val Val Asn Met Gly Gln Ser Ser Cys Thr Phe Asp Asn Leu
    50                  55                  60

Ser Pro Gly Leu Glu Tyr Asn Val Ser Cys Ala Ala Asp Ser Thr Ile
65                  70                  75                  80

Tyr Ala Ser Tyr Tyr Glu Cys Gly His Gly Ile Ser Thr Gly Gly Tyr
                85                  90                  95

Gly Tyr Ser Val Pro Ile Ser Asp Thr Ile Ile Pro Ala Val
            100                 105                 110


<210> 152
<211> 107
<212> PRT
<213> Artificial Sequence

<220>
<223> Description of Artificial Sequence: IMABIS035

<220>
<221> SITE
<222> (1)..(107)

<400> 152
Pro Pro Thr Asp Leu Arg Phe Thr Asn Ile Gly Pro Asp Thr Met Arg
1               5                   10                  15

Val Thr Trp Ala Ala Ser Gly Tyr Thr Ile Gly Pro Thr Asn Phe Leu
            20                  25                  30

Val Arg Tyr Ser Pro Val Lys Asn Glu Glu Asp Val Ala Glu Leu Ser

35                  40                  45

Ile Asn Met Gly Asp Asn Ala Val Val Leu Thr Asn Leu Leu Pro Gly
    50                  55                  60

Thr Glu Tyr Val Val Ser Cys Ala Ala Asp Ser Thr Ile Tyr Ala Ser
65                  70                  75                  80

Tyr Tyr Glu Cys Gly His Gly Ile Ser Thr Gly Gly Tyr Gly Tyr Ser
                85                  90                  95

Thr Pro Leu Arg Gly Arg Gln Lys Thr Gly Leu
            100                 105


<210> 153
<211> 111
<212> PRT
<213> Artificial Sequence

<220>
<223> Description of Artificial Sequence: IMABIS036

<220>
<221> SITE
<222> (1)..(111)

<400> 153
Asn Pro Pro His Asn Leu Ser Val Ile Asn Ser Glu Glu Leu Ser Ser
1               5                   10                  15

Ile Leu Lys Leu Thr Trp Thr Ala Ser Gly Tyr Thr Ile Gly Pro Leu
            20                  25                  30

Lys Tyr Asn Ile Gln Tyr Arg Thr Lys Asp Ala Ser Thr Trp Ser Gln
        35                  40                  45

Ile Pro Pro Glu Asn Met Gly Arg Ser Ser Phe Thr Val Gln Asp Leu
    50                  55                  60

Lys Pro Phe Thr Glu Tyr Val Phe Arg Cys Ala Ala Asp Ser Thr Ile
65                  70                  75                  80

Tyr Ala Ser Tyr Tyr Glu Cys Gly His Gly Ile Ser Thr Gly Gly Tyr
                85                  90                  95

Gly Tyr Ser Asp Trp Ser Glu Glu Ala Ser Gly Ile Thr Tyr Glu
            100                 105                 110


<210> 154
<211> 103
<212> PRT
<213> Artificial Sequence

<220>
<223> Description of Artificial Sequence: IMABIS037

<220>
<221> SITE
<222> (1)..(103)

<400> 154
Pro Cys Gly Tyr Ile Ser Pro Glu Ser Pro Val Val Gln Leu His Ser
1               5                   10                  15

Asn Phe Thr Ala Val Cys Val Ala Ser Gly Tyr Thr Ile Gly Pro Asn
            20                  25                  30

Tyr Ile Val Trp Lys Thr Asn His Phe Thr Ile Pro Lys Asn Met Gly
        35                  40                  45

Ala Ser Ser Val Thr Phe Thr Asp Ile Ala Ser Leu Asn Ile Gln Leu
    50                  55                  60

Thr Cys Ala Ala Asp Ser Thr Ile Tyr Ala Ser Tyr Tyr Glu Cys Gly
65                  70                  75                  80

His Gly Ile Ser Thr Gly Gly Tyr Gly Tyr Glu Gln Asn Val Tyr Gly
                85                  90                  95

Ile Thr Ile Ile Ser Gly Leu
            100


<210> 155
<211> 108
<212> PRT
<213> Artificial Sequence

<220>
<223> Description of Artificial Sequence: IMABIS038

<220>
<221> SITE
<222> (1)..(108)

<400> 155
Glu Lys Pro Lys Asn Leu Ser Cys Ile Val Asn Glu Gly Lys Lys Met
1               5                   10                  15

Arg Cys Glu Trp Asp Ala Ser Gly Tyr Thr Ile Gly Pro Thr Asn Phe
            20                  25                  30

Thr Leu Lys Ser Glu Trp Ala Thr His Lys Phe Ala Asp Cys Lys Ala
        35                  40                  45

Asn Met Gly Pro Thr Ser Cys Thr Val Asp Tyr Ser Thr Val Tyr Phe
    50                  55                  60

Val Asn Ile Glu Val Trp Cys Ala Ala Asp Ser Thr Ile Tyr Ala Ser
65                  70                  75                  80

Tyr Tyr Glu Cys Gly His Gly Ile Ser Thr Gly Gly Tyr Gly Tyr Lys

                  85                  90                  95

Val Thr Ser Asp His Ile Asn Phe Asp Pro Val Tyr
            100                 105


<210> 156
<211> 102
<212> PRT
<213> Artificial Sequence

<220>
<223> Description of Artificial Sequence: IMABIS039

<220>
<221> SITE
<222> (1)..(102)

<400> 156
Arg Phe Ile Val Lys Pro Tyr Gly Thr Glu Val Gly Glu Gly Gln Ser
  1               5                  10                  15

Ala Asn Phe Tyr Cys Arg Ala Ser Gly Tyr Thr Ile Gly Pro Pro Val
             20                  25                  30

Val Thr Trp His Lys Asp Asp Arg Glu Leu Lys Asn Met Gly Asp Tyr
         35                  40                  45

Gly Leu Thr Ile Asn Arg Val Lys Gly Asp Asp Lys Gly Glu Tyr Thr
     50                  55                  60

Cys Ala Ala Asp Ser Thr Ile Tyr Ala Ser Tyr Tyr Glu Cys Gly His
 65                  70                  75                  80

Gly Ile Ser Thr Gly Gly Tyr Gly Tyr Gly Thr Lys Glu Glu Ile Val
                 85                  90                  95

Phe Leu Asn Val Thr Arg
            100


<210> 157
<211> 114
<212> PRT
<213> Artificial Sequence

<220>
<223> Description of Artificial Sequence: IMABIS041

<220>
<221> SITE
<222> (1)..(114)

<400> 157
Ser Glu Pro Gly Arg Leu Ala Phe Asn Val Val Ser Ser Thr Val Thr
  1               5                  10                  15

Gln Leu Ser Trp Ala Ala Ser Gly Tyr Thr Ile Gly Pro Thr Ala Tyr
            20                  25                  30

Glu Val Cys Tyr Gly Leu Val Asn Asp Asp Asn Arg Pro Ile Gly Pro
        35                  40                  45

Met Lys Lys Val Leu Val Asp Asn Met Gly Asn Arg Met Leu Leu Ile
    50                  55                  60

Glu Asn Leu Arg Glu Ser Gln Pro Tyr Arg Tyr Thr Cys Ala Ala Asp
65                  70                  75                  80

Ser Thr Ile Tyr Ala Ser Tyr Tyr Glu Cys Gly His Gly Ile Ser Thr
                85                  90                  95

Gly Gly Tyr Gly Tyr Trp Gly Pro Glu Arg Glu Ala Ile Ile Asn Leu
            100                 105                 110

Ala Thr

<210> 158
<211> 109
<212> PRT
<213> Artificial Sequence

<220>
<223> Description of Artificial Sequence: IMABIS042

<220>
<221> SITE
<222> (1)..(109)

<400> 158
Ala Pro Gln Asn Pro Asn Ala Lys Ala Ala Gly Ser Arg Lys Ile His
 1               5                  10                  15

Phe Asn Trp Leu Ala Ser Gly Tyr Thr Ile Gly Pro Met Gly Tyr Arg
            20                  25                  30

Val Lys Tyr Trp Ile Gln Gly Asp Ser Glu Ser Glu Ala His Leu Leu
        35                  40                  45

Asp Ser Asn Met Gly Val Pro Ser Val Glu Leu Thr Asn Leu Tyr Pro
    50                  55                  60

Tyr Cys Asp Tyr Glu Met Lys Cys Ala Ala Asp Ser Thr Ile Tyr Ala
65                  70                  75                  80

Ser Tyr Tyr Glu Cys Gly His Gly Ile Ser Thr Gly Gly Tyr Gly Tyr
                85                  90                  95

Gly Pro Tyr Ser Ser Leu Val Ser Cys Arg Thr His Gln
            100                 105

<210> 159
<211> 100
<212> PRT
<213> Artificial Sequence

<220>
<223> Description of Artificial Sequence: IMABIS044

<220>
<221> SITE
<222> (1)..(100)

<400> 159
Ile Glu Val Glu Lys Pro Leu Tyr Gly Val Glu Val Phe Val Gly Glu
1               5                   10                  15

Thr Ala His Phe Glu Ile Glu Ala Ser Gly Tyr Thr Ile Gly Pro Val
            20                  25                  30

His Gly Gln Trp Lys Leu Lys Gly Gln Pro Asn Met Gly Lys His Ile
        35                  40                  45

Leu Ile Leu His Asn Cys Gln Leu Gly Met Thr Gly Glu Val Ser Cys
    50                  55                  60

Ala Ala Asp Ser Thr Ile Tyr Ala Ser Tyr Tyr Glu Cys Gly His Gly
65                  70                  75                  80

Ile Ser Thr Gly Gly Tyr Gly Tyr Asn Ala Lys Ser Ala Ala Asn Leu
                85                  90                  95

Lys Val Lys Glu
            100


<210> 160
<211> 102
<212> PRT
<213> Artificial Sequence

<220>
<223> Description of Artificial Sequence: IMABIS045

<220>
<221> SITE
<222> (1)..(102)

<400> 160
Phe Lys Ile Glu Thr Thr Pro Glu Ser Arg Tyr Leu Ala Gln Ile Gly
1               5                   10                  15

Asp Ser Val Ser Leu Thr Cys Ser Ala Ser Gly Tyr Thr Ile Gly Pro
            20                  25                  30

Pro Phe Phe Ser Trp Arg Thr Gln Ile Asp Ser Asn Met Gly Thr Ser
        35                  40                  45

Thr Leu Thr Met Asn Pro Val Ser Phe Gly Asn Glu His Ser Tyr Leu
    50                  55                  60

Cys Ala Ala Asp Ser Thr Ile Tyr Ala Ser Tyr Tyr Glu Cys Gly His
65                  70                  75                  80

Gly Ile Ser Thr Gly Gly Tyr Gly Tyr Arg Lys Leu Glu Lys Gly Ile
                85                  90                  95

Gln Val Glu Ile Tyr Ser
            100


<210> 161
<211> 89
<212> PRT
<213> Artificial Sequence

<220>
<223> Description of Artificial Sequence: iMab102

<220>
<221> SITE
<222> (1)..(89)

<400> 161
Asp Asp Leu Lys Leu Thr Cys Arg Ala Ser Gly Tyr Thr Ile Gly Pro
1               5                   10                  15

Tyr Cys Met Gly Trp Phe Arg Gln Ala Pro Asn Asp Asp Ser Thr Asn
            20                  25                  30

Val Ala Thr Ile Asn Met Gly Thr Val Thr Leu Ser Met Asp Asp Leu
        35                  40                  45

Gln Pro Glu Asp Ser Ala Glu Tyr Asn Cys Ala Ala Asp Ser Thr Ile
    50                  55                  60

Tyr Ala Ser Tyr Tyr Glu Cys Gly His Gly Leu Ser Thr Gly Gly Tyr
65                  70                  75                  80

Gly Tyr Asp Ser His Tyr Arg Gly Thr
                85


<210> 162
<211> 89
<212> PRT
<213> Artificial Sequence

<220>
<223> Description of Artificial Sequence: iMabis050

<220>
<221> SITE
<222> (1)..(89)

<400> 162
Asp Asp Leu Lys Leu Thr Ser Arg Ala Ser Gly Tyr Thr Ile Gly Pro
1               5                   10                  15

Tyr Cys Met Gly Trp Phe Arg Gln Ala Pro Asn Asp Asp Ser Thr Asn
            20                  25                  30

Val Ala Thr Ile Asn Met Gly Thr Val Thr Leu Ser Met Asp Asp Leu
        35                  40                  45

Gln Pro Glu Asp Ser Ala Glu Tyr Asn Ser Ala Cys Asp Ser Thr Ile
    50                  55                  60

Tyr Ala Ser Tyr Tyr Glu Cys Gly His Gly Leu Ser Thr Gly Gly Tyr
65                  70                  75                  80

Gly Tyr Asp Cys Arg Gly Gln Gly Thr
                85


<210> 163
<211> 89
<212> PRT
<213> Artificial Sequence

<220>
<223> Description of Artificial Sequence: iMabis051

<220>
<221> SITE
<222> (1)..(89)

<400> 163
Asp Asp Leu Lys Leu Thr Ser Arg Ala Ser Gly Tyr Thr Ile Gly Pro
1               5                   10                  15

Tyr Cys Met Gly Trp Phe Arg Gln Ala Pro Asn Asp Asp Ser Thr Asn
            20                  25                  30

Val Ala Thr Ile Asn Met Gly Thr Val Thr Leu Ser Met Asp Asp Leu
        35                  40                  45

Gln Pro Glu Asp Ser Ala Glu Tyr Asn Ser Ala Cys Asp Ser Thr Ile
    50                  55                  60

Tyr Ala Ser Tyr Tyr Glu Cys Gly His Gly Leu Ser Thr Gly Gly Tyr
65                  70                  75                  80

Gly Tyr Asp Ser Cys Gly Gln Gly Thr
                85


<210> 164
<211> 89
<212> PRT
<213> Artificial Sequence

<220>
<223> Description of Artificial Sequence: iMabis052

<220>
<221> SITE
<222> (1)..(89)

<400> 164
Gly Ser Leu Arg Leu Ser Ser Ala Ala Ser Gly Tyr Thr Ile Gly Pro
1               5                   10                  15

Tyr Cys Met Gly Trp Phe Arg Gln Ala Pro Gly Asp Asp Arg Glu Gly
            20                  25                  30

Val Ala Ala Ile Asn Met Gly Thr Val Tyr Leu Leu Met Asn Ser Leu
        35                  40                  45

Glu Pro Glu Asp Thr Ala Ile Cys Tyr Ser Ala Ala Asp Ser Thr Ile
    50                  55                  60

Tyr Ala Ser Tyr Tyr Glu Cys Gly His Gly Leu Ser Thr Gly Gly Tyr
65                  70                  75                  80

Gly Tyr Asp Ser Trp Gly Gln Gly Cys
                85


<210> 165
<211> 89
<212> PRT
<213> Artificial Sequence

<220>
<223> Description of Artificial Sequence: iMabis053

<220>
<221> SITE
<222> (1)..(89)

<400> 165
Gly Ser Leu Arg Leu Ser Ser Ala Ala Ser Gly Tyr Thr Ile Gly Pro
1               5                   10                  15

Tyr Cys Met Gly Trp Phe Arg Gln Ala Pro Gly Asp Asp Arg Glu Gly
            20                  25                  30

Val Ala Ala Ile Asn Met Gly Thr Val Tyr Leu Leu Met Asn Ser Leu
        35                  40                  45

Glu Pro Glu Asp Thr Ala Ile Tyr Tyr Ser Cys Ala Asp Ser Thr Ile
    50                  55                  60

Tyr Ala Ser Tyr Tyr Glu Cys Gly His Gly Leu Ser Thr Gly Gly Tyr
65                  70                  75                  80

Gly Tyr Asp Ser Cys Gly Gln Gly Thr
                85

<210> 166
<211> 89
<212> PRT
<213> Artificial Sequence

<220>
<223> Description of Artificial Sequence: iMabis054

<220>
<221> SITE
<222> (1)..(89)

<400> 166
Gly Ser Leu Arg Leu Ser Ser Ala Ala Ser Gly Tyr Thr Ile Gly Pro
1               5                   10                  15

Tyr Cys Met Gly Trp Phe Arg Gln Ala Pro Gly Asp Asp Arg Glu Gly
            20                  25                  30

Val Ala Ala Ile Asn Met Gly Thr Val Tyr Leu Leu Met Asn Ser Leu
        35                  40                  45

Glu Pro Glu Asp Thr Ala Ile Tyr Tyr Cys Ala Ala Asp Ser Thr Ile
    50                  55                  60

Tyr Ala Ser Tyr Tyr Glu Cys Gly His Gly Leu Ser Thr Gly Gly Tyr
65                  70                  75                  80

Gly Tyr Asp Ser Trp Gly Cys Gly Gly
                85


<210> 167
<211> 60
<212> PRT
<213> Artificial Sequence

<220>
<223> Description of Artificial Sequence: iMab100
      sequence

<220>
<221> SITE
<222> (1)..(60)

<400> 167
Asn Val Lys Leu Val Glu Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Cys Met Gly Trp Phe Arg Gln Ala Pro Asn Asp Asp Ser Thr Asn Val
        35                  40                  45

Ala Thr Ile Asn Met Gly Gly Gly Ile Thr Tyr Tyr
    50                  55                  60

<210> 168
<211> 60
<212> PRT
<213> Artificial Sequence

<220>
<223> Description of Artificial Sequence: iMab100
sequence

<220>
<221> SITE
<222> (1)..(60)

<400> 168
Gly Asp Ser Val Lys Glu Arg Phe Asp Ile Arg Arg Asp Asn Ala Ser
1               5                   10                  15

Asn Thr Val Thr Leu Ser Met Asp Asp Leu Gln Pro Glu Asp Ser Ala
            20                  25                  30

Glu Tyr Asn Cys Ala Gly Asp Ser Thr Ile Tyr Ala Ser Tyr Tyr Glu
        35                  40                  45

Cys Gly His Gly Leu Ser Thr Gly Gly Tyr Gly Tyr
    50                  55                  60

<210> 169
<211> 15
<212> PRT
<213> Artificial Sequence

<220>
<223> Description of Artificial Sequence: iMab100
sequence

<220>
<221> SITE
<222> (1)..(15)

<400> 169
Asp Ser His Tyr Arg Gly Gln Gly Thr Asp Val Thr Val Ser Ser
1               5                   10                  15

<210> 170
<211> 5797
<212> DNA
<213> Artificial Sequence

<220>
<223> Description of Artificial Sequence: vector
CM114-IMAB100

<220>
<221> misc_feature
<222> (1)..(5797)

<400> 170
aagaaaccaa ttgtccatat tgcatcagac attgccgtca ctgcgtcttt tactggctct 60
tctcgctaac caaaccggta accccgctta ttaaaagcat tctgtaacaa agcgggacca 120
aagccatgac aaaaacgcgt aacaaaagtg tctataatca cggcagaaaa gtccacattg 180
attatttgca cggcgtcaca ctttgctatg ccatagcatt tttatccata agattagcgg 240
atcctacctg acgcttttta tcgcaactct ctactgtttc tccatacccg ttttttgggc 300
taacaggaga agatatacca tgaaaaaact gttatttgcg attccgctgg tggtgccgtt 360
ttatagccat agcgcgggcg gccgcaatgt gaaactggtt gaaaaaggtg gcaatttcgt 420
cgaaaacgat gacgatctta agctcacgtg ccgtgctgaa ggttacacca ttggcccgta 480
ctgcatgggt tggttccgtc aggcgccgaa cgacgacagt actaacgtgg ccacgatcaa 540
catgggtggc ggtattacgt actacggtga ctccgtcaaa gagcgcttcg atatccgtcg 600
cgacaacgcg tccaacaccg ttaccttatc gatggacgat ctgcaaccgg aagactctgc 660
agaatacaat tgtgcaggtg attctaccat ttacgcgagc tattatgaat gtggtcatgg 720
cctgagtacc ggcggttacg gctacgatag ccactaccgt ggtcagggta ccgacgttac 780
cgtctcgtcg gccagctcgg ccggtggcgg tggcagctat accgatattg aaatgaaccg 840
cctgggcaaa accggcagca gtggtgattc gggcagcgcg tggagtcatc cgcagtttga 900
gaaagcggcg cgcctggaaa ctgttgaaag ttgtttagca aaaccccata cagaaaattc 960
atttactaac gtctggaaag acgacaaaac tttagatcgt tacgctaact atgagggttg 1020
tctgtggaat gctacaggcg ttgtagtttg tactggtgac gaaactcagt gttacggtac 1080
atgggttcct attgggcttg ctatccctga aaatgagggt ggtggctctg agggtggcgg 1140
ttctgagggt ggcggttctg agggtggcgg tactaaacct cctgagtacg gtgatacacc 1200
tattccgggc tatacttata tcaaccctct cgacggcact tatccgcctg gtactgagca 1260
aaaccccgct aatcctaatc cttctcttga ggagtctcag cctcttaata ctttcatgtt 1320
tcagaataat aggttccgaa ataggcaggg ggcattaact gtttatacgg gcactgttac 1380
tcaaggcact gaccccgtta aaacttatta ccagtacact cctgtatcat caaaagccat 1440
gtatgacgct tactggaacg gtaaattcag agactgcgct ttccattctg gctttaatga 1500
ggatccattc gtttgtgaat atcaaggcca atcgtctgac ctgcctcaac ctcctgtcaa 1560
tgctggcggc ggctctggtg gtggttctgg tggcggctct gagggtggtg gctctgaggg 1620
tggcggttct gagggtggcg gctctgaggg aggcggttcc ggtggtggct ctggttccgg 1680
tgattttgat tatgaaaaga tggcaaacgc taataagggg gctatgaccg aaaatgccga 1740
tgaaaacgcg ctacagtctg acgctaaagg caaacttgat tctgtcgcta ctgattacgg 1800
tgctgctatc gatggtttca ttggtgacgt ttccggcctt gctaatggta atggtgctac 1860
tggtgatttt gctggctcta attcccaaat ggctcaagtc ggtgacggtg ataattcacc 1920
tttaatgaat aatttccgtc aatatttacc ttccctccct caatcggttg aatgtcgccc 1980
ttttgtcttt agcgctggta aaccatatga attttctatt gattgtgaca aaataaactt 2040
attccgtggt gtctttgcgt ttcttttata tgttgccacc tttatgtatg tattttctac 2100
gtttgctaac atactgcgta ataaggagtc ttaaggcgcg cctgtaatga acggtctcca 2160
gcttggctgt tttggcggat gagagaagat tttcagcctg atacagatta aatcagaacg 2220
cagaagcggt ctgataaaac agaatttgcc tggcggcagt agcgcggtgg tcccacctga 2280
ccccatgccg aactcagaag tgaaacgccg tagcgccgat ggtagtgtgg ggtctcccca 2340
tgcgagagta gggaactgcc aggcatcaaa taaaacgaaa ggctcagtcg aaagactggg 2400
cctttcgttt tatctgttgt ttgtcggtga acgctctcct gagtaggaca aatccgccgg 2460
gagcggattt gaacgttgcg aagcaacggc ccggagggtg gcgggcagga cgcccgccat 2520
aaactgccag gcatcaaatt aagcagaagg ccatcctgac ggatggcctt tttgcgtttc 2580
tacaaactct ttttgtttat ttttctaaat acattcaaat atgtatccgc tcatgagaca 2640
ataaccctga taaatgcttc aataatattg aaaaaggaag agtatgagta ttcaacattt 2700
ccgtgtcgcc cttattccct tttttgcggc attttgcctt cctgtttttg ctcacccaga 2760
aacgctggtg aaagtaaaag atgctgaaga tcagttgggt gcacgagtgg gttacatcga 2820
actggatctc aacagcggta agatccttga gagttttcgc cccgaagaac gttttccaat 2880
gatgagcact tttaaagttc tgctatgtgg cgcggtatta tcccgtgttg acgccgggca 2940
agagcaactc ggtcgccgca tacactattc tcagaatgac ttggttgagt actcaccagt 3000
cacagaaaag catcttacgg atggcatgac agtaagagaa ttatgcagtg ctgccataac 3060

catgagtgat aacactgcgg ccaacttact tctgacaacg atcggaggac cgaaggagct 3120
aaccgctttt ttgcacaaca tgggggatca tgtaactcgc cttgatcgtt gggaaccgga 3180
gctgaatgaa gccataccaa acgacgagcg tgacaccacg atgcctgtag caatggcaac 3240
aacgttgcgc aaactattaa ctggcgaact acttactcta gcttcccggc aacaattaat 3300
agactggatg gaggcggata aagttgcagg accacttctg cgctcggccc ttccggctgg 3360
ctggtttatt gctgataaat ctggagccgg tgagcgtggg tctcgcggta tcattgcagc 3420
actggggcca gatggtaagc cctcccgtat cgtagttatc tacacgacgg ggagtcaggc 3480
aactatggat gaacgaaata gacagatcgc tgagataggt gcctcactga ttaagcattg 3540
gtaactgtca gaccaagttt actcatatat actttagatt gatttaaaac ttcattttta 3600
atttaaaagg atctaggtga agatcctttt tgataatctc atgaccaaaa tcccttaacg 3660
tgagttttcg ttccactgag cgtcagaccc cgtagaaaag atcaaaggat cttcttgaga 3720
tccttttttt ctgcgcgtaa tctgctgctt gcaaacaaaa aaaccaccgc taccagcggt 3780
ggtttgtttg ccggatcaag agctaccaac tctttttccg aaggtaactg gcttcagcag 3840
agcgcagata ccaaatactg tccttctagt gtagccgtag ttaggccacc acttcaagaa 3900
ctctgtagca ccgcctacat acctcgctct gctaatcctg ttaccagtgg ctgctgccag 3960
tggcgataag tcgtgtctta ccgggttgga ctcaagacga tagttaccgg ataaggcgca 4020
gcggtcgggc tgaacggggg gttcgtgcac acagcccagc ttggagcgaa cgacctacac 4080
cgaactgaga tacctacagc gtgagctatg agaaagcgcc acgcttcccg aagggagaaa 4140
ggcggacagg tatccggtaa gcggcagggt cggaacagga gagcgcacga gggagcttcc 4200
agggggaaac gcctggtatc tttatagtcc tgtcgggttt cgccacctct gacttgagcg 4260
tcgatttttg tgatgctcgt caggggggcg gagcctatgg aaaaacgcca gcaacgcggc 4320
ctttttacgg ttcctggcct tttgctggcc ttttgctcac atgttctttc ctgcgttatc 4380
ccctgattct gtggataacc gtattaccgc ctttgagtga gctgataccg ctcgccgcag 4440
ccgaacgacc gagcgcagcg agtcagtgag cgaggaagcg gaagagcgcc tgatgcggta 4500
ttttctcctt acgcatctgt gcggtatttc acaccgcata tggtgcactc tcagtacaat 4560
ctgctctgat gccgcatagt taagccagta tacactccgc tatcgctacg tgactgggtc 4620
atggctgcgc cccgacaccc gccaacaccc gctgacgcgc cctgacgggc ttgtctgctc 4680
ccggcatccg cttacagaca agctgtgacc gtctccggga gctgcatgtg tcagaggttt 4740
tcaccgtcat caccgaaacg cgcgaggcag cagatcaatt cgcgcgcgaa ggcgaagcgg 4800
catgcataat gtgcctgtca aatggacgaa gcagggattc tgcaaaccct atgctactcc 4860
gtcaagccgt caattgtctg attcgttacc aattatgaca acttgacggc tacatcattc 4920
actttttctt cacaaccggc acggaactcg ctcgggctgg ccccggtgca tttttttaat 4980
acccgcgaga aatagagttg atcgtcaaaa ccaacattgc gaccgacggt ggcgataggc 5040
atccgggtgg tgctcaaaag cagcttcgcc tggctgatac gttggtcctc gcgccagctt 5100
aagacgctaa tccctaactg ctggcggaaa agatgtgaca gacgcgacgg cgacaagcaa 5160
acatgctgtg cgacgctggc gatatcaaaa ttgctgtctg ccaggtgatc gctgatgtac 5220
tgacaagcct cgcgtacccg attatccatc ggtggatgga gcgactcgtt aatcgcttcc 5280
atgcgccgca gtaacaattg ctcaagcaga tttatcgcca gcagctccga atagcgccct 5340
tccccttgcc cggcgttaat gatttgccca aacaggtcgc tgaaatgcgg ctggtgcgct 5400
tcatccgggc gaaagaaccc cgtattggca aatattgacg gccagttaag ccattcatgc 5460
cagtaggcgc gcggacgaaa gtaaacccac tggtgatacc attcgcgagc ctccggatga 5520
cgaccgtagt gatgaatctc tcctggcggg aacagcaaaa tatcacccgg tcggcaaaca 5580
aattctcgtc cctgattttt caccaccccc tgaccgcgaa tggtgagatt gagaatataa 5640
cctttcattc ccagcggtcg gtcgataaaa aaatcgagat aaccgttggc ctcaatcggc 5700
gttaaacccg ccaccagatg ggcattaaac gagtatcccg gcagcagggg atcattttgc 5760
gcttcagcca tacttttcat actcccgcca ttcagag 5797

<210> 171
<211> 5100
<212> DNA
<213> Artificial Sequence

<220>
<223> Description of Artificial Sequence: vector
      CM126-IMAB100

<220>
<221> misc_feature
<222> (1)..(5100)

<400> 171
ttctcatgtt tgacagctta tcatcgataa gctttaatgc ggtagtttat cacagttaaa 60
ttgctaacgc agtcaggcac cgtgtatgaa atctaacaat gcgctcatcg tcatcctcgg 120
caccgtcacc ctggatgctg taggcatagg cttggttatg ccggtactgc cgggcctctt 180
gcgggatatc gtccattccg acagcatcgc cagtcactat ggcgtgctgc tagcgctata 240
tgcgttgatg caatttctat gcgcacccgt tctcggagca ctgtccgacc gctttggccg 300
ccgcccagtc ctgctcgctt cgctacttgg agccactatc gactacgcga tcatggcgac 360
cacacccgtc ctgtggatat ccggatatag ttcctccttt cagcaaaaaa cccctcaaga 420
cccgtttaga ggccccaagg ggttatgcta gttattgctc agcggtggca gcagccaact 480
cagcttcctt tcgggctttg ttagcagccg gatccttagt ggtgatggtg atggtggctt 540
ttgcccaggc ggttcatttc tatatcggta tagctgccac cgccaccggc cgagctggcc 600
gacgagacgg taacgtcggt accctgacca cggtagtggc tatcgtagcc gtaaccgccg 660
gtactcaggc catgaccaca ttcataatag ctcgcgtaaa tggtagaatc acctgcacaa 720
ttgtattctg cagagtcttc cggttgcaga tcgtccatcg ataaggtaac ggtgttggac 780
gcgttgtcgc gacggatatc gaagcgctct ttgacggagt caccgtagta cgtaataccg 840
ccacccatgt tgatcgtggc cacgttagta ctgtcgtcgt tcggcgcctg acggaaccaa 900
cccatgcagt acgggccaat ggtgtaacct tcagcacggc acgtgagctt aagatcgtca 960
tcgttttcga cgaaattgcc acctttttca accagtttca cattcatatg tatatctcct 1020
tcttaaagtt aaacaaaatt atttctagag ggaaaccgtt gtggtctccc tatagtgagt 1080
cgtattaatt tcgcgggatc gagatctcga tcctctacgc cggacgcatc gtggccggca 1140
tcaccggcgc cacaggtgcg gttgctggcg cctatatcgc cgacatcacc gatggggaag 1200
atcgggctcg ccacttcggg ctcatgagcg cttgtttcgg cgtgggtatg gtggcaggcc 1260
ccgtggccgg gggactgttg ggcgccatct ccttgcatgc accattcctt gcggcggcgg 1320
tgctcaacgg cctcaaccta ctactgggct gcttcctaat gcaggagtcg cataagggag 1380
agcgtcgatc gaccgatgcc cttgagagcc ttcaacccag tcagctcctt ccggtgggcg 1440
cggggcatga ctatcgtcgc cgcacttatg actgtcttct ttatcatgca actcgtagga 1500
caggtgccgg cagcgctctg ggtcattttc ggcgaggacc gctttcgctg gagcgcgacg 1560
atgatcggcc tgtcgcttgc ggtattcgga atcttgcacg ccctcgctca agccttcgtc 1620
actggtcccg ccaccaaacg tttcggcgag aagcaggcca ttatcgccgg catggcggcc 1680
gacgcgctgg gctacgtctt gctggcgttc gcgacgcgag gctggatggc cttccccatt 1740
atgattcttc tcgcttccgg cggcatcggg atgcccgcgt tgcaggccat gctgtccagg 1800
caggtagatg acgaccatca gggacagctt caaggatcgc tcgcggctct taccagccta 1860
acttcgatca ctggaccgct gatcgtcacg gcgatttatg ccgcctcggc gagcacatgg 1920
aacgggttgg catggattgt aggcgccgcc ctataccttg tctgcctccc cgcgttgcgt 1980
cgcggtgcat ggagccgggc cacctcgacc tgaatggaag ccggcggcac ctcgctaacg 2040
gattcaccac tccaagaatt ggagccaatc aattcttgcg gagaactgtg aatgcgcaaa 2100
ccaacccttg gcagaacata tccatcgcgt ccgccatctc cagcagccgc acgcggcgca 2160
tctcgggcag cgttgggtcc tggccacggg tgcgcatgat cgtgctcctg tcgttgagga 2220
cccggctagg ctggcggggt tgccttactg gttagcagaa tgaatcaccg atacgcgagc 2280
gaacgtgaag cgactgctgc tgcaaaacgt ctgcgacctg agcaacaaca tgaatggtct 2340
tcggtttccg tgtttcgtaa agtctggaaa cgcggaagtc agcgccctgc accattatgt 2400
tccggatctg catcgcagga tgctgctggc taccctgtgg aacacctaca tctgtattaa 2460
cgaagcgctg gcattgaccc tgagtgattt ttctctggtc ccgccgcatc cataccgcca 2520
gttgtttacc ctcacaacgt tccagtaacc gggcatgttc atcatcagta acccgtatcg 2580
tgagcatcct ctctcgtttc atcggtatca ttacccccat gaacagaaat cccccttaca 2640
cggaggcatc agtgaccaaa caggaaaaaa ccgcccttaa catggcccgc tttatcagaa 2700
gccagacatt aacgcttctg gagaaactca acgagctgga cgcggatgaa caggcagaca 2760
tctgtgaatc gcttcacgac cacgctgatg agctttaccg cagctgcctc gcgcgtttcg 2820
gtgatgacgg tgaaaacctc tgacacatgc agctcccgga gacggtcaca gcttgtctgt 2880
aagcggatgc cgggagcaga caagcccgtc agggcgcgtc agcgggtgtt ggcgggtgtc 2940
ggggcgcagc catgacccag tcacgtagcg atagcggagt gtatactggc ttaactatgc 3000
ggcatcagag cagattgtac tgagagtgca ccatatatgc ggtgtgaaat accgcacaga 3060
tgcgtaagga gaaaataccg catcaggcgc tcttccgctt cctcgctcac tgactcgctg 3120

cgctcggtcg ttcggctgcg gcgagcggta tcagctcact caaaggcggt aatacggtta 3180
tccacagaat caggggataa cgcaggaaag aacatgtgag caaaaggcca gcaaaaggcc 3240
aggaaccgta aaaaggccgc gttgctggcg tttttccata ggctccgccc ccctgacgag 3300
catcacaaaa atcgacgctc aagtcagagg tggcgaaacc cgacaggact ataaagatac 3360
caggcgtttc cccctggaag ctccctcgtg cgctctcctg ttccgaccct gccgcttacc 3420
ggatacctgt ccgcctttct cccttcggga agcgtggcgc tttctcatag ctcacgctgt 3480
aggtatctca gttcggtgta ggtcgttcgc tccaagctgg gctgtgtgca cgaacccccc 3540
gttcagcccg accgctgcgc cttatccggt aactatcgtc ttgagtccaa cccggtaaga 3600
cacgacttat cgccactggc agcagccact ggtaacagga ttagcagagc gaggtatgta 3660
ggcggtgcta cagagttctt gaagtggtgg cctaactacg gctacactag aaggacagta 3720
tttggtatct gcgctctgct gaagccagtt accttcggaa aaagagttgg tagctcttga 3780
tccggcaaac aaaccaccgc tggtagcggt ggtttttttg tttgcaagca gcagattacg 3840
cgcagaaaaa aaggatctca agaagatcct ttgatctttt ctacggggtc tgacgctcag 3900
tggaacgaaa actcacgtta agggattttg gtcatgagat tatcaaaaag gatcttcacc 3960
tagatccttt taaattaaaa atgaagtttt aaatcaatct aaagtatata tgagtaaact 4020
tggtctgaca gttaccaatg cttaatcagt gaggcaccta tctcagcgat ctgtctattt 4080
cgttcatcca tagttgcctg actccccgtc gtgtagataa ctacgatacg ggagggctta 4140
ccatctggcc ccagtgctgc aatgataccg cgagacccac gctcaccggc tccagattta 4200
tcagcaataa accagccagc cggaagggcc gagcgcagaa gtggtcctgc aactttatcc 4260
gcctccatcc agtctattaa ttgttgccgg gaagctagag taagtagttc gccagttaat 4320
agtttgcgca acgttgttgc cattgctgca ggcatcgtgg tgtcacgctc gtcgtttggt 4380
atggcttcat tcagctccgg ttcccaacga tcaaggcgag ttacatgatc ccccatgttg 4440
tgcaaaaaag cggttagctc cttcggtcct ccgatcgttg tcagaagtaa gttggccgca 4500
gtgttatcac tcatggttat ggcagcactg cataattctc ttactgtcat gccatccgta 4560
agatgctttt ctgtgactgg tgagtactca accaagtcat tctgagaata gtgtatgcgg 4620
cgaccgagtt gctcttgccc ggcgtcaaca cgggataata ccgcgccaca tagcagaact 4680
ttaaaagtgc tcatcattgg aaaacgttct tcggggcgaa aactctcaag gatcttaccg 4740
ctgttgagat ccagttcgat gtaacccact cgtgcaccca actgatcttc agcatctttt 4800
actttcacca gcgtttctgg gtgagcaaaa acaggaaggc aaaatgccgc aaaaaaggga 4860
ataagggcga cacggaaatg ttgaatactc atactcttcc tttttcaata ttattgaagc 4920
atttatcagg gttattgtct catgagcgga tacatatttg aatgtattta gaaaaataaa 4980
caaatagggg ttccgcgcac atttccccga aaagtgccac ctgacgtcta agaaaccatt 5040
attatcatga cattaaccta taaaaatagg cgtatcacga ggccctttcg tcttcaagaa 5100

## Description

The invention relates to molecular affinity bodies. It particularly relates to conjugates of such molecular affinity bodies with cosmetic substances, more in particular to molecular affinity bodies linked to fragrances and/or coloured substances and/or conditioning agents. Cosmetic agents are typically delivered non specifically to a general area of application. Such application ranges from massaging shampoo or the like in hair, applying cream, powder or ointment on the body to applying a liquid or solid composition to an amount of water in contact with textiles, etc.

There are several problems associated with such nonspecific delivery. For instance, in many applications the majority of the cosmetic agent is never actually delivered to the target, but is rinsed out by a washing step. Also, many cosmetic agents that are delivered are released prematurely, such that colour or fragrance fades or disappears rather quickly. Furthermore, in the case of colouring agents, such as dyes for hair, the nonspecific application leads to staining of clothes, etc. Moreover, the binding to hair of colour, if it is to last any significant time typically requires harsh chemical treatments.

A further problem with cosmetic agents is that many of them (in particular fragrant agents) are hydrophobic, which hampers their applicability in aqueous environments. Also fragrances are often volatile. The present invention contributes to solving many of these problems and more as will become clear from the following description.

The present invention provides in one embodiment a method for applying a cosmetic substance to a desired target molecule, comprising providing a conjugate of a proteinaceous substance having specific affinity for said target molecule linked to a cosmetic substance, whereby the resulting connection between cosmetic substance and target molecule can be disrupted upon the presence of a chemical and/or physical signal. The present invention uses conjugates of proteinaceous molecules having specific affinity for a target molecule and a cosmetic agent, linked together in such a way that the linkage can be disrupted if desired. The cosmetic agents according to the invention can be fragrances, colouring agents, conditioners and the like. Fragrances are typically delivered in an aqueous composition, a powder-like composition, an oil or a cream/ointment-like composition. Typically the fragrance is desired to linger over longer periods of time. However, until the present invention the typical release of a fragrance has been a burst within a very short time from application and a less than desired release for the remainder. As said the fragrant compositions are typically delivered non specifically to a desired area. According to the invention the fragrant molecules are delivered specifically to a target associated with a desired area of delivery, such as to skin components such as keratin or microorganisms associated with skin, or to hair components, or saliva components, or to microorganisms associated with mucosal secretions or to textile fabric. upon delivery of the conjugate of the fragrant molecule and the targeting molecule, the linkage between the two will become labile through the action of local enzymes, or the action of added enzymes and/or by a change in physical and/or chemical conditions, such as temperature, pH and the like. Thereby the fragrant molecule will be released depending on the selected half life of the bond between targeting molecule and fragrant molecule. The same targeting mechanism is applied when the cosmetic agent is a colouring agent, e.g. a dye for hair. Several different options concerning release are present. The colour may be desired for only a short period of time. In this case a rinse with water or washing with a shampoo should be enough to remove the dye, either by disrupting the linkage of the dye to the targeting molecule, or by selecting a targeting molecule which has a low affinity under conditions of removal (shampoo). If the colour is desired for a longer period of time, a special shampoo providing an enzyme which disrupts the link or which proteolyzes the proteinaceous targeting molecule can be provided. The release can also be provided by a chemical signal (e.g. pH) or a physical signal. However, these conjugate dyes can be used as permanent dyes also, which will only disappear because of hair growth. In the same manner as described above one can provide compositions for delivering fragrances to fabric (for softener compositions and the like), as well as conditioner compositions and any other cosmetic agents that are better when specifically delivered and/or which benefit from any form of controlled release.

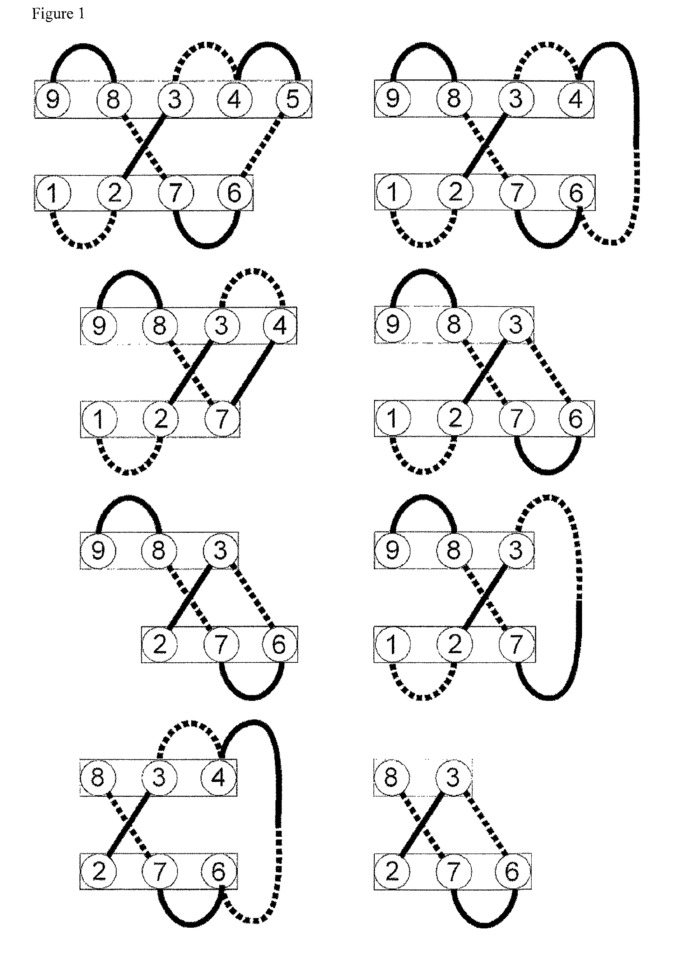

According to the invention versatile affinity proteins are used as targeting molecules. A versatile affinity protein is a molecule which comprises at least a synthetic or recombinant proteinaceous molecule comprising a binding peptide and a core, said core comprising a beta-barrel comprising at least 4 strands, wherein said beta -barrel comprises at least two beta -sheets, wherein each of said beta -sheet comprises two of said strands and wherein said binding peptide is a peptide connecting two strands in said beta -barrel and wherein said binding peptide is outside its natural context. Preferably an affinity protein comprises a beta- barrel, wherein said beta -barrel comprises at least 5 strands, wherein at least one of said sheets comprises 3 of said strands. More preferably an affinity protein comprises a beta -barrel which comprises at least 6 strands, wherein at least two of said sheets comprises 3 of said strands. Preferably an affinity protein comprises a beta -barrel which comprises at least 7 strands, wherein at least one of said sheets comprises 4 of said strands. Preferably an affinity protein comprises a beta-barrel which comprises at least 8 or 9 strands, wherein at least one of said sheets comprises 4 of said strands. The various strands in the core are preferably encoded by a single open reading frame. The loops connecting the various strands may have any type of configuration. So as not to unduly limit the versatility of the core it is preferred that loops connect strands on the same side of the core, i.e. and N-terminus of strand (a) connects to a C-terminus of strand (b) on either the closed side or the open side of the core. Loops may connect strands in the same β-sheet or cross-over to the opposing β-sheet. A preferred arrangements for connecting the various strands in the core are given in the examples and the figures, and in particular figure 1. Strands in the core may be in any orientation (parallel or antiparallel) with respect to each other. Preferably the strands are in the configuration as depicted in figure 1.

In a further preferred embodiment the binding peptide connects two strands of said beta-barrel on the open side of said barrel. Preferably the binding peptide connects at least two beta-sheets of said barrel. In a preferred embodiment the versatile affinity protein comprises more than one, preferably three binding peptides and three peptides connecting beta-sheets and/or beta barrels.

The versatile affinity proteins to be used in the conjugates according to the invention are typically designed to have binding properties and structural properties which are suitable for application in the delivery of cosmetic agents.

These properties are obtained by a selection process as described herein below.

Thus the invention also provides a method according to the invention wherein said proteinaceous molecule has an altered binding property, said property selected for the physical and/or chemical circumstances in which the conjugate is applied, said alteration comprising introducing an alteration in the core of proteinaceous molecules according to the invention, and selecting from said proteinaceous molecules, a proteinaceous molecule with said altered binding property. The invention further provides a method according to the invention wherein said proteinaceous molecule has an altered structural property, said property selected for the physical and/or chemical circumstances in which the conjugate is applied, said alteration comprising introducing an alteration in the core of proteinaceous molecules according to the invention, and selecting from said proteinaceous molecules, a proteinaceous molecule with said altered structural property. These processes are most easily carried out by altering nucleic acid molecules which encode proteinaceous substances according to the invention. However, said alterations may also be post-translational modifications. The invention also provides the novel conjugates comprising a cosmetic agent and a versatile affinity protein liked in any way. The link may be covalent or by coordination or complexing. It may be direct or indirect. The cosmetic agent may be present in a liposom or another vehicle to which the VAP is linked. The conjugate may also be a fusion protein.

Preferably the linkage is labile under certain conditions. Labile linkers are well known in the art of immunotoxins for the treatment of cancer and the like. Such linkers can be applied or adapted to the presently invented conjugates. Also a linker may be a peptide or peptide-like bond, which can be broken by an enzyme. Preferably such an enzyme is normally associated with the target of the conjugate. In an alternative, the enzyme can be added simultaneously or separate. The linker is of course preferably stable under storage conditions. For fragrances the linkers need to be designed such that the disruption occurs exactly at the site which releases the original fragrant substance only. In one embodiment the link between proteinaceous molecule and cosmetic substance is labile under skin and/or hair conditions. This is very advantageous in combiantion with a conjugate which has specific affinity for a target molecule associated with the skin,hair or other body substances exposed to the exterior of said body, in particular keratin. In another embodiment the conjugate has a specific affinity for a target molecule associated with textile fabric. Compositions comprising the conjugates of the invention are also part of the present invention. They include but are not limited to a perfume, a deodorant, a mouth wash or a cleaning composition, a hair dye composition, a lipstick, rouge or another skin colouring composition, a detergent and/or softener composition.

### DETAILED DESCRIPTION

Through molecular modeling, structure analyses and methods based on recent advances in molecular biology, molecular, versatile affinity proteins (VAPs) were devised for specific applications in the field of cosmetics and fragrance industries. These VAPs can provide a versatile context to specifically deliver cosmetic agents to targets associated with skin, hair, nails, saliva, textile fabrics, and tissue-type materials such as diapers, hygiene pads, etc. In order to deliver said cosmetic agents to said target, the agents is coupled (linked) to a VAP, which has specific affinity for the desired target.

### a) Versatile Affinity Proteins

### i) VAP design and Construction

The present invention relates to the design, construction, production, screening and use of proteins that contain one or more regions that may be involved in molecular binding. The invention also relates to naturally occurring proteins provided with artificial binding domains, re-modelled natural occurring proteins provided with extra structural components and provided with one or more artificial binding sites, re-modelled natural occurring proteins disposed of some elements (structural or others) provided with one or more artificial binding sites, artificial proteins containing a standardized core structure motif provided with one or more binding sites. All such proteins are called VAPs (Versatile Affinity Proteins) herein. The invention further relates to novel VAPs identified according to the methods of the invention and the transfer of binding sites on naturally occurring proteins that contain a similar core structure. 3D modelling or mutagenesis of such natural occurring proteins can be desired before transfer in order to restore or ensure antigen binding capabilities by the affinity regions present on the selected VAP. Further, the invention relates to processes that use selected VAPs, as described in the invention, for purification, removal, masking, liberation, inhibition, stimulation, capturing, etc,of the chosen ligand capable of being bound by the selected VAP(s).

### LIGAND BINDING PROTEINS

Many naturally occurring proteins that contain a (putative) molecular binding site comprise two functionally different regions: The actual displayed binding region and the region(s) that is (are) wrapped around the molecular binding site or pocket, called the scaffold herein. These two regions are different in function, structure, composition and physical properties. The scaffold structures ensures a stable 3 dimensional conformation for the whole protein, and act as a steppingstone for the actual recognition region.

Two functional different classes of ligand binding proteins can be discriminated. This discrimination is based upon the presence of a genetically variable or invariable ligand binding region. In general, the invariable ligand binding proteins contain a fixed number, a fixed composition and an invariable sequence of amino acids in the binding pocket in a cell of that species. Examples of such proteins are all cell adhesion molecules, e.g. N-CAM and V-CAM, the enzyme families, e.g. kinases and proteases and the family of growth receptors,e.g EGF-R, bFGF-R. In contrast, the genetically variable class of ligand binding proteins is under control of an active genetic shuffling-, mutational or rearrangement mechanism enabling an organism or cell to change the number, composition and sequence of amino acids in, and possibly around, the binding pocket. Eaxmples of these are all types of light and heavy chain of antibodies, B-cell receptor light and heavy chains and T-cell receptor alfa, beta, gamma and delta chains. The molecular constitution of wild type scaffolds can vary to a large extent. For example, Zinc finger containing DNA binding molecules contain a totally different scaffold (looking at the amino acid composition and structure) than antibodies although both proteins are able to bind to a specific target.

### SCAFFOLDS AND LIGAND BINDING DOMAINS

### Antibodies obtained via immunizations

The class of ligand binding proteins that express variable (putative) antigen binding domains has been shown to be of great value in the search for ligand binding proteins. The classical approach to generate ligand binding proteins makes use of the animal immune system. This system is involved in the protection of an organism against foreign substances. One way of recognizing, binding and clearing the organism of such foreign highly diverse substances is the generation of antibodies against these molecules. The immune system is able to select and multiply antibody producing cells that recognize an antigen. This process can also be mimicked by means of active immunizations. After a series of immunizations antibodies may be formed that recognize and bind the antigen. The possible number of antibodies with different affinity regions that can be formed due to genetic rearrangements and mutations, exceeds the number of 10⁴⁰. However, in practice, a smaller number of antibody types will be screened and optimized by the immune system. The isolation of the correct antibody producing cells and subsequent immortalization of these cells or, alternatively, cloning of the selected antibody genes directly, antigen-antibody pairs can be conserved for future (commercial and non-commercial) use.

The use of antibodies obtained this way is restricted only to a limited number of applications. The structure of animal antibodies is different than antibodies found in human. The introduction of animal derived antibodies in humans eg. for medical applications will almost certainly cause immune responses adversing the effect of the introduced antibody (e.g. HAMA reaction). As it is not allowed to actively immunize men for commercial purposes, it is not or only rarely possible to obtain human antibodies this way. Because of these disadvantages methods have been developed to bypass the generation of animal specific antibodies. One example is the removal of the mouse immune system and the introduction of the human immune system in such mouse. All antibodies produced after immunization are of human origin. However the use of animals has also a couple of important disadvantages. First, animal care has a growing attention from ethologists, investigators, public opinion and government. Immunization belongs to a painful and stressful operation and must be prevented as much as possible. Second, immunizations do not always produce antibodies or do not always produce antibodies that contain required features such as binding strength, antigen specificity, etc. The reason for this can be multiple: the immune system missed by co-incidence such a putative antibody; the initially formed antibody appeared to be toxic or harmful; the initially formed antibody also recognizes animal specific molecules and consequently the cells that produce such antibodies will be destroyed; or the epitope cannot be mapped by the immune system (this can have several reasons).

### Otherwise obtained antibodies

It is clear, as discussed above, that immunization procedures may result in the formation of ligand binding proteins but their use is limited, inflexible and uncontrollable. The invention of methods for the bacterial production of antibody fragments (Skerra and Pluckthun, 1988; Better et al., 1988) provided new powerful tools to circumvent the use of animals and immunization procedures. It is has been shown that cloned antibody fragments, (frameworks, affinity regions and combinations of these) can be expressed in artificial systems, enabling the modulation and production of antibodies and derivatives (Fab, V_{L}, V_{H}, scFv and V_{HH}) that recognize a (putative) specific target *in vitro*. New efficient selection technologies and improved degeneration strategies directed the development of huge artificial (among which human) antibody fragment libraries. Such libraries potentially contain antibodies fragments that can bind one or more ligands of choice. These putative ligand specific antibodies can be retrieved by screening and selection procedures. Thus, ligand binding proteins of specific targets can be engineered and retrieved without the use of animal immunizations.

### Other immunoglobulin superfamily derived scaffolds

Although most energy and effort is put in the development and optimization of natural derived or copied human antibody derived libraries, other scaffolds have also been described as succesful scaffolds as carriers for one or more ligand binding domains. Examples of scaffolds based on natural occurring antibodies encompass minibodies (Pessi et al., 1993), Camelidae V_{HH} proteins (Davies and Riechmann, 1994; Hamers-Casterman et al., 1993) and soluble V_{H} variants (Dimasi et al., 1997; Lauwereys et al., 1998). Two other natural occurring proteins that have been used for affinity region insertions are also member of the immunoglobulin superfamily: the T-cell receptor chains (Kranz et al., WO Patent 0148145) and fibronectin domain-3 regions (Koide US Patent 6,462,189; Koide et al., 1998). The two T-cell receptor chains can each hold three affinity regions according to the inventors while for the fibronectin region the investigators described only two regions.

### Non-immunoglobulin derived scaffolds

Besides immunoglobulin domain derived scaffolds, non-immunoglobulin domain containing scaffolds have been investigated. All proteins investigated contain only one protein chain and one to four affinity related regions. Smith and his colleagues (1998) reported the use of knottins (a group of small disulfide bonded proteins) as a scaffold. They successfully created a library based on knottins that had 7 mutational amino acids. Although the stability and length of the proteins are excellent, the low number of amino acids that can be randomized and the singularity of the affinity region make knottin proteins not very powerful. Ku and Schultz (1995) successfully introduced two randomized regions in the four-helix-bundle structure of cytochrome b₅₆₂. However, selected binders were shown to bind with micromolar K_{d} values instead of the required nanomolar or even better range. Another alternate framework that has been used belongs to the tendamistat family of proteins. McConnell and Hoess (1995) demonstrated that alpha-amylase inhibitor (74 amino acid beta-sheet protein) from *Streptomyces tendae* could serve as a scaffold for ligand binding libraries. Two domains were shown to accept degenerated regions and function in ligand binding. The size and properties of the binders showed that tendamistats could function very well as ligand mimickers, called mimetopes. This option has now been exploited. Lipocalin proteins have also been shown to be succesful scaffolds for a maximum of four affinity regions (Beste et al., 1999; Skerra, 2000 BBA; Skerra, 2001 RMB). Lipocalins are involved in the binding of small molecules like retinoids, arachidonic acid and several different steroids. Each lipocalin has a specialized region that recognizes and binds one or more specific ligands. Skerra (2001) used the lipocalin RBP and lipocalin BBP to introduce variable regions at the site of the ligand binding domain. After the construction of a library and successive screening, the investigators were able to isolate and characterize several unique binders with nanomolar specificity for the chosen ligands. It is currently not known how effective lipocalins can be produced in bacteria or fungal cells. The size of lipocalins (about 170 amino acids) is pretty large in relation to V_{HH} chains (about 100 amino acids),which might be too large for industrial applications.

### CORE STRUCTURE DEVELOPMENT

In commercial industrial applications it is very interesting to use single chain peptides, instead of multiple chain peptides because of low costs and high efficiency of such peptides in production processes. One example that could be used in industrial applications are the V_{HH} antibodies. Such antibodies are very stable, can have high specificities and are relatively small. However, the scaffold has evolutionarily been optimised for an immune dependent function but not for industrial applications. In addition, the highly diverse pool of framework regions that are present in one pool of antibodies prevents the use of modular optimisation methods. Therefore a new scaffold was designed based on the favourable stability of V_{HH} proteins.

3D-modelling and comparative modelling software was used to design a scaffold that meets the requirements of versatile affinity proteins (VAPs).

However, at this moment it is not yet possible to calculate all possible protein structures, protein stability and other features, since this would cost months of computer calculation capacity. Therefore we test the most promising computer designed scaffolds in the laboratory by using display techniques, such as phage display or the like. In this way it is possible to screen large numbers of scaffolds in a relatively short time.

Immunoglobulin-like (ig-like) folds are very common throughout nature. Many proteins, especially in the animal kingdom, have a fold region within the protein that belongs to this class. Reviewing the function of the proteins that contain an ig-like fold and reviewing the function of this ig-like fold within that specific protein, it is apparent that most of these domains, if not all, are involved in ligand binding. Some examples of ig-like fold containing proteins are: V-CAM, immunoglobulin heavy chain variable domains, immunoglobulin light chain variable domains, constant regions of immunglobulines, T-cell receptors, fibronectin, reovirus coat protein, beta-galactosidase, integrins, EPO-receptor, CD58, ribulose carboxylase, desulphoferrodoxine, superoxide likes, biotin decarboxylase and P53 core DNA binding protein. A classification of most ig-like folds can be obtained from the SCOP database (Murzin A. G et al, 1995; http://scop.mrc-lmb.cam.ac.uk/scop) and from CATH (Orengo et al, 1997; http://www.biochem.ucl.ac.uk/bsm/cath new/index.html). SCOP classifies these folds as: all beta proteins, with an immunoglobulin-like beta-sandwich in which the sandwich contains 7 strands in 2 sheets although some members that contain the fold have additional strands. CATH classifies these folds as: Mainly beta proteins with an architecture like a sandwich in an immunoglobulin-like fold designated with code 2.60.40. In structure databases like CE (Shindyalov et al.1998; http://cl.sdsc.edu/ce.htm), VAST (Gibrat et al.,1996; http://www.ncbi.nlm.nih.gov/Structure/VAST/vast.shtml) and FSSP (Holm et al, 1998; http://www.ebi.ac.uk/dali/fssp) similar classifications are used.

Projection of these folds from different proteins using software of Cn3D (NCBI; http://www.ncbi.nlm.nih.gov/Structure/CN3D/cn3d.shtml), InsightII (MSI; http://www.accelrys.com/insight) and other structure viewers, showed that the ig-like folds have different sub-domains. A schematic projection of the structure is depicted in figure 1. The most conserved structure was observed in the centre of the folds, named the core. The core structures hardly vary in length and have a relative conserved spatial constrain, but they were found to vary to a large degree in both sequence and amino acid composition. On both sides of the core, sub-domains are present. These are called connecting loops.

These connecting loops are extremely variable as they can vary in amino acid content, sequence, length and configuration. The core structure is therefore designated as the far most important domain within these proteins. The number of beta- elements that form the core can vary between 7 and 9 although 6 stranded core structures might also be of importance. All beta-elements of the core are arranged in two beta-sheets. Each beta-sheet is build of anti-parallel oriented beta-elements. The minimum number of beta-elements in one beta-sheet that was observed was 3 elements. The maximum number of beta-element in one sheet that was observed was 5 elements, although it can not be excluded that higher number of beta-elements might be possible. Connecting loops connect the beta-elements on one side of the barrel. Some connections cross the beta-sheets while others connect beta-elements that are located within one beta-sheet. Especially the loops that are indicated as L2, L4, L6 and L8 are used in nature for ligand binding and are therefore preferred site for the introduction or modification of binding peptide/affinity region. The high variety in length, structure, sequences and amino acid compositions of the L1, L3, L5 and L7 loops clearly indicates that these loops can also be used for ligand binding, at least in an artificial format.

Amino acid side chains in the beta-elements that form the actual core that are projected towards the interior of the core, and thus fill the space in the centre of the core, can interact with each other via H-bonds, covalent bonds (cysteine bridges) and other forces, and determine the stability of the fold. Because amino acid composition and sequence of the residues of the beta-element parts that line up the interior were found to be extremely variable, it was concluded that many other sequence formats and can be created.

In order to obtain the basic concept of the structure as a starting point for the design of new types of proteins containing this ig-like fold, projections of domains that contain ig-like folds were used. Insight II, Cn3D and Modeller programs were used to determine the minimal elements and lengths. In addition, only C-alpha atoms of the structures were projected because these described the minimal features of the folds. Minor differences in spatial positions (coordinates) of these beta elements were allowed.

PDB files representing the coordinates of the C-alpha atoms of the core of ig-like folds were used for the development of new 9, 8, 7 , 6 and 5 beta-elements containing structures. For 8 stranded structures beta element 1 or 9 can be omitted but also elements 5 or 6 can be omitted. Thus an eight stranded core preferably comprises elements 2-8, and either one or 9. Another preferred eight stranded core comprises elements 1-4, 7-9, and either strand 5 or strand 6. For 7 stranded structures, 2 beta elements can be removed among which combinations of element 1 and 9, 1 and 5, 6 and 9, 9 and 5 and, elements 4 and 5. The exclusion of elements 4 and 5 is preferred because of spatial constrains.

Six stranded structures lack preferably element 1, 4 and 5 or 4, 5 and 9 but also other formats were analyzed with Insight and Modeller and shown to be reliable enough for engineering purposes.

Multiple primary scaffolds were constructed and pooled. All computer designed proteins are just an estimated guess. One mutation or multiple amino acid changes in the primary scaffold may make it a successful scaffold or make it function even better than predicted. To accomplish this the constructed primary scaffolds are subjected to a mild mutational process by PCR amplification that includes error-prone PCR, such as unequimolar dNTP concentration, addition of manganese or other additives, or the addition of nucleotide analogues, such as dITP (Spee et al., 1993) or dPTP (Zaccolo et al., 1996) in the reaction mixture which can ultimately change the amino acid compositions and amino acid sequences of the primary scaffolds. This way new (secondary) scaffolds are generated.

In order to test the functionality, stability and other characteristics required or desired features of the scaffolds, a set of known affinity regions, such as 1MEL for binding lysozyme and 1BZQ for binding RNase were inserted in the primary modularly constructed scaffolds. Functionality, heat and chemical stability of the constructed VAPs were determined by measuring unfolding conditions. Functionality after chemical or heat treatment was determined by binding assays (ELISA), while temperature induced unfolding was measured using a circular dichroism (CD) polarimeter. Phage display techniques were used to select desired scaffolds or for optimisation of scaffolds.

### INITIAL AFFINITY REGIONS FOR LIBRARY CONSTRUCTION

In the present invention new and unique affinity regions are required. Affinity regions can be obtained from natural sources, degenerated primers or stacked DNA triplets. All of these sources have certain important limitations as described above. In our new setting we designed a new and strongly improved source of affinity regions which have less restrictions, can be used in modular systems, are extremely flexible in use and optimization, are fast and easy to generate and modulate, have a low percentage of stop codons, have an extremely low percentage of frameshifts and wherein important structural features will be conserved in a large fraction of the new formed clones and new structural elements can be introduced.

The major important affinity region (CDR3) in both light and heavy chain in normal antibodies has a average length between 11 (mouse) and 13 (human) amino acids. Because in such antibodies the CDR3 in light and heavy chain cooperatively function as antigen binder, the strength of such a binding is a result of both regions together. In contrast, the binding of antigens by V_{HH} antibodies (Camelidae) is a result of one CDR3 region due to the absence of a light chain. With an estimated average length of 16 amino acids these CDR3 regions are significantly longer than regular CDR3 regions (Mol. Immunol. Bang Vu et al., 1997, 34, 1121-1131). It can be emphasized that long or multiple CDR3 regions have potentially more interaction sites with the ligand and can therefore be more specific and bind with more strength. Another exception are the CDR3 regions found in cow (*Bos taurus*) (Berens et al., 1997).

Although the antibodies in cow consists of a light and a heavy chain, their CDR3 regions are much longer than found in mouse and humans and are comparable in length found for camelidae CDR3 regions. Average lengths of the major affinity region(s) should preferably be about 16 amino acids. In order to cover as much as possible potentially functional CDR lengths the major affinity region can vary between 1 and 50 or even more amino acids. As the structure and the structural classes of CDR3 regions (like for CDR1 and CDR2) have not been clarified and understood it is not possible to design long affinity regions in a way that the position and properties of crucial amino acids are correct. Therefore, most libraries were supplied with completely degenerated regions in order to find at least some correct regions.

In the invention we describe the use of natural occurring camelidae V_{HH} CDR3 as well as bovine derived V_{H} CDR3 regions as a template for new affinity regions, but of course other CDR regions (eg CDR1 and CDR2) as well as other varying sequences that corresponds in length might be used. CDR3 regions were amplified from mRNA coding for V_{HH} antibodies originating from various animals of the camelidae group or from various other animals containing long CDR3 regions by means of PCR techniques. Next this pool of about 10⁸ different CDR3 regions, which differ in the coding for amino acid composition, amino acid sequence, putative structural classes and length, is subjected to a mutational process by PCR as described above. The result is that most products will differ from the original templates and thus contain coding regions that potentially have different affinity regions. Other very important consequences are that the products keep their length, the pool keeps their length distribution, a significant part will keep structural important information while others might form non-natural classes of structures, the products do not or only rarely contain frame shifts and the majority of the products will lack stop codons. These new affinity regions can be cloned into the selected scaffolds by means of the Modular Affinity and Scaffold Transfer technology (MAST). This technique is based on the fact that all designed and constructed scaffolds described above have a modular structure such that all loops connecting the beta-strands can be easily replaced by other loops without changing the overall structure of the VAP (see figure ... ) The newly constructed library can be subjected to screening procedures similar to the screening of regular libraries known by an experienced user in the field of the art. Thus further provided is a method for producing a library comprising artificial binding peptides said method comprising providing at least one nucleic acid template wherein said templates encode different specific binding peptides, producing a collection of nucleic acid derivatives of said templates through mutation thereof and providing said collection or a part thereof to a peptide synthesis system to produce said library comprising artificial binding peptides. The complexity of the library increases with increasing number of different templates used to generate the library. In this way an increasing number of different structures used. Thus preferably at least two nucleic acid templates, and better at least 10 nucleic acid templates are provided.

Mutations can be introduced using various means and methods. Preferably the method introduces mutations by changing bases in the nucleic acid template or derivative thereof. With derivative is meant a nucleic acid comprising at least one introduced mutation as compared to the temple. In this way the size of the affinity region is not affected. Suitable modification strategies include amplification strategies such as PCR strategies encompass for example unbalanced concentrations of dNTPs (Cadwell et al., PCR Methods Appl. (1992) 2, 28-33; Leung et al., Technique (1989) 1, 11-15; Kuipers, Methods Mol Biol 57 (1996) 351-356), the addition of dITP (Xu et al., Biotechniques 27 (1999) 1102-1108; Spee et al., Nucleic Acids Res 21 (1993) 777-778; Kuipers, Methods Mol Biol 57 (1996) 351-356), dPTP (Zaccolo et al., J. Mol. Biol. 255 (1996) 589-603), 8-oxo-dG (Zaccolo et al., J. Mol. Biol. 255 (1996) 589-603), Mn²⁺ (Cadwell et al., PCR Methods Appl. (1992) 2, 28-33; Leung et al., Technique (1989) 1, 11-15, Xu et al., Biotechniques 27 (1999) 1102-1108), polymerases with high misincorporation levels (Mutagene ®, Stratagene). Site specific protocols for introducing mutations can of course also be used, however, the considerable time and effort to generate a library using such methods would opt against a strategy solely based on site directed mutagenizes. Hybrid strategies can of course be used. Mutation strategies comprising dITP and/or dPTP incorporation during elongation of a nascent strand are preferred since such strategies are easily controlled with respect to the number of mutations that can be introduced in each cycle. The method does not rely on the use of degenerate primers to introduce complexity.

Therefore in one embodiment said amplification utilizes non-degenerates primers. However, (in part) degenerate primers can be used thus also provided is a method wherein at least one non-degenerate primer further comprises a degenerate region. The methods for generating libraries of binding peptides is especially suited for the generation of the above mentioned preferred larger affinity regions. In these a larger number of changes can be introduced while maintaining the same of similar structure. Thus preferably at least one template encodes a specific binding peptide having an affinity region comprising at least 14 amino acids and preferably at least 16 amino acids.

Though non consecutive regions can be used in this embodiment of the invention it is preferred that the region comprises at least 14 consecutive amino acids. When multiple templates are used it is preferred that the regions comprise an average length of 24 amino acids.

Method for generating a library of binding peptides may favourable be combined be combined with core regions of the invention and method for the generation thereof. For instance, once a suitable binding region is selected a core may be designed or selected to accommodate the particular use envisaged.

However, it is also possible to select a particular core region, for reasons of the intended use of the binding peptide. Subsequently libraries having the core and the mentioned library of binding peptides may be generated. Uses of such libraries are of course manifold. Alternatively, combinations of strategies may be used to generate a library of binding peptides having a library of cores.

Complexities of the respective libraries can of course be controlled to adapt the combination library to the particular use. Thus in a preferred embodiment at least one of said templates encodes a proteinaceous molecule according to the invention. The mentioned peptide, core and combination libraries may be used to select proteinaceous molecules of the invention, thus herein is further provided a method comprising providing a potential binding partner for a peptide in said library of artificial peptides and selecting a peptide capable of specifically binding to said binding partner from said library. A selected proteinaceous molecule obtained using said method is of course also provided.

To allow easy recovery and production of selected proteinaceous molecule it is preferred that at least the core and the binding peptide is displayed on a replicative package comprising nucleic acid encoding the displayed core/peptide proteinaceous molecule. Preferably the replicative package comprises a phage, such as used in phage display strategies. Thus also provided is a phage display library comprising at least one proteinaceous molecule of the invention. As mentioned above, the method for generating a library of binding peptides can advantageously be adapted for core regions.

Thus also provided is a method for producing a library comprising artificial cores said method comprising providing at least one nucleic acid template wherein said templates encode different specific cores, producing a collection of nucleic acid derivatives of said templates through mutation thereof and providing said collection or a part thereof to a peptide synthesis system to produce said library of artificial cores. Preferred binding peptides libraries are derived from templates comprising CDR3 regions from cow (Bos Taurus) or camelidae (preferred lama pacos and lama glama).

Protein-ligand interactions are one of the basic principles of life. All protein-ligand mediated interactions in nature either between proteins, proteins and nucleic acids, proteins and sugars or proteins and other types of molecules are mediated through an interface present at the surface of a protein and the molecular nature of the ligand surface. The very most of protein surfaces that are involved in protein-ligand interactions are conserved throughout the life cycle of an organism. Proteins that belong to these classes are for example receptor proteins, enzymes and structural proteins. The interactive surface area for a certain specific ligand is usually constant. However, some protein classes can modulate their nature of the exposed surface area through e.g. mutations, recombinations or other types of natural genetic engineering programs. The reasons for this action is that their ligands or ligand types can vary to a great extend. Proteins that belong to such classes are e.g. antibodies, B-cell receptors and T-cell receptor proteins. Although there is in principle no difference between both classes of proteins, the speed of surface changes for both classes differ. The first class is mainly sensitive to evolutionary forces (lifespan of the species) while the second class is more sensitive to mutational forces (within the lifespan of the organism).

Binding specificity and affinity between receptors and ligands is mediated by an interaction between exposed interfaces of both molecules. Protein surfaces are dominated by the type of amino acids present at that location. The 20 different amino acids common in nature each have their own side chain with their own chemical and physical properties. It is the accumulated effect of all amino acids in a certain exposed surface area that is responsible for the possibility to interact with other molecules. Electrostatic forces, hydrophobicity, H-bridges, covalent coupling and other types of properties determine the type, specificity and strength of binding with ligands.

The most sophisticated class of proteins involved in protein-ligand interactions is those of antibodies. An ingenious system has been evolved that controls the location and level mutations, recombinations and other genetic changes within the genes that can code for such proteins. Genetic changing forces are mainly focussed to these regions that form the exposed surface area of antibodies that are involved in the binding of putative ligands. The enormous numbers of different antibodies that can be formed (theoretically) indicate the power of antibodies. For example: if the number of amino acids that are directly involved in ligand binding in both the light and heavy chains of antibodies are assumed to be 8 amino acids for each chains (and this is certainly not optimistic) then 20^{2*8} which approximates 10²⁰ (20 amino acids types, 2 chains, 8 residues) different antibodies can be formed. If also indirect effects of nearby located amino acids include and/or increase the actual number of direct interaction amino acids, one ends up with an astronomically large number. Not one organism on earth is ever able to test al these or even just a fraction of these combinations in the choice of antibody against the ligand.

Not all amino acids present at the exposed surface area are equally involved in ligand binding. Some amino acids can be changed into other amino acids without any notable- or only minor changes in ligand binding properties. Also, most surface areas of proteins are very flexible and can under the influence of the ligand surface easily remodel resulting in a fit with the ligand surface that would not occur with an inflexible ligand-binding region. Interacting forces as mentioned above between the protein and the ligand can thus steer or catalyze this remodeling. In general, large but limited number of genetic changes together with redundancy in amino acids and the flexible nature of the surface in combination with binding forces can lead to the production of effective ligand binding proteins.

### Affinity regions (AR's)

Natural derived antibodies and their affinity regions have been optimized to certain degree, during immune selection procedures. These selections are based upon the action of such molecules in an immune system. Antibody applications outside immune systems can be hindered due to the nature and limitations of the immune selection criteria. Therefore, industrial, cosmetic, research and other applications demand often different properties of ligand binding proteins. The environment in which the binding molecules may be applied can be very harsh for antibody structures, e.g. extreme pH conditions, salt conditions, odd temperatures, etc. Depending on the application CDRs might or might not be transplanted from natural antibodies on to a scaffold. For at least some application unusual affinity regions will be required. Thus, artificial constructed and carefully selected scaffolds and affinity regions will be required for other applications.

Affinity regions present on artificial scaffolds can be obtained from several origins. First, natural affinity regions can be used. CDRs of cDNAs coding for antibody fragments can be isolated using PCR and inserted into the scaffold at the correct position. The source for such regions can be of immunized or non-immunized animals. Second, fully synthetic AR's can be constructed using degenerated primers. Third, semi-synthetic AR's can be constructed in which only some regions are degenerated. Fourth, triplets coding for selected amino acids (monospecific or mixtures) can be fused together in a predetermined fashion. Fifth, natural derived affinity regions (either from immunized or naive animals) which are being mutated during amplification procedures (e.g. NASBA or PCR) by introducing mutational conditions (e.g. manganese ions) or agents (e.g. dITP) during the reaction.

Because for reasons mentioned earlier, immunization based CDRs can be successful but the majority of ligands or ligand domains will not be immunogenic. Artificial affinity regions in combinations with powerful selection and optimization strategies become more and more important if not inevitable. Primer based strategies are not very powerful due to high levels of stop codons, frameshifts, difficult sequences, too large randomizations, relative small number of mutational spots (maximum of about 8 spots) and short randomization stretches (no more than 8 amino acids). The power of non-natural derived AR's depends also on the percentage of AR's that putatively folds correctly, i.e. being able to be presented on the scaffold without folding problems of the AR's or even the scaffold. Hardly any information is currently available about structures and regions that are present in AR's. Therefore the percentage of correctly folded and presented artificial AR's constructed via randomizations, especially long AR's, will be reciprocal with the length of constructed ARs. Insight in CDR and AR structures will most likely be available in the future, but is not available yet.

Single scaffold proteins which are used in applications that require high affinity and high specificity in general require at least one long affinity region or multiple medium length ARs in order to have sufficient exposed amino acid side chains for ligand interactions. Synthetic constructed highly functional long ARs, using primer or triplet fusion strategies, will not be very efficient for reasons as discussed above. Libraries containing such synthetic ARs would either be too low in functionality or too large to handle. The only available source for long ARs is those that can be obtained from animal sources (most often CDR3s in heavy chains of antibodies). Especially cow-derived and camelidae-derived CDR3 regions of respectively Vh chains and Vhh chains are unusual long. The length of these regions is in average above 13 amino acids but 30 amino acids or even more are no exceptions. Libraries constructed with such ARs obtained from immunized animals can be successful for those ligands or ligand domains that are immunological active. Non-immunogenic ligands or ligand domains and ligands that appear to be otherwise silent in immune responsiveness (toxic, self recognition, etc) will not trigger the immune system to produce ligand specific long CDRs. Therefore, long CDRs that mediate the binding of such targets can not or hardly be obtained this way and thus their exist a vacuum in technologies that provides one with specific long ARs that can be used on single scaffold proteins. A comparable conclusion has also been drawn by Muyldermans (Reviews in Molecular Biotechnology 74 (2001) 277-302) who analyzed the use of synthetic ARs on lama Vhh scaffolds.

Isolation of CDR regions, especially CDR3 regions, by means of PCR enables one to use all length variations and use all structural variations present in the available CDR regions. The introduction of minor, mild, medium level or high level random mutations via nucleic acid amplification techniques like for example PCR will generate new types of affinity regions. The benefits of such AR pools are that length distributions of such generated regions will be conserved. Also, stop codon introductions and frame shifts will be prevented to a large degree due to the relatively low number of mutations if compared with random primers based methods. Further, depending on the mutational percentage, a significant part or even the majority of the products will code for peptide sequences that exhibit structural information identical or at least partly identical to their original template sequence present in the animal. Due to these mutations altered amino acid sequences will be generated by a vast part of the products and consequently these will have novel binding properties. Binding properties can be altered in respect to the original template not only in strength but also in specificity and selectivity. This way libraries of long AR regions can be generated with strongly reduced technical or physical problems as mentioned above if compared with synthetic, semi synthetic and natural obtained ARs.

In recent years several new and powerful in vitro mutagenesis methods and agents have been developed. One branch of mutagenizing methods produces mutations independently of the location (in contract to site directed mutagenesis methods). PCR strategies encompass for example unbalanced concentrations of dNTPs (Cadwell et al., PCR Methods Appl. (1992) 2, 28-33; Leung et al., Technique (1989) 1, 11-15; Kuipers, Methods Mol Biol 57 (1996) 351-356), the addition of dITP (Xu et al., Biotechniques 27 (1999) 1102-1108; Spee et al., Nucleic Acids Res 21 (1993) 777-778; Kuipers, Methods Mol Biol 57 (1996) 351-356), dPTP (Zaccolo et al., J. Mol. Biol. 255 (1996) 589-603), 8-oxo-dG (Zaccolo et al., J. Mol. Biol. 255 (1996) 589-603), Mn2+ (Cadwell et al., PCR Methods Appl. (1992) 2, 28-33; Leung et al., Technique (1989) 1, 11-15, Xu et al., Biotechniques 27 (1999) 1102-1108), polymerases with high misincorporation levels (Mutagene ®, Stratagene).

### AFFINITY MATURATION

After one or more selection rounds, an enriched population of VAPs is formed that recognizes the ligand selected for. In order to obtain better, different or otherwise changed VAPs against the ligand(s), the VAP coding regions or parts thereof can be the subject of a mutational program as described above due to its modular nature. Several stratagies are possible: First, the whole VAP or VAPs can be used as a template. Second, only one or more affinity regions can be mutated. Third framework regions can be mutated. Fourth, fragments throughout the VAP can be used as a template. Of course itterative processes can be applied to change more regions. The average number of mutations can be varied by changing PCR conditions. This way every desired region can be mutated and every desired level of mutation number can be applied independently. After the mutational procedure, the new formed pool of VAPs can be re-screened and re-selected in order to find new and improved VAPs against the ligand(s). The process of maturation can be re-started and reapplied as much rounds as necessary.

The effect of this mutational program is that not only affinity regions 1 and 2 with desired affinities and specificities can be found but also that minor changes in the selected affinity region 3 can be introduced. It has been shown (REF) that mutational programs in this major ligand binding region can strongly increase ligand binding properties. In conclusion, the invention described here is extremely powerful in the maturation phase.

### ii) Selection for affinity against target compound

VAP's can be selected that are specific for exposed ligands on either hair, skin or nails. In case of skin, obvious targets would be proteins or lipid/protein complexes that are present in the stratum corneum, especially in the stratifying squamous keratinizing epithelium where the soft keratins of type I and type II are expressed (The keratinocyte handbook, ed. Leigh IM, Lane B, Watt FM, 1994, ISBN 052143416 5). Other suitable exposed ligands can be KAPs (keratin associated proteins) such as involucrin, loricrin, filagrin, elafin (trappin2), sciellin, cystatin A, annexin 1, LEP/X5, S100 A1-A13, SPRR1 and 2, and the like. Most of these proteins are specific for skin, i.e. they have not (yet) been detected in hair, but if no cross reaction with hair is desired, it is easy for someone skilled in the art to do a negative selection with naive or matured libraries expressing VAPs with different, randomized affinity regions in ways as described in this patent, thereby circumventing any cross-reactivity with hair cuticle.

For selection of hair-specific binders, preferred VAP-targets would be directed against the hair cuticle, especially ligands exposed on the fibre surface, outer beta-layer and epicuticle of hairs. For example the hard keratins or hard keratin intermediate filaments (Langbein L et al. J Biol Chem 274 19874-84, 1999; Langbein L et al. J Biol Chem 276 35123-32, 2001), or the different classes of KAPs which are uniquely expressed in the hair cuticle (e.g. KAP19.4 of the high glycine-tyrosine class, or KAP13.2 and KAP15.1 of the high sulfur class). Interestingly, some KAPs are strongly expressed in scalp hairs but are low to absent in beard hairs (Rogers MA et al. J Biol Chem 276:19440-51, 2001; Rogers MA et al. J Biol Chem 30 sept, 2002). Other potential ligands are lipids stabilized by isopeptide bonds that form part of the hydrophobic outerlayer of the hairs, with methyleicosanoic acid and C16:0 fatty acid are the major lipid components. Due to its poor extractability, the protein composition of hair cuticle is only starting to be unraveled, but for the selection of hair-specific VAPs, it is not necessary to know the actual molecular target. When a truly hair specific ligans needs to be targeted (e.g. when hair-coloring agents as described in this patent are being used), a negative selection with naïve or matured libraries expressing VAPs with different, randomized affinity regions in ways as described in this patent, thereby circumventing any cross-reactivity with skin epidermis can easily be done by someone skilled in the art.

Besides skin or hair-specific VAPs, other affinity targets can be selected for, such as cotton fibers, flax, fibers, hemp fibers, polyester fibers and the like, being all fibers that are used in fabrics for clothes, linen, clothes etc. Especially cellulose fibers are a preferred embodiment of the invention, as these are used in many clothes, sheets, towels, diapers, hygiene pads etc.

### iii Bulk production of VAPs

Direct production of VAP's in transgenic plant seeds followed by minimal seed processing (such as described in US patent 571,474 or via oilbodies as described in US Patents 6,146,645 and 5,948,682) would be an economically attractive source for bulk quantities of bi-valent VAP's but good alternative sources are industrial micro-organisms where cellular protein can be used as carrier protein to introduce the conditioner agent into the consumer product.

### b) Coupling cosmetic agents to VAPs

For the coupling of cosmetic agents to amino acids comprising the VAPs, several strategies can be chosen, using standard coupling chemistry known to those skilled in the art. Ample literature is available for such coupling chemistry, as is illustrated by the Handbook of Molecular Probes (Eugene, OR, USA), Bioconjugate Techniques (GT Hermanson, Associated Press 1996) and references therein where a wide range of fluorescent dyes is coupled to proteins such as antibodies. The functional groups used for chemical binding include, amino groups, carboxyl groups, aldehyde groups, thiol groups, polysaccharide side chains and the like.

Other versatile coupling methods can also be used for coupling of active agents to VAPs. Examples are Kreatech's Universal Linkage System (ULS) as described in patents or patent applications US 5,580,990; EP 0539466; US 5,714,327, US 5,985,566.

At the most distal region from the affinity region (also described as the tail end of the molecule, the C-terminus), additional amino acids can be added that do not alter the tertiary structure of the scaffold, onto which cosmetic agents can be coupled without interference with the scaffold's stability. Also, 3D-structural modelling and analysis can be used to determine which amino acids are exposed from the scaffold as they are the most hydrophilic and which side chains are available for chemical coupling. Preferably, the target amino acids should not be present in the affinity regions, a feature that can be selected for when panning the display libraries and doing sequence analyses on putative binders.

In the case of cosmetic agents that lack organic functional groups, chemical modifications are necessary to able these molecules with binding regions. Treatments using coupling agents and silicone treatments, such as amino-modified silicone (3-aminopropyl triethoxy silane), SH-modified silicone (3-mercaptopropyl triethoxy silane) and carboxyl-modified silicone can be used for the introduction of organic functional groups on the surface of cosmetic agents. In addition, the binding of the cosmetic agents to the protein moiety has to be designed so that the release conditions will not compromise the cosmetic agent's structure. Chemical modifications have to be therefore introduced in order to ensure that the released molecule's physical, chemical and active properties remain unscathed.

Coupling may be achieved through peptide bonds, direct coupling, though chemical bonds or a combination thereof. A preferred example of a chemical bond comprises aldehyde reacting with amines. Aldehydes will react with amines to form Schiff Bases or Imines. These compounds can then be further reduced to further form more stable bonds. Release of the aldehydes upon formation of the Schiff base can be triggered by moderate acid or basic aqueous solution. A summary of these reactions is shown below:

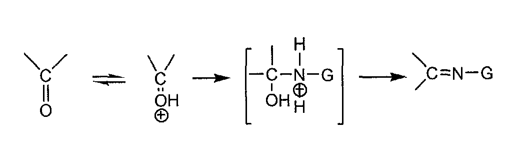

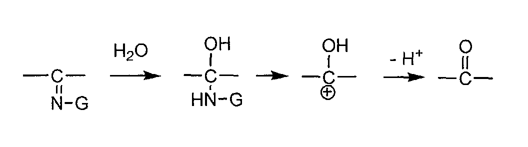

### c) Classes of cosmetic agents

Several classes of cosmetic agents are envisioned in the invention:

### i) Fragrances

One fragrant substance but preferably complexes composed of various molecules known in the classical field of fragrance are tagged to VAPs that can bind to a wide variety of target molecules with e.g. affinity and specificity to skin hair, textiles and tissue type materials. The fragrance molecules can then be released in a timed or conditioned fashion either by normal physiological natural processes such as enzymatic hydrolysis or by specific chemical reactions triggered by conditions brought by formulated products geared towards such conditions.

### ii) Color compounds

A single coloured agent or complexes composed of various molecules known in the classical field of hair dyes are tagged to VAPs that are selected to bind with high specificity and high affinity directly to e.g. the hair surface, circumventing the need for hair dye molecules to penetrate the high and be dimerized under strong oxidative conditions.

### iii) Conditioning compounds

single conditioning compounds and/or complexes composed of various molecules known in the classical field of cosmetic conditioners are tagged to VAPs that are selected to bind with high specificity and high affinity directly to hair, skin or nail surface. The cosmetic agents can be conditioners such as polymeric lubricants, antioxidants, dye fixative agents, conditioners, moisturizers, toners and various other compounds that improve the smell, look, feel, or overall health of the skin, hair or nails.

### d) Fragrance Agents

Fragrances are usually not water-soluble and are applied in solvents such as ethanol or water/solvent mixtures to overcome the hydrophobic nature of the fragrance molecule. Release is temperature dependent and therefore a mixture of fragrances has different odor perceptions over time. A slow release mechanism provides a more controlled volatility and constant perception.

Using VAPs that are charged with various fragrance molecules as delivery agents, one can devise a slow release system based on the skin or hair physiology: sweat, heat, skin and hair natural bacterial flora's exogenous hydrolytic enzymes. In addition, the release of these fragrant molecules from the peptides can also be triggered by the addition of specially formulated products.

Various fragrance structural groups or more molecules of a single group can be attached to these affinity proteins via methods known in the field of organic chemistry. Depending on the fragrance intensity desired, the VAP can be synthetically designed to include side groups, which will optimize the binding of multiple fragrances.

The fragrant molecules that can be attached to these proteins lie in the following chemical classes (with examples, but not limited to these examples):

Acid salts, acetylenes, alcohols, aldehydes, amines, alpha-amino acids, carboxylic acids, esters, acetals, heterocycles, hydrocarbons, ketones, nitriles and cumulated double bonds, sulfides, disulfides and mercaptans and essential oils, Examples are acid salts such as non-aromatic acids salts, sodium acetate, potassium acetate, sodium citrate dihydrate or acetylenes such as 3-pentyn-1-ol, methyl 2-octynoate, methyl 2-nonynoate or alcohols such as 3-isopropylphenol, vanillyl alcohol, , 1-octanol, 3-methyl-3-pentanol, pinacol, 4-hexen-1-ol, isoborneol, decahydro-2-napthol or polyols or aldehydes such as p-tolyacetaldehyde, cinnamaldehyde, 4-ethylbenzaldehyde, isobutyraldehyde, heptanal, 2-methyl-2-pentenal, dihydro-2,4,6-trimethyl-1,3,5(4h)dithiazine or alpha-amino acids such as DL-phenylalanine, DL-isoleucine, DL-methionine or carboxylic acids such as phenylacetic acid, cinnamic acid, propionic acid, isovaleric acid, fumaric acid, levulinic acid or esters, lactones such as benzyl formate, phenethyl formate, alpha-methylbenzyl butyrate, geranyl phenylacetate, ethyl p-anisate, butyl stearate, tripropionin, citronellyl acetate or ethers, acetals such as anisole, isoeugenol, vanillin propylene glycol acetal, caryophyllene oxide or heterocycles such as pyrrole, 2-pentylfuran, furfuryl mercaptan, 3-(2-furyl) acrolein, furfuryl heptanoate or hydrocarbons such as p-cymene, undecane, (r)-(+)-limonene, terpinolene, valencene, beta-caryophyllene or ketones such as 4--methyl-1-phenyl-2-pentanone, vanillylactone, propiophenone, 1-phenyl-1,2-propanedione, 2-decanone or sulfides, disulfides & mercaptans such as 2-phenethyl isothiocyanate, 2-methoxythiophenol, butyl sulfide, isopropyl disulfide, cyclopentanethiol, allyl isothiocyanate. A complete and comprehensive listing of fragrances products can be found in: Arctander's "Perfume and Flavor Chemicals and Perfume and Flavor
Materials of Natural Origin" CD Rom. 1999 Edition.

### i) perfumes, deodorant, shampoos.

Perfumes, deodorants and shampoos may comprise fragrances coupled to VAP directed to skin and or hair components such as keratin and skin bacteria. Release of the fragrance can be achieved through proteolytic cleavage, oxidation or other means.

### ii) laundry detergents, fabric softeners

Laundry detergents and rinsing agents: fragrances coupled to VAPs that are selected for specificity against fabric materials such as cotton fibers, flax, fibers, hemp fibers, polyester fibers and the like, being all fibers that are used in fabrics for clothes, linen, towels etc. Laundry can be treated more effectively with such fragrance-VAP's as there will be very limited release after the rinse, both in wet and dry state of the laundry. Only when the fabric comes in contact with human skin, release of the fragrance molecules is triggered by either skin-or microflora-derived enzymes, or physical changes such as pH.

### iii) Tissues, hygiene pads, diapers

Fragrances coupled to VAPs that are selected for specificity against fabric materials such as cotton fibers, flax, fibers, hemp fibers, polyester fibers and the like, being all fibers that are used in fabrics for tissues, hygiene pads, diapers and the like. Only when the fabric comes in contact with human skin, release of the fragrance molecules is triggered by either skin-or microflora-derived enzymes, or physical changes such as pH, temperature or high moisture conditions.

### e Skin and Nail Conditioning Agents

These conditioning agents can be described as materials, which improve the appearance of dry or damaged skin or nails. Many of these products are designed to remain on the skin (or nails) for a length of time in order to reduce flaking and to act as lubricants. These conditioning agents help maintain the soft, smooth, flexible nature of what is perceived as healthy, young looking skin (or nails).

### i) Occlusive Agents

These agents perform in such a manner that the evaporation of water from the skin surface is substantially blocked. This occlusivity helps to increase the water content of the skin, giving it the desired supple appearance. Typically, occlusive agents are lipids, which, due to their water insolubility provide the best barrier to water vapor transport. The mechanism of skin moisturisation by these lipids is based on their tendency to remain on the skin's surface over time to provide a long lasting occlusive effect. Examples such as naphtenic and isoparaffenic hydrocarbon found in petrolatum are great occlusive agents, which can be delivered and slowly released to the skin by VAPs.

### ii) Humectants

These are conditioning agents that regulate water levels on the skin and hair due to their hygroscopicity (ability to attract and bind water). They have the ability of re-hydrating the skin when delivered from a cream or lotion. These humectants can potentially be tagged onto the side chains of the VAPs to near saturation. By choosing VAPs with very high skin affinity, one can then design permanent organic humectants. Classical humectants include Glycerin, propylene glycol, butylene glycol, 1,3-butylene glycol, polyethylene glycols, sorbitol, sodium pyrrolidone carboxylate, acetamide MEA and many other miscellaneous humectants based on their water-absorbing characteristics (collagens, keratins, glucose esters and ethers etc...)

### iii) Emollients

Emollients are defined as: "agents, which when applied to a dry or inflexible corneum, will affect softening of that tissue by inducing re-hydration". Esters and oils will induce re-hydration by reducing the loss of water in a similar fashion as occlusive agents listed above. Agents such as triglycerides (animal, vegetable and marine oils), lanolin and lanolin derivatives, simple esters, straight chain esters, fatty alcohol component variations, modified chain alcohols, branched chain esters (short branched chain alcohols, branched chain fatty alcohols) and complex esters can be delivered to the skin via VAPs.

### iv) Conditioning Proteins and Amino Acids

### • Enzymes Used as Skin Conditioners

Fusion proteins can be built using VAPs fragments fused to catalytic fragments from various enzymes with application to skin care. Catalytic fragments from enzymes such as: superoxide dismutase (a superoxide radical scavenger, which may thereby function as an anti-inflammatory agent), papain (a protease, an alternative to AHAs as an exfoliant) and various others can now be anchored to the surface of the skin using VAPs properties. Fusion proteins have been previously documented in the literature and other prior arts.

### • Structural Proteins

Natural protein choices for the skin are proteins, which play part in its structural make-up. Such proteins include: collagen and its versions, hydrolyzed elastin etc.

### • Modified VAPs

Quaternized protein hydrolysates have higher isoionic points, enhanced substantivity, and are capable of reducing irritation of anionic surfactants in cleansing formulations. Such examples are polytrimonium gelatin, polytrimonium hydrolyzed collagen etc. By adding polytrimonium groups to the surface side chains of all VAPs, one can reduce the skin irritation to chloroxylenol-based antiseptic cleansers and sodium lauryl sulfate.

By grafting fatty acid residues on primary amino groups of VAPs, these VAPs become film formers with various cosmetic advantages or even transport facilitators across bio-membranes for various active products listed in the document or others.

### • Amino Acids

Collagen peptides are highly hygroscopic and substantive to the skin. The small collagen derived peptide chains can actually be slowly released after tagging such a peptide collagen molecule to a VAP and taking advantage of the proteolytic properties of various enzymes present on the surface of the skin. The slow release of individual collagen amino acids will provide the skin with low molecular weight hydrolysates without the tacky feel of higher molecular weight hydrolysates. Hydrolyzed wheat proteins can also be used as a source of amino acids. Wheat amino acids are of smaller size, allowing penetration of the skin's outer layer, in a manner similar to that observed for the hydrolyzed collagen. In addition to the amino acids mentioned above, individual amino acids have specific effect. Tyrosine and derivatives are used in sun care products because of their involvement in skin coloration processes, synthetic and natural. Glycine derived from gelatin enriched with lysine enhances recovery of skin elasticity and depigmentation of age spots. Acetyl cysteine is an alternative to alpha-hydroxy acids (AHAs) for the removal of dead skin. It can be used to improve skin suppleness and smoothness and to treat acne. All these amino acids can be delivered by VAPs by creating fusion proteins with fragments made out repetitions of these amino acids linked to VAPs. These fragments can be engineered to be susceptible to proteolytic cleavage from endogenous skin enzymes.

### • Organo-Modified Siloxane Polymers

Organo-modified siloxane polymers are derived from chlorosilane monomers via hydrolysis and polymerization and/or polycondensation. They include (poly) dimethylcyclosiloxanes, linear (poly) dimethylsiloxanes, cross linked (poly) dimethylsiloxanes, and other functional siloxanes.

### • Specialty Silicones

These silicones have their organo-functional groups modified to limit their de-foaming properties, increase their solubility, make them less greasy, and decrease their need for emulsification for water-based systems. They include: dimethiconol, dimethycone copolyol, alkyl dimethicone copolyol, trimethylsilylamodimethicone, amodimethicone, dimethicone copolyol amine, silicone quartenium compounds, silicone esters, etc.

### • Cationic Surfactants and Quaternary Derivatives

By general definition, quaternary ammonium salts are "a type of organic compound in which the molecular structure includes a central nitrogen atom joined to four organic groups as well as an acid radical". These quaternary derivatives can be based on fatty acids, proteins, sugars and silicone polymers. They include amines, amphoteric surfactants, amine oxides, amidoamines, alkylamines, alkyl imidazolines, ethoxylated amines, quaternary salts, and others.

It was reported in various previous research findings that quaternized proteins are useful in treating damaged keratinous surfaces (l'Oreal, US Patent 4,796,646). One could therefore genetically engineer a VAP to be quaternized by first adding as many amino acids with NH₃ side chains on the outer surface of the scaffold and/or at the tail end, and then quaternizing these side chains using techniques illustrated in the literature.

### • Polymers as Conditioning Agents

These polymers include cationic and non-cationic polymers. The adsorption of a cationic polymer shows a sharp initial uptake followed by a slow approach to equilibrium. The mechanism appears to involve slow penetration of the skin by the polymer, since the uptake by the polymer far exceeds that of a monolayer. Skin keratin is very reactive to these polymers. Tagging these polymers onto VAPs ensures constant replenishing of the outer skin layer, which is constantly in a state of sloughing.

These polymers include:
- Cationic conditioning polymers such as polyvinylpyrrolidone (PVP), copolymers of PVP (PVP/Vinyl acetate copolymers, PVP-□-olenin copolymers, PVP/Methacrylate copolymers etc.), Dimethyl Diallyl Ammonium Chloride (DDAC) homopolymer (Polyquaternium 6), DDAC/Acrylamide (polyquaternium 7), DDC/Acrylic acid (polyquaternium 22), polymethacrylamideopropyl trimonium chloride, acrylamide/□-methacryloxyethyltrimethyl ammonium methosulfate (polyquaternium 5), adipic acid/dimethylaminohydroxypropyl/diethylenetriamine copolymer, vinyl aldohol hydroxypropyl amine (polyquaternium 19), quaternized polyvinyl octadecyl ether (polyquaternium 20), quaternized ionenes (polyquaterniums 2, 17, 18, 27), polyquaternium 8, Cellulose cationic polymers (such as hydroxypropyl trimethyl ammonium chloride ether of hydroxyethyl cellulose (polyquaternium 10), polyquaternium 24, hydroxyethylcellulose/diallyldimethyl ammonium chloride (polyquaternium 4), alkyl dimonium hydroxypropyl oxyethyl cellulose), polyquaternium 46, cationic polysaccharides such as guar hydroxypropyl trimonium chloride, quaternized lanolin (quaternium 33), quaternized CHITOSAN (polyquaternium 29)
- Quaternized Proteins can also be designed or constructed to be included as additional fragments linked to the VAP. Such proteins can include quaternized collagens, quaternized keratin, quaternized vegetable proteins, quaternized wheat protein, quaternized soy proteins, hydrogenated soy proteins
- Aminosilicones can also be used for skin cleansing preparations since they interact with skin proteins to provide a re-fatting effect, imparting a smooth and supple feel to the cleansed skin.

### • Anti-Ageing proteins and vitamins

Whey protein was found to activate cytokines (immunological regulators, signaling and controlling molecules in cell-regulating pathways). Synthesis of a fusion protein composed of the activating portion of the whey protein along with the VAP will improve skin firmness, touch, smoothness and will increase skin elasticity and thickness. Anti-ageing vitamins such as Vitamin D, vitamin A (retinol) or other vitamins tagged to VAP's.

VAPs can present a stable and long-lasting alternative for the delivery of beneficial enzymes and more specifically catalytic portions of these enzymes. Fusion proteins can therefore be synthesized which will have affinity for skin and at the same time contain the catalytic portion of the beneficial enzyme. The intentional delivery of these active principles (usually in the form of a catalytic portion of an enzyme, hormone or specific peptide as well as many other materials) to living cells for cosmetic purposes, with the aim of providing noticeably "improved" skin texture and topography will be included in this patent. As an illustration, VAP linked to a catalytic fragment of serine protease would be a method to deliver lastingly an agent that will provide skin-smoothing effects.

### f Hair Conditioning Agents

### i) hair "repair" agents

Hair damage results from both mechanical and chemical trauma that alters any of the physical structures of the hair. Conditioning agents cannot enhance repair, since repair does not occur, but can temporarily increase the cosmetic value and functioning of the hair shaft until removal of the conditioner occurs with cleansing. VAPs can provide a slow release mechanism and a vehicle for the delivery of these conditioning agents while enhancing their resistance to washing. Potentially, these conditioners can now resist normal shampooing conditions thanks to these VAPs and hence, provide a constant stable conditioning effect to the hair cuticle onto which they are anchored.

These conditioning agents, which are tagged to the VAPs, cannot only include chemicals listed above but they also may fall in the categories listed below.
- Immobilized enzyme-VAPs fusion proteins applied to damage hair to remove frayed cuticle, thereby enhancing shine, much as Cellulases are used in prior art to remove fuzz from cotton clothing during laundering and thereby brightening colors.
- Modified amino acids on the VAPs targeted to keratin capable to convert cystic acid residues to moieties capable of forming disulfide bonds, thereby allowing strengthening of hair in a much gentler fashion than current methods
- Treatment for greasy hair (seborrheic conditions): the cause for such conditions is usually the disintegration of sebaceous cells (holocrine secretion). This holocrine secretion will subsequently release a sterile mixture of lipids (triglycerides (60%), wax esters of fatty acids and long chain fatty alcohols (20 - 25%); and squalene (15%)) into the pilosebaceous duct, already containing protein and lipids and inhabited by normal skin flora. This population is mainly engaged in enzymatic activity, directly toward the triglycerides releasing fatty acids. Once excreted onto the surface, the modified sebum will mix with another of lipid emanating from epidermal cells, free cholesterol, cholesterol esters, glycerides etc.
   - VAPs coupled with enzymatic fragments from variety of enzymes involved in metabolism of fatty acids (notably saturated chain fatty acids) such as acyl CoA synthase etc. could present a cure to this problem
   - Other methods involve using sulfur containing amino acids and thioethers linked to VAPs or VAPs engineered to contain high proportions of these amino acids (S-carboxymethyl, S-benzhydryl, S-trityl cysteine, 4-thiazoline carboxylic acid, N-acetyl homocysteine thiolactone, thioethers derived from cysteamine, glutathione and pyridoxine, tiolanediol and oxidation derivatives etc.
   - Substances retarding sebum recovery: this method involves the design of VAPs geared towards the slow down of sebum's uptake by hair by depositing an oleophilic film on the surface of the cuticle. By designing a very hydrophobic and lipophobic VAP, one can retard the sebum transfer from scalp to hair.
   - VAPs designed to be grease absorbers. These VAPs will mimic properties of gelatin or casein to absorb sebum and give it a more waxy consistency in order to make the seborrheic state less obvious.
- Design of VAPs to treat dry hair caused by trauma from over-vigorous mechanical or chemical treatments. A further factor in the frailty of hair is weathering from the cumulative effect of climatic exposure, namely sunlight, air pollutants, wind, seawater and spindrift or chlorinated water. These physiochemical changes can be defined and may be measured by a loosening of cuticle scales and increase in the friction coefficient, increase in porosity, a tendency for the hair to break more easily due disruption of salt and cysteine linkages, hydrogen bonds, sulfur content and degradation in polypeptide chains leading to the elimination of oligoproteins. VAPs will help treat dry hair by providing a vehicle for a timely release of amino acids and other microelements it has lost and in essence, restoring its biochemical balance. VAPs linked fatty elements will be a logical step in combating hair dryness. These peptides will be linked with compounds such as:
   - Fatty acids
   - Fatty alcohols
   - Natural triglycerides
   - Natural waxes
   - Fatty esters
   - Oxyethylenated or oxypropylenated derivatives of waxes, alcohol, and fatty acids
   - Partially saturated fatty alcohols
   - Lanolin and its derivatives
   Other agents are listed and will help restore hair to its original biochemical structure.

In general, all conditioning agents for all different forms of keratin can be tagged to this VAP carrier. The conditioning agents can be chosen from the following:
- Amino acids (e.g. cysteine, lysine, alanine, N-phenylalanine, arginine, Glycine, leucine, etc.), oligopeptides, peptides, hydrolyzed or not (modified or unmodified silk or wool hydrolyzed proteins, hydrolyzed wheat proteins etc.), modified or unmodified
- Fatty acids (carboxylic acids from C₈ - C₃₀, such as palmitic, oleic, linoleic, myristic, stearic, lauric, etc. and their mixture) or fatty alcohols, branched or unbranched (C₈ - C₃₀ fatty alcohols such as: palmitic, myristic, stearic, lauric etc. and their mixture)
- Vegetable, animal or mineral fats and their mixture
- Ceramides (e.g. Class I, II, III, and IV according to Downing's classification such as: 2-N-linoleoylamino-octadecane-1,3-diol, 2-N-oleoylamino-octadecane-1,3-diol, 2-N-palmitoylaminooctadecane-1,3-diol, 2-N-stearoylamino-octadecane-1,3-diol, 2-N-behenylamino-octadecane-1,3-diol etc.) and their mixture and the pseudo-ceramides and their mixture
- Hydroxylated organic acids (e.g. citric acid, lactic acid, tartaric acid, malic acid and their mixture)
- UV filters (e.g. UV-A and/or UV-B known for a person trained in the art. They can include derivatives of dibenzoylmethane, p-amino benzoic acid and its esters, salicylic acid salts and their derivatives, Cinnamic acid esters, benzotriazole derivatives, triazine derivatives, derivatives of □□□'-diphenylacrylate 2-phenylbenzimidazole-5-sulfonic acid and its salts, derivatives of benzophenone, derivatives of benzylidene-camphor, silicone filters etc. and their mixtures)
- Anti-oxidants and free radicals scavengers (such as ascorbic acid, ascorbyl dipalmitate, t-butylhydroquinone, polyphenols such as phloroglucinol, sodium sulfite, erythorbic acid, flavonoids, and their mixture)
- Chelating agents (EDTA and its salts, di-potassium EDTA, phosphate compounds such sodium metaphosphate, sodium hexametaphosphate, tetra potassium pyrophosphate, phosphonic acids and their salts, etc. and their mixtures)
- Regulating agents for seborrhea: succinylchitosane, poly-□-alanine, etc. and their mixture
- Anti-dandruff agents: the invention can include many agents from the following compounds: benzethonium chloride, banzalkonium chloride, chlorexidine, chloramines T, chloramines B, 1,3-dibromo-5,5dimethylhydantoine, 1,3-dichloro-5,5,-dimethylhydantoine, 3-bromo 1chloro 5,5-dimethylhydantoine, N-chlorosuccinimide, derivatives of 1-hydroxy-2-pyridone, trihalohenocarbamides, triclosan, azole derivatives, antifungal derivatives such as amphotericine B or nystatine, sulfur derivatives of selenium, sulfur in its different forms, physiologically tolerated acid salts such as nitric, thiocyanic, phosphoric, acetic etc. and various other agents and their mixture)
- Cationic tenso-cosmetic agents: primary, secondary, tertiary fatty amine (polyoxyalkylanated) salts, quaternary ammonium salts, imidazoline salts, cationic amino oxides, etc. and their mixture
- Amphoteric polymers
- Organically or non-organically modified silicones
- Mineral, vegetable or animal oils
- Esters
- Poly-isobutenes and poly-(□-olefin)
- Anionic polymers
- Non-ionic polymers

These compounds can be attached to the VAP individually or mixed together.

### ii) Hair perming agents

One preferred example of the invention is a non-aggressive perming agent that is formed by a bi-valent VAP that has specificity for hair surface proteins, thus where a hair-specific VAP is in fact the delivery agent for a second VAP. The bi-valency would result in cross linking activity and gives the hair a permanent wave look and and more substance feel or, when flexible spacers are used to make the VAP a bi-valent molecule, provide a more gelling agent feel. Many modern hair shampoos, conditioners or other forms of hair treatments already contain 0.5-3% proteinaceous material or protein hydrolysates of natural origin such as from plants for the purpose of hair protection, providing free amino acids and substance. Bi-valent VAP's with hair surface specificity would not have a tendency to be rinsed readily off such as the non-specific proteinaceous materials.

### g Hair Coloring Agents

Hair dye products come in three classes; permanent, semi-permanent and temporary dyes. The latter can be rinsed out instantly. The permanent dyes can be sub-divided into
1) oxidation hair dye products and
2) progressive hair dyes.
Oxidation hair dye products consist of dye intermediates and a solution of hydrogen peroxide. An example of dye intermediates is p-phenylenediamine which form hair dyes on chemical reaction. 2-nitro-p-phenylenediamine is another type of dye intermediates. They are already dyes and are added to achieve the intended shades. The dye intermediates and the hydrogen peroxide solution, often called the developer, are mixed shortly before application to the hair. The applied mixture causes the hair to swell and the dye intermediates penetrate the hair shaft to some extent before they have fully reacted with each other and the hydrogen peroxide in an oxidative condensation reaction and thus forming the hair dye. The necessary high pH (9-10) is usually achieved through the addition of ammonia.

The active ingredient for progressive hair dye products is lead acetate. The most noticeable difference between oxidation and progressive hair dyes is that progressive dyes are intended to give a more gradual change in hair color. In general, the permanent hair dyes are sensitive to UV light from which they are shielded by the keratin hair shaft. They have a quite limited spectrum of color options and gradually loose their intensity after the hair dye process. The molecules bleach and leak out of the hair during subsequent washings.

The semi-permanent dyes are more complex benzene derivatives that are weakly bound directly to the hair surface and usually administered via coal-tar carriers. The size exclusion of the hair shaft prevents deeper binding sites inside the hair. The colors that can be formed with the semi-permanent dyes cover a wider spectrum and some have more intense primary color characteristics, they are less sensitive to UV light as the permanent dyes. A major disadvantage of the semi-permanent dyes is that the binding to the hair surface is relatively weak and can be rinsed out more easily than the permanent hair dyes.

The coloring substances used in the invention included water-soluble dyes (light green SF yellow, patent blue NA, naphthol Green B, Eosine YS and the like) and water-insoluble colorants such as lakes (naphthol blue black-aluminium salt, alizurol-aluminium salt and the like), organic pigments (brilliant fast scarlet, permanent red F5R, lithol red, deep maroon, permanent red or orange, bezidine yellow G and the like) and natural coloring matter (capsanthin, chlorophyll, riboflavin, shisonin, brazilin and the like), in addition to titanium oxides, iron oxides and magnetic particles. They can also be fluorescent, phosphorescent or luminescent dyes. Fairly complete listings of hair coloring substances can be found e.g. in US patent 5,597,386, but the present invention is not limited to the currently known coloring substances. There are a number of reasons why, different or less frequently used color substances can now be applied in a more favorable way for hair coloring:
a) when low molecular weight water-insoluble dyes are coupled to the very hydrophilic VAPs, the water-solubility of the complex may effectively make the coloring substance now water-soluble.
b) the more UV-stable semi-permanent dyes are preferred for surface colorations, thus providing the option to use the wider color gamma of semi-permanent dyes or even the temporary dyes in a now more permanent application.

Dye mixtures can be used when coupling to VAPs when common coupling procedures can be used, or the VAPs/dye complexes can be mixed to achieve required shades. Binding affinity strength provides an additonal way to control the performance of the dye treatment over time.

Other advantages of this invention for hair coloring products include;
- the whole hair-dying process can now be performed under much more benign chemical conditions as there is no need for dimerization inside the hair shaft using oxidative treatments
- In comparison with the permanent dye procedures, there will be no need for the expensive additives that are used to create the strong oxidative conditions
- the binding will be more permanent as a result of the relatively strong affinity body to ligand binding characteristics
- specific shampoos can be developed easily to remove the dye from the hairs again; analogous to methods that dissociate the affinity body to ligand binding that are well known from affinity chromatography procedures, such as a shift in pH or high salt treatments, again relatively benign chemical treatments that are non-damaging to the hair structure. This part of the invention is further described in the selection procedures for hair-specific VAPs selections.

Due to the relative small size of VAPs compared to antibodies, favorable ratios of VAP/dye substance can be obtained, providing both sufficient binding activity and color effect. The color intensity per unit VAPs will depend on the particular dye, background hair color etc. but the coupling of dyes to VAPs is flexible and allows a wide range of ratios. Also molecular modifications of the VAP can be used to increase the number of dye labelling sites; also pre-labelled peptides or other polymeric strands can be bound to VAPs. Furthermore, as the VAPs-mediated hair dyes as described in the present invention have a high and specific affinity for hair, the actual hair coloring process is much more efficient than with conventional hair dyes, where a substantial amount of dye material is lost directly with the first rinse. Also, with conventional dyes, concentration of the coloring compounds can become so high that they cause skin irritation or skin coloring or, in order to prevent these effects, dye concentrations are so low that repeated treatment is necessary before the required hair shade is reached. A much more precise treatment effect can be obtained with dye-charged VAPs. The coloring substances can be tagged on the VAP using functional groups on the macro-carrier such as amino groups, carboxyl groups, aldehyde groups, hydroxyl groups, thiol groups and the like. In manufacturing the aforementioned Dye-VAP molecule, ratios of the VAP to the coloring substances can be changed so that their ratios can be adjusted to obtain desired proportions of their components. Depending on the color intensity desired, the VAP can be synthetically designed to include side groups, which will optimize the binding of the dyes. The weight ratio of the coloring substance to VAP will therefore be dependent on the final color intensity desired and the artificially designed chemical nature of the VAP side chains.

### h VAP-loaded on encapsulation devices as skin or hair conditioning agents

Besides direct application of VAP's coupled with fragrances, colours conditioning agents and the like, the VAP's can easily be applied as an intricate part of a vesicle. The use of vesicles is widely known in the art and may include but are not limited to liposomes, oilbodies, polyethylene glycol micelles, sodium acrylates co-polymers in caprylic/capric triglyceride and water (e.g. Luvigel, BASF, Germany), nanoparticles (a phospholipid monolayer with a hydrophobic center), starch (nano)particles (WO 0069916, WO 0040617) etc. Vesicle-enhanced formulations can offer protein stabilization, prevention of oxidation, increased solubilization of normally recalcitrant compounds and targeted delivery of active ingredients. When charging such vesicles with VAP's, e.g. by addition of a hydrophobic tail region on either the carboxy- or the amino terminus of the VAP and mixing purified VAP's with said vesicles, the vesicles themselves become multivalent and can be used as improved delivery agents for hair and skin applications. Examples of such non-polar hydrophobic tails are known in the art, such as a polyleucine.

### i Release of cosmetic agents from VAPs

Release of the cosmetic agents from the VAPs moieties can either be dependent on a secondary formulations which will trigger conditions ideal for decoupling of the agents or on inherent skin and hair physiological conditions i.e. increase in temperature and decrease in pH through exercise, natural skin fauna secretions or even endogenous skin enzymes. Again, bonding of the peptide to the cosmetic agents should also be optimized for such conditional releases via chemical modification of both peptide VAPs and cosmetic agents.

In a healthy person, the internal tissues, e.g. blood, brain, muscle, etc., are normally free of microorganisms. On the other hand, the surface tissues, e.g. skin and mucous membranes, are constantly in contact with environmental organisms and become readily colonized by certain microbial species. The mixture of organisms regularly found at any anatomical site is referred to as the normal flora.

### i) skin enzymes

The enzymes accumulated in the stratum granulosum and in lamellar granules involved in protein cleavage and/or activation (e.g. profilagrin, involucrin) are profilagrin endopeptidase (Resing KA et al. Biochemistry 32:10036-9, 1993), transglutaminase family members (Akiyama M et al. Br J Dermatol 146:968-76, 2002), cathespin B, C, D, H and L (Tanabe H et al. Biochim Biophys Acta 1094:281-7, 1991; Horikoshi T et al. Br J Dermatol 141:453-9, 1999; Tobin DJ et al. Am J Pathol 160:1807-21, 2002), proprotein convertases (PC) furin, PACE4, PC5/6 and PC7/8 (Pearton DJ et al. Exp.Dermatol 10:193-203, 2001), a chymotrypsin-like enzyme (Egelrud T. Acta Dermatol venerol Supp 208:44-45, 2000), two trypsin-like serine proteinases (Simon M et al. J Biol Chem 276:20292-99, 2001)), and stratum corneum thiol protease (Watkinson A. Arch Dermatol Res 291:260-8, 1999). The hydrolytic enzymes released from the lamellar granules into the intercellular space comprise acid phosphatase, acid lipase, sphingomyelinase, glucosidase and phospholipase A (Grayson S et al. J Invest Dermatol 85:289-94, 1985; Freinkel RK et al. J Invest Dermatol 85:295-8, 1985). Several enzymes are present on the outer surface of the skin, probably involved in maintenance activities for the Stratum Corneum (SC); permeability barrier homeostasis, extracellular lipid processing, SC integrity and cohesion, desquamation and the like. Examples are the serine protease kallikrein, lysozyme (Ric Clin Lab 1978, 8(4):211-31) or the newly discovered phospholipase (Br J Dermat 2000, 142(3): 424-31, BBRC 2002, 295(2):362-9. The localization and the concentration of these enzymes vary greatly in different regions of the body. These enzymes encompass groups such as dehydrogenases, acid phosphatases, esterases, peptidases, phosphorylases and lipases amongst others (Stevens et al. Int J Dermatology ,1980 Vol. 19 No. 6 p 295) and therefor diverse release mechanisms can be applied when cosmetic substances are delivered via VAPs. These enzymes can also be used to deliver a skin benefit via the interaction of the VAP-benefit agent with the enzyme. These VAP attached to the cosmetic agents thus become enzyme-linked benefits with inherent slow release mechanisms.

Furthermore, endogenous hair fiber enzymes have not only been shown to be present, but also to be biologically active. Maturation of hair fiber results in the death of its constituent cells (Tamada *et al*. (1994) Br. J. Dermatol. 131) and this coupled with the increased levels of intracellular cross-linking results in a mature fiber, which is metabolically dead. Unexpectedly, the authors have found that enzyme activity is in fact preserved, rather than denatured, during the process of cellular keratinization and death that occur during fiber growth. Examples of active enzymes identified to date within the mature human fiber include transglutaminase, protease, lipase, steroid sulphatase, catalase and esterase. Ingredients suitable for use as benefiting agents for targeting hair fiber enzymes are any VAP bound to the cosmetic agents and capable of specifically interacting with the enzyme. The bond, which links cosmetic agent to VAP must ideally be recognized by the enzyme as a substrate.

### ii) microflora-derived enzymes

The normal flora of humans is exceedingly complex and consists of more than 400 species of bacteria and fungi. The makeup of the normal flora depends upon various factors, including genetics, age, sex, stress, nutrition and diet of the individual. The normal flora of humans consists of a few eukaryotic fungi and protists, and some methanogenic Archaea that colonize the lower intestinal tract, but the Bacteria are the most numerous and obvious microbial components of the normal flora, mostly present on the skin surface. Bacteria common on skin surface include Staphylococcus epidermis, Staphylococcus aureus, Streptococcus pyogenes, Corynebacterium diphtheriae, Micrococcus luteus and Propionibacterium acnes. Examples of fungi growing on human skin are Malassezia furfur, Pityriasis versicolor, Malassezia folliculitis, Candida albicans, Trycophyton, Microsporum and Epidermophyton. The adult human is covered with approximately 2 square meters of skin. The density and composition of the normal flora of the skin vary with anatomical locale. The high moisture content of the axilla, groin, and areas between the toes supports the activity and growth of relatively high densities of bacterial cells, but the density of bacterial populations at most other sites is fairly low, generally in 100s or 1000s per square cm. Qualitatively, the bacteria on the skin near any body orifice may be similar to those in the orifice. The majority of skin microorganisms are found in the most superficial layers of the epidermis and the upper parts of the hair follicles. These are generally nonpathogenic and considered to be commensal, although mutualistic and parasitic roles have been assigned to them. Sometimes potentially pathogenic Staphylococcus aureus is found on the face and hands, particularly in individuals who are nasal carriers. Nails are common host tissues for fungi such as Aspergillus, Penicillium, Cladosporium, Mucor. The bacteria and other components of the natural skin flora are known to secrete a battery of hydrolytic enzymes as a mechanism of defense against pathogens and antagonistic bacteria. These hydrolytic enzymes can be used to slowly release the cosmetic agents herein mentioned.

### iii) physical release conditions

Non-enzymatic conditions for release of fragrances can be induced by pH-changes such as a pH-decrease on the skin resulting from sweat, both from the eccrine glands and the apocrine glands (Exog. Dermatol. 2002, 1:163-175).

### EXAMPLES

### Example 1

### Determination of core coordinates

Immunoglobulin-like (ig-like) folds are very common throughout nature.many proteins, especially in the animal kingdom, have a fold region within the protein that belongs to this class. Reviewing the function of the proteins that contain an ig-like fold and reviewing the function of this ig-like fold within that specific protein, it is apparent thatmost of these domains, if not all, are involved in ligand binding. Some examples of ig-like fold containing proteins are: V-CAM, immunoglobulin heavy chain variable domains, immunoglobulin light chain variable domains, constant regions of immunglobulines, T-cell receptors, fibronectin, reovirus coat protein, beta-galactosidase, integrins, EPO-receptor, CD58, ribulose carboxylase, desulphoferrodoxine, superoxide likes, biotin decarboxylase and P53 core DNA binding protein. A classification ofmost ig-like folds can be obtained from the SCOP database (Murzin A. G et al, 1995; http://scop.mrc-lmb.cam.ac.uk/scop) and from CATH (Orengo et al, 1997; http://www.biochem.ucl.ac.uk/bsm/cath_new/index.html). SCOP classifies these folds as: all beta proteins, with an immunoglobulin-like beta-sandwich in which the sandwich contains 7 strands in 2 sheets although somemembers that contain the fold have additional strands. CATH classifies these folds as:mainly beta proteins with an architecture like a sandwich in an immunoglobulin-like fold designated with code 2.60.40. In structure database like CE (Shindyalov et al.1998; http://cl.sdsc.edu/ce.htm), VAST (Gibrat et al.,1996; http://www.ncbi.nlm.nih.gov/Structure/VAST/vast.shtml) and FSSP (Holm et al, 1998; http://www.ebi.ac.uk/dali/fssp) similar classifications are used.

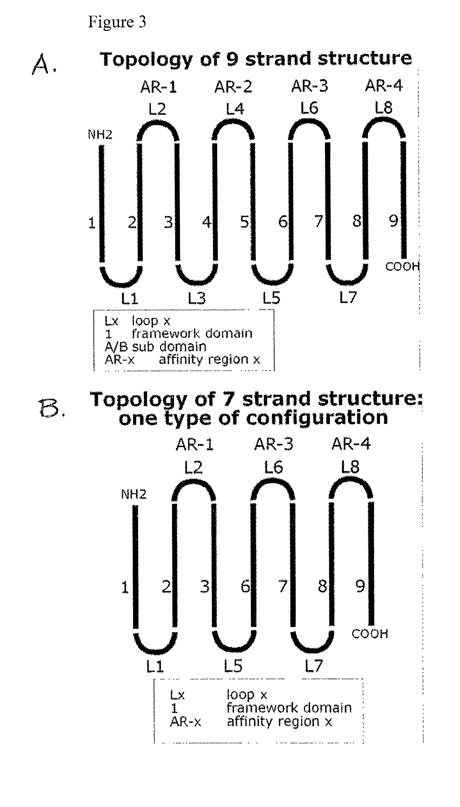

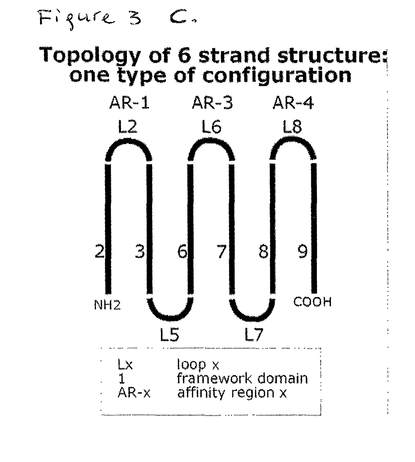

Projection of these folds from different proteins using software of Cn3D (NCBI; http://www.ncbi.nlm.nih.gov/Structure/CN3D/cn3d.shtml), InsightII (MSI; http://www.accelrys.com/insight) and other structure viewers, showed that the ig-like folds have different sub-domains. A schematic projection of the structure is depicted in figure 3A. Themost conserved structure was observed in the centre of the folds, named the core. The core structures hardly vary in length and have a relative conserved spatial constrain, but they were found to vary to a large degree in both sequence and amino acid composition. On both sides of the core, extremely variable sub-domains were present that are called connecting loops. These connecting loops can vary in amino acid content, sequence, length and configuration. The core structure is therefore designated as the farmost important domain within these proteins. The number of beta-elements that form core can vary between 7 and 9 although 6 stranded core structuresmight also be of importance. The beta-elements are all arranged in two beta-sheets. Each beta-sheet is build of anti-parallel beta-element orientations. Theminimum number of beta-elements in one beta-sheet that was observed was 3 elements. Themaximum number of beta-element in one sheet that was observed was 5 elements. Higher number of beta-elementsmight be possible. Connecting loops connect the beta-elements on one side of the barrel. Some connections cross the beta-sheets while others connect beta-elements that are located within one beta-sheet. Especially the loops that are indicated as L2, L4, L6 and L8 are used in nature for ligand binding. The high variety in length, structure, sequences and amino acid compositions of the L1, L3, L5 and L7 loops clearly indicates that these loops can also be used for ligand binding, at least in an artificial format.

Amino acid side chains in the beta-elements that form the actual core that are projected towards the interior of the core and thus fill the space in the centre of the core, can interact with each other via H-bonds, covalent bonds (cysteine bridges) and other forces, to stabilize the fold. Because amino acid composition and sequence of the residues of the beta-element parts that line up the interior were found to be extremely variable it was concluded thatmany other formats and can also be created.

In order to obtain the basic concept of the structure as a starting point for the design of new types of proteins containing this ig-like fold, projections of domains that contain ig-like folds were used. Insight II, Cn3D andmodeller programs were used to determine theminimal elements and lengths. In addition, as amino acid identities were determined as not of any importance, only C-alpha atoms of the structures were projected because these described theminimal features of the folds.minor differences in spatial positions (coordinates) of these beta elements were allowed. Four examples of such structures containing 9 beta elements were determined and converted into PDB formats (coordinate descriptions; see table 1) but many minor differences within the structure were also assumed to be of importance, as long as the fold according to the definitions of an ig-like fold (see e.g. CATH and SCOP).

These PDB files representing the coordinates of the C-alpha atoms of the core of ig-like folds were used for the development of new 9, 8, 7 and 6 beta-elements containing structures. For 8 stranded structures beta element 1 or 9 can be omitted but also elements 4 or 5 can be omitted. For 7 stranded structures, beta elements 1 and 9 were removed or, preferably, elements 4 and 5 were omitted. The exclusion of elements 4 and 5 is preferred because of spatial constrains (figure 3B). Six stranded structures lack preferably element 1, 4 and 5 or 4, 5 and 9 but also other formats were analyzed with Insight and modeller and shown to be reliable enough for engineering purposes (figure 3C).

### Example 2

### Design of 9 strands folds

Protein folding depends on interaction between amino acid backbone atoms and atoms present in the side chains of amino acids. Beta sheets depend on both types of interactions while interactions between two beta sheets, for example in the abovementioned structures, aremainlymediated via amino acid side chain interactions of opposing residues. Spatial constrains, physical and chemical properties of amino acid side chains limit the possibilities for specific structures and folds and thus the types of amino acids that can be used at a certain location in a fold or structure. To obtain amino acid sequences that meet the spatial constrains and properties that fit with the 3D structure of the above described structures (example 1), 3D analysis software (Modeller, Prosa, InsigthII, What if and Procheck) was used. Current computer calculation powers and limitedmodel accuracy and algorithm reliabilities limit the number of residues and putative structures that can be calculated and assessed.

To obtain an amino acid sequence that can form a 9 beta strand folds as described above, different levels of testing are required, starting with a C-alpha backbone trace as described in for example PDB file 1. First the interior of the fold needs to be designed and tested. Secondly, beta-element connecting loops need to be attached and calculated. Thirdly exterior amino acids, i.e. amino acids that expose their amino acid side chains to the environment, need to fit in without disturbing the obtained putative fold. In addition, the exterior amino acid side chains should preferably result in a soluble product. In the fourth and last phase the totalmodel is recalculated for accidentally introduced spatial conflicts. Amino acid residues that provoked incompatibilities are exchanged by an amino acid that exhibits amore accurate and reliable fit.

In the first phase, amino acid sequences aligning the interior of correctly folded double beta-sheet structures thatmeet criteria as described above and also in example 1, were obtained by submitting PDB files for structural alignments in e.g. VAST (http://www.ncbi.nlm.nih.gov/Structure/VAST/vast.shtml). The submission of the PDB files as depicted in PDB file 1 already resulted in thousands of hits. The majority of these proteins were proteins that contained at least one domain that would be classified according to SCOP or CATH (see above) as foldsmeant here.

Several unique sequences aligning the interior of the submitted structure were used for the generation of product examples. Interesting sequences from this structural alignment experiment were selected on criteria of classification, rootmean square deviations (RMSD-value), VAST-score values (higher values representmore accurate fit), sequence identities, origin of species and proposed biological function of the hits. Structures as fibronectin-like protein, antibody related proteins, cell adhesionmolecules, virus core proteins, andmany others. The structures that are represented by the C-alpha backbones are called the core structures.

In the second phase, loops were attached to obtained products. Although several analysismethods can be applied that resolve the structure of the end products, themost challenging feature would be the presentation of affinity regions on core sequences that have full functional ligand binding properties. In order to test the functionality of the end products, affinity loops that recognize known ligands can be transplanted on the core structure. Because anti-chicken lysozyme (structure known as 1MEL) is well documented, and the features of these affinity regions (called CDR's in antibodies) are well described, these loops were inserted at the correct position on core sequences obtained via themethod described in the first phase. Correct positions were determined via structural alignments, i.e. overlap projections of the already obtained folds with the file that describes the 3D structure of 1MEL (PDB file; example). Similar projections and subsequent loop transplantations were carried out for the bovine RNase A binding affinity region that were extracted from the structure described by 1BZQ (PDB). The transplanted affinity loops connect one end of the beta elements with one other. Affinity region 1 connects beta-element 2 with 3 (L2), AR2 connects beta element 4 and 5 (L4), AR3 connects beta elements 6 and 7 (L6) and AR4 connects beta elements 8 and 9 (L8). The other end of each of the beta elements was connected by loops that connect element 1 with 2 (L1), 3 with 4 (L3), 5 with 6 (L5) and 7 with 8 (L7) respectively (see schematic projection in figure 3A). Of course all kinds of loops can be used to connect the beta elements. Sources of loop sequences and loop lengths encompass for example loops obtained via loopmodeling (software) and from available data from natural occurring loops that have been described in the indicated classes of for example SCOP and CATH. C-alpha backbones of loops representing loops 1 (L1), 3 (L3), 5 (L5) and 7 (L7; figure 3A) were selected from structures like for example 1NEU, 1EPF-B, 1QHP-A, 1CWV-A, 1EJ6-A, 1E50-C, 1MEL, 1BZQ and 1F2X, but many others could have been used with similar results. 3D-aligments of the core structures obtained in the first phase as described above, together with loop positions obtained from structural information that is present in the PDB files of the example structures 1EPF, 1NEU, 1CWV, 1F2X, 1QHP, 1E50 and 1EJ6 were realized using powerful computers and Cn3D,modeller and/or Insight software of. Corresponding loops were inserted at the correct position in the first phasemodels. Loops did not have to fit exactly on to the core because a certain degree of energy and/or spatial freedom can be present. The type of amino acids that actually will form the loops and the position of these amino acids within the loop determine this energy freedom of the loops. Loops from different sources can be used to shuffle loop regions. This feature enables new features in the future protein because different loops have different properties, like physical, chemical, expressional, post translationalmodifications, etc. Similarly, structures that contain less loops due to reduced numbers of beta elements can be converted into proteins with 9 beta elements and a compatible number of loops. Here it is demonstrated that the C-alpha trace backbones of the loops of 7 stranded proteins like for example 1EPF, 1QHP, 1E50 and 1CWV could be used as templates for 9 stranded core templates. The additional loop (L3) was in this case retrieved from the 9 stranded template 1F2X but any other loops that were reliable according to assessment analysis could also have been used. The nature of the amino acids side chains that are pointing to the interior of the protein structure was restricted and thus determined by spatial constrains. Therefore several but limited configurations were possible according to 3D-structure projections using themodeling software.

In the third phase, all possible identities of amino acid side chains that are exposed to the exterior, i.e. side chains that stick out of the structure into the environment, were calculated for each location individually. Formost applications it is preferred to use proteins that are very good soluble and therefore amino acids were chosen that are non-hydrophobic. Such amino acids are for example D, E, N, Q, R, S and T.methionine was preferably omitted because the codon belonging tomethionine (ATG) can results in alternative proteins products due to aberrant translational starts. Also, cysteine residues were omitted because free cysteines can lead to cysteine-cysteine bonds. Thus free cysteines can result in undesired covalent protein-protein interactions that contain free cysteines. Glycine residues can be introduced at locations that have extreme spatial constrains. These residues do not have side chains and are thusmore or less neutral in activity. However, the extreme flexibility and lack of interactive side chains of glycine residues can lead to destabilization and therefore glycine residues were not commonly used.

In the fourth phase themodels were assessed usingmodeller.modeller was programmed to accept cysteine-cysteine bridges when appropriate. Next all predicted protein structures were assessed with ProsaII (http://www.came.sbg.ac.at/Services/prosa.html), Procheck and What if(http://www.cmbi.kun.nl/What if). ProsaII zp-comb scores of less then -4.71 were assumed to indicate protein sequences thatmight fold in vivo into the desired betamotif. The seven protein sequences depicted in table 1 represent a collection of acceptable solutions meeting all criteria mentioned above.

Procheck and What ifassessments also indicated that these sequencesmight fit into themodels and thus as being reliable (e.g. pG values larger than 0.80; Sánchez et al., 1998)

### Example 3

### Assembly of synthetic scaffolds

Synthetic VAPs were designed on basis of their, predicted, three dimensional structure. The amino acid sequence (Table 3) was back translated into DNA sequence (Table 4) using the preferred codon usage for enteric bacterial gene expression (Informax Vector Nti). The obtained DNA sequence was checked for undesired restriction sites that could interfere with future cloning steps. Such sites were removed by changing the DNA sequence without changing the amino acid codons. Next the DNA sequence was adapted to create a *NdeI* site at the 5' end to introduce the ATG start codon and at the 3' end a *SfiI* site, both required for unidirectional cloning purposes. PCR assembly consists of four steps: oligo primer design (ordered at Operon's), gene assembly, gene amplification, and cloning. The scaffolds were assembled in the following manner: first both plus and minus strands of the DNA sequence were divided into oligonucleotide primers of approximately 35 bp and the oligonucleotide primer pairs that code for opposite strands were designed in such a way that they have complementary overlaps of approximately 16 - 17 bases. Second, all oligonucleotide primers for each synthetic scaffold weremixed in equimolar amounts, 100 pmol of this primermix was used in a PCR assembly reaction using 1 Unit Taq polymerase (Roche), 1 x PCR buffer +mgCl₂ (Roche) and 0.1mM dNTP (Roche) in a final volume of 50 µl, 35 cycles of; 30 sec. 92°C, 30 sec. 50°C, and 30 sec. 72°C. Third, 5 µl of PCR assembly product was used in a standard PCR amplification reaction using, both outside primers of the synthetic scaffold, 1 Unit Taq polymerase, 1 x PCR buffer +mgCl₂, and 0.1mM dNTP in a final volume of 50 µl, 25 cycles; 30 sec. 92°C, 30 sec. 55°C, 1min. 72°C. Fourth, PCR products were analyzed by agarose gel electrophoresis, PCR products of the correct size were digested with *NdeI* and *SfiI* and ligated into vector pCM126 linearized with *NdeI* and *SfiI*. Ligation products were transformed into TOP10 competent cells (InVitrogen) grown overnight at 37°C on 2xTY plates containing 100microgram/ml ampicillin and 2% glucose. Single colonies were grown in liquidmedium containing 100 µg ampicillin, plasmid DNA was isolated and used for sequence analysis.

### Example 4

### Expression vector CM126 construction

A vector for efficient protein expression (CM126; see figure 4A) based on pET-12a (Novagen) was constructed. A dummy VAP, iMab100, including convenient restriction sites, linker, VSV-tag, 6 times His-tag and stop codon was inserted (see table 4, 3). As a result the signal peptide OmpT was omitted from pET-12a. iMab100 was PCR amplified using forward primer 129 (see table 5) that contains a 5' *NdeI* overhanging sequence and a very long reverse oligonucleotide primer 306 (see table 5) containing all linkers and tag sequences and a *BamHI* overhanging sequence. After amplification the PCR product and pET-12a were digested with *NdeI* and *BamHI*. After gel purification products were purified via the Qiagen gel-elution system according tomanufactures procedures. The vector and PCR fragment were ligated and transformed by electroporation in E.coli TOP10 cells. Correct clones were selected and verified for their sequence by sequencing. This vector including the dummy VAP acted as the basic vector for expression analysis of other VAPs. Insertion of other VAPs was performed by amplification with primers 129 and 51 (see table 5), digestion with *Nde*I and *Sfi*I and ligation into *Nde*I and *Sfi*I digested CM126.

### Example 5

### Expression of iMab100

*E. coli* BL21 (DE3) (Novagen) was transformed with expression vector CM 126-iMab100. Cells were grown in 250ml shaker flasks containing 50ml 2*TYmedium (16 g/l tryptone, 10 g/l yeast extract, 5 g/l NaCl (Merck)) supplemented with ampicillin (200 microgram/ml) and agitated at 30°C. Isopropylthio-β-galactoside (IPTG) was added at a final concentration of 0.2mM to initiate protein expression when OD (600 nm) reached one. The cells were harvested 4 hours after the addition of IPTG, centrifuged (4000g, 15min., 4°C) and pellets were stored at -20°C until used.

Protein expression was analyzed by Sodium Dodecyl Sulphate PolyAcrylamide Gel Electrophoresis (SDS-PAGE). This is demonstrated in Figure X lane 2 for *E. coli* BL21(CM 126-iMab100) expressing iMAb100.

### Example 6

Purification of iMab100 proteins from inclusion bodies using heat.

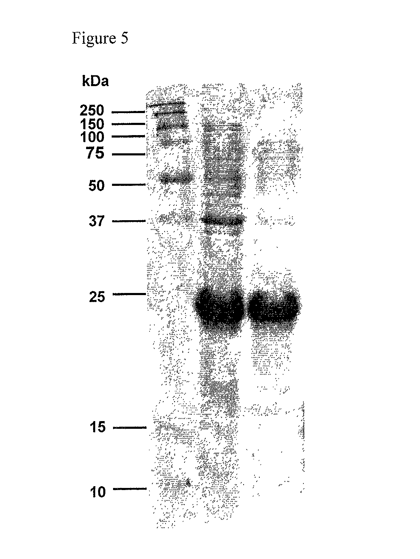

IMab100 was expressed in *E. coli* BL21 (CM 126-iMab100) as described in example 5.most of the expressed iMab100 was deposited in inclusionbodies. This is demonstrated in Figure X lane 3, which represents soluble proteins of *E. coli* BL21 (CM126) after lysis (French press) and subsequent centrifugation (12.000g, 15min). Inclusion bodies were purified as follows. Cell pellets (from a 50ml culture) were resuspended in 5ml PBS pH 8 up to 20 g cdw/l and lysed by 2 passages through a cold French pressure cell (Sim-Aminco). Inclusion bodies were collected by centrifugation (12.000 g, 15min) and resuspended in PBS containing 1 % Tween-20 (ICN) in order to solubilize and removemembrane-bound proteins. After centrifugation (12.000 g, 15min), pellet (containing inclusion bodies) was washed 2 times with PBS. The isolated inclusion bodies were resuspended in PBS pH 8 + 1% Tween-20 and incubated at 60°C for 10minutes. This resulted in nearly complete solubilization of iMab100 as is demonstrated in Figure 5. Lane 1 represents isolated inclusion bodies of iMab100. Lane 2 represents solubilized iMab100 after incubation of the isolated inclusion bodies in PBS pH 8 + 1% Tween-20 at 60 °C for 10minutes.

The supernatant was loaded on a Nickel-Nitrilotriacetic acid (Ni-NTA) superflow column and purified according to a standard protocol as described by Qiagen (The QIAexpressionist™, fifth edition, 2001). The binding of the thus purified iMab100 to chicken lysozyme was analyzed by ELISA (according to example 8) and is summarized in Table 6.

### Example 7

### Purification of iMab100 proteins from inclusion bodies using urea and matrix assisted refolding.

Alternatively, iMab100 was solubilized from inclusion bodies using 8m urea and purified into an active form bymatrix assisted refolding. Inclusion bodies were prepared as described in example 6 and solubilized in 1ml PBS pH 8 + 8m urea. The solubilized proteins were clarified from insolublematerial by centrifugation (12.000 g, 30min.) and subsequently loaded on a Ni-NTA superflow column (Qiagen) equilibrated with PBS pH 8 + 8M urea. Aspecific proteins were released by washing the column with 4 volumes PBS pH 6.2 + 8M urea. The bound His-tagged iMab100 was allowed to refold on the column by a stepwise reduction of the urea concentration in PBS pH 8 at room temperature. The column was washed with 2 volumes of PBS + 4M urea, followed by 2 volumes of PBS + 2M urea, 2 volumes of PBS + 1M urea and 2 volumes of PBS without urea. IMab100 was eluted with PBS pH 8 containing 250mM imidazole. The released iMab100 was dialyzed overnight against PBS pH 8 (4°C), concentrated by freeze drying and characterized for binding and structuremeasurements.

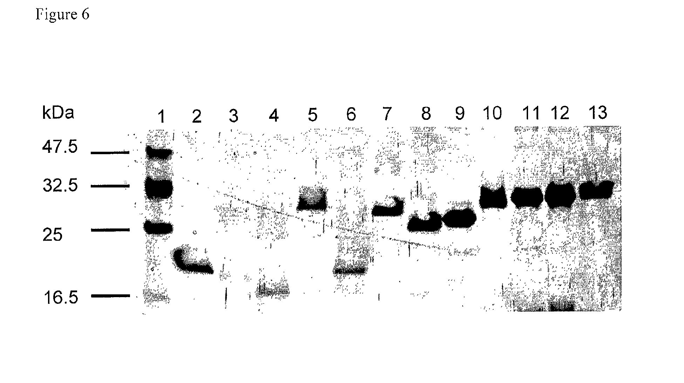

The purified fraction of iMab100 was analysed by SDS-PAGE as is demonstrated in Figure 6. lane 13.

### Example 8

### Specific binding of iMab100 proteins to chicken lysozyme (ELISA)

Binding of iMab proteins to targetmolecules was detected using an Enzyme Linked Immuno Sorption Assay (ELISA). ELISA was performed by coating wells of microtiter plates (Nunc) with the desired antigen (such as chicken lysozyme) and blocked with an appropriate blocking agent such as 3% skimmilk powder solution (ELK). Purified iMab proteins or purified phages (10⁸-10⁹) originating from a single colony were added to each well and incubated for 1 hour at room temperature. Plates were excessively washed with PBS containing 0.1% Tween-20 using a plate washer (Bio-Tek Instruments). Bound iMab proteins or phages were detected by the standard ELISA protocol using anti-VSV-hrp conjugate (Roche) or anti-M13-hrp conjugate (Pharmacia), respectively. Colorimetric assays were performed using Turbo-TMB (3, 3', 5, 5' -tetramethylbenzidine, Pierce) as a substrate.

Binding of iMab100 to chicken lysozyme was assayed as follows. Purified iMab100(∼ 50 ng) in 100 µl was added to a microtiter plate well coated with either ELK (control) or lysozyme (+ ELK as a blocking agent) and incubated for 1 hour at room temperature on a table shaker (300 rpm). Themicrotiter plate was excessively washed with PBS (3 times), PBS + 0.1% Tween-20 (3 times) and PBS (3 times). Bound iMab100 was detected by incubating the wells with 100 µl ELK containing anti-VSV-HRP conjugate (Roche) for 1 hour at room temperature.

After excessive washing using PBS (3 times), PBS + 0.1 % Tween-20 (3 times) and PBS (3 times), wells were incubated with 100 µl Turbo-TMB for 5minutes. Reaction was stopped with 100 µl 2M H₂SO₄ and absorbtion was read at 450 nm using amicrotiter plate reader (Biorad).

Purified iMab100 which has been prepared as described in example 6 and example 7 appeared to bind strongly and specifically to chicken lysozyme which is demonstrated in Table 6.

### Example 9

### Size exclusion chromatography

IMab100 was purified as described in example 7.

The purified iMab100 was analyzed formolecular weight distribution using a Shodex 803 column with 40% acetonitrile, 60%milliQ and 0.1% TFA asmobile phase. 90 % of the protein eluted at a retention time of 14.7minutes corresponding to amolecular weight of 21.5 kD. This is in close agreement with the computer calculated molecular weight (19.5kD) and indicates thatmost of the protein is present in the monomeric form.

### Example 10

### iMab100 stability at 95 °C over time

iMab100 stability was determined at 95 °C by ELISA. 10 microgram/milliliter iMab100 was heated to 95 °C for 10 minutes to 2.5 hours, unheated iMab was used as input control.

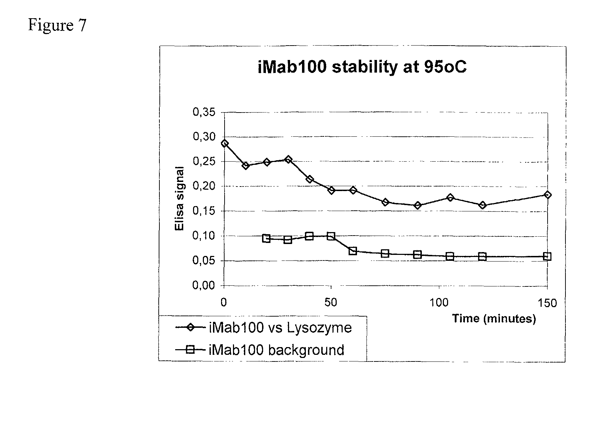

After heating, samples were placed at 20 °C and kept there until assayed. Lysozyme binding of these samples was tested by ELISA measurements using 1:2000 in PBS diluted anti-VSV-hrp (Roche). TMB-ultra (Pierce) was used as a substrate for hrp enzyme levels (figure 7). iMab100 was very stable at high tempertures. A very slow decrease in activity was deteced.

### Example 11

### iMab100 stability over time at 20 °C

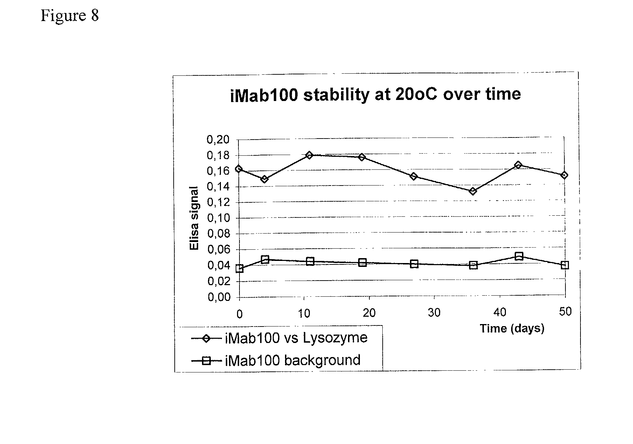

iMab100 stability was determined over a period of 50 days at 20 °C. iMab100 (O.lmilligram/milliliter) was placed at 20 °C. Every 7 days a sample was taken and every sample was stored at -20 °C for at least 2 hours to prevent breakdown and freeze the experimental condition. Samples were diluted 200 times in PBS. Lysozyme binding of these samples was tested by ELISA measurements using 1:2000 in PBS diluted anti-VSV-hrp (Roche). TMB-ultra (Pierce) was used as a substrate for hrp enzyme levels (Figure 8). iMab100 was very stable at room temperture. Activity of iMab100 hardly decreased over time, and thus it can be concluded that the iMab scaffold and its affinity regions are extremely stable.

### Example 12

iMab100 size determination, resistance against pH 4.8 environment, testing by gel and Purified iMab100 (as described in example 6) was brought to pH 4.8 using postassium acetate (final concentration of 50 mM) which resulted in precipitation of the protein. The precipitate was collected by centrifugation (12000 g, 30 minutes), redissolved in PBS pH 7.5 and subsequently filtered through a 0.45 micrometer filter to remove residual precipitates.

The samples fore and after pH shock were analyzed by SDS-PAGE, western blotting and characterized for binding using ELISA (example 8).

It was demonstrates that all iMab100 was precipitated at pH 4.8 and could also be completely recovered after redissolving in PBS pH 7.5 and filtering. ELISA measurements demonstrated that precipitation and subsequent resolubilization did nort result in a loss of activity (Table 7). It was confirmed that the VSV-tag is not lost during purification and precipitation and that no degradation products are formed.

### Example 13

### Structural analysis of scaffolds

The structure of iMab100 was analyzed and compared with another structure using a circular dichroism polarimeter (CD). As a reference, a natural occuring 9 beta strand containing Vhhmolecule, Vhh10-2/271102 (a kind gift ofm. Kwaaitaal, Wagening University), wasmeasured. Both proteins have tags attached to the C-terminal end. The amino acid sequence and length of these tags are identical. The only structural differences between these two proteins are present in the CDR3 (Vhh) corresponding affinity loop 4 (iMab100).

System settings were: sensitivity = standard (100mdeg); start = 260nm; end = 205 nm; interval = 0.1 nm; delay = 1 sec.; speed = 50 nm/min; accumulation = 10.

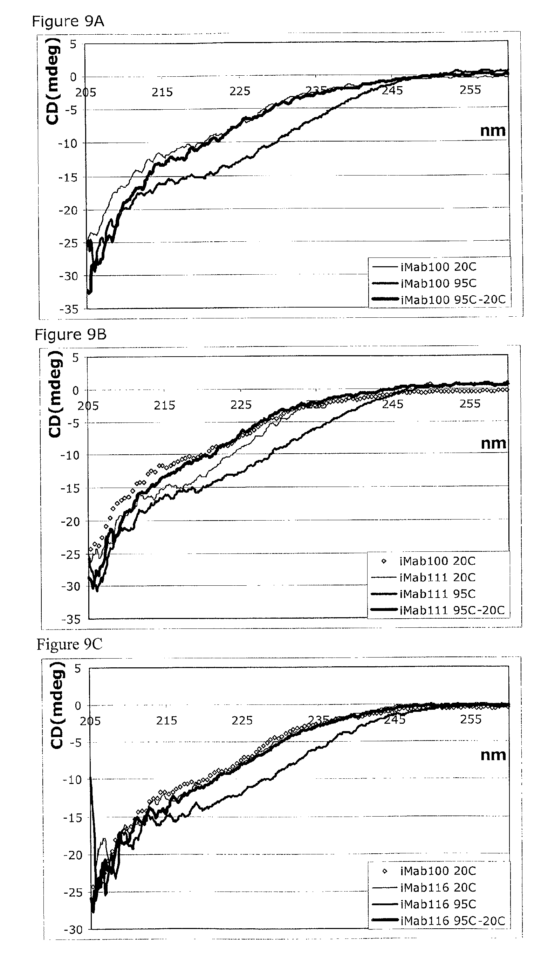

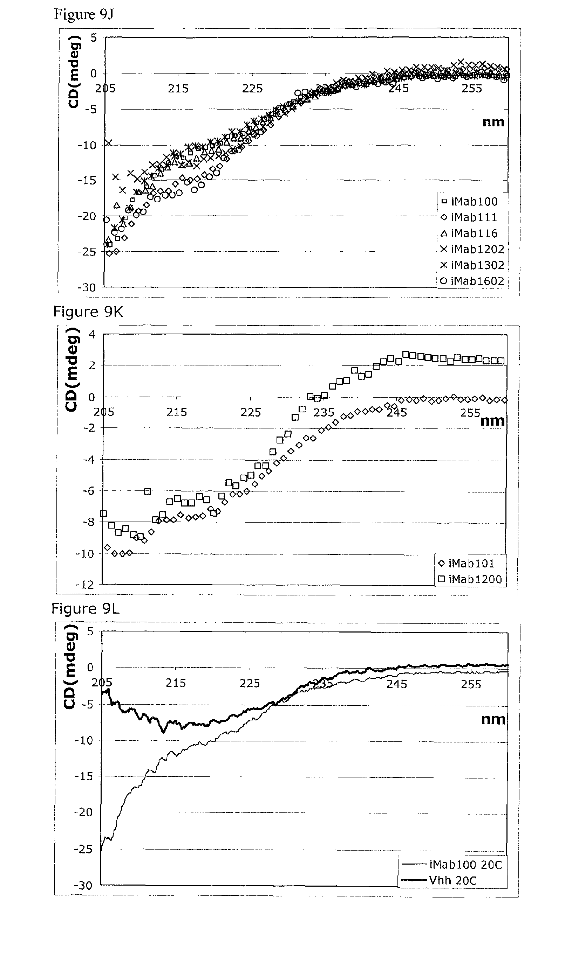

iMab100 and Vhh10-2/271102 were prepared with a purity of 98% in PBS pH 7.5 and OD₂₈₀ ≈ 1.0. Sample was loaded in a 0.1 cm quartz cuvette and the CD spectrummeasured with a computer controlled JASCO Corporation J-715 spectropolarimeter software (Spectramanager version 1.53.00, JASCO Corporation). Baseline corrections were obtained bymeausring the spectrum of PBS. The obtained PBS signal was substracted from allmeasurement to correct for solvent and salt effects. An initialmeasurement with each sample was done to determine themaximum signal. If required, the sample was diluted with 1 times PBS fro optimal resolution of the photomultiplier signal. A solution in PBS of RNase A was used to verify the CD apparatus. The observed spectrum of RNase A was completely different if compared with iMab100 and the Vhh spectrum. Figure 9L represents the CD spectrum of iMab100 and the Vhh proteins in far UV (205-260nm). Large part of the spectral patterns were identical. Spectral difference weremainly observed at wavelengths below 220nm. The observed differences of the spectra are probably due to differences in CDR3/AR4 structural differences. The structure of AR4 in iMab100, which was retreived from 1MEL, can be classified as random coil-like. Also, AR4 present in iMab100 is about 10 amino acids longer than the CDR3 of the Vhh protein.

The temperature stability of the iMab100 protein was determined in a similar way using the CD-meter except that the temperature at which themeasurements were performed were adjusted.

In addition to measurements at room temperture, folding and refolding was assayed at 20, 50, 80 (not shown) and 95 degrees Celsius. Fresh iMab100 protein solution in PBS diluted was first measured at 20 degrees Celsius.

Next, spectra at increasing temperatures were determined and lastly, the 20 degrees Celsius spectrum was re-measured. Baseline corrections were applied with the spectrum of PBS (figure 9A). The results clearly show a gruadual increase in ellipticity at increasing tempertures. The re-appearance of the 20 degrees Celsius spectrum after heating strongly indicates complete refolding of the scaffold. This conclusion was also substantiated by subsequent lysozyme binding capacity detection of the samples by ELISA (data not shown).

### Example 14

*E. coli* BL21 (DE3) (Novagen) was transformed with expression vector CM126 containing various VAP inserts for iMab1302, iMab1602, iMab1202 and iMab122 all containing 9 β-strands. Growth and expression was similar as described in example 5.

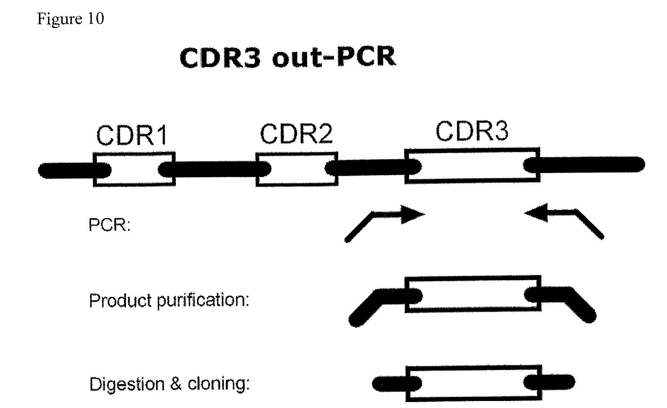

All 9-stranded iMab proteins were purified bymatrix assisted refolding similar as is described in example 7. The purified fractions of iMab1302, iMab1602, iMab1202 and iMab122 were analysed by SDS-PAGE as is demonstrated in Figure 10 lanes10, 9, 8 and 7 respectively.

### Example 15

### Specific binding of various 9 stranded iMab proteins to chicken lysozyme (ELISA)

Purified iMab1302(∼ 50 ng), iMab 1602(∼ 50 ng), iMab1202(∼ 50 ng) and iMab122 (∼ 50 ng) were analyzed for binding to either ELK (control) or lysozyme (+ ELK as a blocking agent) similar as is described in Example 8. ELISA confirmed specific binding of purified iMab1302, iMab 1602, iMab1202 and iMab122 to chicken lysozyme as is demonstrated in Table 6

### Example 16

### CD spectra of various 9 stranded iMab

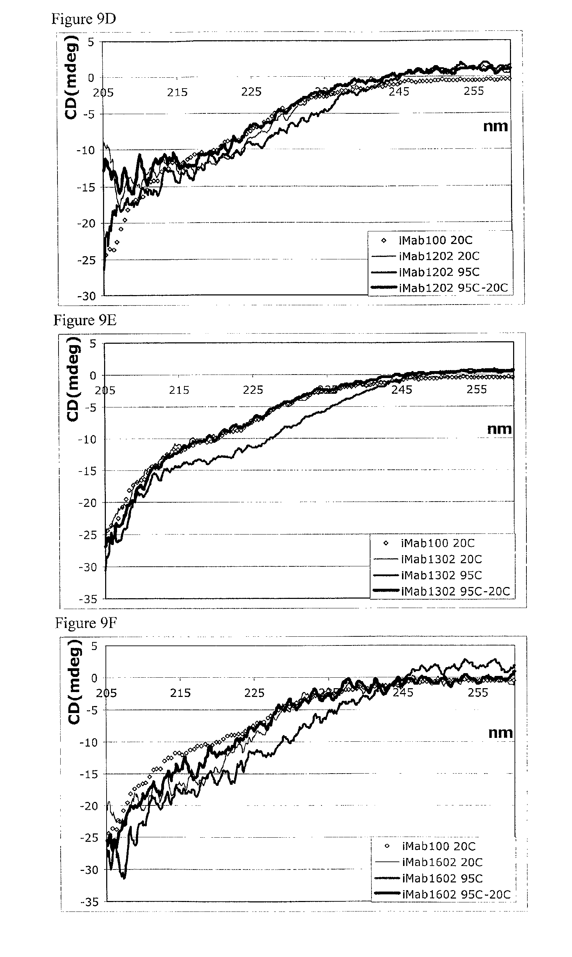

iMab100, iMab1202, Imab1302 and iMab1602 were purified as described in example 14 and analyzed for CD spectra as described in example 13. The spectra of iMab 1202, iMab1302 and iMab 1602 were measured at 20°C, 95°C and back at 20°C to test scaffold stability and refolding characteristics. The corresponding spectra are demonstrated in Figure 9D, 9E and 9F respectively. The spectra measured at 20°C were compared with the spectrum of iMab100 at 20°C to determine the degree of similarity of the secondary structure (see Figure 9J). It can be concluded that all different 9 strand scaffolds behave identical. This indicates that the basic structure of these scaffolds is identical. The data obtained after succesive 20-95-20 degrees Celsius treatments clearly show that all scaffolds return to their original conformation.

### Example 17

### Design of 7 stranded ig-like folds

The procedure as described in example 2 was used for the development of sequences that contain an ig-like fold consisting of 7 beta-elements in the core and 3+3 connecting loops. The procedure involved 4 phases through which the development of the new sequences was guided, identical as the process as described in example 2. In phase 1, the coordinates of C-alpha atoms as indicated in PDB table 1 for 9 stranded core structures were adapted. C-alpha atoms representing beta elements 4 and 5 were removed from the PDB files, resulting in a seven-stranded example of the core (PDB table 8). Amino acid side chains that line up with the interior of the beta-sheets were obtained and inserted as described in detail in example 2. In the second phase connecting loops were added. On one site beta-elements were connected with one other by affinity region retrieved from anti-chicken lysozyme binding region obtained from the structure 1MEL or the bovine RNase A binding regions of 1BZQ (L2, L6 and L8). On the other end of the structure, beta-elements were connected with C-alpha backbone trace loops obtained from several different origins (1E50, 1CWV, 1QHP, 1NEU, 1EPF, 1F2x or 1EJ6). The procedure for the attachment and fit of the loops is described in detail in example 2. In the third phase, amino acid side chains that determine the solubility of the proteins located in the core and loops 1, 3, 7 were determined as described in example 2.

In the last phase, the models were build using Insight. Insight was programmed to accept cysteine-cysteine bridges when appropriate. Next all predicted protein structures build with Insigth were assessed with ProsaII, Procheck and WHAT IF. ProsaII zp-comb scores of less then -4.71 were assumed to indicate protein sequences that might fold in vivo into the desired ig-like beta motif fold (table 9). A number of example sequences depicted in table 10 represent a collection that appeared to be reliable. Procheck and What ifassessments also indicated that these sequencesmight fit into themodels and thus as being reliable (e.g. pG values larger than 0.80; Sánchez et al., 1998).

### Example 18

*E. coli* BL21 (DE3) (Novagen) was transformed with expression vector CM126 containing various VAP inserts for iMab1300, iMab1200, iMab101 and iMab900 all containing 7 beta-strands. Growth and expression was similar as described in example 5.

All 7-strand iMabs were purified bymatrix assisted refolding similar as is described in example 7. The purified fractions of iMab101, iMab1300, iMab1200 and iMab900 were analysed by SDS-PAGE as is demonstrated in Figure 10 lanes 2, 3, 5 and 6 respectively.

### Example 19

Purified iMab1300 (∼ 50 ng), iMab1200(∼ 5 ng), iMab101(∼ 20 ng) and iMab900 (∼ 10 ng) were analyzed for binding to either ELK (control) or lysozyme (+ ELK as a blocking agent) similar as is described in example 8. ELISA confirmed specific binding of purified iMab1300, iMab1200, iMab101 and iMab900 to chicken lysozyme as is demonstrated in table 6.

### Example 20

### CD spectra of various 7 stranded iMab proteins

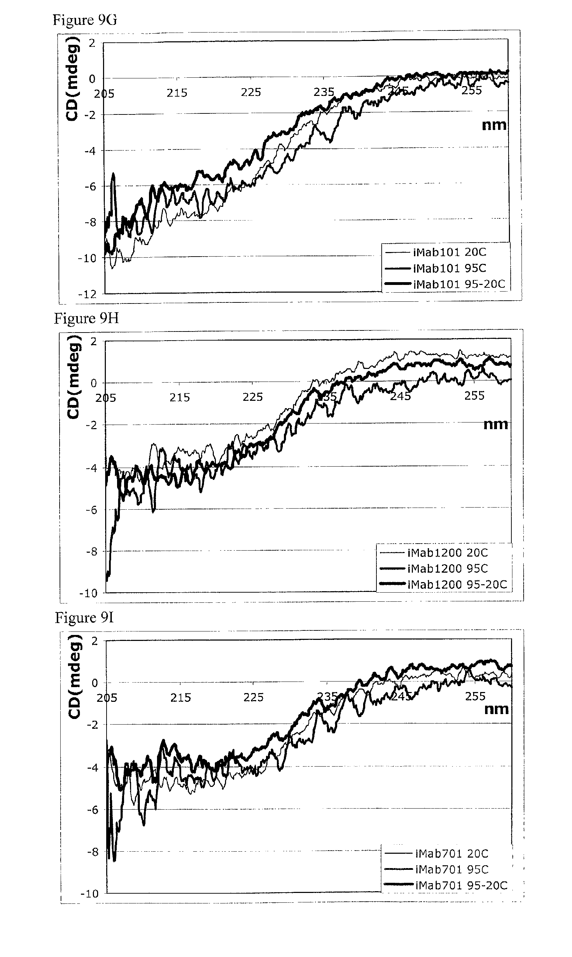

IMab1200 and iMab101 were purified as described in example 18 and analyzed for CD spectra as described in example13. The spectra of iMab1200 and iMab101 were measured at 20°C, 95°C and back at 20°C to test scaffold stability and refolding characteristics. The corresonding spectra are demonstrated in Figure 9H and 9G respectively. The spectra of iMab1200 and iMab101 measured at 20°C were compared with each other to determine the degree of similarity of the secondary structure (see Figure 9K). It can be concluded that the different 7 strand scaffolds behave identical. This indicates that the basic structure of these scaffolds is identical. Even more, as the obtained signals form the 9 stranded scaffolds (example 16) are similar to the signals obeserved for the 7 strands as presented here, it can also be concluded that the both types of scaffolds have an similar conformations. The data obtained after succesive 20-95-20 degrees Celsius treatments clearly show that all scaffolds stay in their original conformation.

### Example 21

### Design of 6 stranded ig-like folds

The procedure as described in example 2 and 3 was used for the development of sequences that contain an ig-like fold consisting of six beta-elements in the core and 3+3 connecting loops. The procedure involved 4 phases through which the development of the new sequences was guided, identical as the process as described in example 2 and 3. In phase one, the coordinates of C-alpha atoms as indicated in PDB table 1 for 9 stranded core structures were adapted. C-alpha atoms representing beta elements 1, 4 and 5 were removed from the PDB files, resulting in a six-stranded example of the core (table 11). Amino acid side chains that line up with the interior of the beta-sheets were obtained and inserted as described in detail in example 2 and 3. In the second phase connecting loops were added. On one site beta-elements were connected with one other by affinity region retrieved from anti-chicken lysozyme binding region obtained from the structure 1MEL or the bovine RNase A binding regions of 1BZQ (L2, L6 and L8). On the other end of the structure, beta-elements were connected with C-alpha backbone trace loops obtained from several different origins (1E50, 1CWV, 1QHP, 1NEU, 1EPF, 1F2x or 1EJ6). The procedure for the attachment and fit of the loops is described in detail in example 2 and 3. In the third phase, amino acid side chains that determine the solubility of the proteins located in the core and loops L1, L3, L7 were determined as described in example 2 and 3. In the last phase, themodels were assessed usingmodeller.modeller was programmed to accept cysteine-cysteine bridges when appropriate. Next all predicted protein structures were assessed with ProsaII, Procheck and WHAT IF. ProsaII zp-comb scores were determined (table 12) to indicate if the created protein sequences might fold in vivo into the desired ig-like beta motif fold. Procheck and What if assessments were applied to check whether sequences might fit into the models (table 13).

### Example 22

### Purification of 6 stranded iMab proteins

*E. coli* BL21 (DE3) (Novagen) was transformed with expression vector CM126 containing an VAP insert for iMab701 containing 6 beta-strands. Growth and expression was similar as described in example 5.

The iMab701 proteins were purified bymatrix assisted refolding similar as is described in example 7. The purified fraction of iMab701 was analysed by SDS-PAGE as is demonstrated in Figure 6 lane 4.

### Example 23

Specific binding of 6 stranded iMab proteins to chicken lysozyme (ELISA) Purified iMab701(∼ 10 ng) was analyzed for binding to either ELK (control) and lysozyme (+ ELK as a blocking agent) similar as is described in Example 8.

ELISA confirmed specific binding of purified iMab701 to chicken lysozyme as is demonstrated in Table 6.

### Example 24

### CD spectra of a 6 stranded iMab proteins

IMab701 was purified as described in example 22 and analyzed for CD spectra as described in example 13. The spectra of iMab701 was measured at 20°C, 95°C and again at 20°C to test scaffold stability and refolding characteristics. The corresponding spectra are demonstrated in Figure 9I. It can be concluded that the 6 strand scaffold behaves identical to the 7 strand scaffolds as described in example 20. This indicates that the basic structure of this scaffold is identical to the structure of the 7 strand containing scaffolds. Even more, as the obtained signals form the 9 stranded scaffolds (example 16) are similar to the signals obeserved for this 6 strand scaffold as presented here, it can also be concluded that the both types of scaffolds have an similar conformations. The data obtained after succesive 20-95-20 degrees Celsius treatments clearly show that all scaffolds stay in their original conformation.

### Example 25

### Design of aminimal primary scaffold

A minimal scaffold is designed according to the requirements and features as described in example 1. However now only four and five beta-elements are used in the scaffold (see figure 1). In the case of 5 beta-elements amino acids side chains of beta-elements 2, 3, 6, 7 and 8 that are forming themantle of the new scaffold need to be adjusted for a watery environment. The immunoglobulin killer receptor 2dl2 (VAST code 2DLI) is used as a template for comparativemodelling to design a new small scaffold consisting of 5 beta-elements.

### Example 26

### Procedure for exchanging surface residues: lysine replacements

Lysine residues contain chemical active amino-groups that are convenient in for example covalent coupling procedures of VAPs. Covalent coupling can be used for immobilization of proteins on surfaces or irreversible coupling of othermolecules to the target.

The spatial position of lysine residues within the VAP determines the positioning of the VAP on the surface after immobilization. Wrong positioning can easily happen with odd located lysine residues exposed on the surface of VAPs. Therefore itmay be required for some VAP structures to remove lysine residues from certain locations, especially from those locations that can result in diminished availability of affinity regions.

As an example of the exchange strategy for residues that are located on the outer surface, iMab100 outer surface lysine residues were changed. 3D imaging indicated that all lysine residues present in iMab100 are actually located on the outer surface. 3Dmodelling and analysis software (InsightII) determined the spatial consequence of such replacements.

Modeller software was programmed in such a way that either cysteine bridge formation between the beta-sheets was taken into account or the cysteine bridges were neglected in analyses. All retrievedmodels were build with ProsaII software formore or less objective result ranking. The zp-comb parameter of ProsaII indicated the reliability of themodels. Results showed that virtually all types of amino acids could replace lysine residues. However, surface exposed amino acid side chains determine the solubility of a protein. Therefore only amino acids that will solubilize the proteins were taken into account andmarked with an X (see table 14).

Sequence of iMab100: underlined lysine residues were exchanged

NVKLVEKGGNFVENDDDLKLTCRAEGYTIGPYCMGWFRQAPNDDSTNVA
TINMGGGITYYGDSVKERFDIRRDNASNTVTLSMDDLQPEDSAEYNCAGDS
TIYASYYECGHGLSTGGYGYDSRGQGTDVTVSS

### Example 27

### Changing amino acids in the exterior: removal of glycosylation site.

N-glycosylation can interfere strongly with protein functions if the glycosylation site is for example present in a putative ligand-binding site. iMab100 proteins were shown to be glycosylated in Pichia pastoris cells and unable to bind to the ligand. Analysis showed that there is a putative N-glycosylation site in AR3. Inspection of the iMab100 structure using template-modeling strategies withmodeller software revealed that this site is potentially blocking ligand binding due to obstruction by glycosylation. This site could be removed in two different ways, by removing the residue being glycosylated or by changing the recognitionmotif for N-glycosylation. Here the glycosylation site itself (..RDNAS..) was removed. All residues could be used to replace the amino acid, after which ProsaII, What ifand Procheck could be used to check the reliability of each individual amino acid. However, some amino acids could introduce chemical or physical properties that are unfavorable. Cysteine for example couldmake the proteins susceptible to covalent dimerization with proteins that also bear a free cysteine group. Also non-hydrophilic amino acids could disturb the folding process and were omitted.methionine, on the other hand, is coded by ATG, which can introduce aberrant start sites in DNA sequences. The introduction of ATG sequencesmight result in alternative protein products due to potential alternative start sites.methionine residues were only assessed if no other amino acids would fit. All other amino acid residues were assessed with ProsaII, What ifand Procheck. Replacement of N with Q was considered to be feasible and reliable. protein sequence from iMab with glycosylation site: 
NVKLVEKGGNFVENDDDLKLTCRAEGYTIGPYCMGWFRQAPNDDSTNVA
TINMGGGITYYGDSVKERFDIRRDNASNTVTLSMDDLQPEDSAEYNCAGDS
TIYASYYECGHGLSTGGYGYDSRGQGTDVTVSS
 protein sequence from iMab without glycosylation site:

NVKLVEKGGNFVENDDDLKLTCRAEGYTIGPYCMGWFRQAPNDDSTNVA
TINMGGGITYYGDSVKERFDIRRDQASNTVTLSMDDLQPEDSAEYNCAGDS
TIYASYYECGHGLSTGGYGYDSRGQGTDVTVSS

Expression of iMab100 in *Pichia pastoris* was performed by amplification of 10 ng of CM114-iMab100 DNA in a 100microliter PCR reactionmix comprising 2 units Taq polymerase (Roche), 200 micromilor of each dNTP (Roche), buffers (Roche Taq buffer system), 2.5 micromolar of primer 107 and 108 in a Primus96 PCRmachine (MWG) with the following program 25 times [94°C 20", 55°C 25", 72°C 30"], digestion with *EcoRI* and *NotI* and ligation in *EcoRI* and *NotI* digested pPIC9 (InVitrogen). Constructs were checked by sequencing and showed all the correct iMab100 sequence. Transformation of *Pichia pastoris* was performed by electroporation according to themanufacturers protocol.

Growth and induction of protein expression bymethanol was performed according to themanufacturers protocol. Expression of iMab100 resulted in the production of a protein that on a SDS-PAGE showed a size of 50 kD, while expressed in *E.coli* the size of iMab100 is 21 kD. This difference ismost likely due to glycosylation of the putative N-glycosylation site present in iMab100 as described above. Therefore this glycosylation site was removed by exchange of the asparagine (N) for a glutamine (Q) in a similar way as described in example 26 except that primer 136 (table 5) was used. This resulted in iMab 115. Expression of iMab115 in *E. coli* resulted in the production of a 21 kD protein. ELISA experiments confirmed specificity of this iMab for lysozyme.

Thus, ARs in iMab115 were positioned correctly and,more specifically, replacement of the asparagine with glutamine in AR3 did not alter AR3 properties.

### Example 28

### Changing amino acids in the interior of the core: removal of cysteine residues.

Obtained sequences that fold in an ig-like structure, can be used for the retrieveal of similarly folded structures but aberant amino acid seqeunces.

Amino acids can be exchanged with other amino acids and thereby putatively changing the physical and chemical properties of the new protein if compared with the template protein. Changes on the out side of the protein structure were shown to be rather straightforward. Here we changed amino acids that are lining up with the interior of the core. Spatial constrains of neighboring amino acid side chains and the spatial constrains of the core structure itself determine and limit the types of side chains that can be present at these locations. In addition, chemical properties of neighboring side chains can also influence the outcome of the replacements. In some replacement studies it might be necessary to replace addition amino acids that are in close proximity of the target residues in order to obtain suitable and reliable replacements. Here were removed the potential to form cysteine bridges in the core. The removal of only one cysteine already prevents the potential to form cysteine bridges in the core. However, dual replacements can also be performed in order to prevent the free cysteine to interact with other free cysteine during folding or re-folding in vivo or in vitro. First, the individual cysteine residues were replaced by any other common amino acid (19 in total). This way 2 times 19models were retrieved. Allmodels were assessed using ProsaII (zp-scores), What if (2^{nd} generation packing quality, backbone conformation) and Procheck (number of residues outside allowed regions). Several reliable models were obtained. Table 15 shows the zp-combined Prosa scores of the cysteine replacements at position 96. The replacement of one of the cysteines with valine was tested in vivo to validate the method. This clone was designated as iMab116 (see table 3) and constructed (table 4) according to the procedure as described in example 3. The complete iMab sequence of this clone was transferred into CM126 in the following manner. The iMab sequence, iMab116, was isolated by PCR using Cys-min iMab116 as a template together with primers pr121 and pr129 (table 5). The resulting PCR fragment was digested with *NdeI* and *SfiI* and ligated into CM126 linearized with *NdeI* and *SfiI*. This clone, designated CM126-iMab116 was selected and used for further testing.

### Example 29

### Purification of iMab116

*E. coli* BL21 (DE3) (Novagen) was transformed with expression vector CM126 containing an VAP insert for iMab116 containing 9 beta-strands and potentially lacking a cysteine bridge in the core (as described in example 27). Growth and expression was similar as described in example 5.

IMab116 was purified bymatrix assisted refolding similar as is described in example 7. The purified fraction of iMab116 was analysed by SDS-PAGE as is demonstrated in Figure 6 lane 11.

### Example 30

### Specific binding of iMab116 to chicken lysozyme (ELISA)

Purified iMab116(∼ 50 ng) was analyzed for binding to either ELK (control) and lysozyme (+ ELK as a blocking agent) similar as is described in Example 8.

ELISA confirmed specific binding of purified iMab116 to chicken lysozyme as is demonstrated in Table 6.

### Example 31

### CD spectra of iMab116 proteins

IMab116 was purified as described in example 28 and analyzed for CD spectra as described in example 13. The spectrum of iMab116 was measured at 20°C, 95°C and again at 20°C to test scaffold stability and refolding characteristics. The corresponding spectra are demonstrated in Figure 9C. The spectra measured at 20°C were compared with the spectrum of iMab100 and other 9-stranded iMab proteins at 20°C to determine the degree of similarity of the secondary structure (see Figure 9J). Because the obtained spectrum is identical to the spectrum obtained from other 9 strand scaffolds, including the iMab100 spectrum, it can be concluded that the cysteine residue removal from the internal core has no effect on the structure itself.

### Example 32

### Introduction of extra cysteine bridge in the core

Chemical bonding of two cysteine residues in a proteins structure (cysteine bridge) can dramatically stabilize a protein structure at temperatures below about 70 degrees Celsius. Above this temperature cysteine bridges can be broken. Some applications demand proteins that aremore stable than the original protein. The spatial constrains of the core of beta strand folds as referred to in example 1, enables cysteine bridges. This conclusion is based on the observation that in some natural occurring proteins with the referred fold a cysteine bridge is present in the center of the core (e.g. all heavy chain variable domains in antibodies). The distance between C-alpha backbone atoms of such cysteines ismost often found to be between 6.3 and 7.4 angstrom. The introduction of new cysteine residues that putatively form bridges in coremotifs was analyzed bymeasurements. The coordinates of C-alpha atoms of a protein written in PDB files can be used to determine potential cysteine bridges. The distance between each C-alpha atom individually and all other C-alpha atoms can be calculated. The position of C-alpha atoms of the iMab100 protein obtained via comparative modeling is shown in figure BBB3. Insight software can be used to determine the distance between C-alpha atoms. However, standardmathematical algorithms that determine distances between two positions in space indicated by coordinates (as represented in a PDB coordinates) can also be used. Excel sheets were used to determine all possible distances. Distance values that appear to be between 6.3 and 7.4 angstrom were regarded as putative cysteine locations. Analysis indicated 33 possible cysteine bridge locations within iMab100. The cys-number indicates the position of the C-alpha atom in the structure that might be used for the insertion of a cysteine (table 16A). However, not all positions in space are very useful; some bridgesmight be to close to an already available cysteine bridge, two cysteines next to each other can be problematic, two cysteine bridges between identical beta strands will not be very helpful, spatial constrains with other amino acid side chains that are located nearby. All 33models were constructed and assayed with iMab100 as a template inmodeller. Zp-scores of assessed models obtained with ProsaII indicated that most cysteine residues are problematic. The best cysteine locations are indicated in table 16B. Twomodels, indicated in bold, were chosen based on the spatial position of these cysteine residues and bridges in relation to the other potential cysteine bridge. Also, somemodels were rejected, though the zp-scores were excellent, because of their position within the fold as reviewed with Insight (MSI).

### Example 33

### Construction of an iMab100 derivative that contains two extra cysteines in the core.

An oligo nucleotidemediated site directed mutagenesis method was used to construct an iMab100 derivative, named iMab111 (table 3), that received two extra cysteine residues. CM114-iMab100 was used as a template for the PCR reactions together with oligo nucleotides pr33, pr35, pr82, pr83 (see table 5). In the first PCR reaction, primers pr82 and pr83 were used to generate a 401 bp fragment. In this PCR fragment a glutamine and a glycine coding residue were changed into cysteine coding sequences. This PCR fragment is used as a template in two parallel PCR reaction: In one reaction the obtained PCR fragment, CM114-iMab100 template and pr33 were used, while in the other reaction the obtained PCR fragment, CM114-iMab100 template and primers 35 were used. The firstmentioned reaction gave a 584 bp product while the second one produced a 531 bp fragment. Both PCR fragments were isolated via agarose gel separation and isolation (Qiagen gel extraction kit). The products weremixed in an equimolar relation and an fragment overlap-PCR reaction with primers pr33 and pr35 resulted in a 714 bp fragment. This PCR fragment was digested with *NotI* and *SfiI*. The resulting 411 bp fragment was isolated via an agarose gel and ligated into CM114 linearized with *NotI* and *SfiI*. Sequencing analysis confirmed the product, i.e. iMab111 (table 4 and 3).

### Example 34

### Expression of iMab111

iMab111 DNA was subcloned in CM126 as described in example 28. CM126-iMab111 transformed BL21(DE3) cells were induced with IPTG and protein was isolated as described in example 7. Protein extracts were analysed on 15% SDS-PAGE gels and showed a strong induction of a 21 KD protein. The expected length of iMab111 including tags is also about 21 kD indicating high production levels of this clone.

### Example 35

### Purification of iMab111

*E. coli* BL21 (DE3) (Novagen) was transformed with expression vector CM126 containing an VAP inserts for iMab111 containing 9 beta-strands potentially containing an extra cysteine bridge (as described in example 32 and 33). Growth and expression was similar as described in example 5 and 34.

iMab111 was purified by matrix assisted refolding similar as is described in example 7. The purified fraction of iMab111 was analysed by SDS-PAGE as is demonstrated in Figure 6 lane 12.

### Example 36

### Specific binding of iMab111 to chicken lysozyme (ELISA)

Purified imab111(∼ 50 ng) was analyzed for binding to either ELK (control) and lysozyme (+ ELK as a blocking agent) similar as is described in Example 8 A 100-fold dilution of the protein extract in an ELISA assay resulted in a signal of approximately 20 fold higher than background signal. ELISA results confirmed specific binding of purified iMab111 to chicken lysozyme as is demonstrated in Table 6.

### Example 37

### CD spectra of iMab111 proteins

IMab111 was purified as described in example 32 and analyzed for CD spectra as described in example 13. The spectrum of iMab116 was measured at 20°C, 95°C and again at 20°C to test scaffold stability and refolding characteristics. The corresponding spectra are demonstrated in Figure 9C. The spectra measured at 20°C were compared with the spectrum of iMab100 and other 9-stranded iMab proteins at 20°C to determine the degree of similarity of the secondary structure (see Figure 9J). Because the obtained spectrum is identical to the spectrum obtained from other 9 strand scaffolds, including the iMab100 spectrum, it can be concluded that the additiona cysteine residue in the centre of the core has no effect on the structure itself.

### Example 38

### Improving properties of scaffolds for specific applications

For certain applications, the properties of a scaffold need to be optimized. For example heat stability, acid tolerance or proteolytic stability can be advantageous or even required in certain environments in order to function well. Amutation and re-selection program can be applied to create a new scaffold with similar binding properties but with improved properties. In this example a selected binding protein is improved to resist proteolytic degradation in a proteolytic environment. New scaffolds can be tested for proteolytic resistance by a treatment with amixture of proteases or alternatively a cascade treatment with specific protease. In addition, new scaffolds can be tested for resistance by introducing the scaffolds in the environment of the future application. In orde to obtain proteolytic restant scaffolds, the gene(s) that codes for the scaffold(s) is (are)mutated usingmutagenesismethods. Next a phage display library is build from themutated PCR products so that the new scaffolds are expressed on the outside of phages as fusion proteins with a coat protein. The phages are added to a the desired proteolytic active environment for a certain time at the desired temperature. Intact phages can be used in a standard panning procedure as described. After extensive washing bound phages are eluted, infected in E.coli cells that bear F-pili and grown overnight on a agar plate that contains appropriate antibiotics. Individual clones are re-checked for their new properties and sequenced. The process ofmutation introduction and selection can be repeated several times or other selection conditions can be applied in further optimization rounds.

### Example 39

### Randommutagenesis of scaffolds regions

Primers annealing just 3 prime and 5 prime of the desired region (affinity regions, frameworks, loops or combinations of these) are used for amplification in the presence of dITP or dPTP as described. Thesemutated fragments are amplified in a second PCR reaction with primers having the identical sequence as the set of primers used in the first PCR but now containing restriction sites for recloning the fragments into the scaffold structure at the which can differ among each other in DNA sequence and thus also in protein sequence. Phage display selection procedures can be used for the retrieval of clones that have desired properties.

### Example 40

### Phage Display vector CM114-iMab100 construction

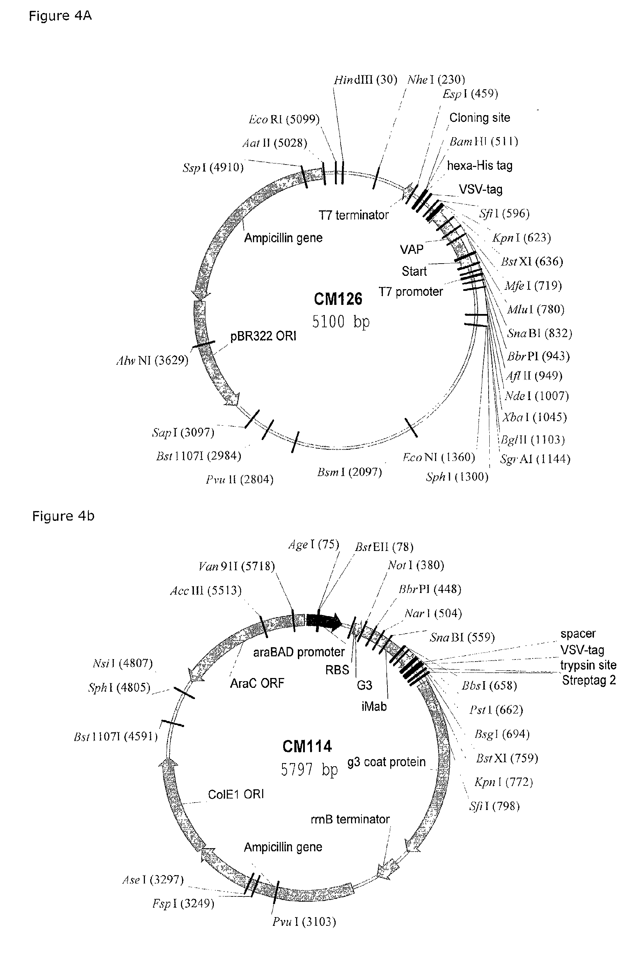

A vector for efficient phage display (CM114-iMab100; see figure 4B) was constructed using part of the backbone of a pBAD (InVitrogen). The required vector part from pBAD was amplified using primers 4 and 5 containing respectively *AscI* and *BamHI* overhanging restriction sites. In parallel a synthetic constructed fragment was made containing the sequence as described in table 4 including a new promoter, optimized g3 secretion leader, *NotI* site, dummy insert, *SfiI* site, linker, VSV-tag, trypsin specific proteolytic site, Strep-tagII and *AscI* site (see figure 4B). After combining the digested fragment and the PCR amplified pBAD vector fragment, the coding region of them13 phage g3 core protein was amplified using *AscI* overhanging sites attached to primers (table 5, primer 6 and 7) and inserted after *AscI* digestion. Vector that contained correct sequences and correct orientations of the inserted fragments were used for further experiments.

### Example 41

### Phage Display vector CM114-iMab113 construction

Cysteine bridges between AR4 and other affinity regions (e.g. AR1 for iMab100) can be involved in certain types of structures and stabilities that are not very likely without cysteine bridge formations. Not only can AR1 be used as an attachment for cysteines present in some affinity regions 4, but also AR2 and AR3 are obvious stabilizing sites for cysteine bridge formation. Because AR2 is an attractive alternative location for cysteine bridge formation with AR4, an expression vector is constructed which is 100% identical to CM114-iMab100 with the exception of the locations of a cysteine codon in AR2 and the lack of such in AR1. 3D-modelling analysis revealed that the best suitable location for cysteine in AR2 is at the location originally determined as a threonine (.VATIN.. into ..VACIN..). Analysis indicated that in addition to the new cysteine location (..VACIN..), the alanine residue just before the threonine residue in AR2 was replaced with a serine residue (..VSCIN..). The original cysteine in AR1 was replaced by a serine that turned out to be a suitable replacement according to 3Dmodelling analysis (table 3).

The new determined sequence, named iMab113, (table 4) was constructed according to the gene construction procedure as described above (example 3) and inserted in CM114 replacing iMab100.

### Example 42

### Phage Display vector CM114-iMab114 construction

Cysteine bridges between AR4 and other regions are not always desired because intermolecular cysteine bridge formations during foldingmight influence the efficiency of expression and percentage of correct folded proteins.

Also, in reducing environments such ARsmight become less active or even inactive. Therefore, scaffolds without cysteine bridges are required.

An expression vector lacking cysteines in AR1, 2 and 3 was constructed. This vector is 100% identical to CM114 with the exception that the cysteine in AR1 (..PYCMG..) has been changed to a serine (..PMSMG..; see table 3). The new determined sequence, named iMab114, (table 4) was constructed according to the gene construction procedure as described above (example 3) and inserted in CM114 replacing iMab100.

### Example 43

### Amplification of camelidae derived CDR3 regions

Lama pacos and Lama glama blood lymphocytes were isolated according to standard procedures as described in Spinelli et al. (Biochemistry 39 (2000) 1217-1222). RNA from these cells was isolated via Qiagen RNeasymethods according tomanufactures protocol. cDNA was generated usingmuMLv or AMV (New England Biolabs) according tomanufactures procedure. CDR3 regions from Vhh cDNA were amplified (see figure 10) using 1 µl cDNA reaction in 100microliter PCR reactionmix comprising 2 units Taq polymerase (Roche), 200 µM of each dNTP (Roche), buffers (Roche Taq buffer system), 2.5 µM of forward and reverse primers in a Primus96 PCRmachine (MWG) with the following program 35 times [94°C 20", 50°C 25", 72°C 30"]. In order to select for CDR3 regions containing at least one cysteine primer 56 (table 5) was used as a forward primer and in case to select for CDR regions that do not contain cysteines primer 76 (table 5) was used in the first PCR round. In both cases primer 16 (table 5) was used as reverse primer. Products were separated on a 1% Agarose gel and products of the correct length (∼ 250 bp) were isolated and purified using Qiagen gel extraction kit. 5 µl of these products were used in a next round of PCR similar as described above in which primer 8 (table 5) and primer 9 (table 5) were used to amplify CDR3 regions. Products were separated on a 2% Agarose gel and products of the correct length (∼80 - 150 bp) were isolated and purified using Qiagen gel extraction kit. In order to adapt the environment of the camelidae CDR3 regions to scaffold iMab100 two extra rounds of PCR similar to the first PCRmethod was performed on 5 µl of the products with the exception that the cycle number was decreased to 15 cycles and in which primer 73 (table 5) and 75 (table 5) were subsequently used as forward primer and primer 49 (table 5) was used as reverse primer.

### Example 44

### Amplification of cow derived CDR3 regions

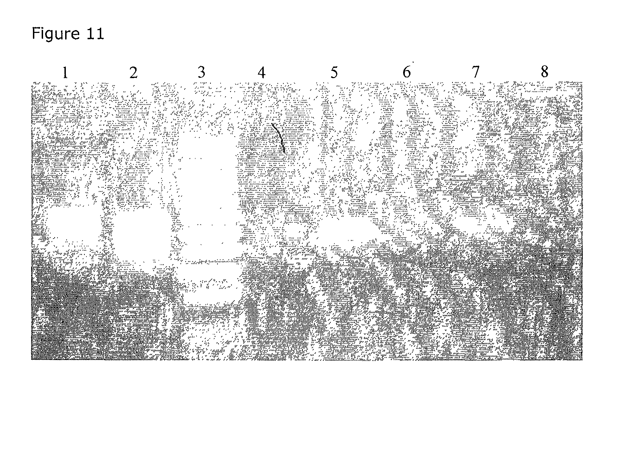

Cow (*Bos taurus*) blood lymphocytes were isolated according to standard procedures as described in Spinelli et al. (Biochemistry 39 (2000) 1217-1222). RNA from these cells was isolated via Qiagen RNeasymethods according tomanufactures protocol. cDNA was generated usingmuMLv or AMV (New England Biolabs) according tomanufactures procedure. CDR3 regions from Vh cDNA was amplified using 1 µl cDNA reaction in 100microliter PCR reactionmix comprising 2 units Taq polymerase (Roche), 200 µM of each dNTP (Roche), buffers (Roche Taq buffer system), 2.5 µM of primer 299 (table 5) and 300 (table 5) in a Primus96 PCRmachine (MWG) with the following program 35 times [94°C 20", 50°C 25", 72°C 30"]. Products were separated on a 2% Agarose gel and products of the correct length were isolated and purified using Qiagen gel extraction kit. The length distribution of the PCR products observed (see figure 11) represents the average length of cow CDR3 regions.

Correcting for framework sequences that are present in primer 299 (21 amino acids; table 5) and 300 (27 amino acids; table 5) it can be concluded that the average length of cow CDR3s is: 120 base average PCR product lengthminus 48 base frameworks determines 72 bases and thus 24 amino acids. This result corresponds very well with the results observed by Spinelli et al. (Biochemistry 39 (2000) 1217-1222). These CDR regions are therefore extremely useful for naive library constructions.

Isolated and purified products can be used to adapt the sequences around the actual CDR3/AR4 location in a way that the coding regions of the frameworks are gradually adapted via several PCRmodifications rounds similarly as described for lama derived ARs (see example 43).

### Example 45

### Libraries containing loop variegations in AR4 by insertion of amplified CDR3 regions

A nucleic acid phage display library having variegations in AR4 was prepared by the followingmethod. Amplified CDR3 regions from lama's immunized with lactoperoxidase and lactoferrin was obtained as described in example 43 and were digested with *PstI* and *KpnI* and ligated with T4 DNA ligase into the *PstI* and *KpnI* digested and alkaline phosphatase treated vector CM114-iMab113 or CM114-iMab114. Cysteine containing CDR3s were cloned into CM114-iMab114 while CDR3s without cysteines were cloned into vector CM114-iMab113. The libraries were constructed by electroporation into *E. coli* TG1 electrocompetent cells by using a BTX electrocellmanipulator ECM 630. Cells were recovered in SOB and grown on plates that contained 4% glucose, 100microgram ampicillin permilliliter in 2*TY-agar. After overnight culture at 37 °C, cells were harvested in 2*TYmedium and stored in 50% glycerol as concentrated dispersions at -80°C. Typically, 5x 10⁸ transformants were obtained with 1 µg DNA and a library contained about 10⁹ independent clones.

### Example 46

### Libraries containing loop variegations in AR4 by insertion of randomized CDR3 regions

A nucleic acid phage display library having variegations in AR4 by insertion of randomized CDR3 regions was prepared by the followingmethod. CDR3 regions from non-immunized and immunized lama's were amplified as described in example 28 except that in the second PCR round dITP according to Spee et al. (1993) or dPTP according to Zaccolo et al. (1996) were included as described in example 35. Preparation of the library was performed as described in example 28. With dITP amutation rate of 2 % was achieved while with dPTP included in the PCR amutation rate of over 20% was obtained.

### Example 47

### Enrichment of VAPs that bind to targetmolecules

About 50microliter of the library stocks was inoculated in 50ml 2*TY/100microgram ampicillin/4% glucose and grown until an OD600 of 0.5 was reached. Next 10¹¹ VCSM13 (Stratagene) helper phages were added. The culture was left at 37°C without shaking for 45minutes to enable infection. Cells were pellet by centrifugation and the supernatant was discarded. Pellets were resuspended in 400ml 2*TY/100microgram ampicillin and cultured for 1 hour at 37°C after which 50µg/ml kanamycine was added. Infected cultures were grown at 30°C for 8 hours on a 200rpm shaking platform. Next, bacteria were removed by pelleting at 5000g at 4°C for 30minutes. The supernatant was filtered through a 0.45micrometer PVDF filtermembrane. Poly-ethylene-glycol and NaCl were added to the flow through with final concentrations of respectively 4% and 0.5M. In this way phages precipitated on ice and were pelleted by centrifugation at 6000g. The phage pellet was solved in 50% glycerol/50% PBS and stored at -20°C.

The selection of phage-displayed VAPs was performed as follows. Approximately 1 µg of a targetmolecule (antigen) was immobilized in an immunotube (Nunc) ormicrotiter plate (Nunc) in 0.1m sodium carbonate buffer (pH 9.4) at 4 °C o/n. After the removal of this solution, the tubes were blocked with a 3% skimmilk powder solution (ELK) in PBS or a similar blocking agent for at least 2 hrs either at room temperature or at 4 °C o/n. After removal of the blocking agent a phagemid library solution containing approximately 10¹²-10¹³ colony forming units (cfu), which was preblocked with blocking buffer for 1 hour at room temperature, was added in blocking buffer. Incubation was performed on a slow rotating platform for 1 hour at room temperature. The tubes were then washed three times with PBS, two times with PBS with 0.1% Tween and again four times with PBS. Bound phages were eluted with an appropriate elution buffer, either 300 µl 0.1m glycine pH 2.2 or 500 µl 0.1 % trypsin in PBS. Recovered phages were immediately neutralized with 700 µl 1m Tris-HCl pH 8.5 if eluted with glycine. Alternatively the bound phages were eluted by incubation with PBS containing the antigen (1-10 µM). Recovered phages were amplified as described above employing *E.coli* XLI-Blue (Stratagene) or Top10F' (InVitrogen) cells as the host. The selection process was repeated several times to concentrate positive clones. After the final round, individual clones were picked and their binding affinities and DNA sequences were determined.

The binding affinities of VAPs were determined by ELISA as described in example 6, either as gIII-fusion protein on the phage particles or after subcloning as a *Nde*I-*Sfi*I into the expression vector CM126 as described in example 4 *E.coli* BL21(DE3) or Origami(DE3) (Novagen) were transformed by electroporation as described in example 5 and transformants were grown in 2x TYmedium supplemented with Ampicillin (100 µg/ml). When the cell cultures reached an OD600 ∼ 1 protein expression was induced by adding IPTG (0.2mM). After 4 hours at 37 °C cells were harvested by centrifugation. Proteins were isolated as described in example 5

### Example 48

### Enrichment for Lactoferrin binding VAPs

Purified Lactoferrin (LF) was supplied by DMV-Campina.

A phage display library with variegations in AR4 as described in example 45 was used to select LF binding VAPs. LF (10 microgram in 1ml sodium bicarbonate buffer (0.1m, pH 9.4)) was immobilized in an immunotube (Nunc) followed by blocking with 3% chicken serum in PBS. Panning was performed as described in example 32. 10¹³ phages were used as input. After the 1^{st} round of panning about 10000 colonies were formed. After the 2^{nd} panning round 500 to 1000 colonies were formed. Individual clones were grown and VAPs were produced and checked by ELISA as described in example 6. Enrichment was found for clones with the following AR4:

CAAQTGGPPAPYYCTEYGSPDSW

### Example 49

### Enrichment for Lactoperoxidase binding VAPs

Purified Lactoperoxidase (LP) was supplied by DMV-Campina.

A phage display library with variegations in AR4 as described in example 45 was used to select LP binding VAPs. LP (10 microgram in 1ml sodium bicarbonate buffer (0.1m, pH 9.4)) was immobilized in an immunotube (Nunc) followed by blocking with 3% chicken serum in PBS. Panning was performed as described in example 32 10¹³ phages were used as input. After the 1^{st} round of panning about 5000 colonies were formed. After the 2^{nd} panning round 500 to 1000 colonies were formed. Individual clones were grown and VAPs were produced and checked by ELISA as described in example 6. Positive clones were sequenced. Enrichment was found for clones with the following AR4:

CAAVLGCGYCDYDDGDVGSW

CAATENFRIAREGYEYDYW
CAATSDFRIAREDYEYDYW

### Example 50

### RNase A binder, constructionmaturation and panning.

A synthetic RNase A binding iMab, iMab130, was synthesized as described in example 3 (table 4, table 3) and subsequently cloned into CM114 forming CM114-iMab130. Chimeric phages with iMab130 as a fusion protein with the g3 coat protein were produced under conditions as described for library amplification procedure in example 32 Panning with these chimeric phages against RNase A coated immunotubes (see example 32 for panning procedure) failed to show RNase A specific binding of iMab130. Functional positioning of the RNase A binding regions had clearly failed, probably due tominor distortions of surrounding amino acid side chains. Smallmodifications of the scaffoldmight help to displace ARs into correct positions. In order to achieve this, the iMab130 coding region wasmutated using the followingmethod: iMab130 present in vector CM114 wasmutagenised using either dITP or dPTP during amplification of the scaffold with primers 120and 121(table...).mutagenizing concentrations of 1.7mM dITP or 300µM, 75 µM or 10 µM dPTP were used. Resulting PCR products were isolated from an agarose gel via Qiagen's gel elution system according tomanufactures procedures. Isolated products were amplified in the presence of 100µM of dNTPs (Roche) in order to generate dITP and dPTP free products. After purification via Qiagen's PCR clean up kit, these PCR fragments were digested with *NotI* and *SfiI* (NEB) and ligated into *NotI* and *SfiI* linearized CM114. Precipitated and 70% ethanol washed ligation products were transformed into TG1 bymeans of electroporation and grown in 2xTYmedium containing 100µg/ml ampicillin and 2% glucose and subsequently infected with VCSM13 helper phage (Stratagene) for chimeric phage production as described in example 32. Part of the transformation was plated on 2xTY plates containing 2% glucose and 100microgram/ml ampicillin to determine transformation frequency:

These phage libraries were used in RNase A panning experiments as described in example 32 RNase A was immobilized in immunotubes and panning was performed. After panning, phages were eluted and used for infection of TOP10 F' (InVitrogen), and grown overnight at 37°C on 2xTY plates containing 2% glucose and 100µg/ml ampicillin and 25microgram/ml tetracycline. The number of retrieved colonies is indicated in table 17.

As can be concluded from the number of colonies obtained after panning with phage libraries derived from differentmutagenesis levels of iMab130, a significant increase of binders can be observed from the library with a mild mutagenesis level, being dITP (table 17)

### Example 51

### Immobilisation procedure

1g of epoxy activated Sepharose 6B (manufacturer Amersham Biosciences) was packed in a column and washed with 10 bed volumes coupling buffer (200mM potassium phosphate, pH 7). The protein to be coupled was dissolved in coupling buffer at a concentration of 1mg/ml and passed over the column at a flow rate of 0.1ml/min. After passing 20 bed volumes of protein solution, the column was washed with coupling buffer. Passing 10 bed volumes of 0.2M ethanolamine/ 200mM potassium phosphate pH 7 blocked the unreacted epoxy groups. The resin was then washed with 20 bed volumes of 50mM potassium phosphate pH 7 after which it was ready for use.

### Example 52

### iMab100 purification via Lysozyme immobilized beads

Lysozyme was immobilized on Eupergit, an activated epoxy-resin from Rohm and used in a column. A solution containing iMab100 was passed on the column and the concentration wasmeasured in a direct bypass and the flow through from the column (A280 nm). The difference indicated the amount of iMab100 that was bound to the column. The bound iMab100 could be released with a CAPS buffer pH11. Control experiments with BSA indicated that the binding of iMab100 to immobilized lysozyme was specific.

### Example 53

### Lysozyme purification via iMab100 immobilized beads

iMab100 was immobilized on Eupergit and used in a column. A solution containing Lysozyme was passed on the column and the concentration wasmeasured and in a direct bypass and the flow through from the column (A280 nm). The difference indicated the amount of Lysozyme that was bound to the column. The bound Lysozyme could be released with a CAPS buffer pH11. Control experiments with BSA indicated that the binding of Lysozyme to immobilized iMab100 was specific.

### Example 54

### Stability of iMab100 in whey fractions

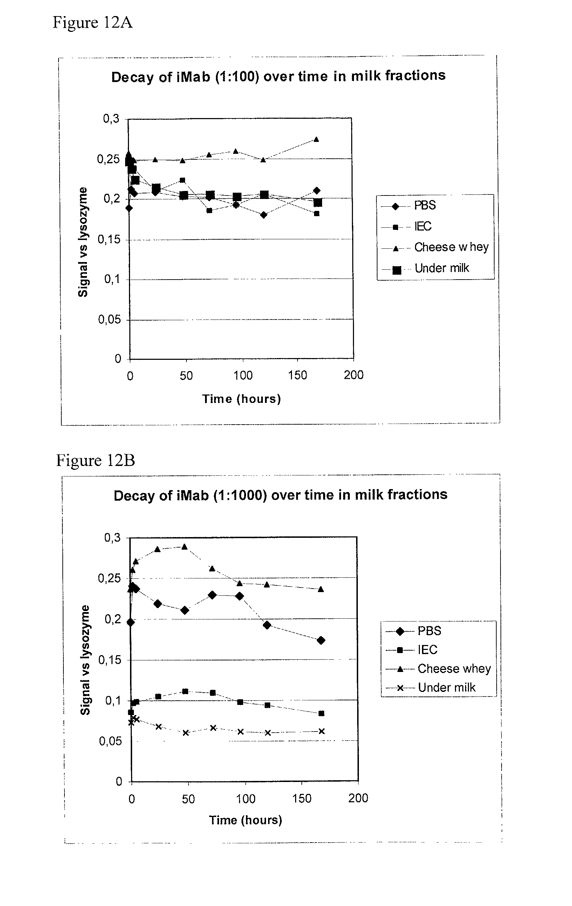

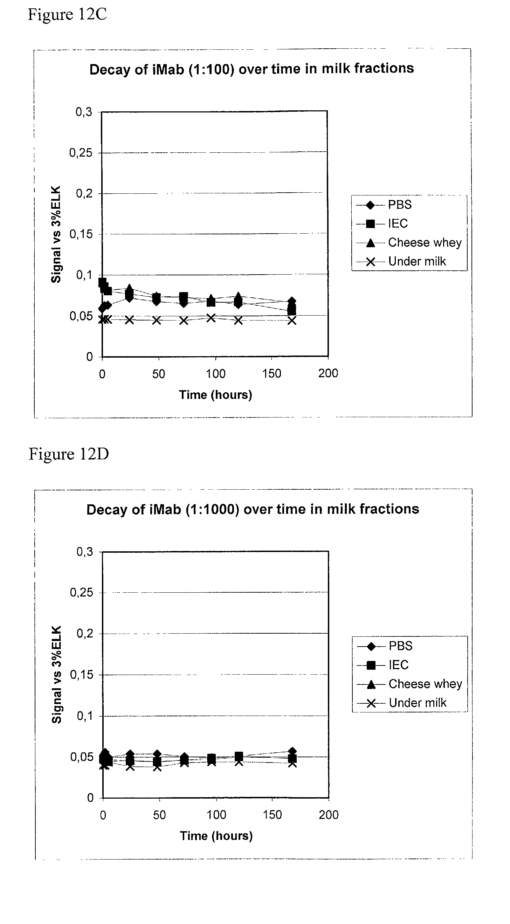

The stability of iMab100 in several milk fractions was measured by lysozyme coated plates via ELISA methods (example 8). If the tags, scaffold regions or affinity regions were proteolytically degraded, a decreased anti-lysozyme activity would be observed. iMab100 was diluted in several different solution: 1xPBS as a control, ion-exchange fraction from cheese-whey, gouda-cheese-whey and low pasteurised undermilk, 1.4 µm filtered to a final concentration of 40 µg/ml. All fractions were stored at 8 °C, samples were taken after:0, 2 and 5 hours and after 1, 2, 3, 4, 5 and 7 days. Samples were placed at -20 °C to prevent further degradation. ELISA detection was performed as described in example 8 and shown in figure 12. The activity pattern of iMab100 remained similar throughout the experiment. Therefore it can be concluded that iMab100, including the tags, were stable in assayed milk fractions.

### Example 55. Preparation of ligands

Skin samples were harvested from two female donors undergoing cosmetic surgery (buttocks and abdomen) and were processed within 2-6 hours after removal with transport to the laboratory on dry ice at 4 degrees C. Before removal, the skin was desinfected with propyl-ethanol based solution and iodine-betadine. Processing was started with three times washes in PBS to remove all blood under sterile conditions. A dermatome set at 0.3 mm thickness was used to shave the epidermis with a thin layer of dermis (the splitskin). The splitskin surface integrity was not preserved during this procedure and the samples were washed three more times in sterile PBS, then frozen to -80 degrees C. To obtain keratin enriched skin fractions, the frozen samples were grinded in liquid nitrogen, rinsed with 2% non-ionic detergent (such as Tween-20, Triton X-100 or Brij-30) or ethanol. External lipids were removed using a mixture of chloroform-methanol (2:1) for 24 h. The delipidized hair was resuspended in an alkaline buffer (such as Tris-HCl pH 9), preferably in 6 M urea, but a range of 5 - 8 M urea is possible, preferably 1 M thiourea but a range of 0-3 M thiourea is possible and 5% of a reducing agent (such as β-mercaptoethanol or dithiothroetol) ) and stirred at 50 °C for 1-3 days. The mixture was filtered and centrifuged (15.000 rpm, 30 min). The supernatant was dialyzed against 10-50 mM of an alkaline buffer (such as Tris-HCl pH 9) to remove low molecular weight impurities. The dialyzation buffer may contain additives such as reducing agents. The obtained protein fraction was used as an antigen and may be treated with iodoacetic acid to prevent reformation of disulfide bonds. The pellet fraction (containing insoluble proteins) was washed with distilled water and grounded using a homogenizer (such as a Wiley Mill) to a small particle size (i.e all of the particles which pass through a 40 mesh screen). The small particles was resuspended in a buffer to a stable suspension, dialyzed and used as an antigen.

Hair samples were harvested from diverse sources, representing different ethnic backgrounds and including, blond, brown, black curly, black straight and grey hairs. Cuticle-enriched fractions were obtained in a similar extraction procedure as described for keratin enriched skin fractions.

### Example 56. Coupling and release of fragrance molecules to VAP

A C-8 Aldehyde (Octanal) was chosen to test labeling of the VAP with a volatile compound and subsequent release by hydrolysis. Octanal (MW 128.21) occurs in several citrus oils, e.g orange oils. It is a colorless liquid with a pungent odor, which becomes citrus-like on dilution. Octanal was first allowed to react with the amino groups of the VAP and form an Imine bond. We then used aqueous solutions ofHCl and NaOH to hydrolyze the bonds and release the volatile aldehyde.

### 1. Formation of an Imine Bond

*Labeling the VAP with Octanal*: 5 mg of VAP were dissolved in 500 □l of phosphate buffer (0.5 N, pH 7.5). 50 □□ of C-8 Aldehyde (Octanal) were then added to the mixture, which was then allowed to incubate at room temperature for 18 hours.

*Purification of the VAP-Fragrance Complex:* The mixture solution from above was purified using a Ni-NTA column (spin column from Qiagen, used according to standard manufacturer procedures). The mixture was purified and all unbound fragrance was eluted using phosphate buffer (0.5N pH 7) by centrifuging 6 times for 2 minutes at 2000 rpm at 700 x g. The column was then further air dried for 30 minutes to rid the column of all background fragrance from unbound Octanal.

*Release of the Fragrance:* Fragrance was released from the Ni-NTA column by adding a solution of either 3.7 % aqueous HCl or a 5M NaOH, spinning for 2 minutes at 2000 rpm at 700 x g in a mini-centrifuge. Using a pump, air was flushed into the column and released fragrance was evaluated by a 6 person-panel. All release was obtained by evaluating the difference in fragrance from the VAP-fragrance complex upon addition of releasing agents.

| | Release with HCl | Release with NaOH |
|---|---|---|
| C8-Aldehyde | ++++ | ++ |
| No Fragrance | - | - |

### 2. Formation of the Amine Bond

To further test our assumption, the imine was further reduced using sodium borohydride to form a much more stable and non-hydrolyzable amine bond.
*a. Labeling of the VAP:* The procedure was repeated as outlined above for the Imine bond. In addition, after 18 hours, 100 □□ of sodium borohydride (0.1 M pH 9) were added and the mixture was incubated at room temperature for 1.5 hours.
b. *Purification of the VAP with fragrance*:*:* The mixture solution from above was purified using a Ni-NTA column (spin column from Qiagen, used according to standard manufacturer procedures). The mixture was purified and all unbound fragrance was eluted using phosphate buffer (0.5N pH 7) by centrifuging 6 times for 2 minutes at 2000 rpm at 700 x g. The column was again allowed to air-dry using an air pump for 1.5 hour.
c. *Testing for Release:* using the same method as previously mentioned, a very minimal release of fragrance was now discernable and the olfactory index was comparable to a control of unbound VAP.
*d. Release of Octanal-VAP complex from the Ni-NTA column*
   The Octanal-VAP complex was eluted from the column according to the standard manufacturer procedures, and diluted with SDS-PAGE sample buffer, boiled for 5 min at 95 degrees C.
e. *Molecular Weight determination:* The Octanal labeled VAP was loaded on SDS-PAGE (15%, denaturing conditions) and was run against a control unbound VAP. The heavier (est. 1 kilodalton) molecular weight shown by the slower migration confirmed binding of the Octanal to VAP through an amide bond.

### Example 57. Coupling of color compound to VAP

Rhodamine and its derivatives are water-soluble basic dyes used in labeling all types of bio-molecules. Tetramethyl-rhodamine-5-(and 6)-isothiocyanate (TRITC) is a derivative of tetramethyl-rhodamine, which reacts with nucleophiles such as amines, sulfihydryls, and the phenolate ion of tyrosine side chains. The only stable product however is with the primary amine groups, and so TRITCC is almost entirely selective for the modification of □- and N-terminal amines in proteins. The reaction involves attack of the nucleophile on the central, electrophilic carbon of the isothiocyanate group.

Five mg of an anti-chickenLysozyme-VAP (antiCLys-VAP) were dissolved in 1 ml of sodium carbonate buffer (0.1M, pH 9). Aliquots of 10 □□ of a Rhodamine TRITCC solution in DMSO (10 mg/ml) were added 5 times with mixing in between. The mixture was incubated at 4 °C in the dark at 5 rotations per minute. 50 □□□ of an ammonium chloride solution (1M) were then added and the mixture was then incubated for 2 hours at 4 °C at 5 rotations per minute.

The labeled protein was then purified using a Sephadex G-25 column. Elutions were then tested for color appearance along with protein content using Bradford's reagent showing coinciding peaks for both protein and rhodamine.

### Example 58. Coupling of antiCLys-VAP to hair

The purpose of this experiment is to directly label hair coated with Lysozyme protein using a Rhodamine-TRITCC-labeled-antiCLys-VAP.

Hair strands (approximately 0.5 grams) were rinsed with potassium phosphate buffer (0.5 M pH 7.6). The hair strands were then immersed in 1.5 ml of a 25% solution of aqueous glutaraldehyde and incubated for 18 hours at 37 °C. The hair was then washed thoroughly with phosphate buffer and water.

Hair strands were then transferred to a 1 ml solution of phosphate buffer, to which was also added 100 □□ of Egg White Lysozyme (0.1g in 1 ml stock solution). The mixture was allowed to react overnight at 4 °C. The hair strands were then thoroughly washed with coupling buffer and then water.

All remaining aldehydes and other double bonds were then eliminated by adding 100 □□ of sodium borohydride (0.1 M). The hair strands were then washed with water and then resuspended in 1000 m phosphate buffer. 10 □□ of TRITCC-labelled antiCLys-VAP with the highest protein content obtained from the G-25 purification step of the previous example was then added to an Eppendorf tube containing hair samples in 100 □□ of 1x PBS buffer (pH 8). The reaction conditions are summarized below, indicating specific binding of TRITCC-labeled VAPs to hair, via the crosslinked lysozyme that was coupled to the hair surface:

| | Sample | fluorescence |
|---|---|---|
| 1 | Hair + blocking agent | weak |
| 2 | Hair | weak |
| 3 | Hair-Lysozyme + blocking agent | strong |
| 4 | Hair-Lysozyme | strong |

An Elisa assay with Lysozyme bound to the surface of the wells confirmed that the TRITCC-coupling did not interfere with the VAP-affinity for lysozyme. The Elisa reaction was done with anti-Vsv-horse radish peroxidase, detecting the Vsv tag that is present on the carboxy terminas of the antiCLys-VAP

| sample size | VAP - TRITCC | VAP | Blocking Buffer |
|---|---|---|---|
| 1000 ng | 0.239 | 0.266 | 0.065 |
| 500 ng | 0.132 | 0.136 | 0.074 |
| 250 ng | 0.089 | 0.099 | 0.044 |
| 100 ng | 0.077 | 0.118 | 0.045 |
| 50 ng | 0.063 | 0.055 | 0.040 |

### Example 58. Coupling of antiHair-VAP to hair

Selected VAPs that were specific for the hair surface, were coupled with TRITCC and other example compounds known to those in the art of hairdyes, were used in similar experiments as described above, demonstrating the feasibility of hair coloring via VAPs under benign chemical conditions.

### Example 60. Bivalent hair-conditioning agents

A VAP with hair keratin-binding specificity was selected from phage display libraries using methods known to those skilled in the art. Bi-valent molecules (mono-specific or bi-specific for keratin) can easily be synthesized by duplicating the corresponding DNA sequence and adding flexible or inflexible, long or short spacers. As an illustrative example, a spacer is described in the sequence SGGGGSGGGGSGGGG. Such bi-valent VAPs are non-aggressive hair perming agents as they tend to crosslink individual hairs directly upon contact. The flexibility of the spacer will determine the strength and feel of the perming agent, ranging from permanent hair waves to slight gelling agent effects. Besides the example spacer, many other spacers are described in scientific literature to fuse proteins together. Even cross-linking agents such as glutharaldehyde can be used to couple mono-valent VAP's in ways that the affinity to hair is not entirely lost.

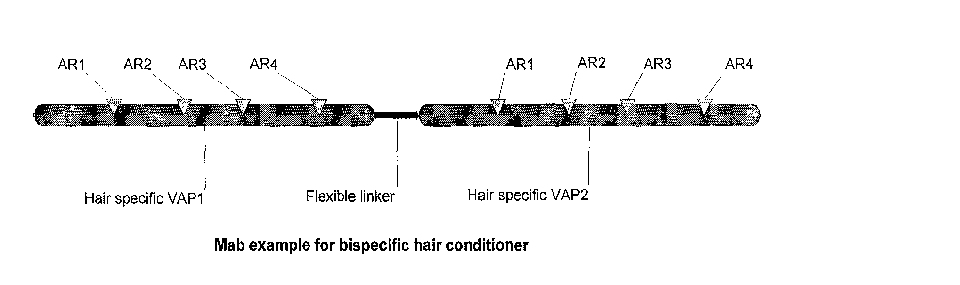

NVKLVEKGGNFVENDDDLKLTCRAEXXXXXXMGWFRQAPNDDSTNVATI
XXXXXXYGDSVKERFDIRRDXXXXXXNTVTLSMDDLQPEDSAEYNCXXXXX
XDSHYRGQGTDVTVSS (VAP1)
ggggsggggsggggs (linker)
NVKLVEKGGNFVENDDDLKLTCRAEXXXXXXMGWFRQAPNDDSTNVATI
XXXXXXYGDSVKERFDIRRDXXXXXXNTVTLSMDDLQPEDSAEYNCXXXXX
XDSHYRGQGTDVTVSS (VAP2)

To apply the perm agent to the hair, the hair is contacted with an effective amount of the bi-valent VAP's as described in the invention (i.e. an amount that is sufficient to achieve a noticable conditioning effect to the hair, depending on the affinity characteristics of the surface binding agent that is isolated from the panning procedure). Preferably, the perm agent is formulated with a suitable diluent that does not react with the perm agent, preferably a water-based diluent. Preferably, bi-valent VAP's are applied to the hair of one human head at a rate of 0.001 g to about 1 g per usage. In another preferred example, the bi-valent VAP is applied directly in a shampoo composition as are widely known in the art.

### Brief description of the tables and drawings

Table 1
   Examples of nine stranded (strands-only) of in PDB format
Table 2
   Example amino acid sequences likely to fold as nine stranded iMab proteins
Table 3: VAP amino acid sequences
Table 4
   iMab DNA sequences
Table 5
   List of primers used.
Table 6.
   Binding characteristics of purified iMab variants to lysozyme.
   Various purified iMabs containing either 6-, 7-, or 9 β-sheets were analyzed for binding to ELK (control) and lysozyme as described in examples 8, 15,19 and 23.
   All iMabs were purified using urea and subsequent matrix assisted refolding (example 7), except for iMab100 which was additionally also purified by heat-induced solubilization of inclusion bodies (example 6).
Table 7
   Effect of pH shock on iMab100, measured in Elisa versus lysozyme before and after precipitation by Potassiumacetate pH 4.8.
Table 8
   Four examples of seven-stranded (strands-only) folds in PDB 2.0 format to indicate spatial conformation.
Table 9
   PROSAII results (zp-comp) and values for the objective function from MODELLER for 7-stranded iMab proteins. Lower values correpond to iMab proteins which are more likely to fold correctly.
Table 10
   Example amino acid sequences less likely to fold as seven stranded iMab proteins
Table 11
   Four examples of six-stranded (strands-only) folds in PDB 2.0 format to indicate spatial conformation.
Table 12
   PROSAII results (zp-comp) and values for the objective function from MODELLER for 6-stranded iMab proteins. Lower values correpond to iMab proteins which are more likely to fold correctly.
Table 13
   Example amino acid sequences likely to fold as six stranded iMab proteins
Table 14
   PROSAII results (zp-comp) from iMab100 derivatives of which lysine was replaced at either position 3, 7, 19 and 65 with all other possible amino acid residues. Models were made with and without native cysteine bridges. The more favourable derivatives (which are hydrophilic) are denoted with X.
Table 15
   PROSAII results (zp-comp) from iMab100 derivatives of which cysteine at position 96 was replaced with all other possible amino acid residues.
Table 16
   A) Amino acid sequence of iMab100 (reference) together with the possible candidates for extra cysteine bridge formation. The position where a cysteine bridge can be formed is indicated.
   B)Preferred locations for cysteine bridges with their corresponding PROSAII score (zp-comp) and the corresponding iMab name.
Table 17
   Effect of mutation frequency of dITP on the number of binders after panning
Table 18
   Sequences of the vectors used in example 40 and in example 4.

### Figure 1

### Schematic 3D-topology of scaffold domains.

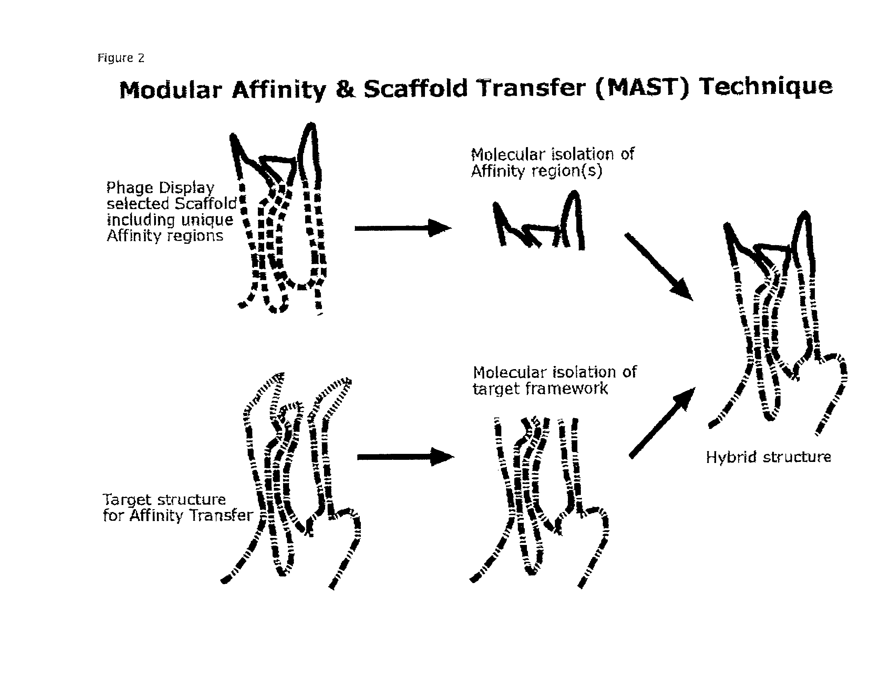

Eight example topologies of protein structures that can be used for the presentation of antigen binding sites are depicted. The basic core beta elements are the nominated in example A. This basic structure contains 9 beta-elements positioned in two plates. One beta-sheets contains elements 1, 2, 6 and 7 and the contains elements 3, 4, 5, 8 and 9. The loops that connect the beta-elements are also depicted. Bold lines are connecting loops between beta-elements that are in top position while dashed lines indicate connecting loops that are located in bottom position. A connection that starts dashed and ends solid indicates a connection between a bottom and top part of beta-elements. The numbers of the beta-elements depicted in the diagram correspond to the numbers and positions mentioned in figures 1 and 2.
A: 9 beta element topology: for example all antibody light and heavy chain variable domains and T-cell receptor variable domains
B: 8 beta element topology: for example interleukin-4 alpha receptor (LIAR)
C: 7a beta element topology: for example immunoglobulin killer receptor 2dl2 (2DLI)
D: 7b beta element topology: for example E-cadherin domain (1FF5)
E: 6a beta strand topology
F: 6b beta element topology: for example Fc epsilon receptor type alpha (1J88)
G: 6c beta element topology: for example interleukin-1 receptor type-1 (1GOY)
H: 5 beta element topology

### Figure 2

### Modular Affinity & Scaffold Transfer (MAST) Technique.

Putative antigen binding proteins that contain a core structure as described here can be used for transfer operations. In addition, individual or multiple elements or regions of the scaffold or core structures can also be used for transfer actions. The transfer operation can occur between structural identical or comparable scaffolds or cores that differ in amino acid composition. Putative affinity regions can be transferred from one scaffold or core to another scaffold or core by for example PCR, restriction digestions, DNA synthesis or other molecular techniques. The results of such transfers is depicted here in a schematic diagram. The putative (coding) binding regions from molecule A (top part, affinity regions) and the scaffold (coding) region of molecule B (bottom part, framework regions) can be isolated by molecular means. After recombination of both elements a new molecule appears (hybrid structure) that has binding properties of molecule A and scaffold properties of scaffold B.

### Figure 3

### Domain notification of immunoglobular structures.

The diagram represents the topologies of protein structures consisting of respectively 9, 7 and 6 beta-elements (indicated 1-9 from N-terminal to C-terminal). Beta elements 1,2, 6 and 7 and elements 3, 4, 5, 8 and 9 form two beta-sheets.

Eight loops (L1-L8) are responsible for the connection of all beta-elements. Loop 2, 4, 6 and 8 are located at the top site of the diagram and this represents the physical location of these loops in example proteins. The function of loops 2,4 and 8 in light and antibody variable domains is to bind antigens, known as CDR regions. The position of L6 (also marked with a patterned region) also allows antigen binding activity, but has not been indicated as a binding region. L2, L4, L6, L8 are determined as affinity region1 (AR1), AR2, AR3 and AR4 respectively. Loops 1, 3, 5 and 7 are located at the opposite site of the proteins.

### Figure 4

A) Schematic overview of vector CM126
B) Schematic overview of vector CM126

### Figure 5

### Solubilization of inclusion bodies of iMab100 using heat (60°C)

Lanes: Molecular weight marker (1), isolated inclusion bodies of iMab100 (2), solubilized iMab100 upon incubation of inclusion bodies in PBS pH 8 + 1% Tween-20 at 60°C for 10 minutes.

### Figure 6

### Purified iMab variants containing either 6-,7- or 9 beta-sheets.

Lanes: Molecular weight marker (1), iMab1300 (2), iMab1200 (3), iMab701 (4), iMab101 (5), iMab900 (6), iMab122 (7), iMab1202 (8), iMab1602 (9), iMab1302 (10), iMab116 (11), iMab111 (12), iMab100 (13).

### Figure 7

### Stability of iMab100 at 95 °C

Purified iMab100 incubated for various times at 95°C was analysed for binding to ELK(squares) and lysozyme (circles).

### Figure 8

### Stability of iMab100 at 20°C

Purified iMab100 incubated for various times at 20°C was analysed for binding to ELK (squares) or chicken lysozyme (circles).

### Figure 9 A-L,

A. far UV CD spectum (205-260 nm) of iMab100 at 20 °C, 95 °C , and again at 20 °C. iMab100 was dissolved in 1xPBS, pH 7.5.
B. iMab111, far UV spectrum determined at 20 °C, (partially) denatured at 95 °C, and refolded at 20 °C, compared to the iMab100 spectrum at 20 °C.
C. iMab116, far UV spectrum determined at 20 °C, (partially) denatured at 95 °C, and refolded at 20 °C, compared to the iMab100 spectrum at 20 °C.
D. iMab1202, far UV spectrum determined at 20 °C, (partially) denatured at 95 °C, and refolded at 20 °C, compared to the iMab100 spectrum at 20 °C.
E. iMab1302, far UV spectrum determined at 20 °C, (partially) denatured at 95 °C, and refolded at 20 °C, compared to the iMab100 spectrum at 20 °C.
F. iMab1602, far UV spectrum determined at 20 °C, (partially) denatured at 95 °C, and refolded at 20 °C, compared to the iMab100 spectrum at 20 °C.
G. iMab101, far UV spectrum determined at 20 °C, (partially) denatured at 95 °C, and refolded at 20 °C
H. iMab1200, far UV spectrum determined at 20 °C, (partially) denatured at 95 °C, and refolded at 20 °C
I. iMab701, far UV spectrum determined at 20 °C, (partially) denatured at 95 °C, and refolded at 20 °C
J. Overlay of native (undenatured) 9 strand iMab scaffolds.
K. Overlay of native (undenatured) 7 strand iMab scaffolds.
L. Far UV CD spectra of iMab100 and a V_{HH} (courtesy Kwaaitaal M, Wageningen University and Research, Wageningen, the Netherlands).

### Figure 10

### Schematic overview of PCR isolation of CDR3 for MAST.

### Figure 11

### Amplification Cow derived CDR3 regions

2% Agarose - TBE gel.
Lane 1. 1 microgram Llama cDNA cyst+, PCR amplified with primers 8 and 9.
Lane 2. 1 microgram Llama cDNA cyst-, PCR amplified with primers 8 and 9.
Lane 3. 25 bp DNA step ladder (Promega).
Lane 4. 0.75 microgram Cow cDNA PCR amplified with primers 299 and 300.
Lane 5. 1.5 microgram Cow cDNA PCR amplified with primers 299 and 300.
Lane 6. 0.75 microgram Cow cDNA PCR amplified with primers 299 and 301.
Lane 7. 1.5 microgram Cow cDNA PCR amplified with primers 299 and 301.
Lane 8. 50 bp GeneRuler DNA ladder (MBI Fermentas).

### Figure 12

Lysozyme binding activity measured with ELISA of iMab100. Several different solutions were tested in time for proteolytic activity on iMab100 proteins. Test samples were diluted 100 times in figures A) and C) while samples were 1000 times diluted in figures B) and D). A) and B) show lysozyme activity while C) and D) show background activity.

**Table 1**

| 1Neu | | | | | | | | | | |
|---|---|---|---|---|---|---|---|---|---|---|
| ATOM | 1 | CA | GLY | 2 | -9.450 | -13.069 | 10.671 | 1.00 | 25.06 | C |
| ATOM | 2 | CA | GLY | 3 | -9.868 | -10.322 | 8.019 | 1.00 | 20.77 | C |
| ATOM | 3 | CA | GLY | 4 | -6.884 | -9.280 | 5.813 | 1.00 | 19.01 | C |
| ATOM | 4 | CA | GLY | 5 | -6.047 | -5.991 | 4.016 | 1.00 | 19.75 | C |
| ATOM | 5 | CA | GLY | 6 | -2.638 | -4.349 | 3.125 | 1.00 | 22.33 | C |
| ATOM | 6 | CA | GLY | 7 | -1.382 | -1.720 | 5.647 | 1.00 | 24.80 | C |
| ATOM | 7 | CA | GLY | 8 | -0.685 | 1.080 | 3.150 | 1.00 | 28.23 | C |
| ATOM | 8 | CA | GLY | 9 | -0.917 | 1.393 | -0.623 | 1.00 | 26.37 | C |
| ATOM | 9 | CA | GLY | 10 | 0.737 | 3.923 | -2.887 | 1.00 | 29.08 | C |
| ATOM | 10 | CA | GLY | 11 | -0.741 | 5.082 | -6.156 | 1.00 | 26.48 | C |
| ATOM | 11 | CA | GLY | 12 | 0.157 | 7.450 | -8.989 | 1.00 | 27.26 | C |
| ATOM | 12 | CA | GLY | 13 | -2.216 | 10.173 | -10.146 | 1.00 | 26.73 | C |
| ATOM | 13 | CA | GLY | 15 | -3.567 | 5.434 | -12.371 | 1.00 | 23.37 | C |
| ATOM | 14 | CA | GLY | 16 | -5.492 | 2.682 | -10.482 | 1.00 | 22.86 | C |
| ATOM | 15 | CA | GLY | 17 | -4.920 | 0.709 | -7.288 | 1.00 | 20.21 | C |
| ATOM | 16 | CA | GLY | 18 | -6.462 | -2.512 | -5.933 | 1.00 | 19.23 | C |
| ATOM | 17 | CA | GLY | 19 | -7.735 | -2.659 | -2.366 | 1.00 | 17.13 | C |
| ATOM | 18 | CA | GLY | 20 | -7.524 | -6.278 | -1.141 | 1.00 | 19.06 | C |
| ATOM | 19 | CA | GLY | 21 | -9.914 | -7.812 | 1.355 | 1.00 | 15.28 | C |
| ATOM | 20 | CA | GLY | 22 | -10.325 | -11.479 | 2.238 | 1.00 | 15.88 | C |
| ATOM | 21 | CA | GLY | 23 | -11.233 | -13.572 | 5.249 | 1.00 | 18.12 | C |
| ATOM | 22 | CA | GLY | 24 | -10.228 | -16.988 | 6.550 | 1.00 | 18.67 | C |
| ATOM | 23 | CA | GLY | 25 | -11.569 | -19.457 | 9.107 | 1.00 | 20.29 | C |
| ATOM | 24 | CA | GLY | 33 | -21.431 | -13.432 | 3.640 | 1.00 | 24.64 | C |
| ATOM | 25 | CA | GLY | 34 | -21.423 | -9.665 | 3.090 | 1.00 | 21.24 | C |
| ATOM | 26 | CA | GLY | 35 | -18.613 | -7.157 | 2.347 | 1.00 | 17.90 | C |
| ATOM | 27 | CA | GLY | 36 | -18.908 | -3.427 | 3.018 | 1.00 | 16.78 | C |
| ATOM | 28 | CA | GLY | 37 | -16.219 | -0.829 | 2.103 | 1.00 | 15.77 | C |
| ATOM | 29 | CA | GLY | 38 | -16.123 | 2.573 | 3.946 | 1.00 | 16.15 | C |
| ATOM | 30 | CA | GLY | 39 | -13.870 | 5.619 | 3.251 | 1.00 | 17.11 | C |
| ATOM | 31 | CA | GLY | 40 | -12.494 | 8.314 | 5.642 | 1.00 | 19.95 | C |
| ATOM | 32 | CA | GLY | 46 | -16.461 | 10.313 | 9.058 | 1.00 | 25.44 | C |
| ATOM | 33 | CA | GLY | 47 | -16.556 | 6.820 | 7.445 | 1.00 | 21.65 | C |
| ATOM | 34 | CA | GLY | 48 | -18.735 | 6.834 | 4.274 | 1.00 | 18.17 | C |
| ATOM | 35 | CA | GLY | 49 | -19.877 | 3.539 | 2.632 | 1.00 | 16.87 | C |
| ATOM | 36 | CA | GLY | 50 | -18.781 | 3.271 | -1.024 | 1.00 | 16.20 | C |
| ATOM | 37 | CA | GLY | 51 | -19.542 | -0.410 | -1.819 | 1.00 | 18.17 | C |
| ATOM | 38 | CA | GLY | 58 | -23.016 | -6.057 | -5.656 | 1.00 | 25.27 | C |
| ATOM | 39 | CA | GLY | 59 | -24.037 | -2.432 | -4.897 | 1.00 | 25.25 | C |
| ATOM | 40 | CA | GLY | 60 | -21.801 | 0.483 | -5.960 | 1.00 | 27.11 | C |
| ATOM | 41 | CA | GLY | 61 | -22.391 | 4.033 | -4.746 | 1.00 | 33.08 | C |
| ATOM | 42 | CA | GLY | 69 | -14.428 | 4.962 | -10.168 | 1.00 | 19.37 | C |
| ATOM | 43 | CA | GLY | 70 | -15.191 | 1.646 | -8.389 | 1.00 | 17.40 | C |
| ATOM | 44 | CA | GLY | 71 | -14.920 | -1.883 | -9.862 | 1.00 | 17.48 | C |
| ATOM | 45 | CA | GLY | 72 | -15.954 | -5.092 | -8.038 | 1.00 | 17.29 | C |
| ATOM | 46 | CA | GLY | 73 | -13.296 | -7.784 | -8.446 | 1.00 | 17.82 | C |
| ATOM | 47 | CA | GLY | 74 | -13.990 | -10.198 | -5.611 | 1.00 | 20.26 | C |
| ATOM | 48 | CA | GLY | 81 | -14.142 | -8.971 | -1.381 | 1.00 | 15.95 | C |
| ATOM | 49 | CA | GLY | 82 | -11.604 | -6.836 | -3.256 | 1.00 | 14.51 | C |
| ATOM | 50 | CA | GLY | 83 | -12.322 | -3.672 | -5.337 | 1.00 | 14.59 | C |
| ATOM | 51 | CA | GLY | 84 | -10.287 | -1.441 | -7.646 | 1.00 | 15.51 | C |
| ATOM | 52 | CA | GLY | 85 | -10.204 | 2.403 | -7.291 | 1.00 | 16.32 | C |
| ATOM | 53 | CA | GLY | 86 | -9.791 | 3.931 | -10.768 | 1.00 | 17.40 | C |
| ATOM | 54 | CA | GLY | 87 | -8.338 | 7.203 | -12.126 | 1.00 | 20.53 | C |
| ATOM | 55 | CA | GLY | 89 | -6.478 | 11.899 | -7.844 | 1.00 | 33.26 | C |
| ATOM | 56 | CA | GLY | 90 | -4.328 | 13.752 | -5.293 | 1.00 | 33.41 | C |
| ATOM | 57 | CA | GLY | 91 | -7.275 | 14.318 | -3.027 | 1.00 | 28.24 | C |
| ATOM | 58 | CA | GLY | 92 | -8.033 | 10.627 | -2.715 | 1.00 | 23.61 | C |
| ATOM | 59 | CA | GLY | 93 | -5.654 | 10.161 | 0.314 | 1.00 | 22.12 | C |
| ATOM | 60 | CA | GLY | 94 | -7.413 | 8.486 | 3.205 | 1.00 | 18.21 | C |
| ATOM | 61 | CA | GLY | 95 | -8.183 | 5.294 | 5.047 | 1.00 | 18.57 | C |
| ATOM | 62 | CA | GLY | 96 | -10.462 | 2.502 | 3.647 | 1.00 | 17.72 | C |
| ATOM | 63 | CA | GLY | 97 | -12.048 | -0.109 | 5.899 | 1.00 | 18.72 | C |
| ATOM | 64 | CA | GLY | 98 | -13.364 | -3.549 | 4.893 | 1.00 | 18.53 | C |
| ATOM | 65 | CA | GLY | 99 | -16.169 | -4.934 | 7.135 | 1.00 | 18.45 | C |
| ATOM | 66 | CA | GLY | 100 | -17.005 | -8.651 | 6.806 | 1.00 | 17.71 | C |
| ATOM | 67 | CA | GLY | 108 | -18.629 | -7.767 | 11.674 | 1.00 | 32.51 | C |
| ATOM | 68 | CA | GLY | 109 | -14.846 | -7.953 | 11.718 | 1.00 | 28.62 | C |
| ATOM | 69 | CA | GLY | 110 | -12.921 | -5.079 | 10.098 | 1.00 | 23.31 | C |
| ATOM | 70 | CA | GLY | 111 | -9.483 | -4.260 | 8.692 | 1.00 | 19.82 | C |
| ATOM | 71 | CA | GLY | 112 | -8.175 | -1.005 | 7.171 | 1.00 | 19.75 | C |
| ATOM | 72 | CA | GLY | 113 | -5.635 | 0.154 | 4.554 | 1.00 | 18.42 | C |
| ATOM | 73 | CA | GLY | 114 | -4.325 | 3.731 | 4.046 | 1.00 | 18.70 | C |
| ATOM | 74 | CA | GLY | 115 | -3.915 | 5.265 | 0.576 | 1.00 | 19.95 | C |
| ATOM | 75 | CA | GLY | 116 | -1.274 | 7.843 | -0.510 | 1.00 | 25.78 | C |
| ATOM | 76 | CA | GLY | 117 | -1.158 | 9.173 | -4.076 | 1.00 | 31.06 | C |
| ATOM | 77 | CA | GLY | 118 | 1.962 | 10.836 | -5.500 | 1.00 | 38.60 | C |
| ATOM | 78 | CA | GLY | 119 | 3.251 | 11.884 | -8.972 | 1.00 | 42.50 | C |
| TER | | | | | | | | | | |
| | | | | | | | | | | |

| 1MEL | | | | | | | | | | |
|---|---|---|---|---|---|---|---|---|---|---|
| ATOM | 79 | CA | GLY A | 3 | -9.610 | -12.241 | 11.306 | 1.00 | 36.96 | C |
| ATOM | 80 | CA | GLY A | 4 | -9.672 | -9.746 | 8.420 | 1.00 | 28.80 | C |
| ATOM | 81 | CA | GLY A | 5 | -6.352 | -8.965 | 6.761 | 1.00 | 31.64 | C |
| ATOM | 82 | CA | GLY A | 6 | -6.225 | -6.212 | 4.154 | 1.00 | 24.68 | C |
| ATOM | 83 | CA | GLY A | 7 | -3.391 | -5.808 | 1.630 | 1.00 | 21.60 | C |
| ATOM | 84 | CA | GLY A | 8 | -2.679 | -3.267 | -1.055 | 1.00 | 17.27 | C |
| ATOM | 85 | CA | GLY A | 9 | -2.837 | 0.450 | -0.715 | 1.00 | 18.58 | C |
| ATOM | 86 | CA | GLY A | 10 | 0.112 | 2.848 | -0.895 | 1.00 | 16.78 | C |
| ATOM | 87 | CA | GLY A | 11 | 0.663 | 5.945 | -3.023 | 1.00 | 11.36 | C |
| ATOM | 88 | CA | GLY A | 12 | -0.001 | 6.528 | -6.640 | 1.00 | 8.84 | C |
| ATOM | 89 | CA | GLY A | 13 | -0.394 | 9.450 | -8.944 | 1.00 | 11.16 | C |
| ATOM | 90 | CA | GLY A | 14 | -3.805 | 10.880 | -9.667 | 1.00 | 10.62 | C |
| ATOM | 91 | CA | GLY A | 16 | -3.498 | 5.586 | -11.682 | 1.00 | 7.90 | C |
| ATOM | 92 | CA | GLY A | 17 | -5.029 | 2.507 | -10.132 | 1.00 | 7.54 | C |
| ATOM | 93 | CA | GLY A | 18 | -4.536 | 0.406 | -6.998 | 1.00 | 6.47 | C |
| ATOM | 94 | CA | GLY A | 19 | -5.823 | -2.962 | -5.868 | 1.00 | 5.43 | C |
| ATOM | 95 | CA | GLY A | 20 | -6.773 | -3.739 | -2.263 | 1.00 | 8.09 | C |
| ATOM | 96 | CA | GLY A | 21 | -7.534 | -7.231 | -0.907 | 1.00 | 8.76 | C |
| ATOM | 97 | CA | GLY A | 22 | -9.056 | -8.745 | 2.173 | 1.00 | 8.33 | C |
| ATOM | 98 | CA | GLY A | 23 | -9.100 | -12.281 | 3.393 | 1.00 | 13.77 | C |
| ATOM | 99 | CA | GLY A | 24 | -11.485 | -13.177 | 6.222 | 1.00 | 17.53 | C |
| ATOM | 100 | CA | GLY A | 25 | -9.970 | -15.910 | 8.360 | 1.00 | 23.23 | C |
| ATOM | 101 | CA | GLY A | 26 | -11.324 | -17.985 | 11.176 | 1.00 | 25.25 | C |
| ATOM | 102 | CA | GLY A | 32 | -22.537 | -12.961 | 4.478 | 1.00 | 5.00 | C |
| ATOM | 103 | CA | GLY A | 33 | -21.061 | -9.574 | 3.450 | 1.00 | 4.64 | C |
| ATOM | 104 | CA | GLY A | 34 | -17.557 | -8.097 | 2.923 | 1.00 | 2.48 | C |
| ATOM | 105 | CA | GLY A | 35 | -17.173 | -4.447 | 1.958 | 1.00 | 2.00 | C |
| ATOM | 106 | CA | GLY A | 36 | -14.942 | -1.420 | 2.147 | 1.00 | 3.40 | C |
| ATOM | 107 | CA | GLY A | 37 | -15.248 | 1.775 | 4.153 | 1.00 | 6.64 | C |
| ATOM | 108 | CA | GLY A | 38 | -12.980 | 4.768 | 4.009 | 1.00 | 8.74 | C |
| ATOM | 109 | CA | GLY A | 39 | -12.174 | 7.634 | 6.381 | 1.00 | 19.24 | C |
| ATOM | 110 | CA | GLY A | 44 | -17.378 | 11.364 | 7.293 | 1.00 | 26.93 | C |
| ATOM | 111 | CA | GLY A | 45 | -16.728 | 7.653 | 7.090 | 1.00 | 16.63 | C |
| ATOM | 112 | CA | GLY A | 46 | -17.836 | 6.562 | 3.651 | 1.00 | 13.26 | C |
| ATOM | 113 | CA | GLY A | 47 | -19.278 | 3.220 | 2.664 | 1.00 | 8.30 | C |
| ATOM | 114 | CA | GLY A | 48 | -17.484 | 2.453 | -0.627 | 1.00 | 6.32 | C |
| ATOM | 115 | CA | GLY A | 49 | -18.387 | -0.943 | -1.936 | 1.00 | 3.71 | C |
| ATOM | 116 | CA | GLY A | 57 | -24.217 | -9.042 | -5.792 | 1.00 | 11.04 | C |
| ATOM | 117 | CA | GLY A | 58 | -22.300 | -5.759 | -5.582 | 1.00 | 7.10 | C |
| ATOM | 118 | CA | GLY A | 59 | -23.147 | -2.237 | -4.440 | 1.00 | 9.00 | C |
| ATOM | 119 | CA | GLY A | 60 | -21.067 | 0.930 | -4.751 | 1.00 | 8.36 | C |
| ATOM | 120 | CA | GLY A | 61 | -21.147 | 4.392 | -3.266 | 1.00 | 15.85 | C |
| ATOM | 121 | CA | GLY A | 67 | -14.348 | 3.577 | -11.091 | 1.00 | 12.68 | C |
| ATOM | 122 | CA | GLY A | 68 | -14.176 | 0.900 | -8.416 | 1.00 | 8.15 | C |
| ATOM | 123 | CA | GLY A | 69 | -15.003 | -2.767 | -8.799 | 1.00 | 8.39 | C |
| ATOM | 124 | CA | GLY A | 70 | -15.301 | -5.266 | -5.949 | 1.00 | 5.47 | C |
| ATOM | 125 | CA | GLY A | 71 | -15.018 | -8.998 | -6.510 | 1.00 | 8.48 | C |
| ATOM | 126 | CA | GLY A | 72 | -14.299 | -12.215 | -4.617 | 1.00 | 18.00 | C |
| ATOM | 127 | CA | GLY A | 79 | -12.288 | -10.021 | -1.938 | 1.00 | 9.55 | C |
| ATOM | 128 | CA | GLY A | 80 | -10.619 | -7.230 | -3.968 | 1.00 | 4.94 | C |
| ATOM | 129 | CA | GLY A | 81 | -11.319 | -3.691 | -4.814 | 1.00 | 4.92 | C |
| ATOM | 130 | CA | GLY A | 82 | -9.808 | -2.556 | -8.096 | 1.00 | 7.44 | C |
| ATOM | 131 | CA | GLY A | 83 | -9.608 | 1.233 | -8.010 | 1.00 | 6.55 | C |
| ATOM | 132 | CA | GLY A | 84 | -9.109 | 2.986 | -11.359 | 1.00 | 9.49 | C |
| ATOM | 133 | CA | GLY A | 85 | -9.157 | 6.689 | -12.211 | 1.00 | 12.54 | C |
| ATOM | 134 | CA | GLY A | 87 | -8.265 | 11.163 | -7.900 | 1.00 | 15.46 | C |
| ATOM | 135 | CA | GLY A | 88 | -6.724 | 12.875 | -4.855 | 1.00 | 13.94 | C |
| ATOM | 136 | CA | GLY A | 89 | -10.223 | 13.046 | -3.286 | 1.00 | 18.68 | C |
| ATOM | 137 | CA | GLY A | 90 | -9.896 | 9.253 | -2.924 | 1.00 | 9.55 | C |
| ATOM | 138 | CA | GLY A | 91 | -7.043 | 9.782 | -0.407 | 1.00 | 6.54 | C |
| ATOM | 139 | CA | GLY A | 92 | -8.201 | 8.111 | 2.809 | 1.00 | 6.38 | C |
| ATOM | 140 | CA | GLY A | 93 | -7.841 | 5.198 | 5.205 | 1.00 | 6.33 | C |
| ATOM | 141 | CA | GLY A | 94 | -9.509 | 2.130 | 3.746 | 1.00 | 6.08 | C |
| ATOM | 142 | CA | GLY A | 95 | -11.083 | -0.367 | 6.028 | 1.00 | 10.12 | C |
| ATOM | 143 | CA | GLY A | 96 | -12.264 | -3.845 | 5.241 | 1.00 | 10.76 | C |
| ATOM | 144 | CA | GLY A | 97 | -15.411 | -5.059 | 6.995 | 1.00 | 9.08 | C |
| ATOM | 145 | CA | GLY A | 98 | -17.534 | -8.225 | 7.154 | 1.00 | 8.28 | C |
| ATOM | 146 | CA | GLY A | 122 | -15.862 | -7.486 | 11.967 | 1.00 | 15.49 | C |
| ATOM | 147 | CA | GLY A | 123 | -13.351 | -4.917 | 11.000 | 1.00 | 12.37 | C |
| ATOM | 148 | CA | GLY A | 124 | -9.811 | -4.988 | 9.801 | 1.00 | 14.34 | C |
| ATOM | 149 | CA | GLY A | 125 | -6.730 | -2.842 | 10.015 | 1.00 | 22.49 | C |
| ATOM | 150 | CA | GLY A | 126 | -6.910 | 0.334 | 7.957 | 1.00 | 17.72 | C |
| ATOM | 151 | CA | GLY A | 127 | -4.732 | 0.802 | 4.921 | 1.00 | 16.51 | C |
| ATOM | 152 | CA | GLY A | 128 | -3.822 | 4.319 | 3.804 | 1.00 | 16.84 | C |
| ATOM | 153 | CA | GLY A | 129 | -4.119 | 5.119 | 0.160 | 1.00 | 11.90 | C |
| ATOM | 154 | CA | GLY A | 130 | -2.710 | 8.445 | -0.901 | 1.00 | 8.75 | C |
| ATOM | 155 | CA | GLY A | 131 | -3.277 | 9.842 | -4.344 | 1.00 | 14.37 | C |
| ATOM | 156 | CA | GLY A | 132 | -0.480 | 12.243 | -5.478 | 1.00 | 23.32 | C |
| ATOM | 157 | CA | GLY A | 133 | -0.447 | 15.425 | -7.580 | 1.00 | 36.14 | C |
| TER | | | | | | | | | | |
| | | | | | | | | | | |

| 1F97 | | | | | | | | | | |
|---|---|---|---|---|---|---|---|---|---|---|
| ATOM | 158 | CA | GLY A | 29 | -9.830 | -13.499 | 10.551 | 1.00 | 41.25 | C |
| ATOM | 159 | CA | GLY A | 30 | -9.746 | -10.552 | 8.150 | 1.00 | 22.43 | C |
| ATOM | 160 | CA | GLY A | 31 | -6.475 | -9.224 | 6.722 | 1.00 | 24.73 | C |
| ATOM | 161 | CA | GLY A | 32 | -4.787 | -7.203 | 3.981 | 1.00 | 20.95 | C |
| ATOM | 162 | CA | GLY A | 33 | -1.574 | -7.581 | 1.983 | 1.00 | 28.77 | C |
| ATOM | 163 | CA | GLY A | 34 | -0.760 | -3.875 | 2.262 | 1.00 | 33.48 | C |
| ATOM | 164 | CA | GLY A | 35 | -2.198 | -1.487 | 4.855 | 1.00 | 27.47 | C |
| ATOM | 165 | CA | GLY A | 36 | -0.223 | 1.510 | 3.544 | 1.00 | 29.20 | C |
| ATOM | 166 | CA | GLY A | 37 | -0.984 | 1.885 | -0.160 | 1.00 | 23.99 | C |
| ATOM | 167 | CA | GLY A | 38 | 0.681 | 4.472 | -2.392 | 1.00 | 24.19 | C |
| ATOM | 168 | CA | GLY A | 39 | -0.199 | 4.783 | -6.071 | 1.00 | 15.35 | C |
| ATOM | 169 | CA | GLY A | 40 | 0.260 | 7.491 | -8.737 | 1.00 | 12.64 | C |
| ATOM | 170 | CA | GLY A | 41 | -2.766 | 9.641 | -9.587 | 1.00 | 9.24 | C |
| ATOM | 171 | CA | GLY A | 43 | -3.890 | 4.861 | -12.131 | 1.00 | 14.44 | C |
| ATOM | 172 | CA | GLY A | 44 | -5.807 | 1.735 | -11.160 | 1.00 | 21.52 | C |
| ATOM | 173 | CA | GLY A | 45 | -5.444 | 0.202 | -7.726 | 1.00 | 22.48 | C |
| ATOM | 174 | CA | GLY A | 46 | -6.964 | -2.720 | -5.861 | 1.00 | 21.22 | C |
| ATOM | 175 | CA | GLY A | 47 | -7.458 | -2.391 | -2.118 | 1.00 | 20.06 | C |
| ATOM | 176 | CA | GLY A | 48 | -7.106 | -5.988 | -0.927 | 1.00 | 13.56 | C |
| ATOM | 177 | CA | GLY A | 49 | -9.118 | -7.626 | 1.851 | 1.00 | 19.14 | C |
| ATOM | 178 | CA | GLY A | 50 | -8.738 | -11.362 | 2.401 | 1.00 | 22.49 | C |
| ATOM | 179 | CA | GLY A | 51 | -10.667 | -13.442 | 4.932 | 1.00 | 20.58 | C |
| ATOM | 180 | CA | GLY A | 52 | -11.032 | -17.051 | 6.076 | 1.00 | 24.55 | C |
| ATOM | 181 | CA | GLY A | 53 | -13.512 | -18.935 | 8.234 | 1.00 | 17.82 | C |
| ATOM | 182 | CA | GLY A | 57 | -19.932 | -13.290 | 4.102 | 1.00 | 10.39 | C |
| ATOM | 183 | CA | GLY A | 58 | -21.330 | -9.790 | 3.738 | 1.00 | 8.00 | C |
| ATOM | 184 | CA | GLY A | 59 | -18.524 | -7.465 | 2.656 | 1.00 | 7.62 | C |
| ATOM | 185 | CA | GLY A | 60 | -18.773 | -3.717 | 3.244 | 1.00 | 6.84 | C |
| ATOM | 186 | CA | GLY A | 61 | -16.384 | -0.804 | 2.777 | 1.00 | 8.48 | C |
| ATOM | 187 | CA | GLY A | 62 | -16.301 | 2.656 | 4.292 | 1.00 | 13.92 | C |
| ATOM | 188 | CA | GLY A | 63 | -14.245 | 5.726 | 3.449 | 1.00 | 11.89 | C |
| ATOM | 189 | CA | GLY A | 64 | -13.094 | 8.083 | 6.189 | 1.00 | 23.18 | C |
| ATOM | 190 | CA | GLY A | 68 | -16.689 | 12.612 | 6.413 | 1.00 | 46.37 | C |
| ATOM | 191 | CA | GLY A | 69 | -17.861 | 8.988 | 6.532 | 1.00 | 32.33 | C |
| ATOM | 192 | CA | GLY A | 70 | -19.096 | 7.427 | 3.276 | 1.00 | 18.88 | C |
| ATOM | 193 | CA | GLY A | 71 | -19.976 | 3.841 | 2.373 | 1.00 | 12.11 | C |
| ATOM | 194 | CA | GLY A | 72 | -18.208 | 2.531 | -0.728 | 1.00 | 9.54 | C |
| ATOM | 195 | CA | GLY A | 73 | -19.659 | -0.955 | -0.492 | 1.00 | 9.17 | C |
| ATOM | 196 | CA | GLY A | 77 | -22.346 | -4.092 | -3.568 | 1.00 | 17.96 | C |
| ATOM | 197 | CA | GLY A | 78 | -20.630 | -0.882 | -4.664 | 1.00 | 11.41 | C |
| ATOM | 198 | CA | GLY A | 79 | -22.720 | 2.163 | -3.704 | 1.00 | 8.01 | C |
| ATOM | 199 | CA | GLY A | 85 | -15.239 | 3.577 | -11.142 | 1.00 | 17.93 | C |
| ATOM | 200 | CA | GLY A | 86 | -15.128 | 0.968 | -8.358 | 1.00 | 15.44 | C |
| ATOM | 201 | CA | GLY A | 87 | -15.569 | -2.740 | -9.020 | 1.00 | 16.79 | C |
| ATOM | 202 | CA | GLY A | 88 | -16.272 | -5.311 | -6.312 | 1.00 | 14.07 | C |
| ATOM | 203 | CA | GLY A | 89 | -14.727 | -8.756 | -5.888 | 1.00 | 16.75 | C |
| ATOM | 204 | CA | GLY A | 91 | -10.820 | -10.288 | -2.524 | 1.00 | 17.13 | C |
| ATOM | 205 | CA | GLY A | 92 | -11.033 | -6.489 | -2.552 | 1.00 | 12.84 | C |
| ATOM | 206 | CA | GLY A | 93 | -12.232 | -3.404 | -4.409 | 1.00 | 12.33 | C |
| ATOM | 207 | CA | GLY A | 94 | -10.610 | -2.146 | -7.602 | 1.00 | 12.43 | C |
| ATOM | 208 | CA | GLY A | 95 | -10.518 | 1.519 | -8.590 | 1.00 | 11.12 | C |
| ATOM | 209 | CA | GLY A | 96 | -10.279 | 1.926 | -12.355 | 1.00 | 16.95 | C |
| ATOM | 210 | CA | GLY A | 97 | -8.644 | 5.292 | -11.532 | 1.00 | 21.11 | C |
| ATOM | 211 | CA | GLY A | 99 | -7.443 | 11.068 | -7.844 | 1.00 | 22.37 | C |
| ATOM | 212 | CA | GLY A | 100 | -5.937 | 13.065 | -4.977 | 1.00 | 23.75 | C |
| ATOM | 213 | CA | GLY A | 101 | -9.515 | 13.338 | -3.639 | 1.00 | 22.84 | C |
| ATOM | 214 | CA | GLY A | 102 | -9.383 | 9.634 | -2.783 | 1.00 | 15.51 | C |
| ATOM | 215 | CA | GLY A | 103 | -6.718 | 10.015 | -0.070 | 1.00 | 11.57 | C |
| ATOM | 216 | CA | GLY A | 104 | -7.876 | 8.577 | 3.237 | 1.00 | 9.52 | C |
| ATOM | 217 | CA | GLY A | 105 | -8.530 | 5.333 | 5.050 | 1.00 | 12.64 | C |
| ATOM | 218 | CA | GLY A | 106 | -10.727 | 2.534 | 3.753 | 1.00 | 7.32 | C |
| ATOM | 219 | CA | GLY A | 107 | -12.073 | -0.044 | 6.175 | 1.00 | 7.52 | C |
| ATOM | 220 | CA | GLY A | 108 | -13.078 | -3.483 | 4.952 | 1.00 | 9.97 | C |
| ATOM | 221 | CA | GLY A | 109 | -15.819 | -4.936 | 7.140 | 1.00 | 12.91 | C |
| ATOM | 222 | CA | GLY A | 110 | -16.559 | -8.639 | 6.792 | 1.00 | 11.27 | C |
| ATOM | 223 | CA | GLY A | 118 | -17.046 | -10.415 | 12.491 | 1.00 | 13.29 | C |
| ATOM | 224 | CA | GLY A | 119 | -13.800 | -8.756 | 11.482 | 1.00 | 15.55 | C |
| ATOM | 225 | CA | GLY A | 120 | -12.342 | -5.613 | 9.917 | 1.00 | 11.36 | C |
| ATOM | 226 | CA | GLY A | 121 | -9.139 | -4.141 | 8.532 | 1.00 | 11.36 | C |
| ATOM | 227 | CA | GLY A | 122 | -8.151 | -0.571 | 7.641 | 1.00 | 11.14 | C |
| ATOM | 228 | CA | GLY A | 123 | -6.080 | 0.520 | 4.643 | 1.00 | 11.58 | C |
| ATOM | 229 | CA | GLY A | 124 | -4.595 | 3.978 | 4.206 | 1.00 | 15.52 | C |
| ATOM | 230 | CA | GLY A | 125 | -4.504 | 5.200 | 0.621 | 1.00 | 9.61 | C |
| ATOM | 231 | CA | GLY A | 126 | -2.079 | 7.899 | -0.493 | 1.00 | 10.84 | C |
| ATOM | 232 | CA | GLY A | 127 | -2.415 | 9.054 | -4.098 | 1.00 | 10.58 | C |
| ATOM | 233 | CA | GLY A | 128 | 0.985 | 10.216 | -5.373 | 1.00 | 14.41 | C |
| ATOM | 234 | CA | GLY A | 129 | 1.308 | 13.675 | -6.915 | 1.00 | 12.32 | C |
| TER | | | | | | | | | | |
| | | | | | | | | | | |

| 1DQT | | | | | | | | | | |
|---|---|---|---|---|---|---|---|---|---|---|
| ATOM | 235 | CA | GLY C | 2 | -10.005 | -8.876 | 13.603 | 1.00 | 35.96 | C |
| ATOM | 236 | CA | GLY C | 3 | -10.267 | -7.502 | 10.101 | 1.00 | 30.20 | C |
| ATOM | 237 | CA | GLY C | 4 | -7.171 | -6.498 | 8.221 | 1.00 | 27.24 | C |
| ATOM | 238 | CA | GLY C | 5 | -6.397 | -5.009 | 4.845 | 1.00 | 23.16 | C |
| ATOM | 239 | CA | GLY C | 6 | -3.219 | -3.760 | 3.070 | 1.00 | 22.73 | C |
| ATOM | 240 | CA | GLY C | 7 | -1.859 | -0.343 | 3.998 | 1.00 | 24.33 | C |
| ATOM | 241 | CA | GLY C | 8 | -1.267 | 0.851 | 0.436 | 1.00 | 21.64 | C |
| ATOM | 242 | CA | GLY C | 9 | -2.613 | 0.109 | -3.024 | 1.00 | 20.25 | C |
| ATOM | 243 | CA | GLY C | 10 | -1.486 | 1.813 | -6.246 | 1.00 | 20.37 | C |
| ATOM | 244 | CA | GLY C | 11 | -4.559 | 2.055 | -8.480 | 1.00 | 22.46 | C |
| ATOM | 245 | CA | GLY C | 12 | -4.228 | 1.091 | -12.139 | 1.00 | 24.30 | C |
| ATOM | 246 | CA | GLY C | 14 | -7.812 | 2.779 | -15.613 | 1.00 | 30.82 | C |
| ATOM | 247 | CA | GLY C | 15 | -8.831 | 3.617 | -12.060 | 1.00 | 25.29 | C |
| ATOM | 248 | CA | GLY C | 16 | -8.949 | 0.054 | -10.709 | 1.00 | 21.63 | C |
| ATOM | 249 | CA | GLY C | 17 | -7.920 | -0.828 | -7.174 | 1.00 | 20.22 | C |
| ATOM | 250 | CA | GLY C | 18 | -8.037 | -4.397 | -5.930 | 1.00 | 20.86 | C |
| ATOM | 251 | CA | GLY C | 19 | -7.062 | -5.746 | -2.558 | 1.00 | 21.25 | C |
| ATOM | 252 | CA | GLY C | 20 | -7.903 | -8.418 | 0.003 | 1.00 | 21.73 | C |
| ATOM | 253 | CA | GLY C | 21 | -9.906 | -7.860 | 3.174 | 1.00 | 22.97 | C |
| ATOM | 254 | CA | GLY C | 22 | -9.210 | -10.573 | 5.688 | 1.00 | 26.59 | C |
| ATOM | 255 | CA | GLY C | 23 | -10.945 | -11.564 | 8.878 | 1.00 | 26.47 | C |
| ATOM | 256 | CA | GLY C | 24 | -10.701 | -13.907 | 11.826 | 1.00 | 31.54 | C |
| ATOM | 257 | CA | GLY C | 25 | -11.932 | -16.084 | 13.335 | 1.00 | 31.33 | C |
| ATOM | 258 | CA | GLY C | 32 | -20.611 | -12.339 | 5.419 | 1.00 | 21.25 | C |
| ATOM | 259 | CA | GLY C | 33 | -21.785 | -8.834 | 4.607 | 1.00 | 21.86 | C |
| ATOM | 260 | CA | GLY C | 34 | -18.854 | -6.717 | 3.456 | 1.00 | 19.78 | C |
| ATOM | 261 | CA | GLY C | 35 | -18.920 | -2.932 | 3.364 | 1.00 | 19.11 | C |
| ATOM | 262 | CA | GLY C | 36 | -16.430 | -0.515 | 1.806 | 1.00 | 19.23 | C |
| ATOM | 263 | CA | GLY C | 37 | -16.229 | 2.889 | 3.460 | 1.00 | 25.95 | C |
| ATOM | 264 | CA | GLY C | 38 | -14.212 | 5.923 | 2.415 | 1.00 | 30.90 | C |
| ATOM | 265 | CA | GLY C | 39 | -12.916 | 7.802 | 5.426 | 1.00 | 40.84 | C |
| ATOM | 266 | CA | GLY C | 43 | -16.713 | 11.053 | 8.779 | 1.00 | 46.74 | C |
| ATOM | 267 | CA | GLY C | 44 | -17.290 | 8.066 | 6.505 | 1.00 | 36.18 | C |
| ATOM | 268 | CA | GLY C | 45 | -19.030 | 7.541 | 3.173 | 1.00 | 28.21 | C |
| ATOM | 269 | CA | GLY C | 46 | -20.279 | 4.093 | 2.143 | 1.00 | 25.48 | C |
| ATOM | 270 | CA | GLY C | 47 | -18.891 | 3.200 | -1.269 | 1.00 | 23.13 | C |
| ATOM | 271 | CA | GLY C | 48 | -20.541 | -0.174 | -1.750 | 1.00 | 20.54 | C |
| ATOM | 272 | CA | GLY C | 58 | -19.057 | -12.667 | -7.642 | 1.00 | 24.08 | C |
| ATOM | 273 | CA | GLY C | 59 | -20.627 | -9.992 | -5.444 | 1.00 | 22.25 | C |
| ATOM | 274 | CA | GLY C | 60 | -21.579 | -6.311 | -5.553 | 1.00 | 22.96 | C |
| ATOM | 275 | CA | GLY C | 61 | -23.676 | -7.234 | -8.605 | 1.00 | 28.83 | C |
| ATOM | 276 | CA | GLY C | 62 | -25.740 | -4.088 | -8.210 | 1.00 | 32.00 | C |
| ATOM | 277 | CA | GLY C | 63 | -22.747 | -1.772 | -7.854 | 1.00 | 30.87 | C |
| ATOM | 278 | CA | GLY C | 66 | -17.371 | -2.468 | -7.103 | 1.00 | 23.17 | C |
| ATOM | 279 | CA | GLY C | 67 | -16.897 | -6.161 | -7.488 | 1.00 | 22.56 | C |
| ATOM | 280 | CA | GLY C | 68 | -15.405 | -9.022 | -5.517 | 1.00 | 21.60 | C |
| ATOM | 281 | CA | GLY C | 69 | -15.312 | -12.649 | -4.466 | 1.00 | 22.69 | C |
| ATOM | 282 | CA | GLY C | 75 | -12.905 | -11.173 | 0.577 | 1.00 | 23.03 | C |
| ATOM | 283 | CA | GLY C | 76 | -11.171 | -10.003 | -2.613 | 1.00 | 24.30 | C |
| ATOM | 284 | CA | GLY C | 77 | -12.370 | -6.459 | -3.308 | 1.00 | 23.46 | C |
| ATOM | 285 | CA | GLY C | 78 | -12.157 | -4.457 | -6.510 | 1.00 | 25.74 | C |
| ATOM | 286 | CA | GLY C | 79 | -13.139 | -0.780 | -6.670 | 1.00 | 26.08 | C |
| ATOM | 287 | CA | GLY C | 80 | -13.383 | 0.660 | -10.196 | 1.00 | 29.15 | C |
| ATOM | 288 | CA | GLY C | 81 | -13.950 | 3.973 | -11.951 | 1.00 | 26.23 | C |
| ATOM | 289 | CA | GLY C | 84 | -7.281 | 11.072 | -8.931 | 1.00 | 24.79 | C |
| ATOM | 290 | CA | GLY C | 85 | -9.649 | 12.909 | -6.605 | 1.00 | 25.37 | C |
| ATOM | 291 | CA | GLY C | 86 | -10.734 | 9.536 | -5.170 | 1.00 | 25.21 | C |
| ATOM | 292 | CA | GLY C | 87 | -7.287 | 9.058 | -3.657 | 1.00 | 23.95 | C |
| ATOM | 293 | CA | GLY C | 88 | -7.874 | 8.354 | 0.014 | 1.00 | 23.67 | C |
| ATOM | 294 | CA | GLY C | 89 | -8.483 | 5.896 | 2.834 | 1.00 | 22.75 | C |
| ATOM | 295 | CA | GLY C | 90 | -10.866 | 2.985 | 2.312 | 1.00 | 23.39 | C |
| ATOM | 296 | CA | GLY C | 91 | -12.127 | 0.902 | 5.210 | 1.00 | 22.55 | C |
| ATOM | 297 | CA | GLY C | 92 | -13.188 | -2.683 | 4.810 | 1.00 | 22.29 | C |
| ATOM | 298 | CA | GLY C | 93 | -15.931 | -3.797 | 7.205 | 1.00 | 20.45 | C |
| ATOM | 299 | CA | GLY C | 94 | -16.933 | -7.418 | 7.719 | 1.00 | 20.73 | C |
| ATOM | 300 | CA | GLY C | 105 | -15.090 | -5.968 | 12.589 | 1.00 | 24.94 | C |
| ATOM | 301 | CA | GLY C | 106 | -13.327 | -3.332 | 10.512 | 1.00 | 25.24 | C |
| ATOM | 302 | CA | GLY C | 107 | -9.792 | -2.872 | 9.205 | 1.00 | 24.05 | C |
| ATOM | 303 | CA | GLY C | 108 | -7.671 | 0.283 | 9.656 | 1.00 | 25.85 | C |
| ATOM | 304 | CA | GLY C | 109 | -8.117 | 1.175 | 6.019 | 1.00 | 23.77 | C |
| ATOM | 305 | CA | GLY C | 110 | -6.209 | 0.891 | 2.770 | 1.00 | 22.46 | C |
| ATOM | 306 | CA | GLY C | 111 | -4.626 | 4.045 | 1.330 | 1.00 | 24.13 | C |
| ATOM | 307 | CA | GLY C | 112 | -5.545 | 4.027 | -2.339 | 1.00 | 22.83 | C |
| ATOM | 308 | CA | GLY C | 113 | -3.438 | 6.352 | -4.496 | 1.00 | 23.40 | C |
| ATOM | 309 | CA | GLY C | 114 | -4.906 | 7.221 | -7.840 | 1.00 | 25.42 | C |
| END | | | | | | | | | | |

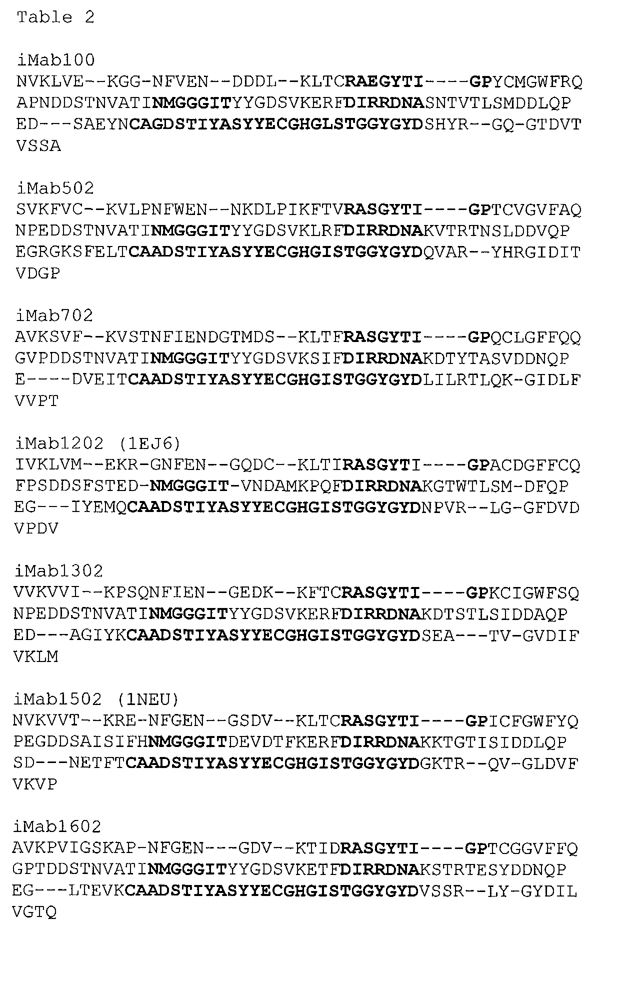

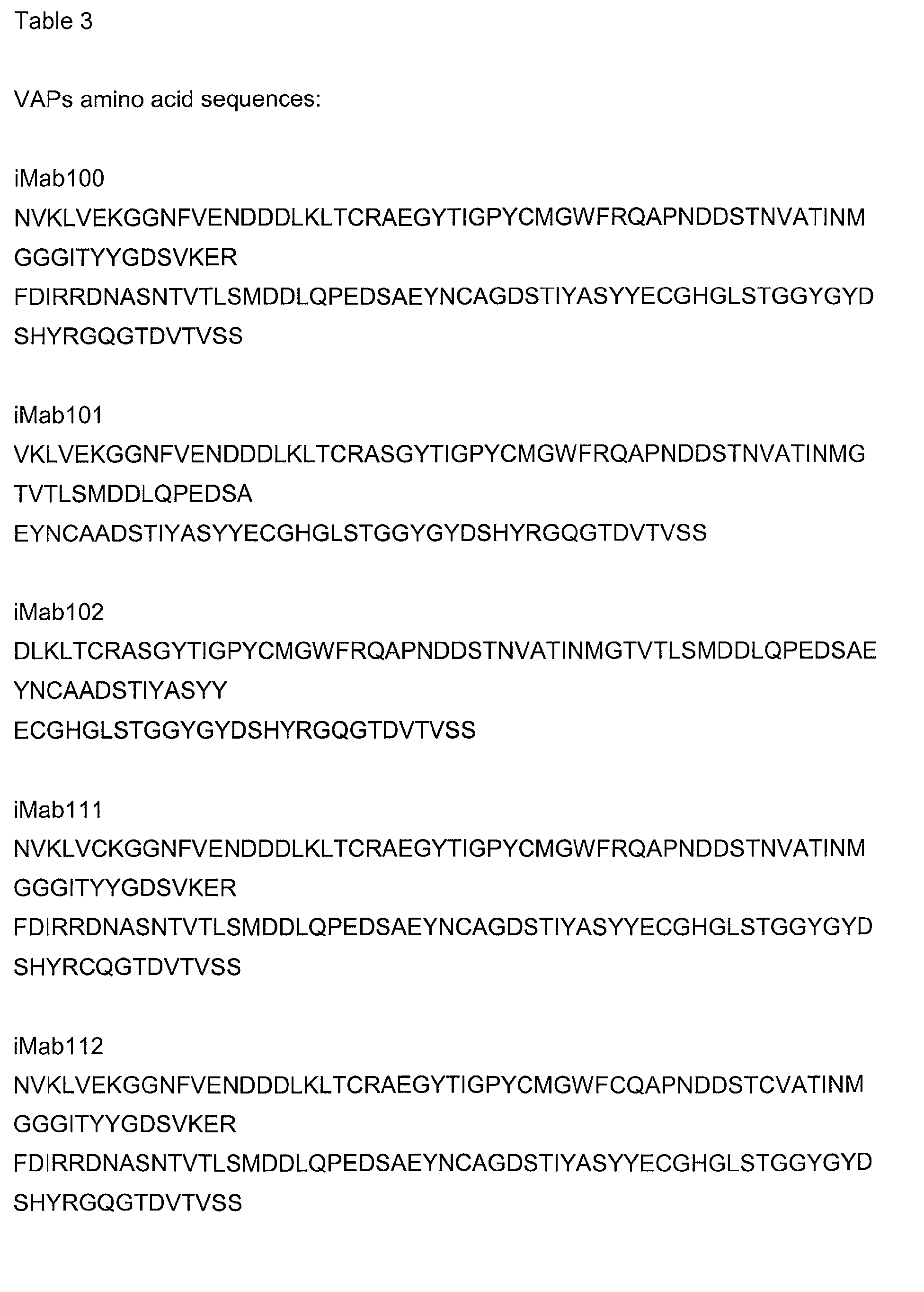

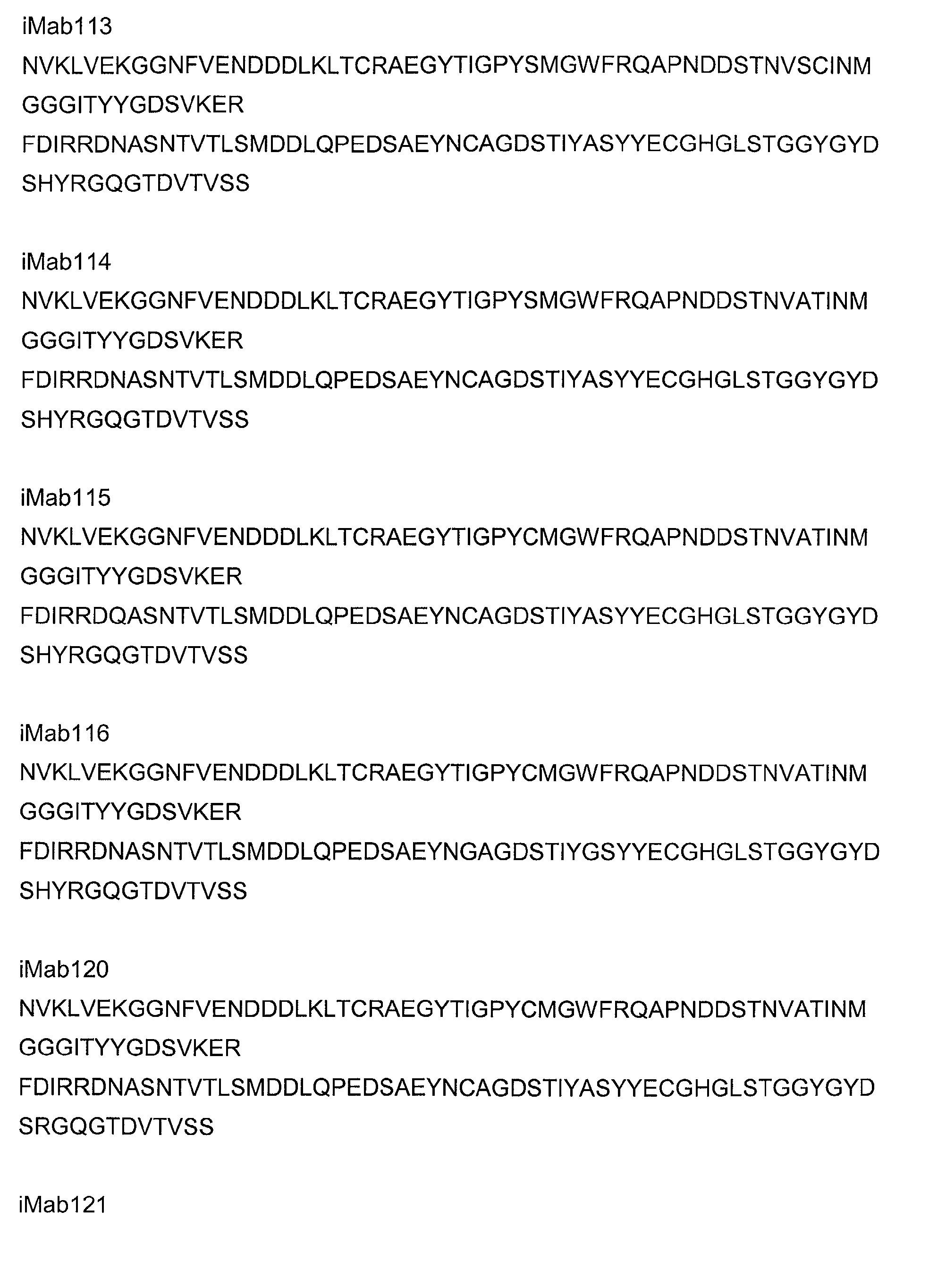

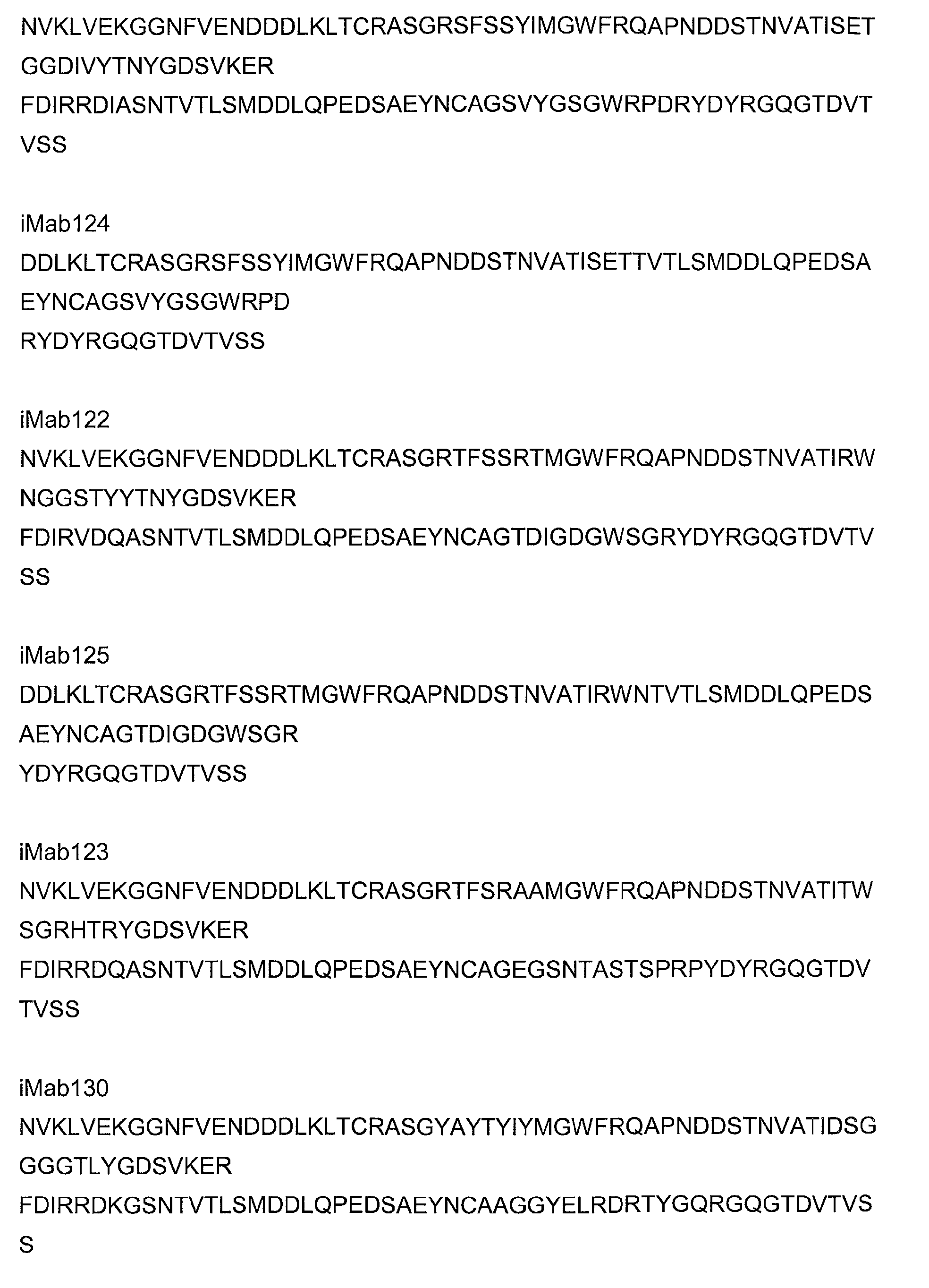

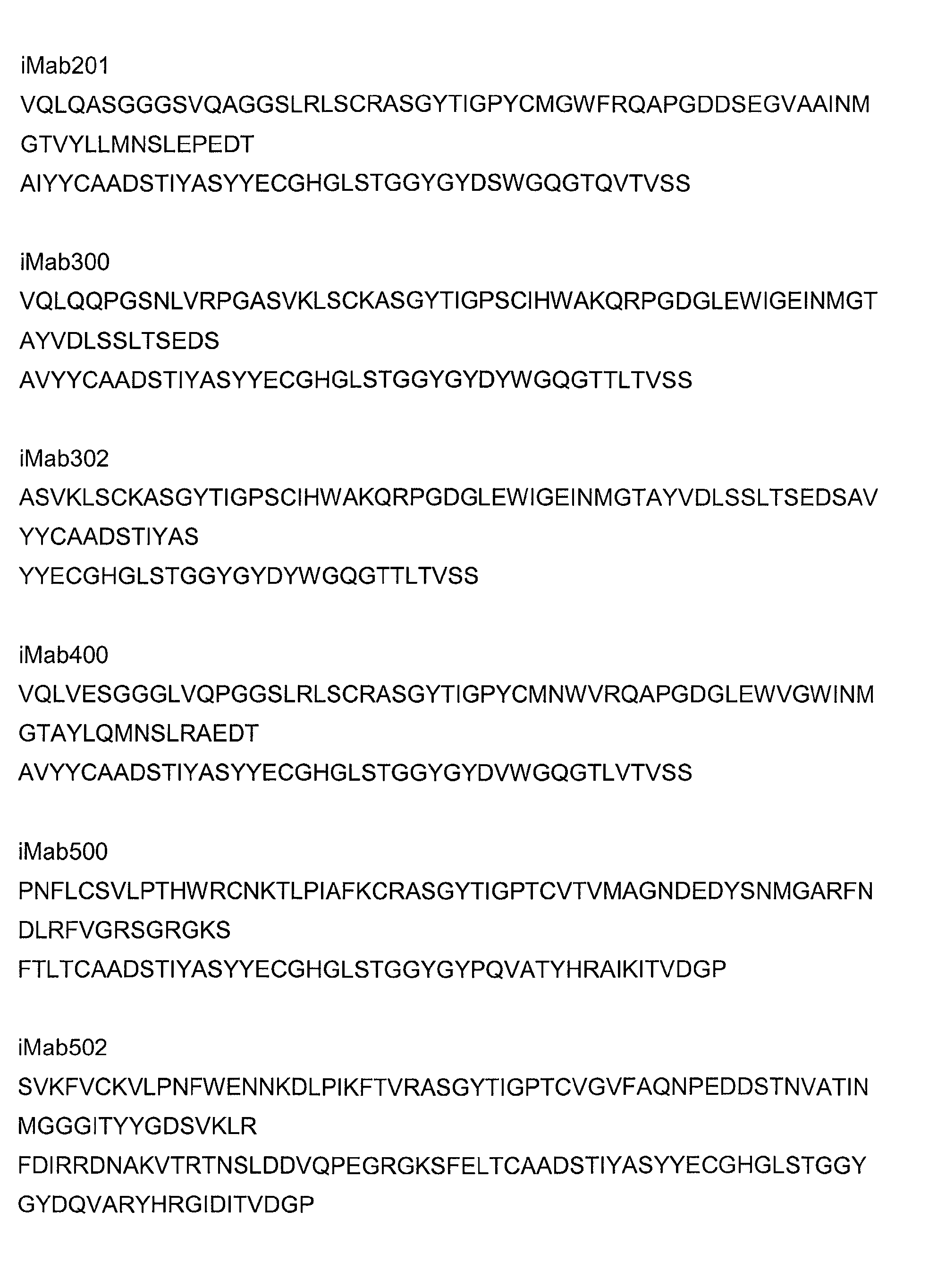

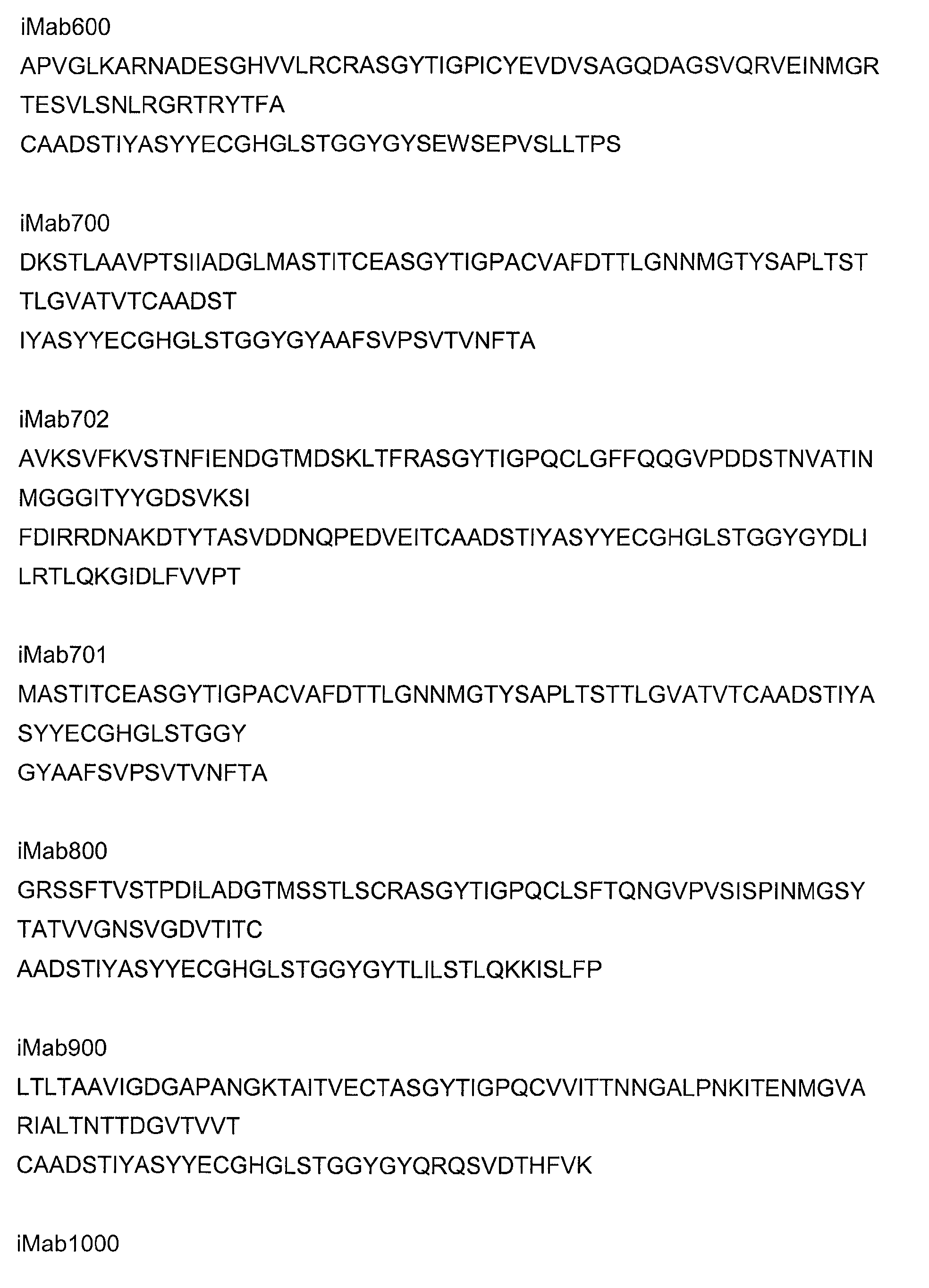

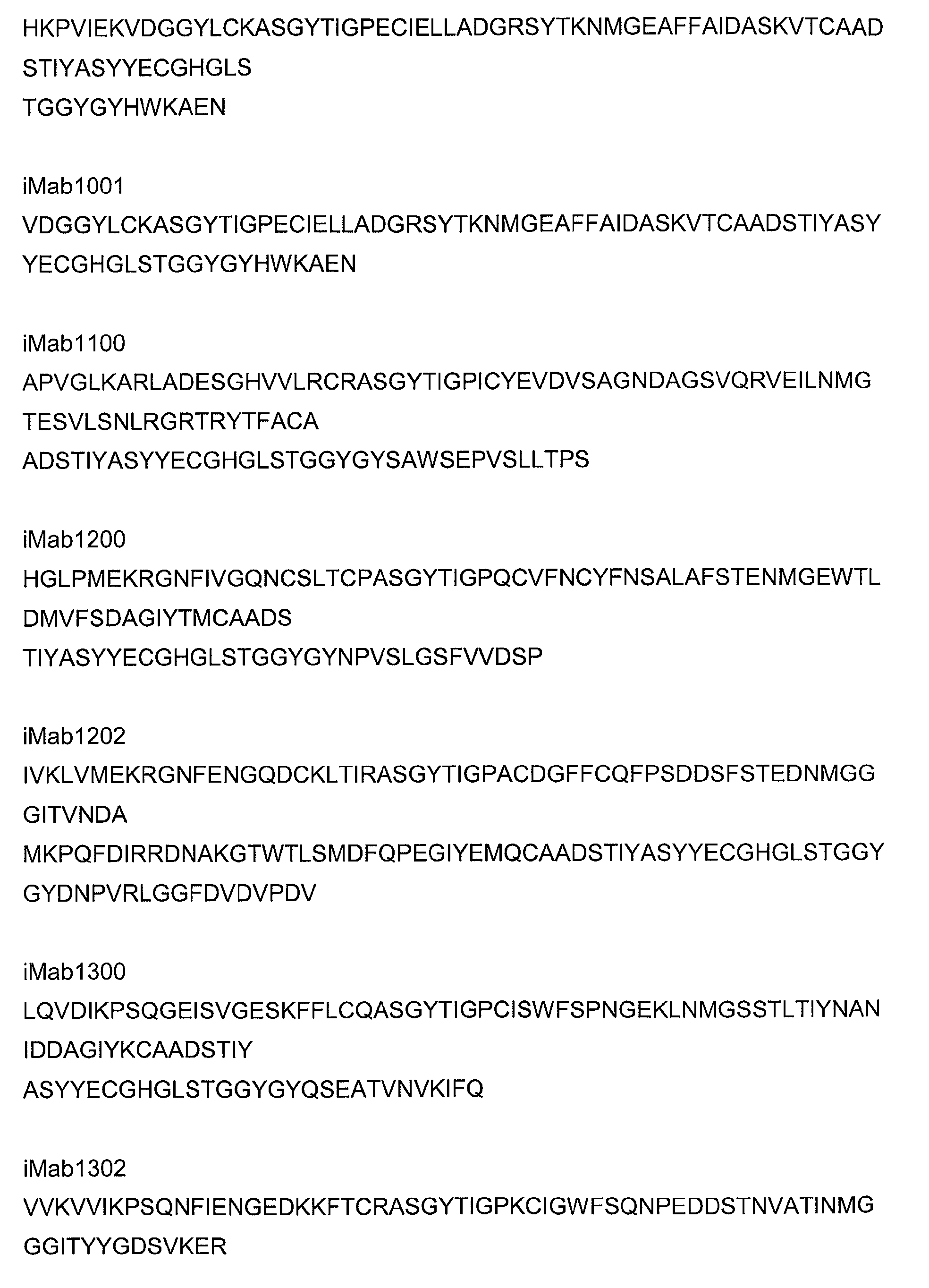

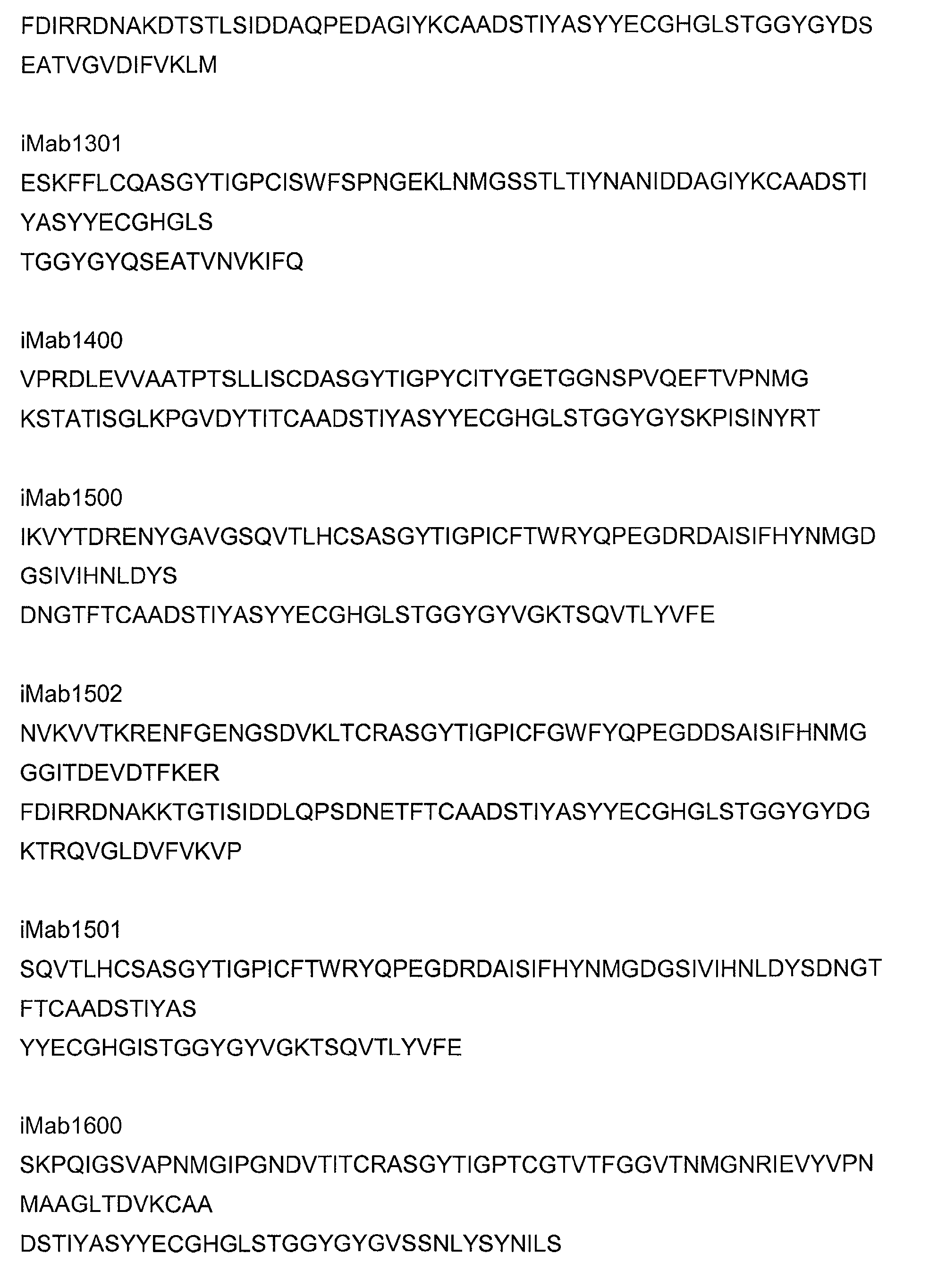

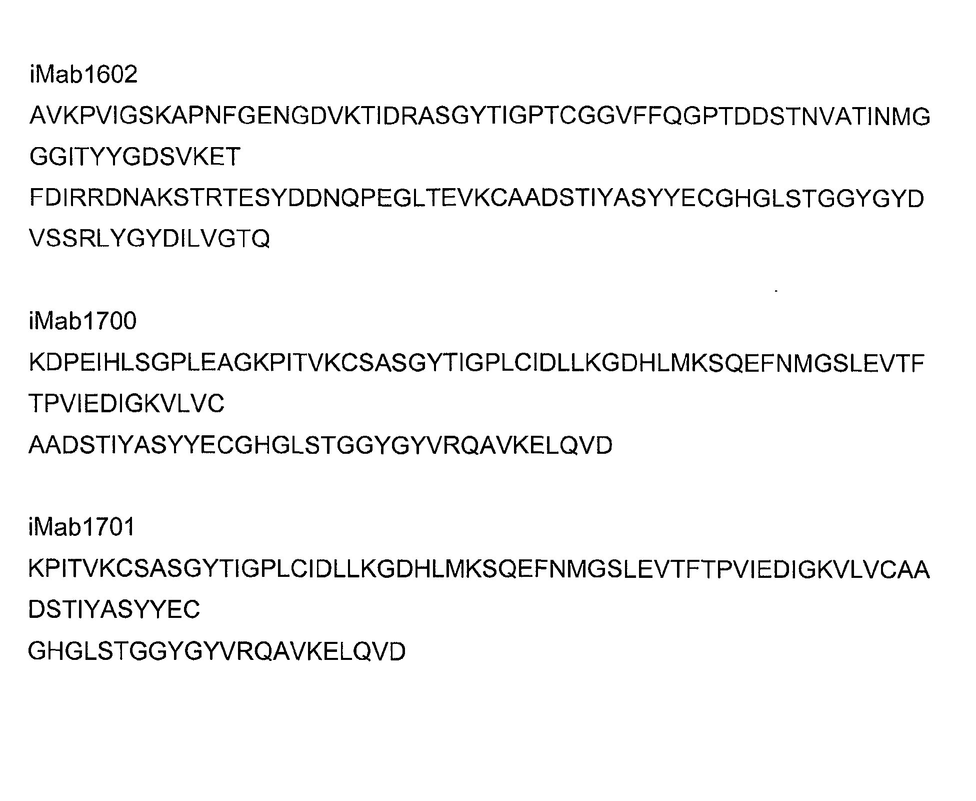

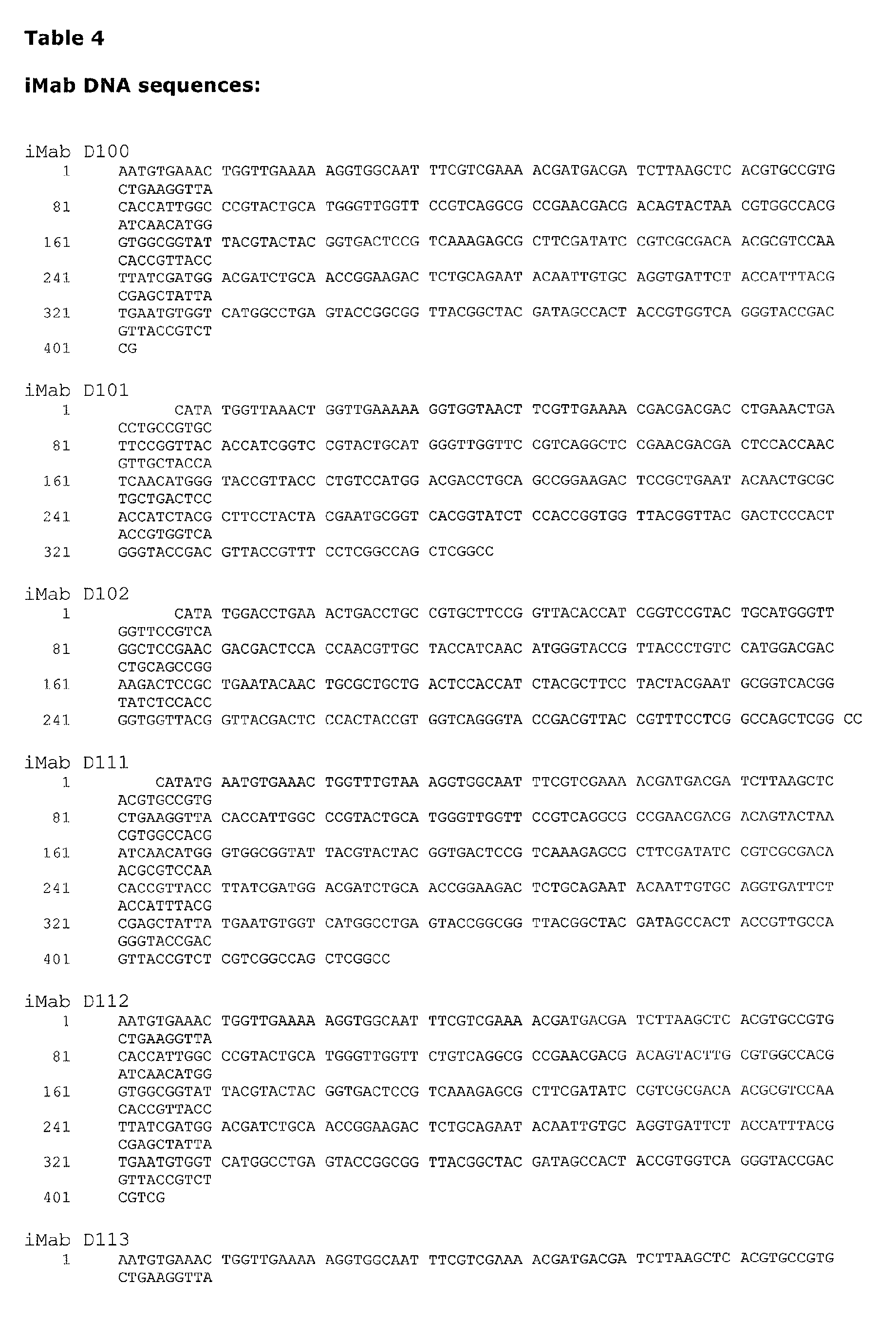

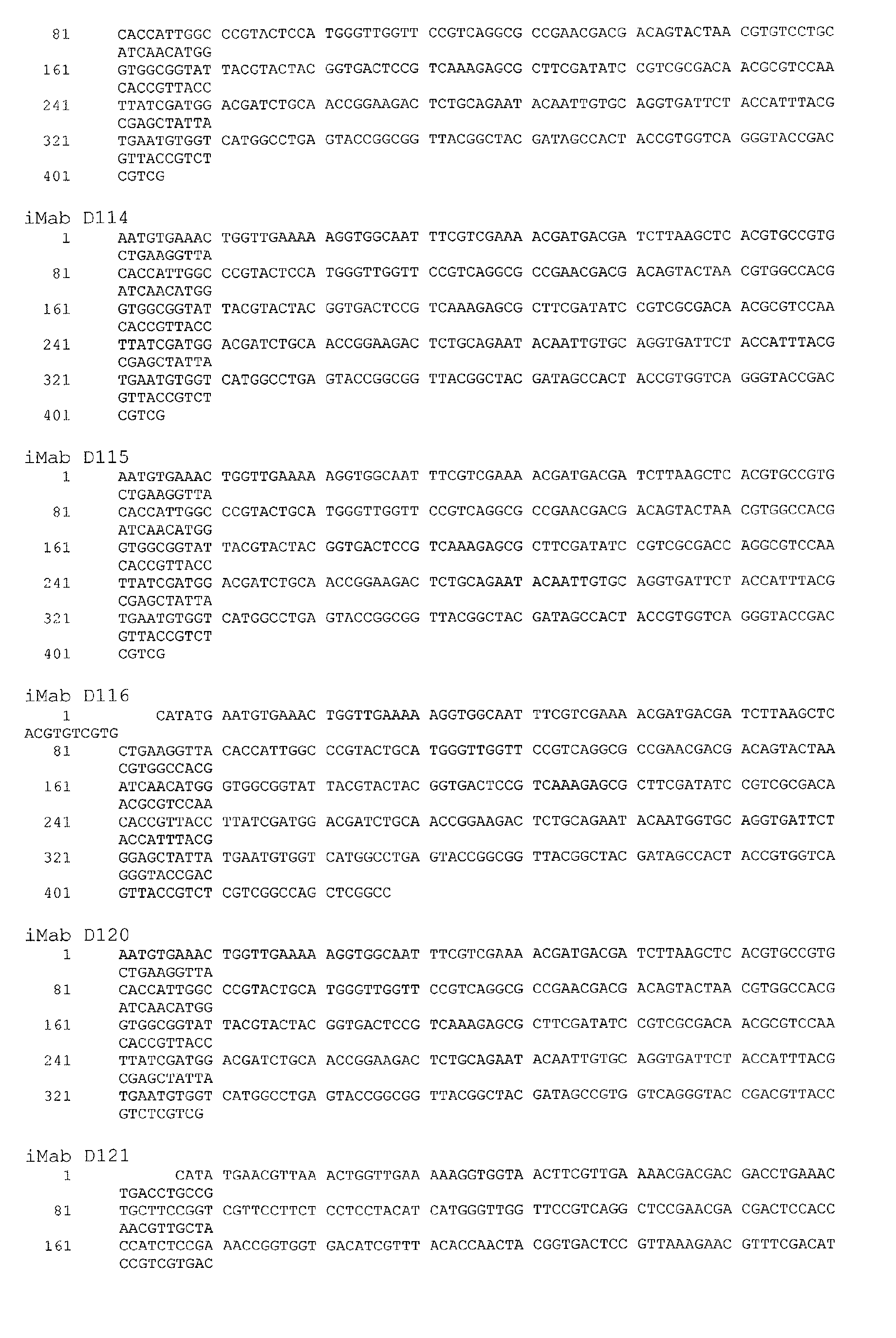

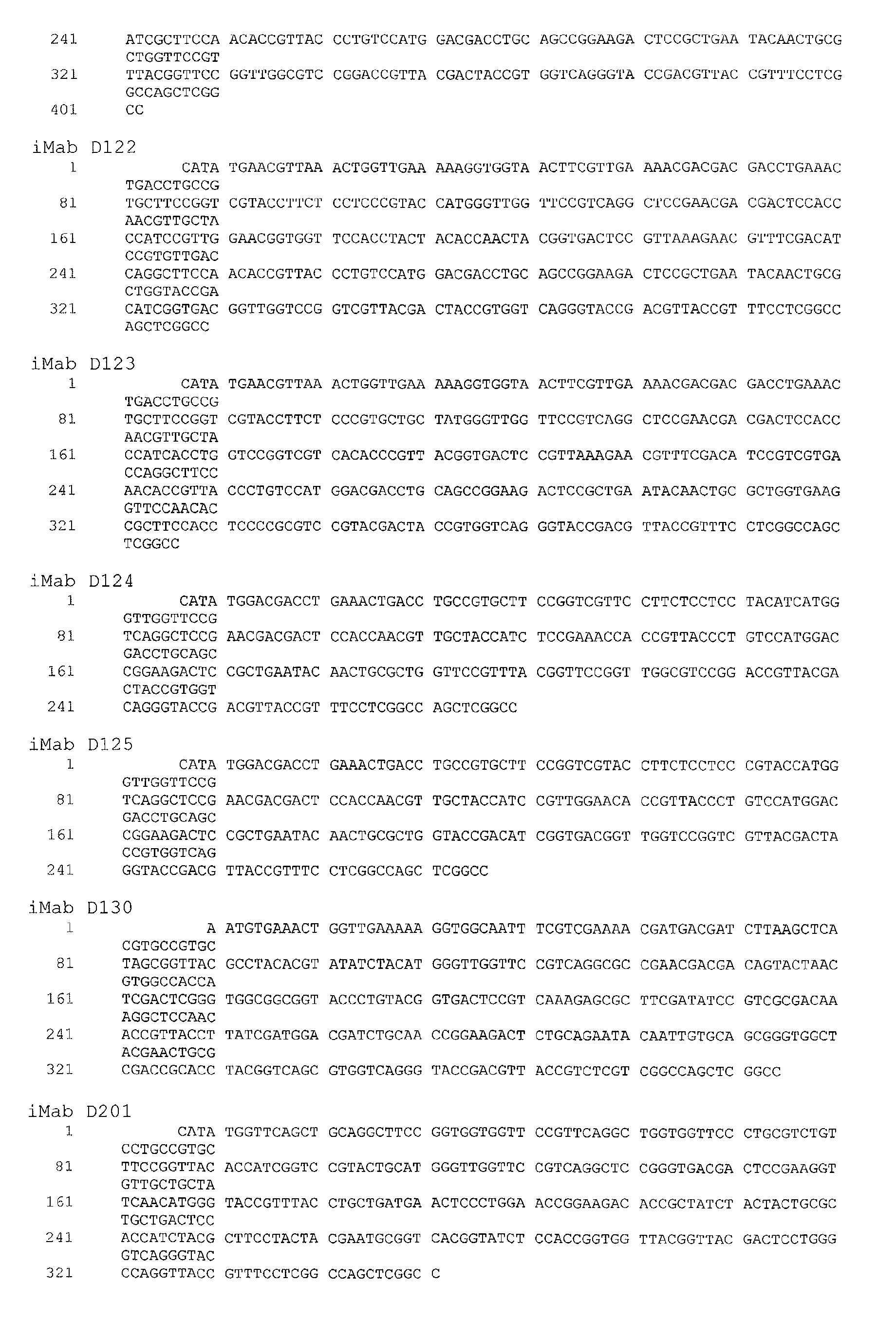

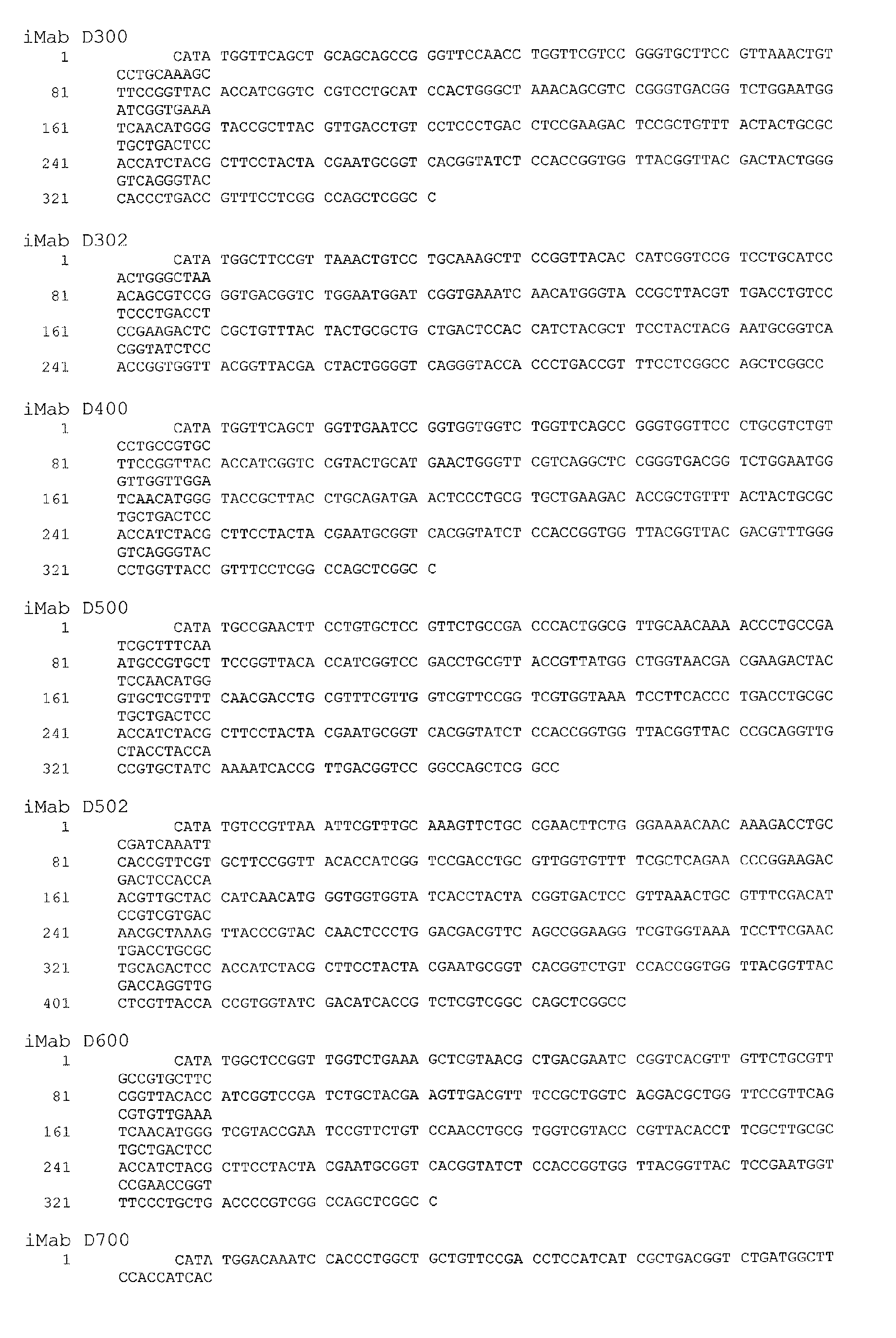

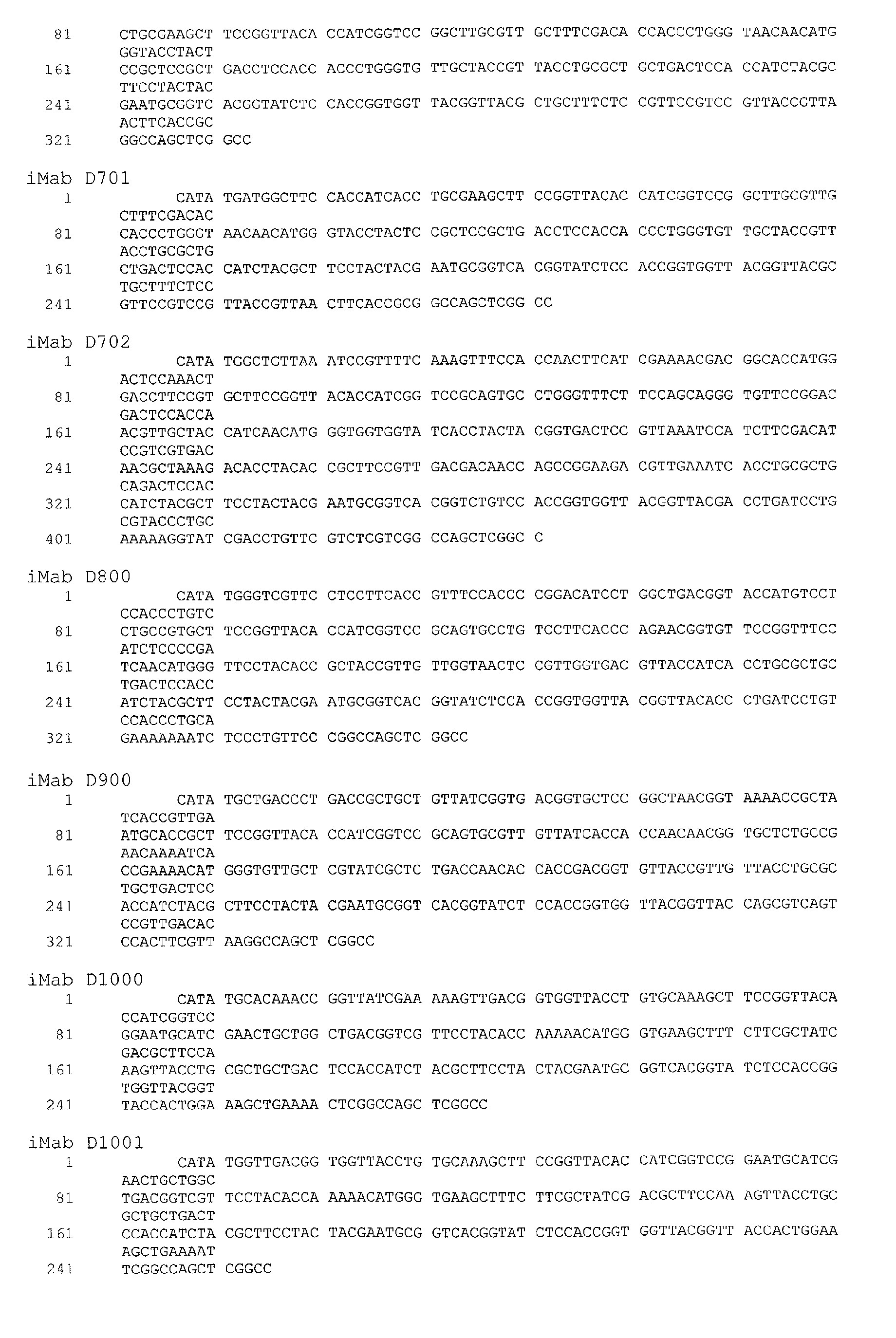

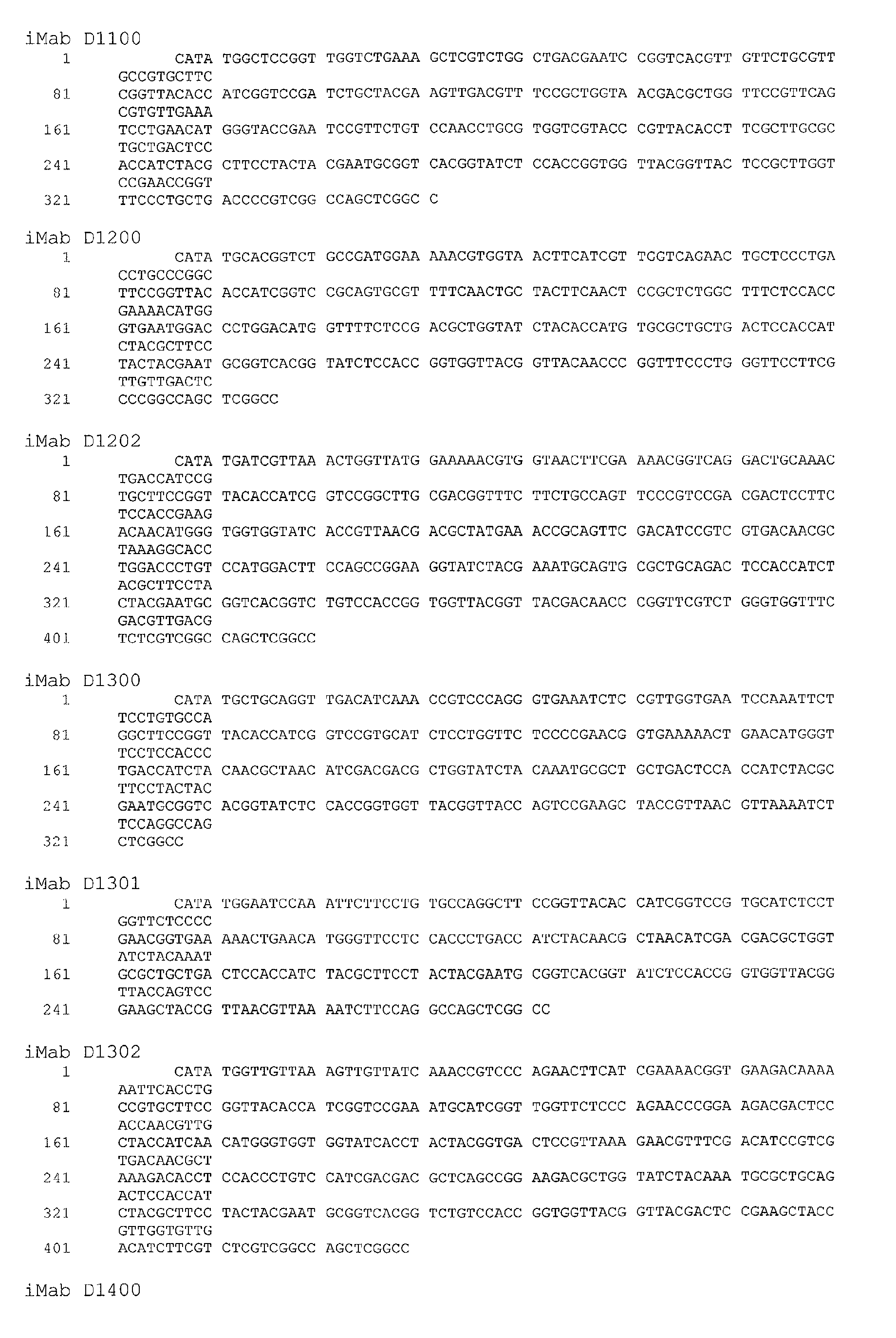

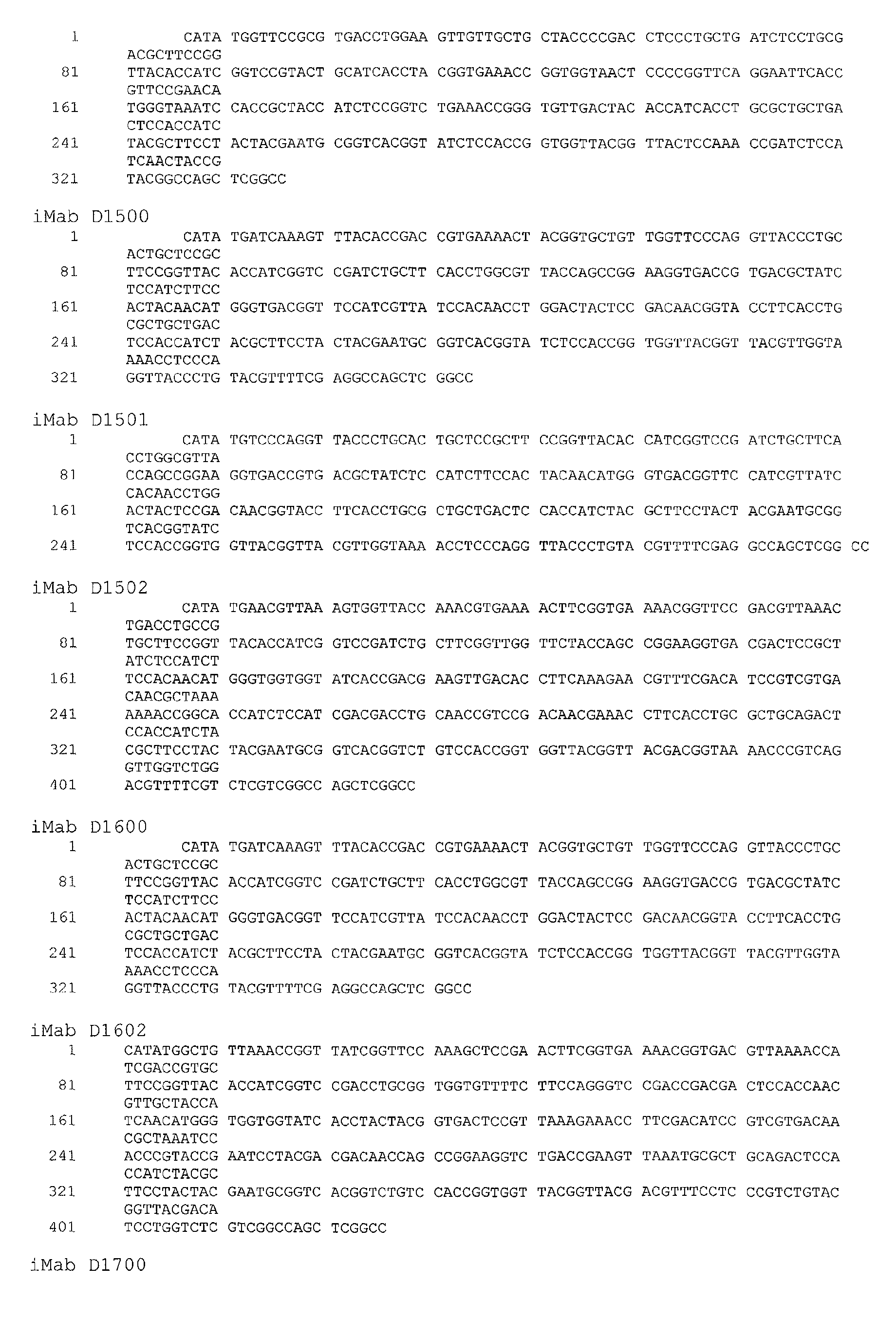

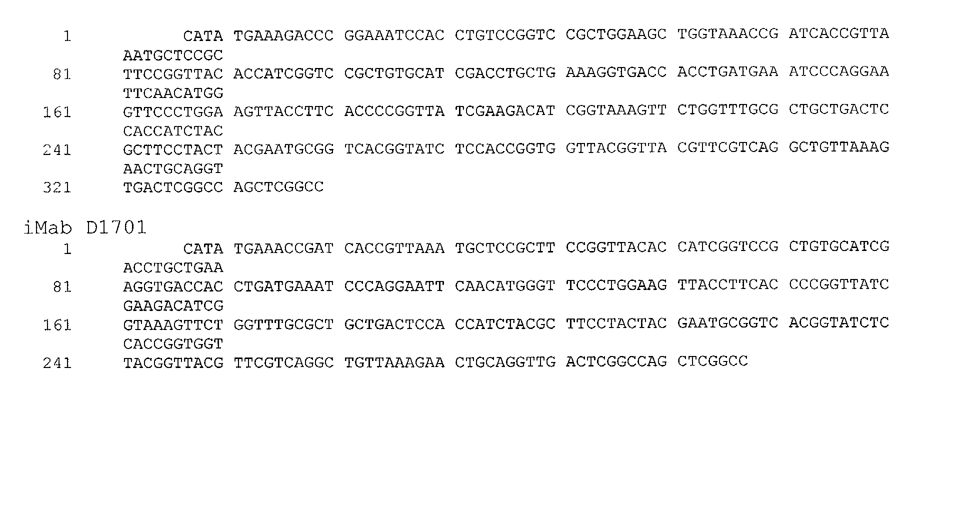

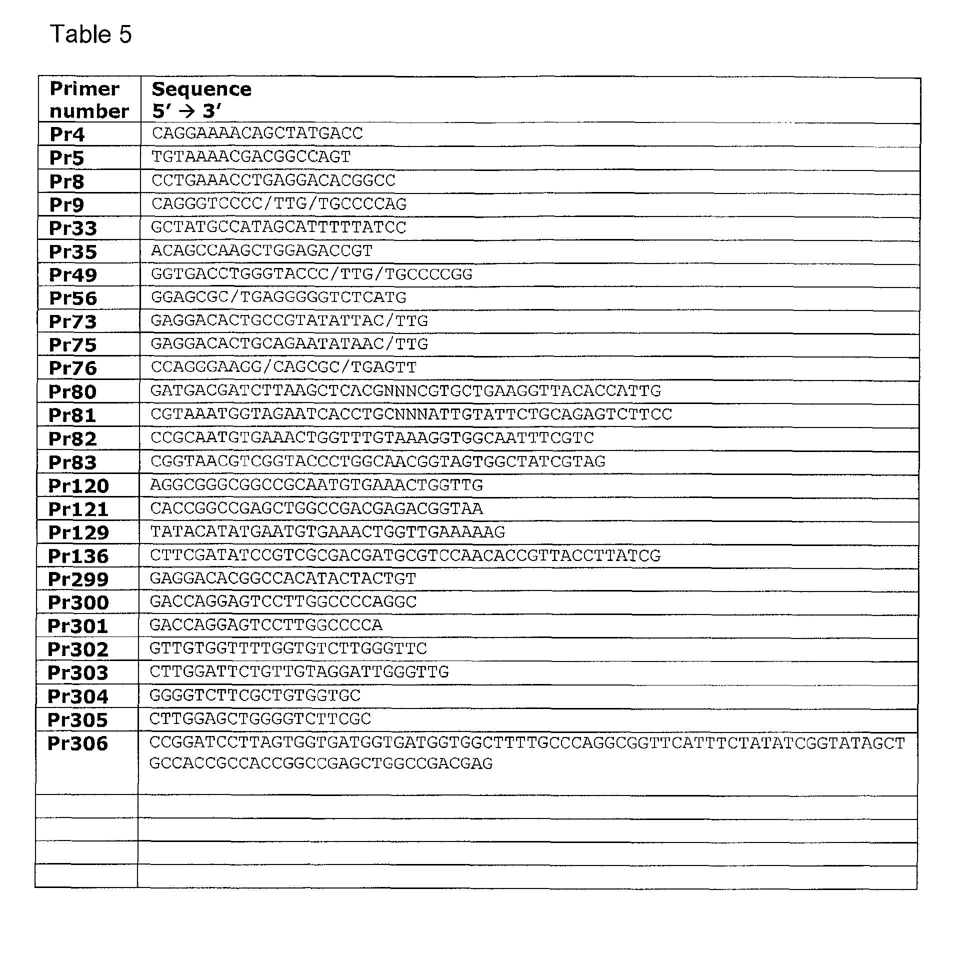

**Table 6**

| | | | | Absorbtion (450 nm) | |
|---|---|---|---|---|---|
| iMab | No. of β-sheets | Purification procedure | Amount of iMab applied per well (in 100 µl) | ELK (control) | Lysozyme (100 µg/ml) |
| 1302 | 9 | urea | ∼50 ng | 0.045 | 0.345 |
| 1602 | 9 | urea | ∼50 ng | 0.043 | 0.357 |
| 1202 | 9 | urea | ∼50 ng | 0.041 | 0.317 |
| 116 | 9 | urea | ∼50 ng | 0.042 | 0.238 |
| 101 | 7 | urea | ∼20 ng | 0.043 | 0.142 |
| 111 | 9 | urea | ∼50 ng | 0.043 | 0.420 |
| 701 | 6 | urea | ∼10 ng | 0.069 | 0.094 |
| 122 | 9 | urea | ∼50 ng | 0.051 | 0.271 |
| 1300 | 7 | urea | ∼50 ng | 0.041 | 0.325 |
| 1200 | 7 | urea | ∼5 ng | 0.040 | 0.061 |
| 900 | 7 | urea | ∼10 ng | 0.043 | 0.087 |
| 100 | 9 | urea | ∼50 ng | 0.040 | 0.494 |
| 100 | 9 | heat (60°C) | ∼50 ng | 0.041 | 0.369 |

### Table X. Binding characteristics of purified iMab variants to lysozyme.

Various purified iMabs containing either 6-, 7-, or 9 β-sheets were analyzed for binding to ELK (control) and lysozyme as described in examples 8, 15,19 and 23. All iMabs were purified using urea and subsequent matrix assisted refolding (example 7), except for iMab100 which was additionally also purified by heat-induced solubilization of inclusion bodies (example 6).

**Table 7**

| iMab dilution | Signal on Elisa of input iMab100 | Signal on Elisa of pH shocked iMab100 |
|---|---|---|
| 1:10 | 0.360 | 0.390 |
| 1:100 | 0.228 | 0.263 |
| 1:1000 | 0.128 | 0.169 |
| 1:10,000 | 0.059 | 0.059 |

**Table 8**

| 1NEU | | | | | | | | | | |
|---|---|---|---|---|---|---|---|---|---|---|
| ATOM | 1 | CA | GLY | 2 | -33.839 | -10.967 | -0.688 | 1.00 | 25.06 | C |
| ATOM | 2 | CA | GLY | 3 | -31.347 | -8.590 | -2.388 | 1.00 | 20.77 | C |
| ATOM | 3 | CA | GLY | 4 | -29.325 | -6.068 | -0.288 | 1.00 | 19.01 | C |
| ATOM | 4 | CA | GLY | 5 | -27.767 | -2.669 | -1.162 | 1.00 | 19.75 | C |
| ATOM | 5 | CA | GLY | 6 | -27.109 | 0.487 | 1.010 | 1.00 | 22.33 | C |
| ATOM | 6 | CA | GLY | 7 | -29.834 | 3.204 | 0.812 | 1.00 | 24.80 | C |
| ATOM | 7 | CA | GLY | 8 | -27.542 | 6.161 | 0.057 | 1.00 | 28.23 | C |
| ATOM | 8 | CA | GLY | 9 | -23.790 | 6.593 | -0.286 | 1.00 | 26.37 | C |
| ATOM | 9 | CA | GLY | 10 | -21.750 | 9.765 | -0.074 | 1.00 | 29.08 | C |
| ATOM | 10 | CA | GLY | 11 | -18.505 | 10.289 | -1.920 | 1.00 | 26.48 | C |
| ATOM | 11 | CA | GLY | 12 | -15.859 | 12.991 | -2.286 | 1.00 | 27.26 | C |
| ATOM | 12 | CA | GLY | 13 | -14.782 | 14.286 | -5.685 | 1.00 | 26.73 | C |
| ATOM | 13 | CA | GLY | 15 | -12.221 | 9.666 | -4.538 | 1.00 | 23.37 | C |
| ATOM | 14 | CA | GLY | 16 | -13.862 | 6.196 | -4.876 | 1.00 | 22.86 | C |
| ATOM | 15 | CA | GLY | 17 | -16.948 | 4.527 | -3.422 | 1.00 | 20.21 | C |
| ATOM | 16 | CA | GLY | 18 | -18.042 | 0.875 | -3.195 | 1.00 | 19.23 | C |
| ATOM | 17 | CA | GLY | 19 | -21.543 | -0.132 | -4.244 | 1.00 | 17.13 | C |
| ATOM | 18 | CA | GLY | 20 | -22.550 | -3.266 | -2.294 | 1.00 | 19.06 | C |
| ATOM | 19 | CA | GLY | 21 | -24.854 | -5.946 | -3.635 | 1.00 | 15.28 | C |
| ATOM | 20 | CA | GLY | 22 | -25.493 | -9.401 | -2.203 | 1.00 | 15.88 | C |
| ATOM | 21 | CA | GLY | 23 | -28.333 | -11.882 | -1.980 | 1.00 | 18.12 | C |
| ATOM | 22 | CA | GLY | 24 | -29.458 | -14.458 | 0.564 | 1.00 | 18.67 | C |
| ATOM | 23 | CA | GLY | 25 | -31.806 | -17.445 | 0.594 | 1.00 | 20.29 | C |
| ATOM | 24 | CA | GLY | 33 | -26.348 | -16.618 | -10.937 | 1.00 | 24.64 | C |
| ATOM | 25 | CA | GLY | 34 | -26.032 | -13.298 | -12.772 | 1.00 | 21.24 | C |
| ATOM | 26 | CA | GLY | 35 | -25.552 | -9.691 | -11.546 | 1.00 | 17.90 | C |
| ATOM | 27 | CA | GLY | 36 | -26.440 | -6.639 | -13.630 | 1.00 | 16.78 | C |
| ATOM | 28 | CA | GLY | 37 | -25.790 | -3.001 | -12.553 | 1.00 | 15.77 | C |
| ATOM | 29 | CA | GLY | 38 | -27.841 | -0.127 | -14.139 | 1.00 | 16.15 | C |
| ATOM | 30 | CA | GLY | 39 | -27.421 | 3.671 | -13.662 | 1.00 | 17.11 | C |
| ATOM | 31 | CA | GLY | 40 | -30.023 | 6.514 | -13.788 | 1.00 | 19.95 | C |
| ATOM | 32 | CA | GLY | 69 | -13.975 | 3.798 | -13.786 | 1.00 | 19.37 | C |
| ATOM | 33 | CA | GLY | 70 | -15.517 | 0.412 | -12.834 | 1.00 | 17.40 | C |
| ATOM | 34 | CA | GLY | 71 | -13.840 | -2.419 | -10.867 | 1.00 | 17.48 | C |
| ATOM | 35 | CA | GLY | 72 | -15.422 | -5.847 | -10.205 | 1.00 | 17.29 | C |
| ATOM | 36 | CA | GLY | 73 | -14.951 | -6.863 | -6.568 | 1.00 | 17.82 | C |
| ATOM | 37 | CA | GLY | 74 | -17.604 | -9.507 | -6.001 | 1.00 | 20.26 | C |
| ATOM | 38 | CA | GLY | 81 | -21.892 | -8.819 | -6.747 | 1.00 | 15.95 | C |
| ATOM | 39 | CA | GLY | 82 | -20.250 | -5.588 | -5.572 | 1.00 | 14.51 | C |
| ATOM | 40 | CA | GLY | 83 | -18.342 | -3.035 | -7.740 | 1.00 | 14.59 | C |
| ATOM | 41 | CA | GLY | 84 | -16.254 | 0.065 | -7.050 | 1.00 | 15.51 | C |
| ATOM | 42 | CA | GLY | 85 | -16.847 | 3.424 | -8.859 | 1.00 | 16.32 | C |
| ATOM | 43 | CA | GLY | 86 | -13.490 | 5.206 | -9.235 | 1.00 | 17.40 | C |
| ATOM | 44 | CA | GLY | 87 | -12.392 | 8.860 | -9.565 | 1.00 | 20.53 | C |
| ATOM | 45 | CA | GLY | 89 | -17.022 | 13.543 | -10.254 | 1.00 | 33.26 | C |
| ATOM | 46 | CA | GLY | 90 | -19.763 | 16.019 | -9.293 | 1.00 | 33.41 | C |
| ATOM | 47 | CA | GLY | 91 | -21.946 | 14.910 | -12.147 | 1.00 | 28.24 | C |
| ATOM | 48 | CA | GLY | 92 | -22.001 | 11.307 | -11.004 | 1.00 | 23.61 | C |
| ATOM | 49 | CA | GLY | 93 | -25.084 | 11.842 | -8.710 | 1.00 | 22.12 | C |
| ATOM | 50 | CA | GLY | 94 | -27.797 | 9.318 | -9.433 | 1.00 | 18.21 | C |
| ATOM | 51 | CA | GLY | 95 | -29.408 | 6.033 | -8.549 | 1.00 | 18.57 | C |
| ATOM | 52 | CA | GLY | 96 | -27.753 | 2.594 | -9.167 | 1.00 | 17.72 | C |
| ATOM | 53 | CA | GLY | 97 | -29.778 | -0.614 | -9.279 | 1.00 | 18.72 | C |
| ATOM | 54 | CA | GLY | 98 | -28.513 | -4.176 | -8.741 | 1.00 | 18.53 | C |
| ATOM | 55 | CA | GLY | 99 | -30.558 | -6.911 | -10.517 | 1.00 | 18.45 | C |
| ATOM | 56 | CA | GLY | 100 | -29.969 | -10.529 | -9.427 | 1.00 | 17.71 | C |
| ATOM | 57 | CA | GLY | 108 | -34.816 | -10.904 | -11.290 | 1.00 | 32.51 | C |
| ATOM | 58 | CA | GLY | 109 | -34.993 | -9.224 | -7.900 | 1.00 | 28.62 | C |
| ATOM | 59 | CA | GLY | 110 | -33.628 | -5.668 | -7.622 | 1.00 | 23.31 | C |
| ATOM | 60 | CA | GLY | 111 | -32.406 | -3.176 | -5.020 | 1.00 | 19.82 | C |
| ATOM | 61 | CA | GLY | 112 | -31.140 | 0.403 | -5.467 | 1.00 | 19.75 | C |
| ATOM | 62 | CA | GLY | 113 | -28.697 | 2.839 | -3.811 | 1.00 | 18.42 | C |
| ATOM | 63 | CA | GLY | 114 | -28.461 | 6.627 | -4.417 | 1.00 | 18.70 | C |
| ATOM | 64 | CA | GLY | 115 | -25.110 | 8.413 | -4.799 | 1.00 | 19.95 | C |
| ATOM | 65 | CA | GLY | 116 | -24.286 | 12.022 | -3.753 | 1.00 | 25.78 | C |
| ATOM | 66 | CA | GLY | 117 | -20.816 | 13.493 | -4.292 | 1.00 | 31.06 | C |
| ATOM | 67 | CA | GLY | 118 | -19.616 | 16.565 | -2.380 | 1.00 | 38.60 | C |
| ATOM | 68 | CA | GLY | 119 | -16.267 | 18.356 | -1.757 | 1.00 | 42.50 | C |
| TER | | | | | | | | | | |
| | | | | | | | | | | |

| 1MEL | | | | | | | | | | |
|---|---|---|---|---|---|---|---|---|---|---|
| ATOM | 69 | CA | GLY A | 3 | -34.517 | -10.371 | -1.234 | 1.00 | 36.96 | C |
| ATOM | 70 | CA | GLY A | 4 | -31.790 | -8.022 | -2.500 | 1.00 | 28.80 | C |
| ATOM | 71 | CA | GLY A | 5 | -30.310 | -5.603 | 0.018 | 1.00 | 31.64 | C |
| ATOM | 72 | CA | GLY A | 6 | -27.884 | -2.957 | -1.209 | 1.00 | 24.68 | C |
| ATOM | 73 | CA | GLY A | 7 | -25.500 | -1.042 | 1.073 | 1.00 | 21.60 | C |
| ATOM | 74 | CA | GLY A | 8 | -23.005 | 1.710 | 0.458 | 1.00 | 17.27 | C |
| ATOM | 75 | CA | GLY A | 9 | -23.567 | 4.843 | -1.499 | 1.00 | 18.58 | C |
| ATOM | 76 | CA | GLY A | 10 | -23.648 | 8.381 | -0.100 | 1.00 | 16.78 | C |
| ATOM | 77 | CA | GLY A | 11 | -21.736 | 11.497 | -1.127 | 1.00 | 11.36 | C |
| ATOM | 78 | CA | GLY A | 12 | -18.140 | 11.942 | -1.980 | 1.00 | 8.84 | C |
| ATOM | 79 | CA | GLY A | 13 | -16.006 | 14.459 | -3.746 | 1.00 | 11.16 | C |
| ATOM | 80 | CA | GLY A | 14 | -15.243 | 14.091 | -7.418 | 1.00 | 10.62 | C |
| ATOM | 81 | CA | GLY A | 16 | -12.920 | 9.782 | -4.554 | 1.00 | 7.90 | C |
| ATOM | 82 | CA | GLY A | 17 | -14.217 | 6.244 | -4.387 | 1.00 | 7.54 | C |
| ATOM | 83 | CA | GLY A | 18 | -17.233 | 4.430 | -2.940 | 1.00 | 6.47 | C |
| ATOM | 84 | CA | GLY A | 19 | -18.104 | 0.790 | -2.418 | 1.00 | 5.43 | C |
| ATOM | 85 | CA | GLY A | 20 | -21.615 | -0.610 | -2.878 | 1.00 | 8.09 | C |
| ATOM | 86 | CA | GLY A | 21 | -22.724 | -4.116 | -1.837 | 1.00 | 8.76 | C |
| ATOM | 87 | CA | GLY A | 22 | -25.644 | -6.399 | -2.433 | 1.00 | 8.33 | C |
| ATOM | 88 | CA | GLY A | 23 | -26.642 | -9.585 | -0.745 | 1.00 | 13.77 | C |
| ATOM | 89 | CA | GLY A | 24 | -29.318 | -11.732 | -2.396 | 1.00 | 17.53 | C |
| ATOM | 90 | CA | GLY A | 25 | -31.340 | -13.525 | 0.256 | 1.00 | 23.23 | C |
| ATOM | 91 | CA | GLY A | 26 | -33.969 | -16.194 | 0.081 | 1.00 | 25.25 | C |
| ATOM | 92 | CA | GLY A | 32 | -27.171 | -16.809 | -12.135 | 1.00 | 5.00 | C |
| ATOM | 93 | CA | GLY A | 33 | -26.411 | -13.068 | -12.502 | 1.00 | 4.64 | C |
| ATOM | 94 | CA | GLY A | 34 | -26.109 | -10.036 | -10.166 | 1.00 | 2.48 | C |
| ATOM | 95 | CA | GLY A | 35 | -25.386 | -6.603 | -11.615 | 1.00 | 2.00 | C |
| ATOM | 96 | CA | GLY A | 36 | -25.845 | -2.895 | -11.151 | 1.00 | 3.40 | C |
| ATOM | 97 | CA | GLY A | 37 | -28.032 | -0.410 | -12.986 | 1.00 | 6.64 | C |
| ATOM | 98 | CA | GLY A | 38 | -28.158 | 3.311 | -12.472 | 1.00 | 8.74 | C |
| ATOM | 99 | CA | GLY A | 39 | -30.731 | 6.025 | -13.179 | 1.00 | 19.24 | C |
| ATOM | 100 | CA | GLY A | 67 | -12.972 | 2.698 | -13.036 | 1.00 | 12.68 | C |
| ATOM | 101 | CA | GLY A | 68 | -15.482 | 0.261 | -11.584 | 1.00 | 8.15 | C |
| ATOM | 102 | CA | GLY A | 69 | -14.843 | -3.306 | -10.511 | 1.00 | 8.39 | C |
| ATOM | 103 | CA | GLY A | 70 | -17.520 | -5.831 | -9.556 | 1.00 | 5.47 | C |
| ATOM | 104 | CA | GLY A | 71 | -16.742 | -8.900 | -7.482 | 1.00 | 8.48 | C |
| ATOM | 105 | CA | GLY A | 72 | -18.459 | -11.484 | -5.287 | 1.00 | 18.00 | C |
| ATOM | 106 | CA | GLY A | 79 | -21.342 | -8.788 | -4.615 | 1.00 | 9.55 | C |
| ATOM | 107 | CA | GLY A | 80 | -19.553 | -5.399 | -4.519 | 1.00 | 4.94 | C |
| ATOM | 108 | CA | GLY A | 81 | -18.901 | -2.601 | -6.858 | 1.00 | 4.92 | C |
| ATOM | 109 | CA | GLY A | 82 | -15.755 | -0.638 | -6.085 | 1.00 | 7.44 | C |
| ATOM | 110 | CA | GLY A | 83 | -16.081 | 2.748 | -7.765 | 1.00 | 6.55 | C |
| ATOM | 111 | CA | GLY A | 84 | -12.869 | 4.759 | -8.177 | 1.00 | 9.49 | C |
| ATOM | 112 | CA | GLY A | 85 | -12.245 | 8.019 | -10.028 | 1.00 | 12.54 | C |
| ATOM | 113 | CA | GLY A | 87 | -16.853 | 12.035 | -11.452 | 1.00 | 15.46 | C |
| ATOM | 114 | CA | GLY A | 88 | -20.054 | 14.055 | -10.955 | 1.00 | 13.94 | C |
| ATOM | 115 | CA | GLY A | 89 | -21.497 | 12.384 | -14.095 | 1.00 | 18.68 | C |
| ATOM | 116 | CA | GLY A | 90 | -21.637 | 9.217 | -11.955 | 1.00 | 9.55 | C |
| ATOM | 117 | CA | GLY A | 91 | -24.288 | 10.888 | -9.734 | 1.00 | 6.54 | C |
| ATOM | 118 | CA | GLY A | 92 | -27.349 | 8.637 | -9.936 | 1.00 | 6.38 | C |
| ATOM | 119 | CA | GLY A | 93 | -29.573 | 6.105 | -8.204 | 1.00 | 6.33 | C |
| ATOM | 120 | CA | GLY A | 94 | -27.866 | 2.727 | -8.154 | 1.00 | 6.08 | C |
| ATOM | 121 | CA | GLY A | 95 | -29.928 | -0.377 | -8.312 | 1.00 | 10.12 | C |
| ATOM | 122 | CA | GLY A | 96 | -28.884 | -3.923 | -7.638 | 1.00 | 10.76 | C |
| ATOM | 123 | CA | GLY A | 97 | -30.439 | -6.641 | -9.794 | 1.00 | 9.08 | C |
| ATOM | 124 | CA | GLY A | 98 | -30.321 | -10.442 | -10.097 | 1.00 | 8.28 | C |
| ATOM | 125 | CA | GLY A | 122 | -35.231 | -9.331 | -9.016 | 1.00 | 15.49 | C |
| ATOM | 126 | CA | GLY A | 123 | -34.520 | -5.802 | -8.079 | 1.00 | 12.37 | C |
| ATOM | 127 | CA | GLY A | 124 | -33.455 | -4.050 | -4.953 | 1.00 | 14.34 | C |
| ATOM | 128 | CA | GLY A | 125 | -33.918 | -0.696 | -3.317 | 1.00 | 22.49 | C |
| ATOM | 129 | CA | GLY A | 126 | -32.054 | 2.128 | -5.022 | 1.00 | 17.72 | C |
| ATOM | 130 | CA | GLY A | 127 | -29.137 | 3.817 | -3.342 | 1.00 | 16.51 | C |
| ATOM | 131 | CA | GLY A | 128 | -28.275 | 7.401 | -4.265 | 1.00 | 16.84 | C |
| ATOM | 132 | CA | GLY A | 129 | -24.678 | 8.216 | -4.904 | 1.00 | 11.90 | C |
| ATOM | 133 | CA | GLY A | 130 | -23.878 | 11.873 | -5.299 | 1.00 | 8.75 | C |
| ATOM | 134 | CA | GLY A | 131 | -20.508 | 13.061 | -6.466 | 1.00 | 14.37 | C |
| ATOM | 135 | CA | GLY A | 132 | -19.632 | 16.597 | -5.198 | 1.00 | 23.32 | C |
| ATOM | 136 | CA | GLY A | 133 | -17.732 | 19.533 | -6.720 | 1.00 | 36.14 | C |
| TER | | | | | | | | | | |
| | | | | | | | | | | |

| 1F97 | | | | | | | | | | |
|---|---|---|---|---|---|---|---|---|---|---|
| ATOM | 137 | CA | GLY A | 29 | -33.679 | -11.517 | -0.808 | 1.00 | 41.25 | C |
| ATOM | 138 | CA | GLY A | 30 | -31.468 | -8.740 | -2.170 | 1.00 | 22.43 | C |
| ATOM | 139 | CA | GLY A | 31 | -30.250 | -5.886 | 0.038 | 1.00 | 24.73 | C |
| ATOM | 140 | CA | GLY A | 32 | -27.706 | -3.105 | 0.530 | 1.00 | 20.95 | C |
| ATOM | 141 | CA | GLY A | 33 | -25.811 | -1.723 | 3.523 | 1.00 | 28.77 | C |
| ATOM | 142 | CA | GLY A | 34 | -26.349 | 1.878 | 2.419 | 1.00 | 33.48 | C |
| ATOM | 143 | CA | GLY A | 35 | -29.027 | 3.067 | -0.012 | 1.00 | 27.47 | C |
| ATOM | 144 | CA | GLY A | 36 | -27.980 | 6.732 | 0.249 | 1.00 | 29.20 | C |
| ATOM | 145 | CA | GLY A | 37 | -24.279 | 6.955 | -0.586 | 1.00 | 23.99 | C |
| ATOM | 146 | CA | GLY A | 38 | -22.275 | 10.179 | -0.393 | 1.00 | 24.19 | C |
| ATOM | 147 | CA | GLY A | 39 | -18.592 | 10.286 | -1.302 | 1.00 | 15.35 | C |
| ATOM | 148 | CA | GLY A | 40 | -16.117 | 13.059 | -2.218 | 1.00 | 12.64 | C |
| ATOM | 149 | CA | GLY A | 41 | -15.286 | 13.515 | -5.906 | 1.00 | 9.24 | C |
| ATOM | 150 | CA | GLY A | 43 | -12.412 | 8.992 | -4.540 | 1.00 | 14.44 | C |
| ATOM | 151 | CA | GLY A | 44 | -13.115 | 5.267 | -4.685 | 1.00 | 21.52 | C |
| ATOM | 152 | CA | GLY A | 45 | -16.460 | 3.862 | -3.630 | 1.00 | 22.48 | C |
| ATOM | 153 | CA | GLY A | 46 | -18.082 | 0.444 | -3.532 | 1.00 | 21.22 | C |
| ATOM | 154 | CA | GLY A | 47 | -21.817 | 0.219 | -4.135 | 1.00 | 20.06 | C |
| ATOM | 155 | CA | GLY A | 48 | -22.797 | -2.825 | -2.072 | 1.00 | 13.56 | C |
| ATOM | 156 | CA | GLY A | 49 | -25.390 | -5.432 | -3.034 | 1.00 | 19.14 | C |
| ATOM | 157 | CA | GLY A | 50 | -25.724 | -8.537 | -0.876 | 1.00 | 22.49 | C |
| ATOM | 158 | CA | GLY A | 51 | -28.046 | -11.470 | -1.549 | 1.00 | 20.58 | C |
| ATOM | 159 | CA | GLY A | 52 | -28.951 | -14.871 | -0.105 | 1.00 | 24.55 | C |
| ATOM | 160 | CA | GLY A | 53 | -30.893 | -17.875 | -1.353 | 1.00 | 17.82 | C |
| ATOM | 161 | CA | GLY A | 57 | -26.875 | -15.797 | -9.701 | 1.00 | 10.39 | C |
| ATOM | 162 | CA | GLY A | 58 | -26.674 | -13.408 | -12.632 | 1.00 | 8.00 | C |
| ATOM | 163 | CA | GLY A | 59 | -25.845 | -9.939 | -11.318 | 1.00 | 7.62 | C |
| ATOM | 164 | CA | GLY A | 60 | -26.653 | -6.841 | -13.371 | 1.00 | 6.84 | C |
| ATOM | 165 | CA | GLY A | 61 | -26.457 | -3.109 | -12.711 | 1.00 | 8.48 | C |
| ATOM | 166 | CA | GLY A | 62 | -28.184 | -0.168 | -14.336 | 1.00 | 13.92 | C |
| ATOM | 167 | CA | GLY A | 63 | -27.611 | 3.567 | -14.043 | 1.00 | 11.89 | C |
| ATOM | 168 | CA | GLY A | 64 | -30.532 | 5.981 | -14.200 | 1.00 | 23.18 | C |
| ATOM | 169 | CA | GLY A | 85 | -12.888 | 2.268 | -13.813 | 1.00 | 17.93 | C |
| ATOM | 170 | CA | GLY A | 86 | -15.508 | -0.149 | -12.447 | 1.00 | 15.44 | C |
| ATOM | 171 | CA | GLY A | 87 | -14.603 | -3.542 | -11.016 | 1.00 | 16.79 | C |
| ATOM | 172 | CA | GLY A | 88 | -17.118 | -6.318 | -10.381 | 1.00 | 14.07 | C |
| ATOM | 173 | CA | GLY A | 89 | -17.389 | -8.592 | -7.349 | 1.00 | 16.75 | C |
| ATOM | 174 | CA | GLY A | 91 | -20.798 | -8.262 | -3.202 | 1.00 | 17.13 | C |
| ATOM | 175 | CA | GLY A | 92 | -20.995 | -5.059 | -5.247 | 1.00 | 12.84 | C |
| ATOM | 176 | CA | GLY A | 93 | -19.287 | -2.826 | -7.795 | 1.00 | 12.33 | C |
| ATOM | 177 | CA | GLY A | 94 | -16.243 | -0.709 | -6.986 | 1.00 | 12.43 | C |
| ATOM | 178 | CA | GLY A | 95 | -15.485 | 2.596 | -8.696 | 1.00 | 11.12 | C |
| ATOM | 179 | CA | GLY A | 96 | -11.765 | 3.339 | -8.675 | 1.00 | 16.95 | C |
| ATOM | 180 | CA | GLY A | 97 | -12.855 | 7.004 | -8.899 | 1.00 | 21.11 | C |
| ATOM | 181 | CA | GLY A | 99 | -16.935 | 12.349 | -10.689 | 1.00 | 22.37 | C |
| ATOM | 182 | CA | GLY A | 100 | -19.974 | 14.613 | -10.361 | 1.00 | 23.75 | C |
| ATOM | 183 | CA | GLY A | 101 | -21.190 | 13.009 | -13.619 | 1.00 | 22.84 | C |
| ATOM | 184 | CA | GLY A | 102 | -21.820 | 9.789 | -11.695 | 1.00 | 15.51 | C |
| ATOM | 185 | CA | GLY A | 103 | -24.651 | 11.224 | -9.565 | 1.00 | 11.57 | C |
| ATOM | 186 | CA | GLY A | 104 | -27.817 | 9.170 | -9.882 | 1.00 | 9.52 | C |
| ATOM | 187 | CA | GLY A | 105 | -29.401 | 5.897 | -8.871 | 1.00 | 12.64 | C |
| ATOM | 188 | CA | GLY A | 106 | -27.851 | 2.484 | -9.413 | 1.00 | 7.32 | C |
| ATOM | 189 | CA | GLY A | 107 | -30.057 | -0.590 | -9.334 | 1.00 | 7.52 | C |
| ATOM | 190 | CA | GLY A | 108 | -28.588 | -3.984 | -8.524 | 1.00 | 9.97 | C |
| ATOM | 191 | CA | GLY A | 109 | -30.576 | -6.743 | -10.211 | 1.00 | 12.91 | C |
| ATOM | 192 | CA | GLY A | 110 | -29.973 | -10.300 | -9.043 | 1.00 | 11.27 | C |
| ATOM | 193 | CA | GLY A | 118 | -35.528 | -12.495 | -8.616 | 1.00 | 13.29 | C |
| ATOM | 194 | CA | GLY A | 119 | -34.748 | -9.395 | -6.594 | 1.00 | 15.55 | C |
| ATOM | 195 | CA | GLY A | 120 | -33.436 | -5.837 | -6.855 | 1.00 | 11.36 | C |
| ATOM | 196 | CA | GLY A | 121 | -32.267 | -2.894 | -4.778 | 1.00 | 11.36 | C |
| ATOM | 197 | CA | GLY A | 122 | -31.635 | 0.758 | -5.660 | 1.00 | 11.14 | C |
| ATOM | 198 | CA | GLY A | 123 | -28.791 | 2.935 | -4.379 | 1.00 | 11.58 | C |
| ATOM | 199 | CA | GLY A | 124 | -28.626 | 6.699 | -4.773 | 1.00 | 15.52 | C |
| ATOM | 200 | CA | GLY A | 125 | -25.128 | 8.065 | -5.281 | 1.00 | 9.61 | C |
| ATOM | 201 | CA | GLY A | 126 | -24.275 | 11.677 | -4.483 | 1.00 | 10.84 | C |
| ATOM | 202 | CA | GLY A | 127 | -20.739 | 12.778 | -5.330 | 1.00 | 10.58 | C |
| ATOM | 203 | CA | GLYA | 128 | -19.667 | 15.540 | -2.929 | 1.00 | 14.41 | C |
| ATOM | 204 | CA | GLY A | 129 | -18.355 | 18.818 | -4.335 | 1.00 | 12.32 | C |
| TER | | | | | | | | | | |
| | | | | | | | | | | |

| 1DQT | | | | | | | | | | |
|---|---|---|---|---|---|---|---|---|---|---|
| ATOM | 205 | CA | GLY C | 2 | -37.000 | -7.803 | -3.232 | 1.00 | 35.96 | C |
| ATOM | 206 | CA | GLY C | 3 | -33.582 | -6.481 | -4.122 | 1.00 | 30.20 | C |
| ATOM | 207 | CA | GLY C | 4 | -31.887 | -3.962 | -1.908 | 1.00 | 27.24 | C |
| ATOM | 208 | CA | GLY C | 5 | -28.640 | -2.044 | -1.950 | 1.00 | 23.16 | C |
| ATOM | 209 | CA | GLY C | 6 | -27.069 | 0.720 | 0.216 | 1.00 | 22.73 | C |
| ATOM | 210 | CA | GLY C | 7 | -28.256 | 4.289 | -0.273 | 1.00 | 24.33 | C |
| ATOM | 211 | CA | GLY C | 8 | -24.800 | 5.874 | -0.329 | 1.00 | 21.64 | C |
| ATOM | 212 | CA | GLY C | 9 | -21.252 | 4.822 | -1.130 | 1.00 | 20.25 | C |
| ATOM | 213 | CA | GLY C | 10 | -18.186 | 7.087 | -0.970 | 1.00 | 20.37 | C |
| ATOM | 214 | CA | GLY C | 11 | -15.855 | 5.961 | -3.761 | 1.00 | 22.46 | C |
| ATOM | 215 | CA | GLY C | 12 | -12.159 | 5.548 | -2.990 | 1.00 | 24.30 | C |
| ATOM | 216 | CA | GLY C | 14 | -8.661 | 5.520 | -6.931 | 1.00 | 30.82 | C |
| ATOM | 217 | CA | GLY C | 15 | -12.218 | 5.495 | -8.241 | 1.00 | 25.29 | C |
| ATOM | 218 | CA | GLY C | 16 | -13.342 | 2.240 | -6.604 | 1.00 | 21.63 | C |
| ATOM | 219 | CA | GLY C | 17 | -16.853 | 1.719 | -5.287 | 1.00 | 20.22 | C |
| ATOM | 220 | CA | GLY C | 18 | -17.869 | -1.533 | -3.647 | 1.00 | 20.86 | C |
| ATOM | 221 | CA | GLY C | 19 | -21.187 | -2.475 | -2.147 | 1.00 | 21.25 | C |
| ATOM | 222 | CA | GLY C | 20 | -23.544 | -5.395 | -1.581 | 1.00 | 21.73 | C |
| ATOM | 223 | CA | GLY C | 21 | -26.665 | -6.116 | -3.611 | 1.00 | 22.97 | C |
| ATOM | 224 | CA | GLY C | 22 | -29.032 | -8.318 | -1.684 | 1.00 | 26.59 | C |
| ATOM | 225 | CA | GLY C | 23 | -32.087 | -10.258 | -2.722 | 1.00 | 26.47 | C |
| ATOM | 226 | CA | GLY C | 24 | -34.892 | -12.390 | -1.373 | 1.00 | 31.54 | C |
| ATOM | 227 | CA | GLY C | 25 | -36.216 | -14.994 | -1.386 | 1.00 | 31.33 | C |
| ATOM | 228 | CA | GLY C | 32 | -28.221 | -15.396 | -10.763 | 1.00 | 21.25 | C |
| ATOM | 229 | CA | GLY C | 33 | -27.582 | -12.861 | -13.499 | 1.00 | 21.86 | C |
| ATOM | 230 | CA | GLY C | 34 | -26.676 | -9.507 | -11.974 | 1.00 | 19.78 | C |
| ATOM | 231 | CA | GLY C | 35 | -26.814 | -6.238 | -13.884 | 1.00 | 19.11 | C |
| ATOM | 232 | CA | GLY C | 36 | -25.505 | -2.810 | -12.890 | 1.00 | 19.23 | C |
| ATOM | 233 | CA | GLY C | 37 | -27.371 | 0.131 | -14.384 | 1.00 | 25.95 | C |
| ATOM | 234 | CA | GLY C | 38 | -26.592 | 3.830 | -14.106 | 1.00 | 30.90 | C |
| ATOM | 235 | CA | GLY C | 39 | -29.761 | 5.879 | -13.906 | 1.00 | 40.84 | C |
| ATOM | 236 | CA | GLY C | 66 | -16.463 | -4.323 | -12.728 | 1.00 | 23.17 | C |
| ATOM | 237 | CA | GLY C | 67 | -15.869 | -7.277 | -10.506 | 1.00 | 22.56 | C |
| ATOM | 238 | CA | GLY C | 68 | -17.716 | -9.180 | -7.811 | 1.00 | 21.60 | C |
| ATOM | 239 | CA | GLY C | 69 | -18.545 | -12.367 | -5.959 | 1.00 | 22.69 | C |
| ATOM | 240 | CA | GLY C | 75 | -23.757 | -10.273 | -4.597 | 1.00 | 23.03 | C |
| ATOM | 241 | CA | GLY C | 76 | -20.713 | -8.179 | -3.648 | 1.00 | 24.30 | C |
| ATOM | 242 | CA | GLY C | 77 | -20.192 | -5.631 | -6.425 | 1.00 | 23.46 | C |
| ATOM | 243 | CA | GLY C | 78 | -17.130 | -3.554 | -7.209 | 1.00 | 25.74 | C |
| ATOM | 244 | CA | GLY C | 79 | -17.159 | -0.822 | -9.864 | 1.00 | 26.08 | C |
| ATOM | 245 | CA | GLY C | 80 | -13.722 | 0.567 | -10.770 | 1.00 | 29.15 | C |
| ATOM | 246 | CA | GLY C | 81 | -12.154 | 3.299 | -12.879 | 1.00 | 26.23 | C |
| ATOM | 247 | CA | GLY C | 84 | -15.857 | 12.510 | -10.546 | 1.00 | 24.79 | C |
| ATOM | 248 | CA | GLY C | 85 | -18.200 | 12.785 | -13.517 | 1.00 | 25.37 | C |
| ATOM | 249 | CA | GLY C | 86 | -19.382 | 9.218 | -12.817 | 1.00 | 25.21 | C |
| ATOM | 250 | CA | GLY C | 87 | -20.993 | 10.374 | -9.582 | 1.00 | 23.95 | C |
| ATOM | 251 | CA | GLY C | 88 | -24.588 | 9.210 | -9.761 | 1.00 | 23.67 | C |
| ATOM | 252 | CA | GLY C | 89 | -27.227 | 6.570 | -9.098 | 1.00 | 22.75 | C |
| ATOM | 253 | CA | GLY C | 90 | -26.436 | 2.913 | -9.751 | 1.00 | 23.39 | C |
| ATOM | 254 | CA | GLY C | 91 | -29.150 | 0.276 | -9.841 | 1.00 | 22.55 | C |
| ATOM | 255 | CA | GLY C | 92 | -28.491 | -3.332 | -9.011 | 1.00 | 22.29 | C |
| ATOM | 256 | CA | GLY C | 93 | -30.706 | -5.811 | -10.865 | 1.00 | 20.45 | C |
| ATOM | 257 | CA | GLY C | 94 | -30.958 | -9.487 | -9.970 | 1.00 | 20.73 | C |
| ATOM | 258 | CA | GLY C | 105 | -35.975 | -7.679 | -9.086 | 1.00 | 24.94 | C |
| ATOM | 259 | CA | GLY C | 106 | -34.133 | -4.376 | -8.832 | 1.00 | 25.24 | C |
| ATOM | 260 | CA | GLY C | 107 | -32.992 | -2.157 | -5.970 | 1.00 | 24.05 | C |
| ATOM | 261 | CA | GLY C | 108 | -33.717 | 1.590 | -5.665 | 1.00 | 25.85 | C |
| ATOM | 262 | CA | GLY C | 109 | -30.127 | 2.411 | -6.479 | 1.00 | 23.77 | C |
| ATOM | 263 | CA | GLY C | 110 | -26.942 | 3.329 | -4.666 | 1.00 | 22.46 | C |
| ATOM | 264 | CA | GLY C | 111 | -25.759 | 6.950 | -4.824 | 1.00 | 24.13 | C |
| ATOM | 265 | CA | GLY C | 112 | -22.065 | 6.752 | -5.605 | 1.00 | 22.83 | C |
| ATOM | 266 | CA | GLY C | 113 | -20.136 | 9.957 | -4.898 | 1.00 | 23.40 | C |
| ATOM | 267 | CA | GLY C | 114 | -16.799 | 10.239 | -6.594 | 1.00 | 25.42 | C |
| END | | | | | | | | | | |

**Table 9**

| molecule | zp-comb (lower is better) | objective function (lower is better) |
|---|---|---|
| iMab 101 | -5.63 | 853 |
| iMab 201 | -5.34 | 683 |
| iMab IS003 | -4.29 | 860 |
| iMab IS004 | -1.49 | 2744 |
| iMab 300 | -6.28 | 854 |
| iMab IS006 | -3.29 | 912 |
| iMab IS007 | -2.71 | 1558 |
| iMab IS008 | -1.10 | 808 |
| iMab IS009 | -3.70 | 1623 |
| iMab IS0010 | -2.85 | 2704 |
| iMab 400 | -5.58 | 734 |
| iMab IS0012 | -5.35 | 889 |
| iMab IS0013 | -2.85 | 1162 |
| iMab IS0014 | -2.92 | 924 |
| iMab IS0015 | -3.48 | 925 |
| iMab IS0016 | -3.23 | 837 |
| iMab 500 | -3.94 | 1356 |
| iMab IS0018 | -2.97 | 867 |
| iMab IS0019 | -3.11 | 1366 |
| iMab 600 | -4.15 | 880 |
| iMab 700 | -3.94 | 1111 |
| iMab 800 | -3.68 | 653 |
| iMab 900 | -4.65 | 833 |
| iMab 1000 | -3.57 | 631 |
| iMab IS0025 | -2.79 | 1080 |
| iMab 1100 | -4.07 | 823 |
| iMab IS0027 | -3.59 | 809 |
| iMab IS0028 | -3.51 | 1431 |
| iMab 1200 | -2.66 | 783 |
| iMab 1300 | -3.18 | 1463 |
| iMab IS0031 | -2.98 | 1263 |
| iMab IS0032 | -3.84 | 896 |
| iMab 1400 | -5.17 | 939 |
| iMab IS0034 | -4.38 | 966 |
| iMab IS0035 | -3.86 | 966 |
| iMab IS0036 | -3.29 | 862 |
| iMab IS0037 | -3.45 | 874 |
| iMab IS0038 | -2.80 | 792 |
| iMab IS0039 | -4.44 | 1858 |
| iMab IS0040 | -5.01 | 751 |
| iMab IS0041 | -2.70 | 907 |
| iMab IS0042 | -3.14 | 837 |
| iMab IS0043 | -2.80 | 1425 |
| iMab IS0044 | -3.27 | 1492 |
| iMab IS0045 | -3.56 | 1794 |
| imab IS0046 | -3.79 | 832 |

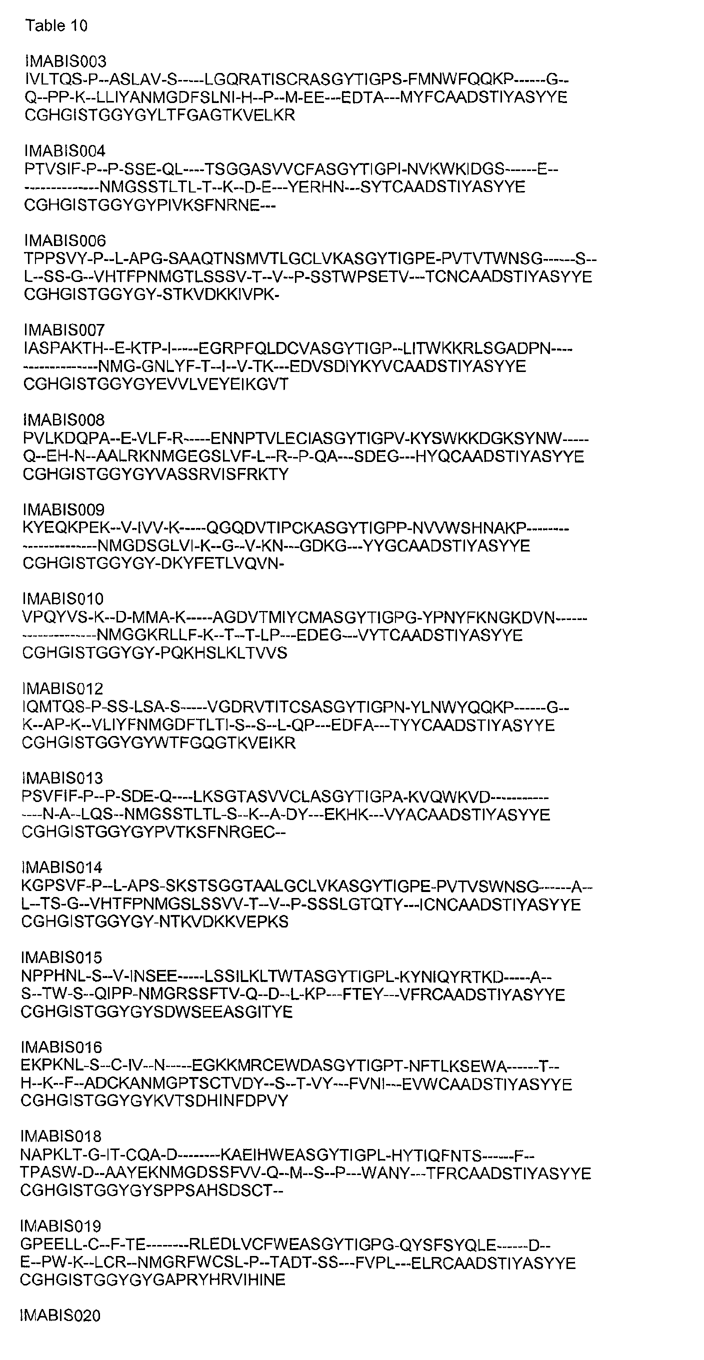

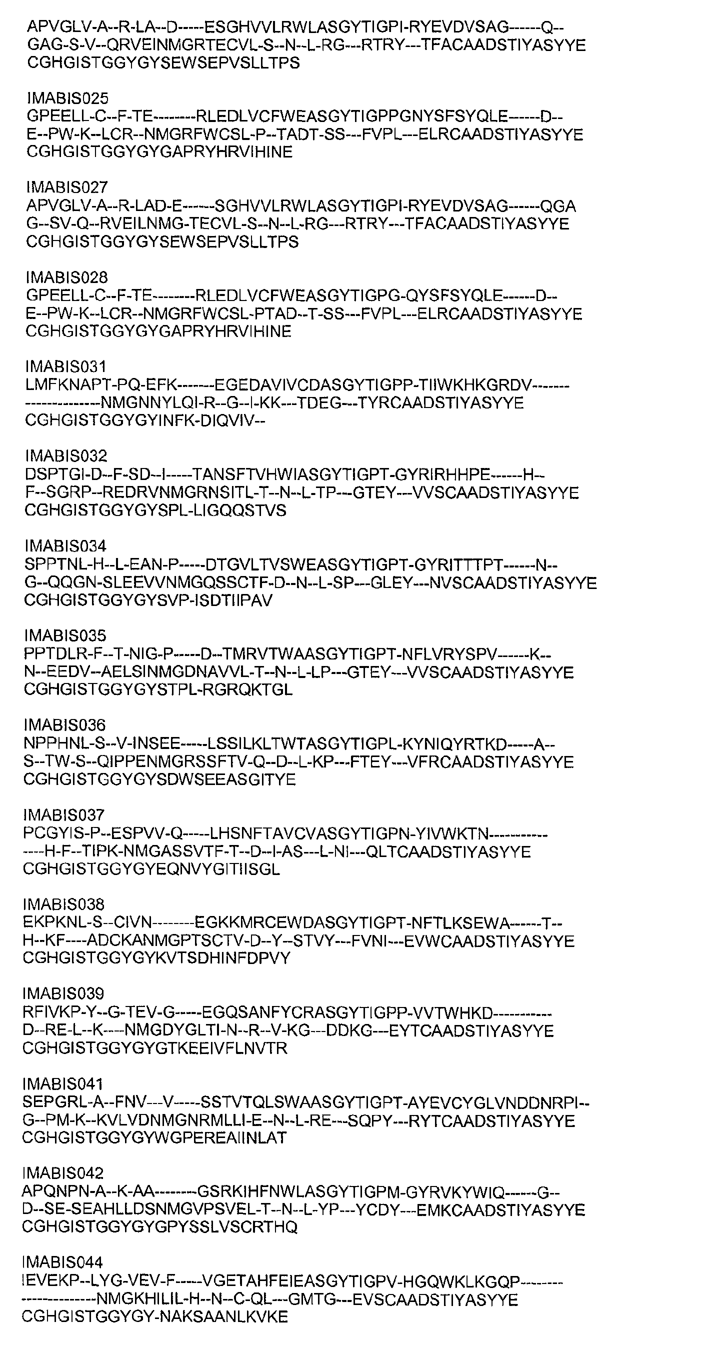

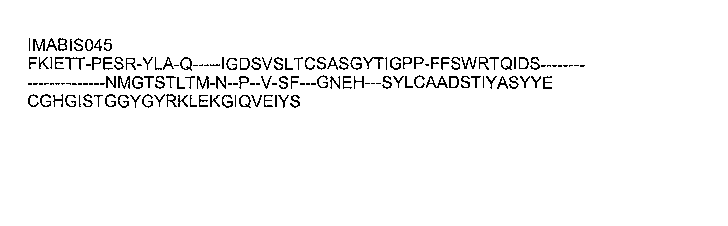

**Table 11**

| 1NEU | | | | | | | | | | |
|---|---|---|---|---|---|---|---|---|---|---|
| ATOM | 1 | CA | GLY | 15 | -13.154 | 9.208 | -3.380 | 1.00 | 23.37 | C |
| ATOM | 2 | CA | GLY | 16 | -14.293 | 5.561 | -3.888 | 1.00 | 22.86 | C |
| ATOM | 3 | CA | GLY | 17 | -16.782 | 3.259 | -2.179 | 1.00 | 20.21 | C |
| ATOM | 4 | CA | GLY | 18 | -17.260 | -0.530 | -2.245 | 1.00 | 19.23 | C |
| ATOM | 5 | CA | GLY | 19 | -20.702 | -2.004 | -2.834 | 1.00 | 17.13 | C |
| ATOM | 6 | CA | GLY | 20 | -20.862 | -5.442 | -1.161 | 1.00 | 19.06 | C |
| ATOM | 7 | CA | GLY | 21 | -22.944 | -8.326 | -2.443 | 1.00 | 15.28 | C |
| ATOM | 8 | CA | GLY | 22 | -22.792 | -11.964 | -1.378 | 1.00 | 15.88 | C |
| ATOM | 9 | CA | GLY | 23 | -25.143 | -14.899 | -1.017 | 1.00 | 18.12 | C |
| ATOM | 10 | CA | GLY | 24 | -25.395 | -17.869 | 1.327 | 1.00 | 18.67 | C |
| ATOM | 11 | CA | GLY | 25 | -27.226 | -21.197 | 1.356 | 1.00 | 20.29 | C |
| ATOM | 12 | CA | GLY | 33 | -24.118 | -18.315 | -10.706 | 1.00 | 24.64 | C |
| ATOM | 13 | CA | GLY | 34 | -24.637 | -14.823 | -12.130 | 1.00 | 21.24 | C |
| ATOM | 14 | CA | GLY | 35 | -24.488 | -11.328 | -10.549 | 1.00 | 17.90 | C |
| ATOM | 15 | CA | GLY | 36 | -26.181 | -8.277 | -12.056 | 1.00 | 16.78 | C |
| ATOM | 16 | CA | GLY | 37 | -25.898 | -4.707 | -10.644 | 1.00 | 15.77 | C |
| ATOM | 17 | CA | GLY | 38 | -28.607 | -2.078 | -11.499 | 1.00 | 16.15 | C |
| ATOM | 18 | CA | GLY | 39 | -28.680 | 1.671 | -10.617 | 1.00 | 17.11 | C |
| ATOM | 19 | CA | GLY | 40 | -31.657 | 4.031 | -9.957 | 1.00 | 19.95 | C |
| ATOM | 20 | CA | GLY | 69 | -15.648 | 4.079 | -12.895 | 1.00 | 19.37 | C |
| ATOM | 21 | CA | GLY | 70 | -16.470 | 0.404 | -12.145 | 1.00 | 17.40 | C |
| ATOM | 22 | CA | GLY | 71 | -14.063 | -2.286 | -10.851 | 1.00 | 17.48 | C |
| ATOM | 23 | CA | GLY | 72 | -14.971 | -5.980 | -10.384 | 1.00 | 17.29 | C |
| ATOM | 24 | CA | GLY | 73 | -13.707 | -7.259 | -7.030 | 1.00 | 17.82 | C |
| ATOM | 25 | CA | GLY | 74 | -15.790 | -10.357 | -6.383 | 1.00 | 20.26 | C |
| ATOM | 26 | CA | GLY | 81 | -20.196 | -10.332 | -6.333 | 1.00 | 15.95 | C |
| ATOM | 27 | CA | GLY | 82 | -18.870 | -7.004 | -5.037 | 1.00 | 14.51 | C |
| ATOM | 28 | CA | GLY | 83 | -17.786 | -3.962 | -7.142 | 1.00 | 14.59 | C |
| ATOM | 29 | CA | GLY | 84 | -16.094 | -0.638 | -6.409 | 1.00 | 15.51 | C |
| ATOM | 30 | CA | GLY | 85 | -17.498 | 2.735 | -7.656 | 1.00 | 16.32 | C |
| ATOM | 31 | CA | GLY | 86 | -14.567 | 5.088 | -8.340 | 1.00 | 17.40 | C |
| ATOM | 32 | CA | GLY | 87 | -14.105 | 8.889 | -8.375 | 1.00 | 20.53 | C |
| ATOM | 33 | CA | GLY | 89 | -19.429 | 12.768 | -7.705 | 1.00 | 33.26 | C |
| ATOM | 34 | CA | GLY | 90 | -22.291 | 14.637 | -6.007 | 1.00 | 33.41 | C |
| ATOM | 35 | CA | GLY | 91 | -24.761 | 13.465 | -8.588 | 1.00 | 28.24 | C |
| ATOM | 36 | CA | GLY | 92 | -24.071 | 9.808 | -7.919 | 1.00 | 23.61 | C |
| ATOM | 37 | CA | GLY | 93 | -26.736 | 9.584 | -5.107 | 1.00 | 22.12 | C |
| ATOM | 38 | CA | GLY | 94 | -29.127 | 6.723 | -5.698 | 1.00 | 18.21 | C |
| ATOM | 39 | CA | GLY | 95 | -30.045 | 3.141 | -4.991 | 1.00 | 18.57 | C |
| ATOM | 40 | CA | GLY | 96 | -28.033 | 0.110 | -6.301 | 1.00 | 17.72 | C |
| ATOM | 41 | CA | GLY | 97 | -29.544 | -3.367 | -6.491 | 1.00 | 18.72 | C |
| ATOM | 42 | CA | GLY | 98 | -27.685 | -6.700 | -6.623 | 1.00 | 18.53 | C |
| ATOM | 43 | CA | GLY | 99 | -29.584 | -9.550 | -8.379 | 1.00 | 18.45 | C |
| ATOM | 44 | CA | GLY | 100 | -28.276 | -13.106 | -7.867 | 1.00 | 17.71 | C |
| ATOM | 45 | CA | GLY | 108 | -33.268 | -14.108 | -8.956 | 1.00 | 32.51 | C |
| ATOM | 46 | CA | GLY | 109 | -33.081 | -12.828 | -5.395 | 1.00 | 28.62 | C |
| ATOM | 47 | CA | GLY | 110 | -32.234 | -9.138 | -4.891 | 1.00 | 23.31 | C |
| ATOM | 48 | CA | GLY | 111 | -30.950 | -6.748 | -2.225 | 1.00 | 19.82 | C |
| ATOM | 49 | CA | GLY | 112 | -30.333 | -2.979 | -2.412 | 1.00 | 19.75 | C |
| ATOM | 50 | CA | GLY | 113 | -28.024 | -0.343 | -0.875 | 1.00 | 18.42 | C |
| ATOM | 51 | CA | GLY | 114 | -28.469 | 3.472 | -1.024 | 1.00 | 18.70 | C |
| ATOM | 52 | CA | GLY | 115 | -25.546 | 5.827 | -1.713 | 1.00 | 19.95 | C |
| ATOM | 53 | CA | GLY | 116 | -25.095 | 9.402 | -0.363 | 1.00 | 25.78 | C |
| ATOM | 54 | CA | GLY | 117 | -22.037 | 11.484 | -1.264 | 1.00 | 31.06 | C |
| ATOM | 55 | CA | GLY | 118 | -20.985 | 14.508 | 0.805 | 1.00 | 38.60 | C |
| ATOM | 56 | CA | GLY | 119 | -17.884 | 16.768 | 1.101 | 1.00 | 42.50 | C |
| TER | | | | | | | | | | |
| | | | | | | | | | | |

| 1MEL | | | | | | | | | | |
|---|---|---|---|---|---|---|---|---|---|---|
| ATOM | 57 | CA | GLY A | 16 | -13.853 | 9.205 | -3.269 | 1.00 | 7.90 | C |
| ATOM | 58 | CA | GLY A | 17 | -14.557 | 5.500 | -3.346 | 1.00 | 7.54 | C |
| ATOM | 59 | CA | GLY A | 18 | -16.958 | 3.068 | -1.674 | 1.00 | 6.47 | C |
| ATOM | 60 | CA | GLY A | 19 | -17.168 | -0.701 | -1.485 | 1.00 | 5.43 | C |
| ATOM | 61 | CA | GLY A | 20 | -20.455 | -2.621 | -1.546 | 1.00 | 8.09 | C |
| ATOM | 62 | CA | GLY A | 21 | -20.823 | -6.351 | -0.794 | 1.00 | 8.76 | C |
| ATOM | 63 | CA | GLY A | 22 | -23.429 | -9.023 | -1.196 | 1.00 | 8.33 | C |
| ATOM | 64 | CA | GLY A | 23 | -23.620 | -12.484 | 0.211 | 1.00 | 13.77 | C |
| ATOM | 65 | CA | GLY A | 24 | -26.198 | -14.877 | -1.245 | 1.00 | 17.53 | C |
| ATOM | 66 | CA | GLY A | 25 | -27.419 | -17.241 | 1.448 | 1.00 | 23.23 | C |
| ATOM | 67 | CA | GLY A | 26 | -29.608 | -20.285 | 1.362 | 1.00 | 25.25 | C |
| ATOM | 68 | CA | GLY A | 32 | -25.105 | -18.522 | -11.770 | 1.00 | 5.00 | C |
| ATOM | 69 | CA | GLY A | 33 | -24.991 | -14.689 | -11.775 | 1.00 | 4.64 | C |
| ATOM | 70 | CA | GLY A | 34 | -24.729 | -11.897 | -9.152 | 1.00 | 2.48 | C |
| ATOM | 71 | CA | GLY A | 35 | -24.799 | -8.264 | -10.249 | 1.00 | 2.00 | C |
| ATOM | 72 | CA | GLY A | 36 | -25.716 | -4.753 | -9.249 | 1.00 | 3.40 | C |
| ATOM | 73 | CA | GLY A | 37 | -28.543 | -2.502 | -10.376 | 1.00 | 6.64 | C |
| ATOM | 74 | CA | GLY A | 38 | -29.128 | 1.074 | -9.379 | 1.00 | 8.74 | C |
| ATOM | 75 | CA | GLY A | 39 | -32.163 | 3.371 | -9.309 | 1.00 | 19.24 | C |
| ATOM | 76 | CA | GLY A | 67 | -14.374 | 3.095 | -12.462 | 1.00 | 12.68 | C |
| ATOM | 77 | CA | GLY A | 68 | -16.189 | 0.136 | -10.946 | 1.00 | 8.15 | C |
| ATOM | 78 | CA | GLY A | 69 | -14.842 | -3.360 | -10.453 | 1.00 | 8.39 | C |
| ATOM | 79 | CA | GLY A | 70 | -16.896 | -6.384 | -9.408 | 1.00 | 5.47 | C |
| ATOM | 80 | CA | GLY A | 71 | -15.309 | -9.468 | -7.894 | 1.00 | 8.48 | C |
| ATOM | 81 | CA | GLY A | 72 | -16.197 | -12.511 | -5.798 | 1.00 | 18.00 | C |
| ATOM | 82 | CA | GLY A | 79 | -19.282 | -10.422 | -4.331 | 1.00 | 9.55 | C |
| ATOM | 83 | CA | GLY A | 80 | -18.031 | -6.806 | -4.095 | 1.00 | 4.94 | C |
| ATOM | 84 | CA | GLY A | 81 | -18.236 | -3.718 | -6.133 | 1.00 | 4.92 | C |
| ATOM | 85 | CA | GLY A | 82 | -15.331 | -1.340 | -5.635 | 1.00 | 7.44 | C |
| ATOM | 86 | CA | GLY A | 83 | -16.455 | 2.094 | -6.795 | 1.00 | 6.55 | C |
| ATOM | 87 | CA | GLY A | 84 | -13.706 | 4.649 | -7.462 | 1.00 | 9.49 | C |
| ATOM | 88 | CA | GLY A | 85 | -13.919 | 8.136 | -8.959 | 1.00 | 12.54 | C |
| ATOM | 89 | CA | GLY A | 87 | -19.256 | 11.437 | -9.096 | 1.00 | 15.46 | C |
| ATOM | 90 | CA | GLY A | 88 | -22.580 | 12.828 | -7.836 | 1.00 | 13.94 | C |
| ATOM | 91 | CA | GLY A | 89 | -24.298 | 11.258 | -10.888 | 1.00 | 18.68 | C |
| ATOM | 92 | CA | GLY A | 90 | -23.577 | 7.916 | -9.175 | 1.00 | 9.55 | C |
| ATOM | 93 | CA | GLY A | 91 | -26.004 | 8.885 | -6.360 | 1.00 | 6.54 | C |
| ATOM | 94 | CA | GLY A | 92 | -28.681 | 6.180 | -6.349 | 1.00 | 6.38 | C |
| ATOM | 95 | CA | GLY A | 93 | -30.154 | 3.149 | -4.618 | 1.00 | 6.33 | C |
| ATOM | 96 | CA | GLY A | 94 | -27.980 | 0.121 | -5.274 | 1.00 | 6.08 | C |
| ATOM | 97 | CA | GLY A | 95 | -29.551 | -3.256 | -5.491 | 1.00 | 10.12 | C |
| ATOM | 98 | CA | GLY A | 96 | -27.885 | -6.624 | -5.451 | 1.00 | 10.76 | C |
| ATOM | 99 | CA | GLY A | 97 | -29.379 | -9.337 | -7.656 | 1.00 | 9.08 | C |
| ATOM | 100 | CA | GLY A | 98 | -28.752 | -13.014 | -8.455 | 1.00 | 8.28 | C |
| ATOM | 101 | CA | GLY A | 122 | -33.497 | -12.862 | -6.462 | 1.00 | 15.49 | C |
| ATOM | 102 | CA | GLY A | 123 | -33.164 | -9.374 | -5.211 | 1.00 | 12.37 | C |
| ATOM | 103 | CA | GLY A | 124 | -31.827 | -7.789 | -2.102 | 1.00 | 14.34 | C |
| ATOM | 104 | CA | GLY A | 125 | -32.483 | -4.741 | 0.002 | 1.00 | 22.49 | C |
| ATOM | 105 | CA | GLY A | 126 | -31.400 | -1.487 | -1.608 | 1.00 | 17.72 | C |
| ATOM | 106 | CA | GLY A | 127 | -28.513 | 0.495 | -0.221 | 1.00 | 16.51 | C |
| ATOM | 107 | CA | GLY A | 128 | -28.376 | 4.247 | -0.807 | 1.00 | 16.84 | C |
| ATOM | 108 | CA | GLY A | 129 | -25.116 | 5.718 | -1.910 | 1.00 | 11.90 | C |
| ATOM | 109 | CA | GLY A | 130 | -24.954 | 9.478 | -1.961 | 1.00 | 8.75 | C |
| ATOM | 110 | CA | GLY A | 131 | -22.066 | 11.329 | -3.496 | 1.00 | 14.37 | C |
| ATOM | 111 | CA | GLY A | 132 | -21.513 | 14.817 | -1.947 | 1.00 | 23.32 | C |
| ATOM | 112 | CA | GLY A | 133 | -20.378 | 18.169 | -3.369 | 1.00 | 36.14 | C |
| TER | | | | | | | | | | |
| | | | | | | | | | | |

| 1F97 | | | | | | | | | | |
|---|---|---|---|---|---|---|---|---|---|---|
| ATOM | 113 | CA | GLY A | 43 | -13.239 | 8.515 | -3.437 | 1.00 | 14.44 | C |
| ATOM | 114 | CA | GLY A | 44 | -13.393 | 4.758 | -3.940 | 1.00 | 21.52 | C |
| ATOM | 115 | CA | GLY A | 45 | -16.246 | 2.710 | -2.546 | 1.00 | 22.48 | C |
| ATOM | 116 | CA | GLY A | 46 | -17.296 | -0.926 | -2.623 | 1.00 | 21.22 | C |
| ATOM | 117 | CA | GLY A | 47 | -21.001 | -1.717 | -2.638 | 1.00 | 20.06 | C |
| ATOM | 118 | CA | GLY A | 48 | -21.128 | -5.074 | -0.848 | 1.00 | 13.56 | C |
| ATOM | 119 | CA | GLY A | 49 | -23.434 | -7.972 | -1.703 | 1.00 | 19.14 | C |
| ATOM | 120 | CA | GLY A | 50 | -22.907 | -11.288 | 0.067 | 1.00 | 22.49 | C |
| ATOM | 121 | CA | GLY A | 51 | -24.848 | -14.490 | -0.589 | 1.00 | 20.58 | C |
| ATOM | 122 | CA | GLY A | 52 | -24.961 | -18.121 | 0.538 | 1.00 | 24.55 | C |
| ATOM | 123 | CA | GLY A | 53 | -26.625 | -21.271 | -0.752 | 1.00 | 17.82 | C |
| ATOM | 124 | CA | GLY A | 57 | -24.531 | -17.722 | -9.307 | 1.00 | 10.39 | C |
| ATOM | 125 | CA | GLY A | 58 | -25.219 | -15.054 | -11.903 | 1.00 | 8.00 | C |
| ATOM | 126 | CA | GLY A | 59 | -24.694 | -11.642 | -10.310 | 1.00 | 7.62 | C |
| ATOM | 127 | CA | GLY A | 60 | -26.312 | -8.537 | -11.794 | 1.00 | 6.84 | C |
| ATOM | 128 | CA | GLY A | 61 | -26.560 | -4.910 | -10.704 | 1.00 | 8.48 | C |
| ATOM | 129 | CA | GLY A | 62 | -28.971 | -2.156 | -11.641 | 1.00 | 13.92 | C |
| ATOM | 130 | CA | GLY A | 63 | -28.917 | 1.574 | -10.971 | 1.00 | 11.89 | C |
| ATOM | 131 | CA | GLY A | 64 | -32.146 | 3.463 | -10.346 | 1.00 | 23.18 | C |
| ATOM | 132 | CA | GLY A | 85 | -14.367 | 2.765 | -13.291 | 1.00 | 17.93 | C |
| ATOM | 133 | CA | GLY A | 86 | -16.308 | -0.184 | -11.839 | 1.00 | 15.44 | C |
| ATOM | 134 | CA | GLY A | 87 | -14.665 | -3.500 | -11.017 | 1.00 | 16.79 | C |
| ATOM | 135 | CA | GLY A | 88 | -16.581 | -6.711 | -10.341 | 1.00 | 14.07 | C |
| ATOM | 136 | CA | GLY A | 89 | -15.959 | -9.289 | -7.620 | 1.00 | 16.75 | C |
| ATOM | 137 | CA | GLY A | 91 | -18.578 | -9.954 | -2.969 | 1.00 | 17.13 | C |
| ATOM | 138 | CA | GLY A | 92 | -19.615 | -6.643 | -4.531 | 1.00 | 12.84 | C |
| ATOM | 139 | CA | GLY A | 93 | -18.746 | -3.911 | -7.017 | 1.00 | 12.33 | C |
| ATOM | 140 | CA | GLY A | 94 | -15.956 | -1.401 | -6.447 | 1.00 | 12.43 | C |
| ATOM | 141 | CA | GLY A | 95 | -16.021 | 2.137 | -7.822 | 1.00 | 11.12 | C |
| ATOM | 142 | CA | GLY A | 96 | -12.511 | 3.493 | -8.309 | 1.00 | 16.95 | C |
| ATOM | 143 | CA | GLY A | 97 | -14.158 | 6.925 | -7.885 | 1.00 | 21.11 | C |
| ATOM | 144 | CA | GLY A | 99 | -19.244 | 11.655 | -8.297 | 1.00 | 22.37 | C |
| ATOM | 145 | CA | GLY A | 100 | -22.479 | 13.329 | -7.197 | 1.00 | 23.75 | C |
| ATOM | 146 | CA | GLY A | 101 | -24.007 | 11.875 | -10.393 | 1.00 | 22.84 | C |
| ATOM | 147 | CA | GLY A | 102 | -23.793 | 8.420 | -8.818 | 1.00 | 15.51 | C |
| ATOM | 148 | CA | GLY A | 103 | -26.377 | 9.137 | -6.093 | 1.00 | 11.57 | C |
| ATOM | 149 | CA | GLY A | 104 | -29.205 | 6.618 | -6.153 | 1.00 | 9.52 | C |
| ATOM | 150 | CA | GLY A | 105 | -30.075 | 3.041 | -5.324 | 1.00 | 12.64 | C |
| ATOM | 151 | CA | GLY A | 106 | -28.157 | 0.010 | -6.540 | 1.00 | 7.32 | C |
| ATOM | 152 | CA | GLY A | 107 | -29.828 | -3.384 | -6.497 | 1.00 | 7.52 | C |
| ATOM | 153 | CA | GLY A | 108 | -27.747 | -6.545 | -6.374 | 1.00 | 9.97 | C |
| ATOM | 154 | CA | GLY A | 109 | -29.572 | -9.419 | -8.055 | 1.00 | 12.91 | C |
| ATOM | 155 | CA | GLY A | 110 | -28.245 | -12.921 | -7.462 | 1.00 | 11.27 | C |
| ATOM | 156 | CA | GLY A | 118 | -33.242 | -16.054 | -6.426 | 1.00 | 13.29 | C |
| ATOM | 157 | CA | GLY A | 119 | -32.582 | -13.085 | -4.179 | 1.00 | 15.55 | C |
| ATOM | 158 | CA | GLY A | 120 | -31.884 | -9.348 | -4.193 | 1.00 | 11.36 | C |
| ATOM | 159 | CA | GLY A | 121 | -30.813 | -6.471 | -1.975 | 1.00 | 11.36 | C |
| ATOM | 160 | CA | GLY A | 122 | -30.903 | -2.696 | -2.475 | 1.00 | 11.14 | C |
| ATOM | 161 | CA | GLY A | 123 | -28.232 | -0.209 | -1.404 | 1.00 | 11.58 | C |
| ATOM | 162 | CA | GLY A | 124 | -28.703 | 3.550 | -1.338 | 1.00 | 15.52 | C |
| ATOM | 163 | CA | GLY A | 125 | -25.598 | 5.531 | -2.225 | 1.00 | 9.61 | C |
| ATOM | 164 | CA | GLY A | 126 | -25.164 | 9.137 | -1.123 | 1.00 | 10.84 | C |
| ATOM | 165 | CA | GLY A | 127 | -22.043 | 10.899 | -2.383 | 1.00 | 10.58 | C |
| ATOM | 166 | CA | GLY A | 128 | -20.981 | 13.549 | 0.145 | 1.00 | 14.41 | C |
| ATOM | 167 | CA | GLY A | 129 | -20.447 | 17.126 | -1.025 | 1.00 | 12.32 | C |
| TER | | | | | | | | | | |
| | | | | | | | | | | |

| 1DQT | | | | | | | | | | |
|---|---|---|---|---|---|---|---|---|---|---|
| ATOM | 168 | CA | GLY C | 14 | -9.505 | 5.982 | -6.825 | 1.00 | 30.82 | C |
| ATOM | 169 | CA | GLY C | 15 | -13.195 | 5.487 | -7.536 | 1.00 | 25.29 | C |
| ATOM | 170 | CA | GLY C | 16 | -13.507 | 1.942 | -6.167 | 1.00 | 21.63 | C |
| ATOM | 171 | CA | GLY C | 17 | -16.606 | 0.707 | -4.377 | 1.00 | 20.22 | C |
| ATOM | 172 | CA | GLY C | 18 | -16.814 | -2.817 | -3.021 | 1.00 | 20.86 | C |
| ATOM | 173 | CA | GLY C | 19 | -19.629 | -4.451 | -1.137 | 1.00 | 21.25 | C |
| ATOM | 174 | CA | GLY C | 20 | -21.383 | -7.769 | -0.574 | 1.00 | 21.73 | C |
| ATOM | 175 | CA | GLY C | 21 | -24.675 | -8.800 | -2.148 | 1.00 | 22.97 | C |
| ATOM | 176 | CA | GLY C | 22 | -26.301 | -11.552 | -0.160 | 1.00 | 26.59 | C |
| ATOM | 177 | CA | GLY C | 23 | -29.169 | -13.867 | -0.930 | 1.00 | 26.47 | C |
| ATOM | 178 | CA | GLY C | 24 | -31.335 | -16.565 | 0.573 | 1.00 | 31.54 | C |
| ATOM | 179 | CA | GLY C | 25 | -32.236 | -19.342 | 0.443 | 1.00 | 31.33 | C |
| ATOM | 180 | CA | GLY C | 32 | -26.091 | -17.451 | -10.077 | 1.00 | 21.25 | C |
| ATOM | 181 | CA | GLY C | 33 | -26.340 | -14.584 | -12.535 | 1.00 | 21.86 | C |
| ATOM | 182 | CA | GLY C | 34 | -25.686 | -11.294 | -10.762 | 1.00 | 19.78 | C |
| ATOM | 183 | CA | GLY C | 35 | -26.651 | -7.922 | -12.193 | 1.00 | 19.11 | C |
| ATOM | 184 | CA | GLY C | 36 | -25.711 | -4.438 | -10.995 | 1.00 | 19.23 | C |
| ATOM | 185 | CA | GLY C | 37 | -28.233 | -1.721 | -11.782 | 1.00 | 25.95 | C |
| ATOM | 186 | CA | GLY C | 38 | -27.978 | 2.010 | -11.165 | 1.00 | 30.90 | C |
| ATOM | 187 | CA | GLY C | 39 | -31.328 | 3.464 | -10.196 | 1.00 | 40.84 | C |
| ATOM | 188 | CA | GLY C | 66 | -16.664 | -4.410 | -12.490 | 1.00 | 23.17 | C |
| ATOM | 189 | CA | GLY C | 67 | -15.247 | -7.428 | -10.788 | 1.00 | 22.56 | C |
| ATOM | 190 | CA | GLY C | 68 | -16.273 | -9.875 | -8.095 | 1.00 | 21.60 | C |
| ATOM | 191 | CA | GLY C | 69 | -16.270 | -13.324 | -6.554 | 1.00 | 22.69 | C |
| ATOM | 192 | CA | GLY C | 75 | -21.405 | -12.287 | -4.112 | 1.00 | 23.03 | C |
| ATOM | 193 | CA | GLY C | 76 | -18.587 | -9.814 | -3.409 | 1.00 | 24.30 | C |
| ATOM | 194 | CA | GLY C | 77 | -18.961 | -6.951 | -5.886 | 1.00 | 23.46 | C |
| ATOM | 195 | CA | GLY C | 78 | -16.435 | -4.318 | -6.884 | 1.00 | 25.74 | C |
| ATOM | 196 | CA | GLY C | 79 | -17.349 | -1.380 | -9.129 | 1.00 | 26.08 | C |
| ATOM | 197 | CA | GLY C | 80 | -14.377 | 0.652 | -10.395 | 1.00 | 29.15 | C |
| ATOM | 198 | CA | GLY C | 81 | -13.639 | 3.807 | -12.365 | 1.00 | 26.23 | C |
| ATOM | 199 | CA | GLY C | 84 | -18.194 | 11.980 | -8.310 | 1.00 | 24.79 | C |
| ATOM | 200 | CA | GLY C | 85 | -21.048 | 12.151 | -10.804 | 1.00 | 25.37 | C |
| ATOM | 201 | CA | GLY C | 86 | -21.540 | 8.383 | -10.383 | 1.00 | 25.21 | C |
| ATOM | 202 | CA | GLY C | 87 | -22.696 | 8.922 | -6.809 | 1.00 | 23.95 | C |
| ATOM | 203 | CA | GLY C | 88 | -26.050 | 7.191 | -6.551 | 1.00 | 23.67 | C |
| ATOM | 204 | CA | GLY C | 89 | -28.103 | 4.091 | -5.812 | 1.00 | 22.75 | C |
| ATOM | 205 | CA | GLY C | 90 | -26.905 | 0.703 | -7.044 | 1.00 | 23.39 | C |
| ATOM | 206 | CA | GLY C | 91 | -29.167 | -2.332 | -7.030 | 1.00 | 22.55 | C |
| ATOM | 207 | CA | GLY C | 92 | -27.838 | -5.841 | -6.783 | 1.00 | 22.29 | C |
| ATOM | 208 | CA | GLY C | 93 | -29.956 | -8.462 | -8.555 | 1.00 | 20.45 | C |
| ATOM | 209 | CA | GLY C | 94 | -29.490 | -12.198 | -8.107 | 1.00 | 20.73 | C |
| ATOM | 210 | CA | GLY C | 105 | -34.479 | -11.361 | -6.201 | 1.00 | 24.94 | C |
| ATOM | 211 | CA | GLY C | 106 | -33.136 | -7.836 | -5.829 | 1.00 | 25.24 | C |
| ATOM | 212 | CA | GLY C | 107 | -31.842 | -5.752 | -2.931 | 1.00 | 24.05 | C |
| ATOM | 213 | CA | GLY C | 108 | -33.051 | -2.231 | -2.038 | 1.00 | 25.85 | C |
| ATOM | 214 | CA | GLY C | 109 | -29.830 | -0.739 | -3.310 | 1.00 | 23.77 | C |
| ATOM | 215 | CA | GLY C | 110 | -26.544 | 0.520 | -1.934 | 1.00 | 22.46 | C |
| ATOM | 216 | CA | GLY C | 111 | -25.963 | 4.285 | -1.818 | 1.00 | 24.13 | C |
| ATOM | 217 | CA | GLY C | 112 | -22.482 | 4.793 | -3.205 | 1.00 | 22.83 | C |
| ATOM | 218 | CA | GLY C | 113 | -20.958 | 8.192 | -2.417 | 1.00 | 23.40 | C |
| ATOM | 219 | CA | GLY C | 114 | -18.061 | 9.201 | -4.580 | 1.00 | 25.42 | C |
| END | | | | | | | | | | |

**Table 12**

| imab nummer | objective funct. | zp-comb |
|---|---|---|
| iMabis050 | 617 | -1,83 |
| Mabis051 | 636 | -0,5 |
| iMab102 | 598 | -0,38 |
| Mabis052 | 586 | -0,88 |
| Mabis053 | 592 | -0,73 |
| Mabis054 | 540 | -0,42 |

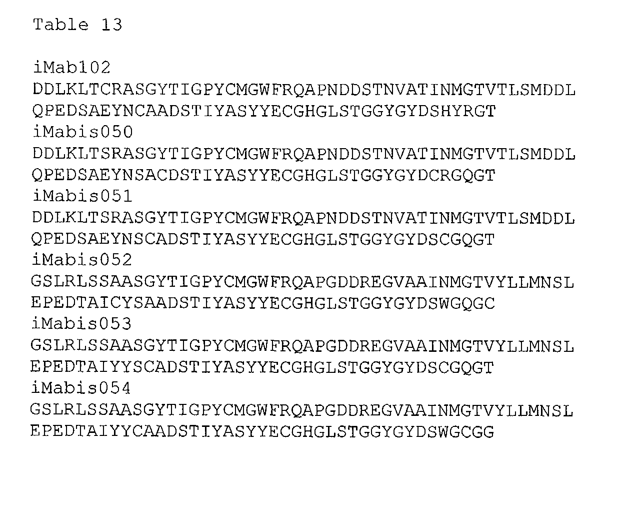

**Table 14**

| results | | without cysteine bridges | with cysteine bridges | |
|---|---|---|---|---|
| Residue replacement solubility | | zp-comp | zp-comb | |
| IMABA_K_3_A | | -6.61 | -6.85 | |
| IMABA_K_3_C | | -6.72 | -6.62 | |
| IMABA_K_3_D X | | -6.65 | -6.54 | |
| IMABA_K_3_E X | X | -6.63 | -6.48 | |
| IMABA_K_3_F | | -6.61 | -6.44 | |
| IMABA_K_3_G | | -6.70 | -6.63 | |
| IMABA_K_3_H | | -6.70 | -6.79 | |
| IMABA_K_3_I | | -6.65 | -6.47 | |
| IMABA_K_3_L | | -6.42 | -6.55 | |
| IMABA_K_3_M | | -6.34 | -6.57 | |
| IMABA_K_3_N X | X | -6.57 | -6.41 | |
| IMABA_K_3_P | | -6.74 | -6.46 | |
| IMABA_K_3_Q X | X | -6.64 | -6.56 | |
| IMABA_K_3_R X | X | -6.91 | -6.56 | best fit |
| IMABA_K_3_S X | X | -6.52 | -6.61 | |
| IMABA_K_3_T X | X | -6.69 | -6.61 | |
| IMABA_K_3_V | | -6.60 | -6.63 | |
| IMABA_K_3_W | | -6.61 | -6.57 | |
| IMABA_K_3_Y | | -6.54 | -6.54 | |
| | | | | |
| IMABA_K_7_A | | -6.58 | -6.51 | |
| IMABA_K_7_C | | -6.73 | -6.6 | |
| IMABA_K_7_D X | X | -6.47 | -6.67 | |
| IMABA_K_7_E X | X | -6.83 | -6.61 | |
| IMABA_K_7_F | | -6.66 | -6.58 | |
| IMABA_K_7_G | | -6.65 | -6.76 | |
| IMABA_K_7_H | | -6.80 | -6.57 | |
| IMABA_K_7_I | | -6.62 | -6.28 | |
| IMABA_K_7_L | | -6.76 | -6.66 | |
| IMABA_K_7_M | | -6.69 | -6.62 | |
| IMABA_K_7_N X | X | -6.34 | -6.61 | |
| IMABA_K_7_P | | -6.48 | -6.94 | |
| IMABA_K_7_Q X | X | -6.72 | -6.61 | |
| IMABA_K_7_R X | X | -6.63 | -6.94 | best fit |
| IMABA_K_7_S X | X | -6.83 | -6.61 | |
| IMABA_K_7_T X | X | -6.80 | -6.54 | |
| IMABA_K_7_V | | -6.78 | -6.58 | |
| IMABA_K_7_W | | -6.87 | -6.61 | |
| IMABA_K_7_W | | -6.60 | -6.63 | |
| | | | | |
| IMABA_K_19_A | | -6.67 | -6.47 | |
| IMABA_K_19_C | | -6.46 | -6.41 | |
| IMABA_K_19_D X | X | -6.5 | -6.41 | |
| IMABA_K_19_E X | X | -6.69 | -6.77 | |
| IMABA_K_19_F | | -6.61 | -6.55 | |
| IMABA_K_19_G | | -6.94 | -6.54 | |
| IMABA_K_19_H | | -6.48 | -6.62 | |
| IMABA_K_19_I | | -6.74 | -6.51 | |
| IMABA_K_19_L | | -6.52 | -6.72 | |
| IMABA_K_19_M | | -6.60 | -6.16 | |
| IMABA_K_19_N X | X | -6.49 | -6.84 | |
| IMABA_K_19_P P | | -6.56 | -6.41 | |
| IMABA_K_19_Q X | X | -6.91 | -6.65 | |
| IMABA_K_19_R X | X | -6.72 | -6.73 | |
| IMABA_K_19_S X | X | -6.57 | -6.61 | |
| IMABA_K_19_T X | X | -6.85 | -6.61 | best fit |
| IMABA_K_19_V | | -6.75 | -6.81 | |
| IMABA_K_19_W | | -6.4 | -6.43 | |
| IMABA_K_19_Y | | -6.53 | -6.48 | |
| | | | | |
| IMABA_K_65_A | | -6.52 | 6.22 | |
| IMABA_K_65_C | | -6.43 | 6.23 | |
| IMABA_K_65_D X | X | -6.79 | 6.72 | |
| IMABA_K_65_E X | X | -6.82 | 6.70 | best fit |
| IMABA_K_65_F | | -6.52 | 6.26 | |
| IMABA_K_65_G | | -6.66 | 6.58 | |
| IMABA_K_65_H | | -6.54 | 6.33 | |
| IMABA_K_65_I | | -6.35 | 6.18 | |
| IMABA_K_65_L | | -6.23 | 6.34 | |
| IMABA_K_6 5_M | | -6.44 | 6.72 | |
| IMABA_K_65_N X | X | -6.74 | 6.62 | |
| IMABA_K_65_P | | -6.50 | 6.42 | |
| IMABA_K_65_Q X | X | -6.62 | 6.59 | |
| IMABA_K_65_R X | X | -6.63 | 6.53 | |
| IMABA_K_65_S X | X | -6.68 | 6.41 | |
| IMABA_K_65_T X | X | -6.47 | 6.41 | |
| IMABA_K_65_V | | -6.30 | 6.25 | |
| IMABA K 65_W | | -6.50 | 6.39 | |
| IMABA_K_65_Y | | -6.48 | 6.72 | |

**Table 15**

| molecule | zp-comb |
|---|---|
| IMAB_C_96_A | -6,52 |
| IMAB_C_96_C | -7,11 |
| IMAB_C_96_D | -6,26 |
| IMAB_C_96_E | -5,75 |
| IMAB_C_96_F | -6,70 |
| IMAB_C_96_G | -6,38 |
| IMAB_C_96_H | -6,26 |
| IMAB_C_96_I | -6,66 |
| IMAB_C_96_K | -5,56 |
| IMAB_C_96_L | -6,37 |
| IMAB_C_96_M | -6,51 |
| IMAB_C_96_N | -6,53 |
| IMAB_C_96_P | -6,48 |
| IMAB_C_96_Q | -6,19 |
| IMAB_C_96_R | -6,08 |
| IMAB_C_96_S | -6,39 |
| IMAB_C_96_T | -6,38 |
| IMAB_C_96_V | -6,75 |
| IMAB_C_96_W | -6,22 |
| IMAB_C_96_Y | -6,60 |

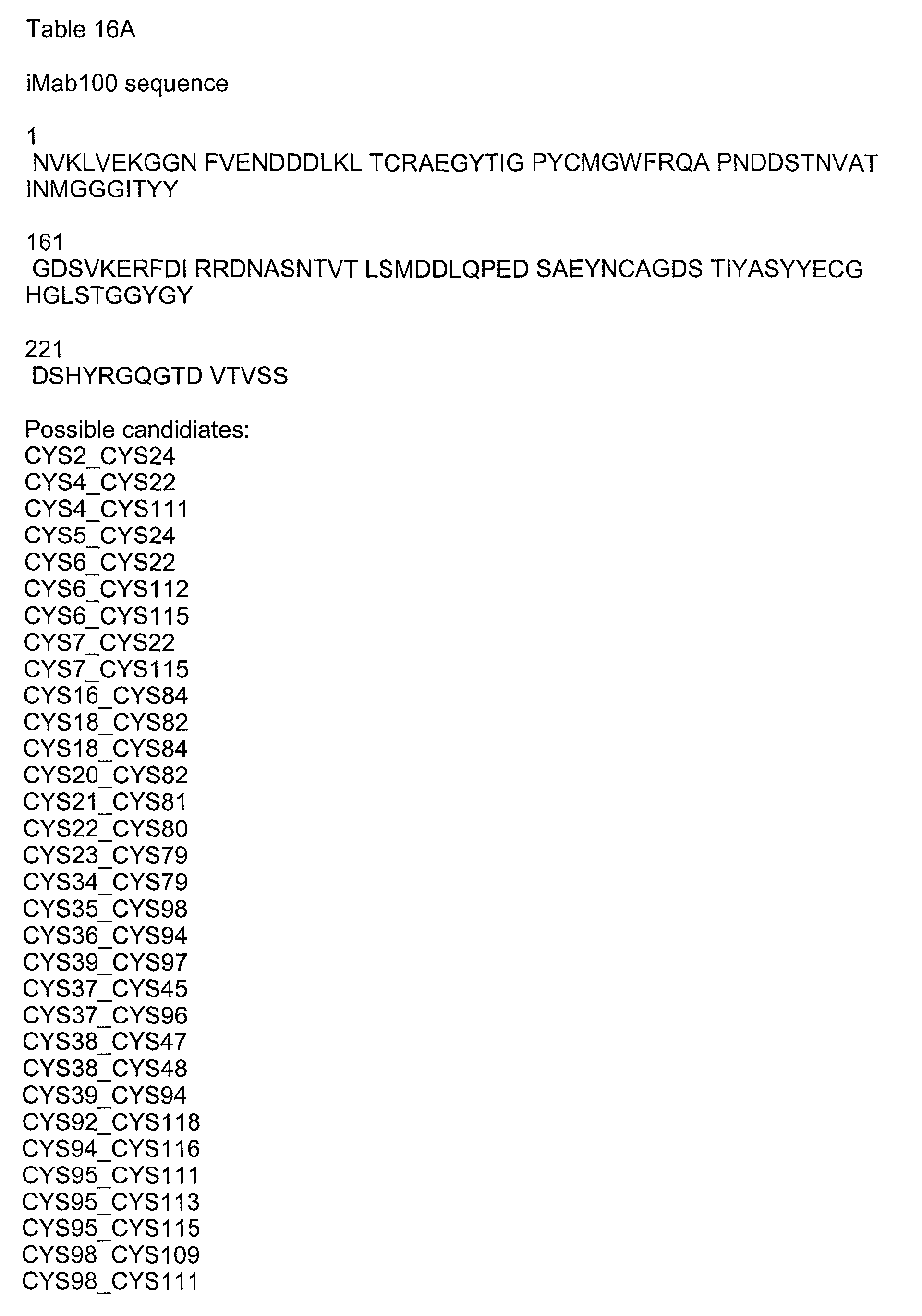

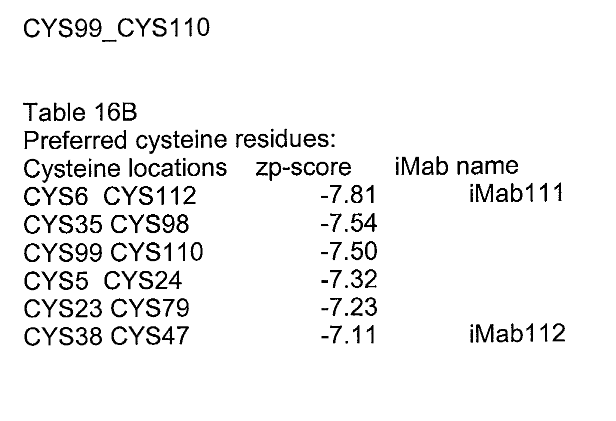

**Table 17**

| Mutation frequency | number of transformants | number of binders |
|---|---|---|
| 0 | 9.3 *10E6 | 50 |
| 2 | 8.1*10E6 | 1000 |
| 3,5 | 5.4*10E6 | 75 |
| 8 | 7.4*10E6 | 100 |
| 13 | 22*10E6 | 100 |

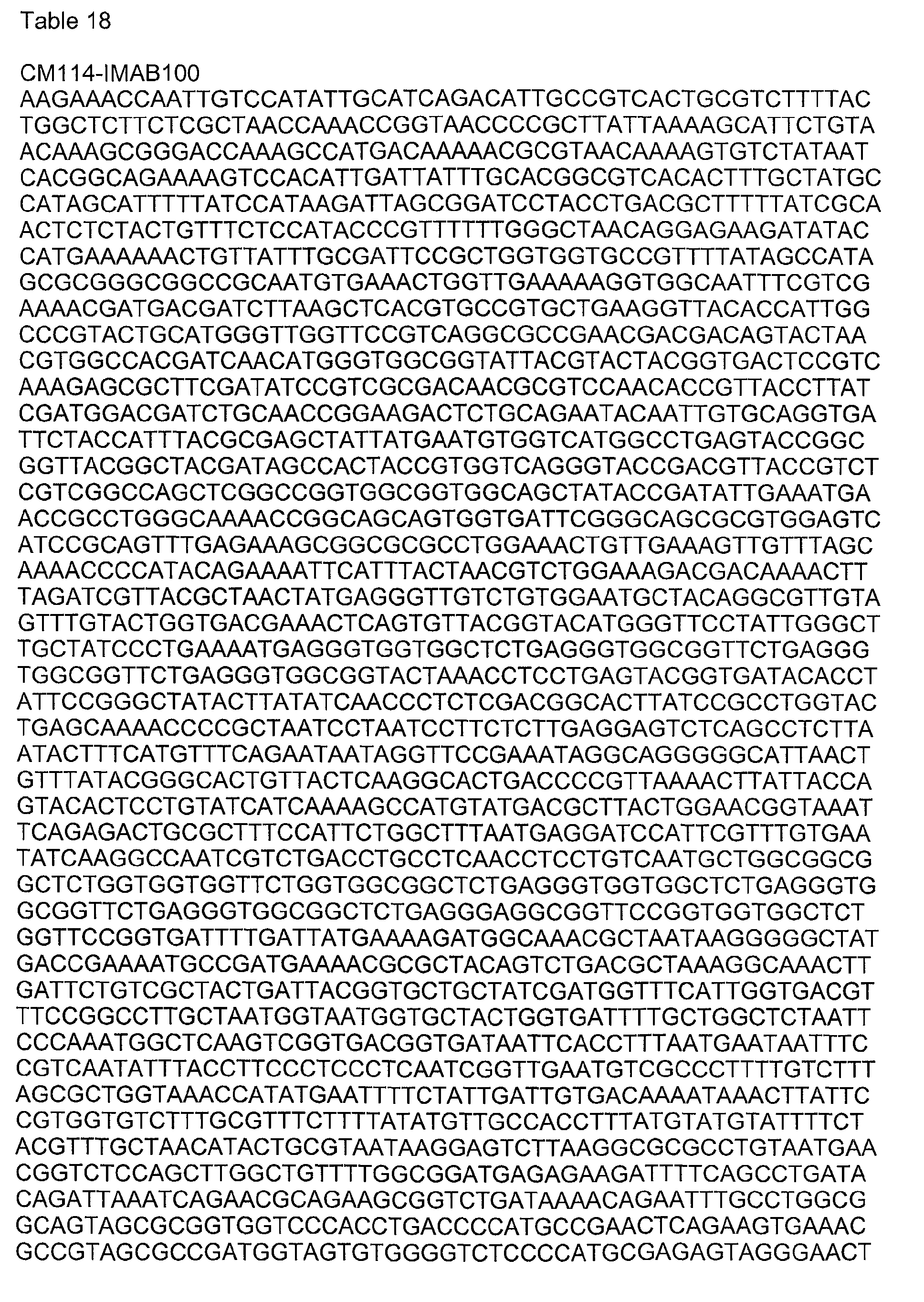

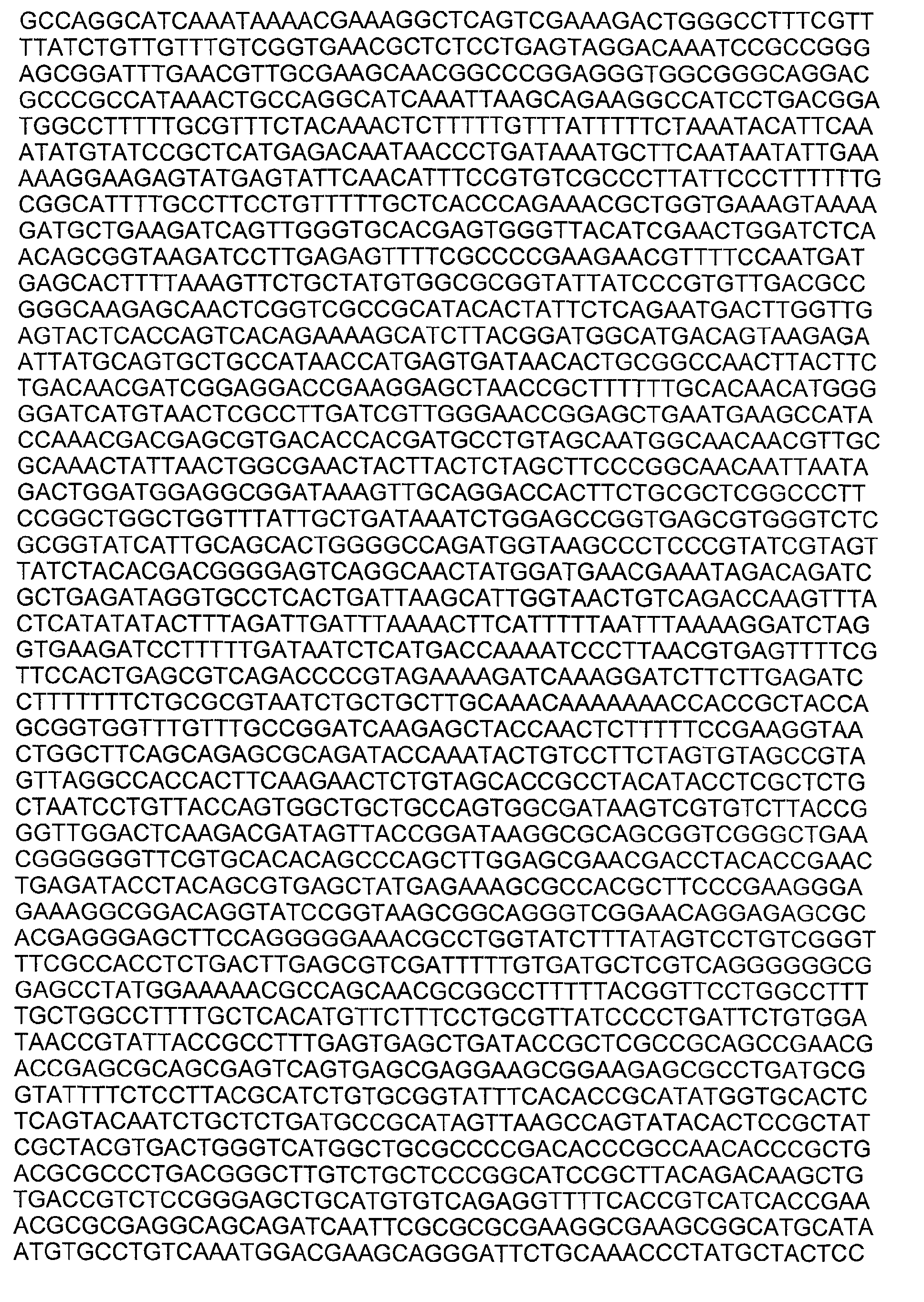

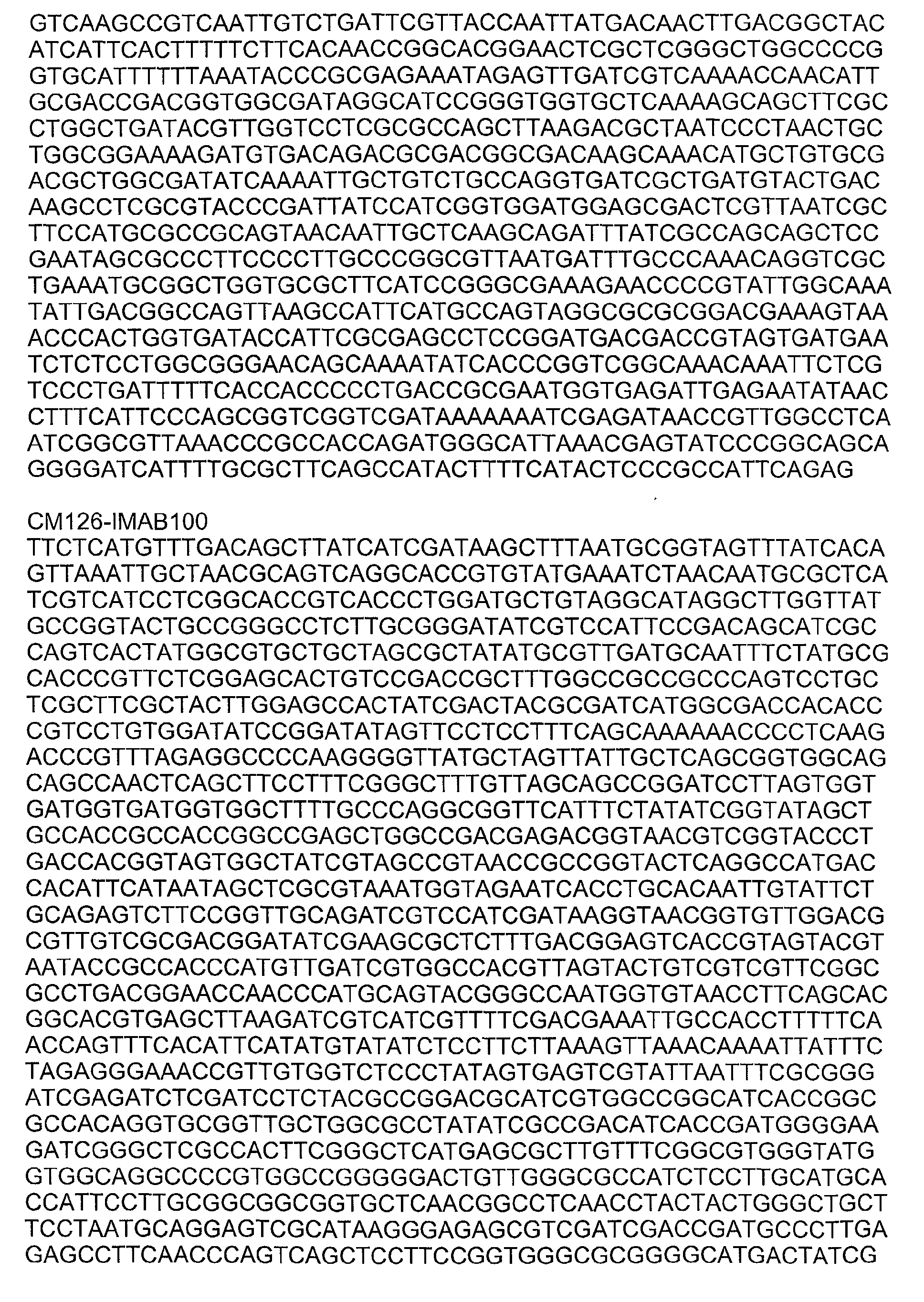

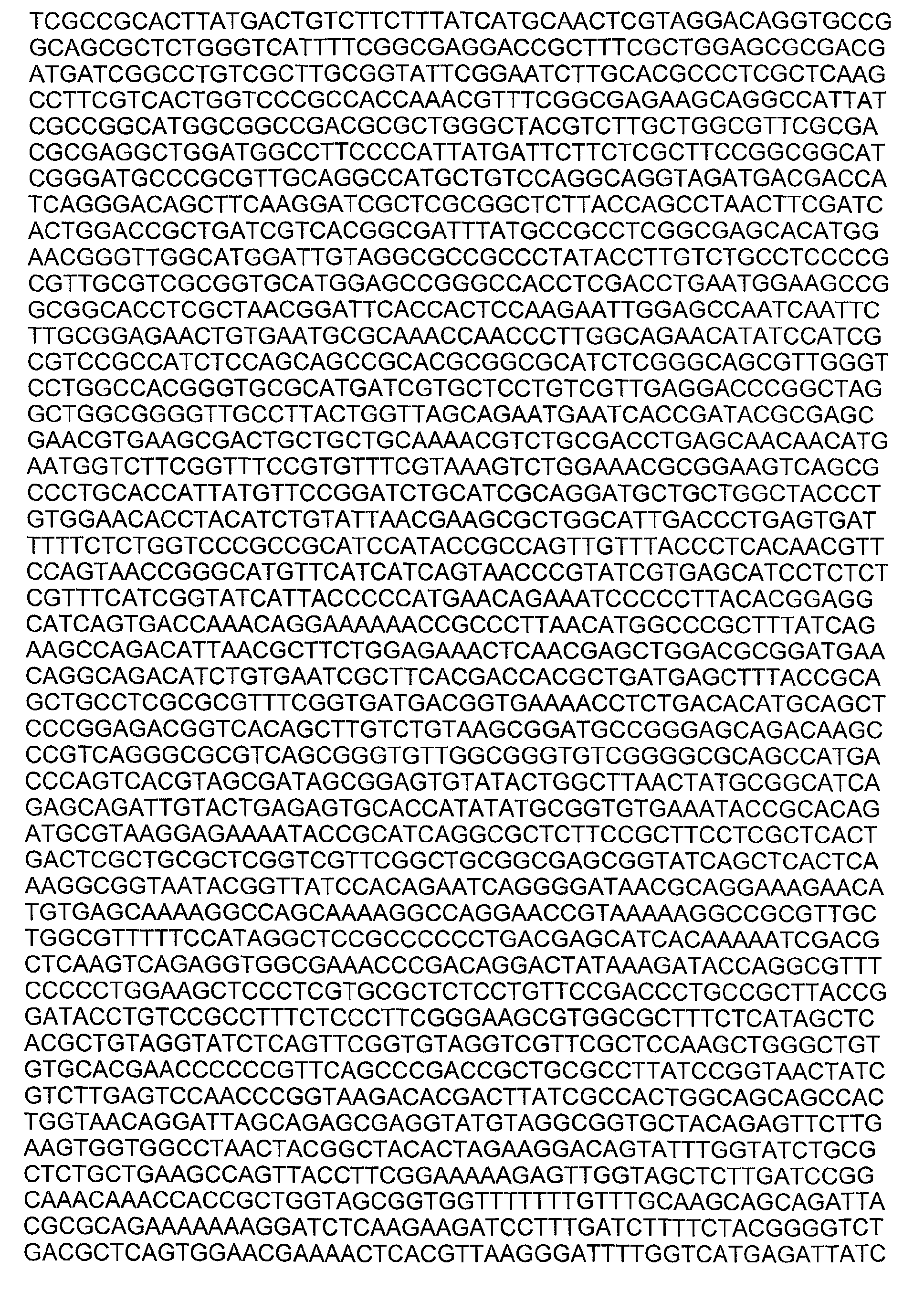

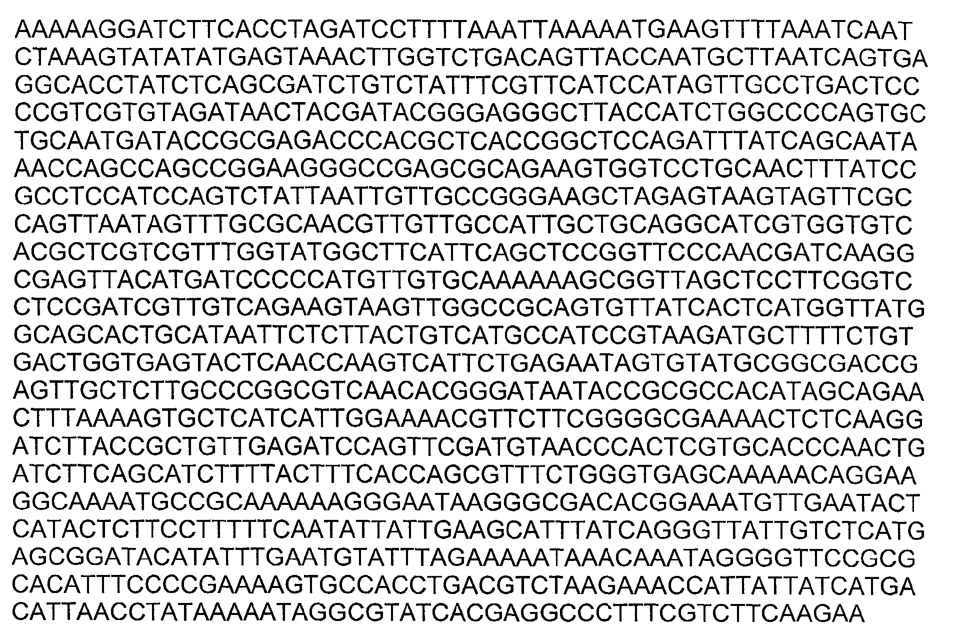

### REFERENCES

Anonymous. FPLC purification of 6* His-tagged proteins from *E.coli* using Ni-NTA Superflow under native conditions. in QIAexpressionist™, Fifth edition, 83-84, (2001)
Berens SJ, Wylie DE, Lopez OJ. Int Immunol, 9(1):189-99, (1997).
Beste G, Schmidt FS, Stibora T, Skerra A. Proc Natl Acad Sci U S A., 96, 1898-1903, (1999).
Better M, Chang CP, Robinson RR, Horwitz AH. Science, 240(4855):1041-3, (1988)
Cadwell et al., PCR Methods Appl., 2, 28-33, (1992)
Crane LJ, Tibtech, 8, 12-16, (1990)
Dimasi N, Martin F, Volpari C, Brunetti M, Biasiol G, Altamura S, Cortese R, De Francesco R, Steinkuhler C, Sollazzo M. J Virol. (10):7461-9., (1997)
Gibrat, JF, Madej, T, Bryant, SH, Curr. Op. Struct. Biol., 6(3), 377-385, (1996)
Hamers-Casterman C, Atarhouch T, Muyldermans S, Robinson G, Hamers C, Songa EB,
Holm, L and Sander, C. Nucl. Acids Res., 26, 316-319, (1998a)
Holm, L and Sander, C. Proteins, 33, 88-96, (1998b)
Koide S., Artificial antibody polypeptides, WO 98/56915,, (1998)
Koide,, US Patent 6,462,189.
Koide A, Bailey CW, Huang X, Koide S, J. Mol. Biol. 284 (4):1141-1151, (1998).
Kranz et al., WO Patent 0148145
Ku J, Schultz PG. Proc Natl Acad Sci U S A. 92(14):6552-6, (1995)
Kuipers, Methods Mol Biol, 57 351-356, (1996)
Lauwereys M, Arbabi Ghahroudi M, Desmyter A, Kinne J, Holzer W, De Genst E, Wyns L, McConnell SJ, Hoess RH. J Mol Biol., 250(4):460-70, (1995)
Leung et al., Technique 1, 11-15, (1989)
Muyldermans S. EMBO J.,17(13):3512-20, (1998)
Murzin A. G et al. J. Mol. Biol., 247, 536-540, (1995)
Orengo CA, Jones DT, Thornton JM. Structure, 5(8) 1093-1108, (1997)
Rodger A. & Nordén B. in *Circular dichroism and linear dichroism,* Oxford University press, Oxford., (1997)
Shindyalov and Bourne Protein Engineering 11 (9) 739-747, (1998)
Skerra A. Biochim Biophys Acta. Oct 18;1482(1-2):337-50, 2000.
Skerra A. J Biotechnol. Jun;74(4):257-75, (2001)
Skerra A, Pluckthun A. Science May 20;240(4855):1038-41, (1988)
Smith, GP, Patel SU, Windass JD, Thornton JM, Winter G, Griffiths AD. J Mol Biol. Mar 27;277(2):317-32, (1998)
Spee, JH, de Vos WM, Kuipers OP. Nucleic Acids Res 21 (3):777-8, (1993)
Vriend, G. J. Mol. Graph. 8, 52-56, (1990)
Vu, KB, Ghahroudi, M.A. , Wyns, L. , Muyldermans, S. Mol. Immunol. 34, 1121-1131, (1997)
Xu et al., Biotechniques, 27, 1102-1108, (1999)
Zaccolo M, Williams DM, Brown DM, Gherardi E. J Mol Biol, 255(4):589-603, (1996)

## Claims

1. A method for applying a cosmetic substance to a desired target molecule, comprising providing a conjugate of a proteinaceous substance having specific affinity for said target molecule linked to a cosmetic substance, whereby the resulting connection between cosmetic substance and target molecule can be disrupted upon the presence of a chemical and/or physical signal.

2. A method according to claim 1, whereby said proteinaceous substance comprises a synthetic or recombinant proteinaceous molecule comprising a binding peptide and a core, said core comprising a beta-barrel comprising at least 4 strands, wherein said beta -barrel comprises at least two beta -sheets, wherein each of said beta -sheet comprises two of said strands and wherein said binding peptide is a peptide connecting two strands in said beta -barrel and wherein said binding peptide is outside its natural context.

3. A method according to claim 2, wherein said proteinaceous molecule comprises a beta barrel, wherein said beta -barrel comprises at least 5 strands, wherein at least one of said sheets comprises 3 of said strands.

4. A method according to claim 3, wherein said beta-barrel comprises at least 6 strands, wherein at least two of said sheets comprises 3 of said strands.

5. A method according to any one of claims 1-4, wherein said beta-barrel comprises at least 7 strands, wherein at least one of said sheets comprises 4 of said strands.

6. A method according to any one of claims 1-5, wherein said beta-barrel comprises at least 8 strands, wherein at least one of said sheets comprises 4 of said strands.

7. A method according to any one of claims 1-6, wherein said beta-barrel comprises at least 9 strands, wherein at least one of said sheets comprises 4 of said strands.

8. A method according to any one of claims 1-7, wherein said binding peptide connects two strands of said beta-barrel on the open side of said barrel.

9. A method according to any one of claims 1-8, wherein said binding peptide connects said at least two beta-sheets of said barrel.

10. A method according to any one of claims 1-9, wherein said proteinaceous molecule comprises at least one further binding peptide

11. A method according to any one of claims 1-10, wherein said proteinaceous molecule comprises three binding peptides and three connecting peptide sequences.

12. A method according to any one of claims 1-11, wherein said proteinaceous molecule comprises at least 4 binding peptides.

13. A method according to claim 12, wherein at least one binding peptide recognizes another target molecule than at least one of the other binding peptides.

14. A method according to any one of claims 1-13 wherein said proteinaceous molecule has an altered binding property, said property selected for the physical and/or chemical circumstances in which the conjugate is applied, said alteration comprising introducing an alteration in the core of proteinaceous molecules according to any one of claims 1-13, and selecting from said proteinaceous molecules, a proteinaceous molecule with said altered binding property.

15. A method according to any one of claims 1-13 wherein said proteinaceous molecule has an altered structural property, said property selected for the physical and/or chemical circumstances in which the conjugate is applied, said alteration comprising introducing an alteration in the core of proteinaceous molecules according to any one of claims 1-13, and selecting from said proteinaceous molecules, a proteinaceous molecule with said altered structural property.

16. A method according to claim 14 or 15, wherein said alteration comprises a post-translational modification.

17. A method according to any one of claims 14-15, wherein said alteration is introduced into a nucleic acid coding for said at least one proteinaceous molecule, the method further comprising expressing said nucleic acid in an expression system that is capable of producing said proteinaceous molecule.

18. A conjugate obtainable by a method according to any one of claims 1-17.

19. A conjugate according to claim 18 in which the proteinaceous molecule is derived from the immunoglobulin superfamily.

20. A conjugate according to claim 18 or 19 in which the exterior of the proteinaceous molecule is immunologically similar to the immunoglobulin superfamily molecule it was derived from.

21. A conjugate according to any one of claims 18-20, comprising a fusion protein.

22. A conjugate according to any one of claims 18-21 in which said proteinaceous substance is covalent ly linked to said cosmetic substance by an organic linker.

23. A conjugate according to claim 22, in which said linker is labile in certain physicochemical conditions and stable in different physicochemical conditions.

24. A conjugate according to anyone of claims 18-23, in which the link between proteinaceous molecule and cosmetic substance can be proteolyzed.

25. A conjugate according to any one of claims 18-24, in which the link between proteinaceous molecule and cosmetic substance is labile under skin and/or hair conditions.

26. A conjugate according to any one of claims 18-25, which has specific affinity for a target molecule associated with the skin,hair or other body substances exposed to the exterior of said body.

27. A conjugate according to claim 26, which has specific affinity for keratin.

28. A conjugate according to any one of claims 18-25, which has a specific affinity for a target molecule associated with textile fabric.

29. A conjugate according to any one of claims 18-28, in which the cosmetic substance comprises a fragrant substance.

30. A conjugate according to any one of claims 18- 29, in which the cosmetic substance comprises a coloured substance.

31. A conjugate according to any one of claims 18-30, which is water soluble.

32. A cosmetic composition comprising a conjugate according to any one of claims 18-27 or 29-31.

33. A cosmetic composition according to claim 32, which is a perfume, a deodorant, a mouth wash or a cleaning composition.

34. A cosmetic composition according to claim 32, which is a hair dye composition, a lipstick, rouge or another skin colouring composition.

35. A detergent and/or softener composition comprising a conjugate according to any one of claims 18-31.
